# FASCICULE DE BREVET EUROPEEN

(11) **EP 3 155 010 B1**
(45) Date de publication et mention de la délivrance du brevet: **18.09.2019**
(21) Numéro de dépôt: 15738477.7
(22) Date de dépôt: 16.06.2015
(51) Int. Cl.: C07K 14/715, G01N 33/574, A61K 39/395, A61K 31/00, A61K 31/713

(54) **INHIBITION DE LA CHIMIOKINE CCL7 OU DE SON RECEPTEUR CCR3 POUR LE TRAITEMENT ET LE DIAGNOSTIC DU CANCER DE LA PROSTATE**
HEMMUNG VON CHEMOKIN CCL7 ODER DES REZEPTORS CCR3 DAVON ZUR BEHANDLUNG UND DIAGNOSE VON PROSTATAKREBS
INHIBITION OF CHEMOKINE CCL7 OR RECEPTOR CCR3 OF SAME FOR THE TREATMENT AND DIAGNOSIS OF PROSTATE CANCER

(30) Priorité: 16.06.2014 FR 1455491
(43) Date de publication de la demande: 19.04.2017
(73) Titulaire: Université Paul Sabatier (Toulouse III), 31062 Toulouse Cedex 9 (FR); Centre National de la Recherche Scientifique, 75016 Paris (FR); Centre Hospitalier Universitaire de Toulouse, 31059 Toulouse Cedex 9 (FR); Institut National de la Santé et de la Recherche Médicale (INSERM), 75013 Paris (FR)
(72) Inventeur: MULLER, Catherine, F-31400 Toulouse (FR); LAURENT, Victor, F-81370 Saint Sulpice (FR); GUERARD, Adrien, F-31400 Toulouse (FR); VALET, Philippe, F-31400 Toulouse (FR); MALAVAUD, Bernard, F-31500 Toulouse (FR)
(74) Mandataire: Gevers & Orès
(86) Numéro de dépôt international: PCT/IB2015/054550
(87) Numéro de publication internationale: WO 2015/193813

(56) Documents cités:
- WO-A2-2005/106492
- WO-A2-2008/121132
- WO-A2-2010/123956
- WO-A2-2012/122499
- ZHU F ET AL: "Eotaxin-1 promotes prostate cancer cell invasion via activation of the CCR3-ERK pathway and upregulation of MMP-3 expression", ONCOLOGY REPORTS MAY 2014 SPANDIDOS PUBLICATIONS GRC, vol. 31, no. 5, mai 2014 (2014-05), pages 2049-2054, XP009182432, ISSN: 1021-335X cité dans la demande
- SABROE I ET AL: "A small molecule antagonist of chemokine receptors CCR1 and CCR3. Potent inhibition of eosinophil function and CCR3-mediated HIV-1 entry", JOURNAL OF BIOLOGICAL CHEMISTRY, AMERICAN SOCIETY FOR BIOCHEMISTRY AND MOLECULAR BIOLOGY, US, vol. 75, no. 34, 25 août 2000 (2000-08-25) , pages 25985-25992, XP002468414, ISSN: 0021-9258, DOI: 10.1074/JBC.M908864199 cité dans la demande
- E. L. WISE ET AL: "Small Molecule Receptor Agonists and Antagonists of CCR3 Provide Insight into Mechanisms of Chemokine Receptor Activation", JOURNAL OF BIOLOGICAL CHEMISTRY, vol. 282, no. 38, 21 septembre 2007 (2007-09-21), pages 27935-27943, XP055168336, ISSN: 0021-9258, DOI: 10.1074/jbc.M703255200
- JOEHRER K ET AL: "UP-REGULATION OF FUNCTIONAL CHEMOKINE RECEPTOR CCR3 IN HUMAN RENAL CELL CARCINOMA", CLINICAL CANCER RESEARCH, THE AMERICAN ASSOCIATION FOR CANCER RESEARCH, US, vol. 11, no. 7, avril 2005 (2005-04), pages 2459-2465, XP008054225, ISSN: 1078-0432, DOI: 10.1158/1078-0432.CCR-04-0405
- KAI-YUAN CAO: "Screening and identification of significant genes related to tumor metastasis and PSMA in prostate cancer using microarray analysis", ONCOLOGY REPORTS, 5 août 2013 (2013-08-05), XP055168393, ISSN: 1021-335X, DOI: 10.3892/or.2013.2656
- MORGAN O'HAYRE ET AL: "Chemokines and cancer: migration, intracellular signalling and intercellular communication in the microenvironment", BIOCHEMICAL JOURNAL, vol. 409, no. 3, février 2008 (2008-02), page 635, XP055168402, ISSN: 0264-6021, DOI: 10.1042/BJ20071493
- AFERO: "Congrès AFERO 2015 (31eréunion scientifique)", OBÉSITÉ, LAVOISIER, PARIS, vol. 9, no. 4, 21 novembre 2014 (2014-11-21), pages 331-343, XP035414235, ISSN: 1951-5995, DOI: 10.1007/S11690-014-0468-Z [extrait le 2014-11-21]

### SEQUENCE LISTING

<110> Université Paul SABATIER -Toulouse III
   Centre National de la Recherche Scientifique
   Centre Hospitalier Universitaire de Toulouse
   Institut National de la Santé et de la Recherche Médicale
   MULLER, Catherine
   LAURENT, Victor
   GUERARD, Adrien
   VALET, Philippe
   MALAVAUD, Bernard
<120> INHIBITION DE LA CHIMIOKINE CCL7 OU DE SON RECEPTEUR CCR3 POUR LE TRAITEMENT ET LE DIAGNOSTIC DU CANCER DE LA PROSTATE
<130> F2541-1-FR_XRN/clg
<150> FR1455491
   <151> 2014-06-16
<160> 25
<170> PatentIn version 3.5
<210> 1
   <211> 67
   <212> DNA
   <213> Artificial Sequence
<220>
   <223> shRNA
<400> 1
   cgcgtcccca gaccacaccc tatgaatatt caagagatat tcatagggtg tggtcttttt        60
   tggaaat                                                                  67
<210> 2
   <211> 67
   <212> DNA
   <213> Artificial Sequence
<220>
   <223> shRNA
<400> 2
   cgcgtccccg accacaccct atgaatattt caagagaata ttcatagggt gtggtctttt 60
   tggaaat 67
<210> 3
   <211> 68
   <212> DNA
   <213> Artificial Sequence
<220>
   <223> shRNA
<400> 3
   cgccgtcccc ggtgagaggt tccggaaact tcaagagagt ttccggaacc tctcaccttt 60
   ttggaaat 68
<210> 4
   <211> 23
   <212> DNA
   <213> Artificial Sequence
<220>
   <223> Primer
<400> 4
   aaacctccaa ttctcatgtg gaa 23
<210> 5
   <211> 21
   <212> DNA
   <213> Artificial Sequence
<220>
   <223> Primer
<400> 5
   cagaagtgct gcagaggctt t 21
<210> 6
   <211> 23
   <212> DNA
   <213> Artificial Sequence
<220>
   <223> Primer
<400> 6
   aaacctccaa ttctcatgtg gaa 23
<210> 7
   <211> 20
   <212> DNA
   <213> Artificial Sequence
<220>
   <223> Primer
<400> 7
   cttcccaggg acaccgacta 20
<210> 8
   <211> 20
   <212> DNA
   <213> Artificial Sequence
<220>
   <223> Primer
<400> 8
   tgcaccacca actgcttagc 20
<210> 9
   <211> 21
   <212> DNA
   <213> Artificial Sequence
<220>
   <223> Primer
<400> 9
   ggcatggact gtggtcatga g 21
<210> 10
   <211> 21
   <212> DNA
   <213> Artificial Sequence
<220>
   <223> Primer
<400> 10
   tgacactggc aaaacaatgc a 21
<210> 11
   <211> 20
   <212> DNA
   <213> Artificial Sequence
<220>
   <223> Primer
<400> 11
   gcttgcgacc ttgaccatct 20
<210> 12
   <211> 21
   <212> DNA
   <213> Artificial Sequence
<220>
   <223> Primer
<400> 12
   tggccatctg cctagtaaag c 21
<210> 13
   <211> 21
   <212> DNA
   <213> Artificial Sequence
<220>
   <223> Primer
<400> 13
   ggacgcagca actgacattt c 21
<210> 14
   <211> 19
   <212> DNA
   <213> Artificial Sequence
<220>
   <223> siRNA
<220>
   <221> modified_base
   <222> (9) .. (9)
   <223> Replace = 2,6-Diaminopurine
<220>
   <221> modified_base
   <222> (15)..(15)
   <223> Replace = 2,6-Diaminopurine
<400> 14
   gggtctgcag cgggatggt 19
<210> 15
   <211> 21
   <212> DNA
   <213> Artificial Sequence
<220>
   <223> siRNA
<220>
   <221> modified_base
   <222> (4) .. (4)
   <223> Replace = 2,6-Diaminopurine
<220>
   <221> modified_base
   <222> (7) .. (7)
   <223> Replace = 2,6-Diaminopurine
<220>
   <221> modified_base
   <222> (13) .. (13)
   <223> Replace = 2,6-Diaminopurine
<400> 15
   gttactactt ccacctgcct g 21
<210> 16
   <211> 19
   <212> DNA
   <213> Artificial Sequence
<220>
   <223> Antisens oligonucleotide
<220>
   <221> misc_feature
   <222> (1)..(19)
   <223> All linkages are phophorothioate
<400> 16
   cacctctgtc accagcatg 19
<210> 17
   <211> 19
   <212> DNA
   <213> Artificial Sequence
<220>
   <223> Antisens oligonucleotide
<220>
   <221> misc_feature
   <222> (1)..(2)
   <223> Linkage to next nucleotide is phophorothioate
<220>
   <221> modified_base
   <222> (2) .. (2)
   <223> Replace = 2-amino-2'-deoxyadenosine
<220>
   <221> misc_feature
   <222> (5)..(6)
   <223> Linkage to next nucleotide is phophorothioate
<220>
   <221> misc_feature
   <222> (9) .. (10)
   <223> Linkage to next nucleotide is phophorothioate
<220>
   <221> modified_base
   <222> (11)..(11)
   <223> Replace = 2-amino-2'-deoxyadenosine
<220>
   <221> misc_feature
   <222> (11)..(11)
   <223> n is a, c, g, or t
<220>
   <221> misc_feature
   <222> (13)..(14)
   <223> Linkage to next nucleotide is phophorothioate
<220>
   <221> modified_base
   <222> (14)..(14)
   <223> Replace = 2-amino-2'-deoxyadenosine
<220>
   <221> modified_base
   <222> (17)..(17)
   <223> Replace = 2-amino-2'-deoxyadenosine
<220>
   <221> misc_feature
   <222> (17)..(18)
   <223> Linkage to next nucleotide is phophorothioate
<400> 17
   cacctctgtc nccagcatg 19
<210> 18
   <211> 19
   <212> DNA
   <213> Artificial Sequence
<220>
   <223> Antisens oligonucleotide
<220>
   <221> misc_feature
   <222> (1)..(3)
   <223> Linkage to next nucleotide is phophorothioate
<220>
   <221> modified_base
   <222> (2) .. (2)
   <223> Replace = 2-amino-2'-deoxyadenosine
<220>
   <221> misc_feature
   <222> (6) .. (7)
   <223> Linkage to next nucleotide is phophorothioate
<220>
   <221> modified_base
   <222> (11)..(11)
   <223> Replace = 2-amino-2'-deoxyadenosine
<220>
   <221> misc_feature
   <222> (11)..(11)
   <223> n is a, c, g, or t
<220>
   <221> misc_feature
   <222> (12)..(13)
   <223> Linkage to next nucleotide is phophorothioate
<220>
   <221> modified_base
   <222> (14)..(14)
   <223> Replace = 2-amino-2'-deoxyadenosine
<220>
   <221> misc_feature
   <222> (14)..(14)
   <223> n is a, c, g, or t
<220>
   <221> misc_feature
   <222> (16)..(18)
   <223> Linkage to next nucleotide is phophorothioate
<220>
   <221> modified_base
   <222> (17)..(17)
   <223> Replace = 2-amino-2'-deoxyadenosine
<400> 18
   cacctctgtc nccngcatg 19
<210> 19
   <211> 19
   <212> DNA
   <213> Artificial Sequence
<220>
   <223> Antisens oligonucleotide
<220>
   <221> misc_feature
   <222> (1)..(2)
   <223> Linkage to next nucleotide is phophorothioate
<220>
   <221> modified_base
   <222> (2) .. (2)
   <223> Replace = 2-amino-2'-deoxyadenosine
<220>
   <221> misc_feature
   <222> (6) .. (7)
   <223> Linkage to next nucleotide is phophorothioate
<220>
   <221> modified_base
   <222> (11)..(11)
   <223> Replace = 2-amino-2'-deoxyadenosine
<220>
   <221> misc_feature
   <222> (11)..(12)
   <223> Linkage to next nucleotide is phophorothioate
<220>
   <221> modified_base
   <222> (14)..(14)
   <223> Replace = 2-amino-2'-deoxyadenosine
<220>
   <221> misc_feature
   <222> (14)..(14)
   <223> n is a, c, g, or t
<220>
   <221> misc_feature
   <222> (16)..(18)
   <223> Linkage to next nucleotide is phophorothioate
<220>
   <221> modified_base
   <222> (17)..(17)
   <223> Replace = 2-amino-2'-deoxyadenosine
<400> 19
   cacctctgtc accngcatg 19
<210> 20
   <211> 19
   <212> DNA
   <213> Artificial Sequence
<220>
   <223> Antisens oligonucleotide
<220>
   <221> misc_feature
   <222> (1)..(2)
   <223> Linkage to next nucleotide is phophorothioate
<220>
   <221> modified_base
   <222> (2) .. (2)
   <223> Replace = 2-amino-2'-deoxyadenosine
<220>
   <221> misc_feature
   <222> (6) .. (7)
   <223> Linkage to next nucleotide is phophorothioate
<220>
   <221> modified_base
   <222> (11) .. (11)
   <223> Replace = 2-amino-2'-deoxyadenosine
<220>
   <221> misc_feature
   <222> (11)..(12)
   <223> Linkage to next nucleotide is phophorothioate
<220>
   <221> modified_base
   <222> (14)..(14)
   <223> Replace = 2-amino-2'-deoxyadenosine
<220>
   <221> misc_feature
   <222> (14)..(14)
   <223> n is a, c, g, or t
<220>
   <221> modified_base
   <222> (17)..(17)
   <223> Replace = 2-amino-2'-deoxyadenosine
<220>
   <221> misc_feature
   <222> (17)..(18)
   <223> Linkage to next nucleotide is phophorothioate
<400> 20
   cacctctgtc accngcatg 19
<210> 21
   <211> 19
   <212> DNA
   <213> Artificial Sequence
<220>
   <223> Antisens oligonucleotide
<220>
   <221> misc_feature
   <222> (1)..(3)
   <223> Linkage to next nucleotide is phophorothioate
<220>
   <221> modified_base
   <222> (2) .. (2)
   <223> Replace = 2-amino-2'-deoxyadenosine
<220>
   <221> misc_feature
   <222> (5)..(6)
   <223> Linkage to next nucleotide is phophorothioate
<220>
   <221> misc_feature
   <222> (8) .. (10)
   <223> Linkage to next nucleotide is phophorothioate
<220>
   <221> modified_base
   <222> (11) .. (11)
   <223> Replace = 2-amino-2'-deoxyadenosine
<220>
   <221> misc_feature
   <222> (11)..(11)
   <223> n is a, c, g, or t
<220>
   <221> misc_feature
   <222> (12)..(15)
   <223> Linkage to next nucleotide is phophorothioate
<220>
   <221> modified_base
   <222> (14)..(14)
   <223> Replace = 2-amino-2'-deoxyadenosine
<220>
   <221> modified_base
   <222> (17)..(17)
   <223> Replace = 2-amino-2'-deoxyadenosine
<220>
   <221> misc_feature
   <222> (17)..(18)
   <223> Linkage to next nucleotide is phophorothioate
<400> 21
   cacctctgtc nccagcatg 19
<210> 22
   <211> 19
   <212> DNA
   <213> Artificial Sequence
<220>
   <223> Antisens oligonucleotide
<220>
   <221> misc_feature
   <222> (1)..(2)
   <223> Linkage to next nucleotide is phophorothioate
<220>
   <221> modified_base
   <222> (2)..(2)
   <223> Replace = 2-amino-2'-deoxyadenosine
<220>
   <221> misc_feature
   <222> (5)..(8)
   <223> Linkage to next nucleotide is phophorothioate
<220>
   <221> modified_base
   <222> (11)..(11)
   <223> Replace = 2-amino-2'-deoxyadenosine
<220>
   <221> misc_feature
   <222> (11)..(13)
   <223> Linkage to next nucleotide is phophorothioate
<220>
   <221> modified_base
   <222> (14)..(14)
   <223> Replace = 2-amino-2'-deoxyadenosine
<220>
   <221> misc_feature
   <222> (14)..(14)
   <223> n is a, c, g, or t
<220>
   <221> misc_feature
   <222> (16)..(18)
   <223> Linkage to next nucleotide is phophorothioate
<220>
   <221> modified_base
   <222> (17)..(17)
   <223> Replace = 2-amino-2'-deoxyadenosine
<400> 22
   cacctctgtc accngcatg 19
<210> 23
   <211> 19
   <212> DNA
   <213> Artificial Sequence
<220>
   <223> Antisens oligonucleotide
<220>
   <221> misc_feature
   <222> (1)..(6)
   <223> Linkage to next nucleotide is phophorothioate
<220>
   <221> modified_base
   <222> (2)..(2)
   <223> Replace = 2-amino-2'-deoxyadenosine
<220>
   <221> modified_base
   <222> (11)..(11)
   <223> Replace = 2-amino-2'-deoxyadenosine
<220>
   <221> misc_feature
   <222> (11)..(11)
   <223> n is a, c, g, or t
<220>
   <221> misc_feature
   <222> (13)..(18)
   <223> Linkage to next nucleotide is phophorothioate
<220>
   <221> modified_base
   <222> (14)..(14)
   <223> Replace = 2-amino-2'-deoxyadenosine
<220>
   <221> modified_base
   <222> (17)..(17)
   <223> Replace = 2-amino-2'-deoxyadenosine
<400> 23
   cacctctgtc nccagcatg 19
<210> 24
   <211> 19
   <212> DNA
   <213> Artificial Sequence
<220>
   <223> Antisens oligonucleotide
<220>
   <221> misc_feature
   <222> (1)..(7)
   <223> Linkage to next nucleotide is phophorothioate
<220>
   <221> modified_base
   <222> (2)..(2)
   <223> Replace = 2-amino-2'-deoxyadenosine
<220>
   <221> modified_base
   <222> (11)..(11)
   <223> Replace = 2-amino-2'-deoxyadenosine
<220>
   <221> misc_feature
   <222> (11)..(11)
   <223> n is a, c, g, or t
<220>
   <221> misc_feature
   <222> (12)..(18)
   <223> Linkage to next nucleotide is phophorothioate
<220>
   <221> modified_base
   <222> (14)..(14)
   <223> Replace = 2-amino-2'-deoxyadenosine
<220>
   <221> modified_base
   <222> (17)..(17)
   <223> Replace = 2-amino-2'-deoxyadenosine
<400> 24
   cacctctgtc nccagcatg 19
<210> 25
   <211> 19
   <212> DNA
   <213> Artificial Sequence
<220>
   <223> Antisens oligonucleotide
<220>
   <221> misc_feature
   <222> (1)..(2)
   <223> Linkage to next nucleotide is phophorothioate
<220>
   <221> modified_base
   <222> (2)..(2)
   <223> Replace = 2-amino-2'-deoxyadenosine
<220>
   <221> misc_feature
   <222> (6)..(12)
   <223> Linkage to next nucleotide is phophorothioate
<220>
   <221> modified_base
   <222> (11)..(11)
   <223> Replace = 2-amino-2'-deoxyadenosine
<220>
   <221> modified_base
   <222> (14)..(14)
   <223> Replace = 2-amino-2'-deoxyadenosine
<220>
   <221> misc_feature
   <222> (14)..(14)
   <223> n is a, c, g, or t
<220>
   <221> misc_feature
   <222> (16)..(18)
   <223> Linkage to next nucleotide is phophorothioate
<220>
   <221> modified_base
   <222> (17)..(17)
   <223> Replace = 2-amino-2'-deoxyadenosine
<400> 25
   cacctctgtc accngcatg 19

## Description

La présente invention concerne le domaine du diagnostic et des médicaments destinés au traitement du cancer de la prostate.
Le cancer de la prostate est le cancer le plus fréquemment observé chez les hommes de plus de 50 ans et représente la deuxième cause de décès par cancer dans les pays développés. Comme l'ensemble des cancers solides, l'extension du cancer de la prostate repose sur la classification TNM (T pour tumeur, N pour « *nodes* » ou atteinte ganglionnaire, M pour métastase) (Ohori, Wheeler et al. 1994 ; Salomon, Azria et al. 2010).

Les cancers de la prostate sont ainsi classés en 4 stades :
1) le cancer de la prostate localisé : T1 [tumeur non palpable ou non visible en imagerie] et T2 [tumeur limitée à la prostate (apex et capsule compris)], N0, M0 ;
2) le cancer de la prostate localement avancé : T3 [extension au-delà de la capsule prostatique] et T4 [Extension aux organes adjacents (col vésical, sphincter urétral, rectum, paroi pelvienne) ou tumeur fixée à la paroi pelvienne], N0, M0 ;
3) le cancer de la prostate avec atteinte ganglionnaire pelvienne : tous T, N1, M0 ; et
4) le cancer de la prostate métastatique : tous T, tous N, M1.

La prostate est entourée de tissu adipeux (tissu adipeux péri-prostatique ou TAPP). La présence de la tumeur dans le tissu adipeux péri-prostatique représente donc la première étape de l'extension locale du cancer de la prostate (stade pT3a) et est un critère établi de mauvais pronostic (Magi-Galluzzi, Evans et al. 2011 ; Houimel and Mazzucchelli 2013). L'envahissement des tissus environnants peut être suivi par une dissémination à distance des cellules cancéreuses (métastase). Ces métastases sont principalement localisées à l'os et sont à l'origine de la majorité des décès liés à la maladie (taux de mortalité d'environ 90%). Les os, principalement constitués de tissu osseux, contiennent également du tissu hématopoïétique, du tissu adipeux, des vaisseaux, des nerfs, du tissu cartilagineux et du tissu conjonctif. Ainsi, le tissu adipeux est présent à la fois aux étapes de dissémination locale et à distance du cancer de la prostate.

Parmi les cellules qui composent le tissu adipeux, notamment le tissu adipeux péri-prostatique, on retrouve majoritairement des adipocytes matures ainsi que, dans une fraction dite « stroma vasculaire », d'autres cellules telles que des progéniteurs (*Adipose Derived Stem Cells* [ADSCs] et préadipocytes), des fibroblastes, des macrophages, des lymphocytes, des péricytes et que des cellules endothéliales (Ouchi, Parker et al. 2011). Les adipocytes ont un rôle dans les fluctuations énergétiques induites par l'apport alimentaire ou les états de jeûne en stockant l'énergie sous forme de triglycérides ou en la libérant sous forme d'acides gras (AG). Les adipocytes sont également des cellules endocrines actives qui sécrètent une grande variété de molécules (appelées adipokines), qui sont impliquées notamment dans la régulation de l'appétit et de la balance énergétique, le métabolisme lipidique, la sensibilité à l'insuline et la régulation de la pression artérielle (Ouchi, Parker et al. 2011). Parmi les sécrétions adipocytaires, on retrouve des facteurs de croissance, des chimiokines, des molécules pro-angiogéniques ou des molécules pro-inflammatoires (Ouchi, Parker et al. 2011). Tourniaire, Romier-Crouzet et al. (2013) ont rapporté que dans des conditions d'obésité, le profil sécrétoire des adipocytes est modifié, et la sécrétion de certaines chimiokines peut être augmentée. En outre, comme résumé dans Allott, Masko et al. (2013), un ensemble convaincant de données épidémiologiques montre que l'obésité est un facteur de mauvais pronostic dans le cancer de la prostate avec une augmentation de la dissémination locale et à distance. Enfin, indépendamment de l'obésité, des données récentes montrent que l'importance du TAPP est positivement associée à l'agressivité du cancer de la prostate (Kiss, Longden et al. 2009).

Les chimiokines représentent un groupe important de petites protéines (d'environ 8 à 11 kDa) qui interagissent avec des récepteurs appartenant à la superfamille des récepteurs à sept domaines transmembranaires couplés aux protéines G (RCPG). La plupart d'entre elles possèdent quatre résidus cystéine caractéristiques qui forment deux ponts disulfures, un entre le premier et le troisième résidu cystéine et un entre le deuxième et le quatrième résidu cystéine. Selon le nombre d'acides aminés présents entre les deux premières cystéines (situées dans la partie N-terminale), les chimiokines sont classées en quatre groupes : C, CC, CXC et CX3C. Les chimiokines possèdent diverses fonctions dont la plus étudiée est l'attraction (chimiotactisme) et le contrôle de l'état d'activation des cellules du système immunitaire. Dans le cancer, ces chimiokines et leurs récepteurs sont connus pour réguler le processus tumoral (prolifération, migration, invasion, angiogenèse, infiltration des leucocytes). Certaines d'entre elles sont produites par les cellules tumorales elles-mêmes, mais également par les cellules stromales telles que les fibroblastes associés au cancer (CAF), les cellules endothéliales ou des cellules immunitaires (macrophages, lymphocytes) (Balkwill 2004).

Parmi les récepteurs aux chimiokines potentiellement impliqués dans le cancer, le récepteur CXCR4 est actuellement considéré comme l'un des récepteurs les plus importants. L'interaction de CXCR4 avec son ligand CXCL12 joue un rôle dans la migration cellulaire de nombreux cancers, dont la prostate, en particulier dans le contexte de la métastase osseuse (Taichman, Cooper et al. 2002). Des études *in vitro* ont montré que CXCL12 stimule le caractère invasif des cellules tumorales prostatiques et qu'il peut être bloqué par l'utilisation d'un inhibiteur pharmacologique (par exemple l'antagoniste de CXCR4 dénommé AMD3100) ou d'un anticorps bloquant. L'interaction entre le récepteur CCR2 et la chimiokine CCL2 a également été identifiée pour son implication dans la migration des cellules tumorales prostatiques (Loberg, Day et al. 2006) et semble jouer un rôle clé dans la métastase du cancer de la prostate (Zhang, Patel et al. 2010). De plus l'expression du récepteur CCR2 est corrélée à l'agressivité des tumeurs prostatiques (Lu, Cai et al. 2007). D'autres récepteur aux chimiokines comme CXCR1 et CXCR2 ont été décrits comme jouant un rôle dans l'invasion et la croissance des cellules tumorales prostatiques via les chimiokines CXCL1, CXCL5 ou CXCL8 (Inoue, Slaton et al. 2000 ; Begley, Kasina et al. 2008).

D'autres récepteurs aux chimiokines pouvant intervenir dans la migration des cellules tumorales dans d'autres types de cancers ont également été identifiés, comme par exemple les récepteurs CCR1 (GeneID :1230) (Loetscher, Pellegrino et al. 2001) et CCR3 (GeneID:1232) (Loetscher, Pellegrino et al. 2001 ; Jung, Che et al. 2010 ; Johrer, Zelle-Rieser et al. 2005 ; Lee, Kim et al. 2010). Ces derniers sont très fortement exprimés par les éosinophiles ou les basophiles et de manière moins importante par les lymphocytes (Th1 et Th2) et certaines cellules épithéliales. CCR1 et CCR3 sont également impliqués dans la régulation de la migration des éosinophiles et joue un rôle majeur dans de nombreuses pathologies inflammatoires comme l'asthme (Joubert, Lajoie-Kadoch et al. 2008). A ce jour, un certain nombre de ligands de CCR3 ont été identifiés : CCL5, CCL7, CCL11, CCL13, CCL15, CCL24, CCL26 et CCL28. Jung, Che et al. (2010) ont montré dans les carcinomes des voies aériennes supérieures que CCR3 peut jouer un rôle dans la migration cellulaires via la chimiokine CCL7 (GeneID:6354 ; Kuri-Harcuch and Green 1978). Il a été montré que le récepteur CCR3 est surexprimé dans les tumeurs rénales (Jung, Che et al. 2010) et le mélanome (Lee, Kim et al. 2010). De façon très récente, une étude préliminaire portant sur une seule lignée de cancer de la prostate Du-145 montre que le récepteur CCR3 favorise, *via* CCL11 (une chimiokine différente de CCL7), la migration et l'invasion *in vitro* des cellules cancéreuses. Dans cette étude, les auteurs ne se sont intéressés en aucune façon aux chimiokines sécrétées par le tissu adipeux (Zhu, Liao et al. 2013). De plus, l'effet *in vivo* de l'inhibition du récepteur CCR3 et son expression dans les tumeurs prostatiques humaines n'ont pas été abordés dans cette étude

Dans toutes ces études portant sur le rôle des chimiokines dans la progression du cancer de la prostate, le rôle des sécrétions du TAPP n'a pas été envisagé ni le lien avec l'obésité et/ou l'importance du tissu adipeux périprostatique évoqué.

La stratégie thérapeutique pour les tumeurs localisées s'appuie actuellement sur la classification d'Amico si l'on considère exclusivement les caractéristiques du cancer (Guide ALD30, HAS/INCA, 2012). En effet, d'autres facteurs tels que les caractéristiques du patient et ses préférences (en particulier dans le cas où il existe des alternatives thérapeutiques présentant un rapport bénéfice/risque équivalent) sont aussi considérés. La classification D'Amico (Agrawal, Maxwell et al. 2009 ; Ohori, Wheeler et al. 1994) permet de prendre en compte les critères suivants : la taille de la tumeur, le score de Gleason (Kiss, Longden et al. 2009) et le marqueur sérique PSA (*Prostate Specific Antigen*). La classification de Gleason est fondée sur le degré de différentiation de la tumeur, coté du grade 1 (différencié) à 5 (très indifférencié). Ce score est la somme des deux grades les plus fréquemment représentés dans la tumeur analysée. Il varie de 2 à 10. Le score de 2 correspond à une tumeur très proche d'un tissu bénin. Plus le score est élevé, plus la tumeur est agressive. La classification d'Amico établit 3 sous-groupes de cancer de la prostate localisé selon le risque de rechute (on parle de risque de rechute biologique 10 ans après un traitement local), c'est-à-dire 3 niveaux de risque de progression du cancer : un risque faible, un risque intermédiaire et un risque élevé. Les 3 sous-groupes définis en fonction de leurs caractéristiques cliniques et biologiques sont :
1) le cancer de la prostate localisé à faible risque : TNM ≤ T2a (atteinte de la moitié d'un lobe ou moins) et Score de Gleason ≤ 6 et Valeur du PSA (ng/ml) ≤ 10 ;
2) le cancer de la prostate à risque intermédiaire : TNM = T2b (atteinte de plus de la moitié d'un lobe sans atteinte de l'autre lobe) ou Score de Gleason = 7 ou Valeur du PSA (ng/ml) = 10-20 ; et
3) le cancer de la prostate localisé à risque élevé : TNM ≥ T2c (atteinte des deux lobes) ou Score de Gleason ≥ 8 ou valeur du PSA (ng/ml) > 20.

Il existe actuellement un besoin de développer des traitements permettant de retarder voire d'empêcher lesétapes de la dissémination locale et à distance afin de permettre non seulement d'éviter le développement de la maladie métastatique mais aussi de maintenir le plus longtemps possible le cancer de la prostate sous une forme potentiellement curable.

Il existe également un besoin d'identifier par une méthode de diagnostic les tumeurs localisées du cancer de la prostate présentant un haut potentiel d'agressivité afin de les traiter de façon adaptée.

Dans le cadre de leurs travaux de recherche, les inventeurs ont caractérisé un axe spécifique de régulation du processus de dissémination locale du cancer de la prostate via l'implication du récepteur à chimiokine CCR3 sous la dépendance d'un de ces ligands, CCL7, exprimé par le TAPP.

Les inventeurs ont montré *in vitro* qu'un milieu conditionné d'adipocytes matures (MC-Ad) obtenus à partir de la différentiation de la lignée pré-adipocytaire murines F442A stimule la migration de cellules prostatiques humaines et murines. Cette migration est inhibée par l'utilisation d'un inhibiteur de CCR1/CCR3 connu (l'UCB35625) dans tous les modèles de lignées de cancer de la prostate étudiés alors que CCR1 n'est pas exprimée dans ces lignées. L'importance de CCR3 dans la migration induite par le MC-Ad des cellules cancéreuses prostatiques a été confirmée par l'absence d'effet de l'UCB35625 sur la migration de différentes lignées tumorales représentatives de différents cancer (sein, colon, pancréas, mélanome), en dehors d'un effet faible sur la migration de la lignée de cancer du sein agressive MDA-MB 231. Par ailleurs, l'utilisation d'inhibiteurs dirigés contre d'autres récepteurs aux chimiokines exprimés par les cellules tumorales prostatiques (CCR2, CXCR1, CXCR2, CXCR4) a un faible effet sur la migration induite par le MC-Ad. Une inhibition a été aussi observée pour des anticorps bloquants dirigés contre CCR3 dans tous les modèles étudiés ainsi que lorsque l'expression de CCR3 est invalidée. Dans le MC-Ad, un seul ligand de CCR3 a été retrouvé : CCL7. L'inhibition de ce ligand par un anticorps bloquant inhibe la migration des cellules prostatiques humaines et murines contre le MC-Ad. Ce ligand est aussi présent dans le milieu conditionné de tissu adipeux périgonadique de souris et périprostatique chez l'homme et le milieu conditionné d'adipocytes primaires isolés à partir de tissu adipeux périgonadique de souris. La capacité à induire la migration des cellules tumorales prostatiques selon un axe CCR3/CCL7 a aussi été retrouvée pour le milieu conditionné de tissu adipeux périgonadique de souris et périprostatique chez l'homme ainsi que pour le milieu conditionné d'adipocytes primaires isolés à partir de tissu adipeux périgonadique de souris.

Les inventeurs ont également montré *in vivo* que l'injection en intraprostatique de lignées tumorales invalidée pour CCR3 conduit à une diminution de la masse tumorale ainsi qu'à une absence de disparition du tissu adipeux périprostatique sous l'effet de l'infiltration tumorale. En outre, les inventeurs ont montré que le récepteur CCR3 est exprimé dans les tumeurs humaines et que cette expression est liée à l'agressivité des tumeurs (corrélation avec le score de Gleason, le pourcentage de cellules indifférenciées, la localisation périphérique de la tumeur) ainsi qu'à leur dissémination extra-prostatique (stade TNM). L'expression du récepteur CCR3 est aussi corrélée à la rechute biologique et à l'échec du traitement chirurgical. Enfin, les adipocytes médullaires sont capables de sécréter CCL7 et le milieu conditionné de ces adipocytes induit la migration des cellules tumorales prostatiques, migration qui est inhibée par l'UCB35625 ou un anticorps bloquant dirigé contre CCR3 et/ou CCL7. Ces résultats ont été obtenus avec des adipocytes médullaires murins (obtenus à partir de la différentiation *ex vivo* de progéniteurs adipocytaires) et humains (directement isolés de moelle jaune humaine). L'ensemble de ces données montrent que l'axe CCR3/CCL7 semble être impliqué dans la dissémination à distance (métastase osseuse) du cancer de la prostate via la capacité des adipocytes médullaires à sécréter CCL7. Ces données montrent également l'intérêt à inhiber CCR3 ou CCL7 dans le cancer de la prostate en vue d'inhiber l'extension locale ou à distance.

En outre, cette stratégie visant à inhiber CCR3 ou CCL7 présente un intérêt particulier chez les sujets obèses ou présentant un TAPP abondant (dont la surface est supérieure ou égale à 10 cm²). En effet, les inventeurs ont montré que l'expression génique de CCL7 est augmentée dans les tissus adipeux de souris et d'individus obèses. Sa sécrétion est augmentée dans le milieu conditionné de tissu adipeux périgonadique de souris obèses et le milieu conditionné d'adipocytes primaires isolés à partir de tissu adipeux périgonadique de souris obèses. La migration des cellules cancéreuses prostatiques humaines contre le milieu conditionné de tissu adipeux périgonadique de souris obèses et le milieu conditionné d'adipocytes primaires isolés à partir de tissu adipeux périgonadique de souris obèses est augmentée par rapport aux mêmes milieux obtenus avec des animaux de poids normal. Cette augmentation est totalement inhibée par l'UCB35625 ou des anticorps bloquants dirigés contre CCR3 ou CCL7. *In vivo*, l'augmentation de la masse tumorale observée en conditions d'obésité n'est plus présente lorsque le récepteur de CCR3 est invalidé. Chez l'homme, l'expression du récepteur CCR3 est significativement augmentée chez les sujets obèses. De plus, il existe une corrélation entre l'importance du TAPP visualisé par imagerie par résonnance magnétique nucléaire (IRM) ou tomodensidométrie et l'expression de CCR3. Enfin, chez l'homme, les adipocytes médullaires obtenus à partir de sujets obèses possèdent une capacité augmentée à induire la migration des cellules tumorales prostatiques et cet effet est inhibé par l'UCB35625.

La présente invention a en conséquence pour objet un inhibiteur de l'interaction CCL7/CCR3 pour leur utilisation pour prévenir ou traiter l'extension du cancer de la prostate hors de la capsule prostatique chez un sujet selon la revendication 1.

Il est décrit un inhibiteur de l'expression de la chimiokine CCL7 ou un inhibiteur de l'expression du récepteur CCR3 ou un inhibiteur de l'interaction CCL7/CCR3 pour leur utilisation pour prévenir ou traiter l'extension du cancer de la prostate hors de la capsule prostatique chez un sujet.

On entend par « extension du cancer de la prostate hors de la capsule prostatique » le cancer de la prostate localement avancé (selon la classification TNM : T3 ou T4, N0, MO), le cancer de la prostate avec atteinte ganglionnaire pelvienne (selon la classification TNM : tous T, N1, M0) ou le cancer de la prostate métastatique (selon la classification TNM : tous T, tous N, M1).

Selon un mode de réalisation préféré de l'invention, ledit inhibiteur est utilisé chez un homme obèse ou présentant un tissu adipeux péri-prostatique abondant.

L'obésité est un état caractérisé par un excès de masse adipeuse. L'Indice de Masse Corporelle (IMC) est une norme internationale pour mesurer l'excès de poids et l'obésité. Il est défini comme le poids divisé par le carré de la taille, exprimé en kg/m². Un individu est considéré comme obèse lorsque cette valeur est supérieure ou égale à 30 kg/m².

On entend par tissu adipeux péri-prostatique (TAPP) abondant, un TAPP dont la surface est supérieure ou égale à 10 cm². Avantageusement, la surface du TAPP est mesurée en utilisant une méthode de mesure et de calcul d'aire elliptique à partir d'images, obtenues par exemple par imagerie scanner ou IRM chez ledit sujet, de sections transverses au niveau fémoral à l'endroit où la fusion de la symphyse pubienne débute. Le dépôt de tissu adipeux péri-prostatique pour lequel une image est obtenue se situe entre la partie antérieure de la prostate et la symphyse pubienne. Une telle méthode est décrite dans la partie Matériel et Méthodes ci-dessous.

La limite de 10 cm² a été définie statistiquement comme étant la surface au-delà de laquelle tous les patients ont un cancer significativement plus agressif (score de Gleason supérieur ou égal à 7).

Les inhibiteurs de l'expression de la CCL7 ou de CCR3 incluent des acides nucléiques tels que des oligonucléotides antisens modifiés ou non, des ARN interférents, des petits ARN en épingle à cheveux (shRNA ou miRNA) ou des ribozymes, ciblant le gène codant CCL7 ou le gène codant CCR3. De tels inhibiteurs sont décrits par Zhu, Liao et al. 2013 ; Agrawal, Maxwell et al. 2009 ; Zhu, Liu et al. 2014.

Selon un mode réalisation préféré, l'inhibiteur de l'expression de CCR3 est un shRNA ciblant le gène CCR3. Il est avantageusement choisi parmi les shRNA : m4CCR3 (SEQ ID NO : 1), m5CCR3 (SEQ ID NO : 2), m6CCR3 (SEQ ID NO : 3).

Selon un autre mode réalisation préféré, l'inhibiteur de l'expression de CCR3 est un oligonucléotide antisens, éventuellement modifié, ciblant le gène CCR3. Il est avantageusement choisi dans le groupe constitué par les oligonucléotides antisens TOP004 possédant la séquence SEQ ID NO : 14, TOP005 possédant la séquence SEQ ID NO : 15, TOP030 possédant la séquence SEQ ID NO : 16, TOP030-P²M-7-DAP possédant la séquence SEQ ID NO : 17, TQP030-P²M-8-DAP possédant la séquence SEQ ID NO : 18, TOP030-P²M-9-DAP possédant la séquence SEQ ID NO : 19, TOP030-P²M-10-DAP possédant la séquence SEQ ID NO : 20, TOP030-P²M-11-DAP possédant la séquence SEQ ID NO : 21, TOP030-P²M-12-DAP possédant la séquence SEQ ID NO: 22, TOP030-P²M-13-DAP possédant la séquence SEQ ID NO : 23, TOP030-P²M-14-DAP possédant la séquence SEQ ID NO : 24 and TOP030-P²M-15-DAP possédant la séquence SEQ ID NO : 25.

On entend par « un inhibiteur de l'interaction CCL7/CCR3 » un composé capable d'inhiber l'activation de CCR3 par CCL7, sans activer lui-même CCR3. Un inhibiteur de l'interaction CCL7/CCR3 inclue les composés capables d'interagir avec CCL7 et d'inhiber sa liaison avec CCR3 ou d'inhiber l'activation de CCR3 résultant de ladite liaison, ainsi que les composés capables d'interagir avec CCR3, et d'inhiber leur liaison avec CCL7 ou leur activation résultant de ladite liaison. En particulier, un inhibiteur de l'interaction CCL7/CCR3 inclue des antagonistes compétitifs et non-compétitifs de CCR3 (appelés antagonistes de CCR3), de préférences les antagonistes compétitifs de CCR3.

Les inhibiteurs de l'interaction CCL7/CCR3 incluent les molécules organiques. A titre d'exemple de molécules organiques inhibitrices de l'interaction CCL7/CCR3 on peut citer les composés de formules I à DCCLXXXIV décrits ci-dessous, de préférence le composé UCB35625 (C₃₀H₃₇Cl₂IN₂O₂) de formule DLI (ci-dessous).

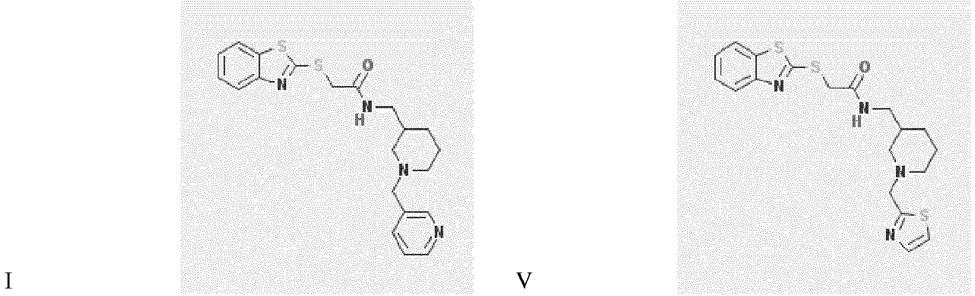

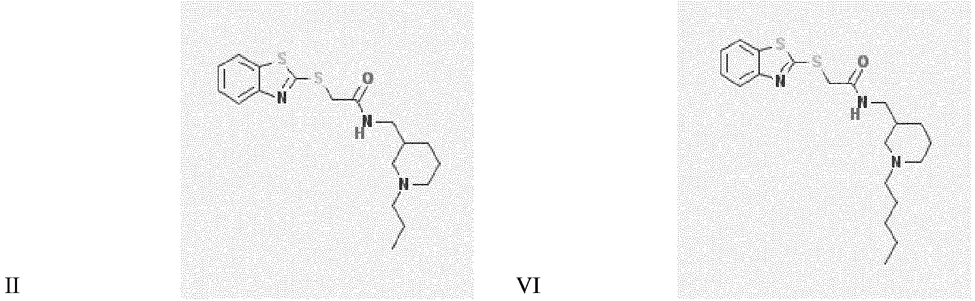

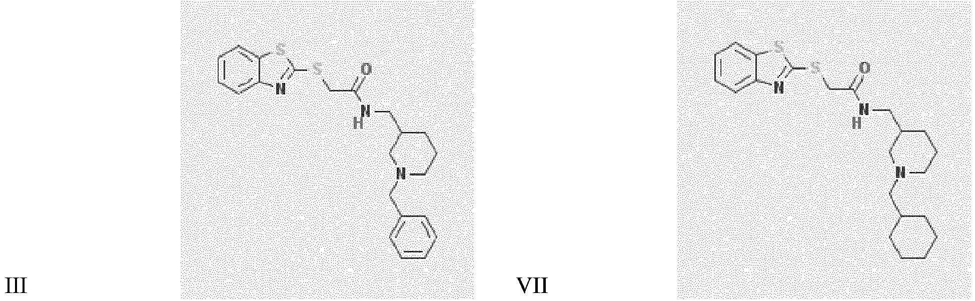

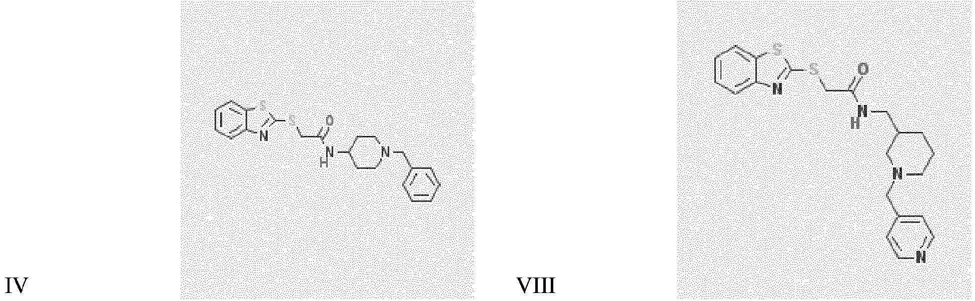

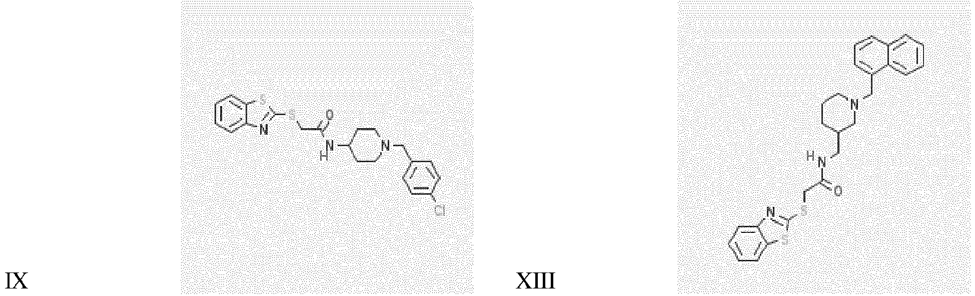

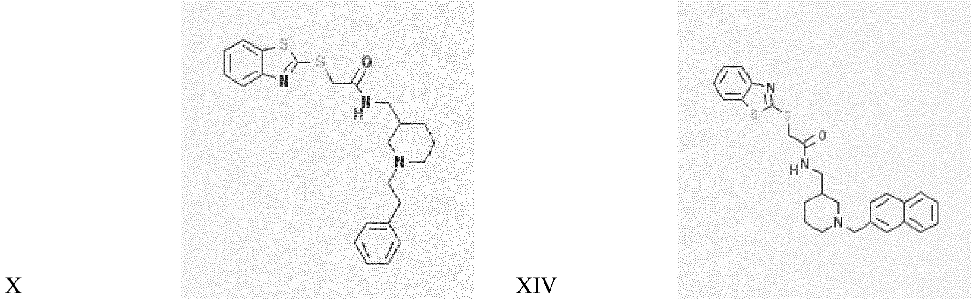

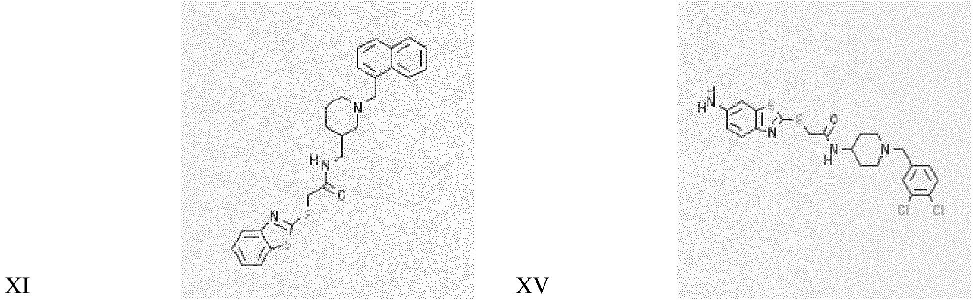

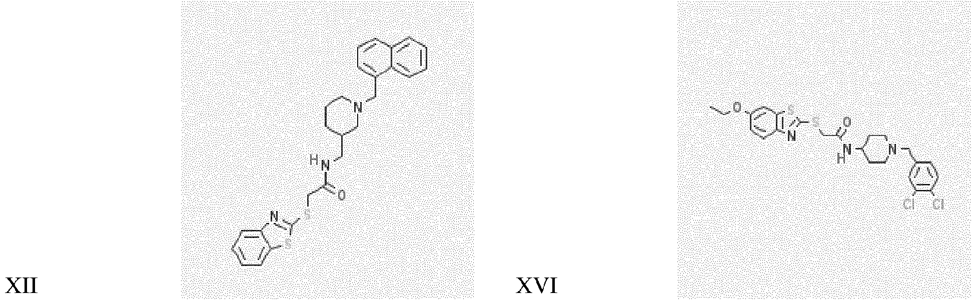

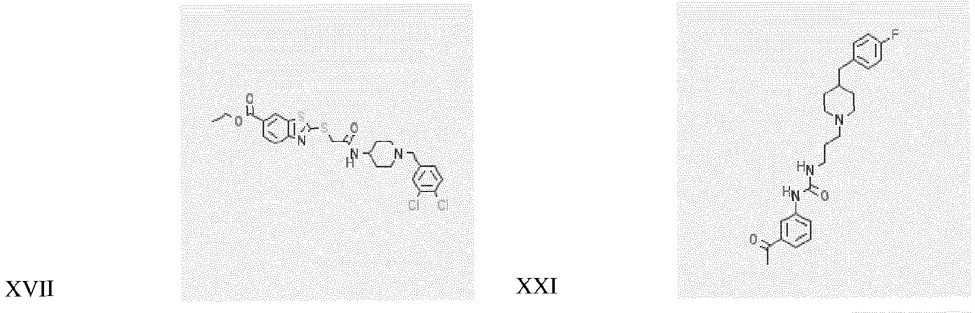

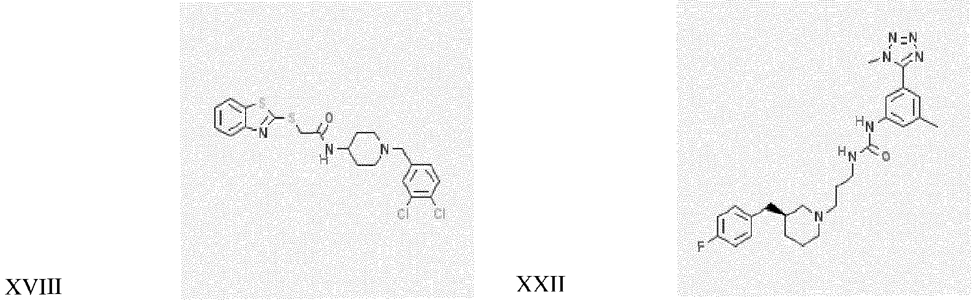

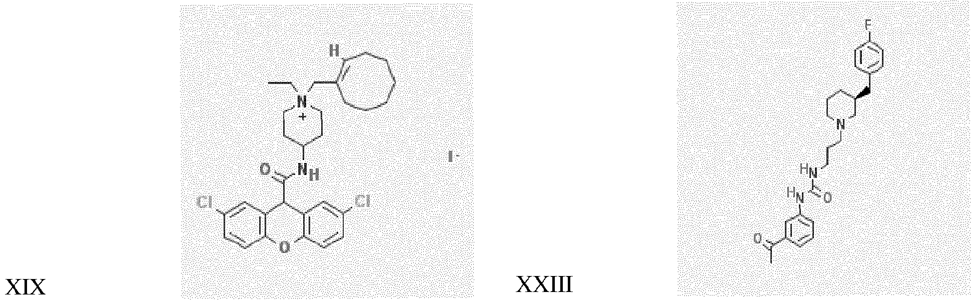

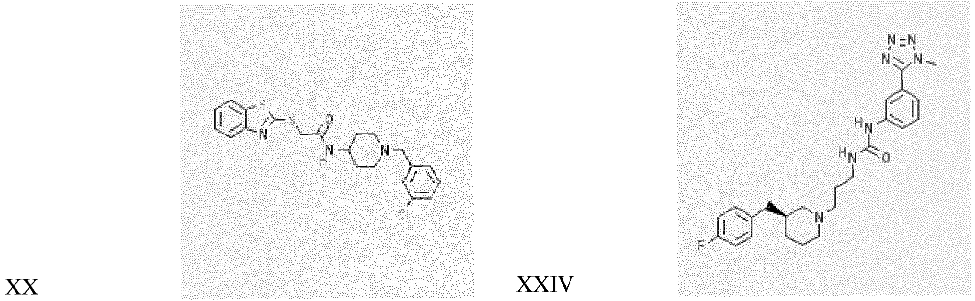

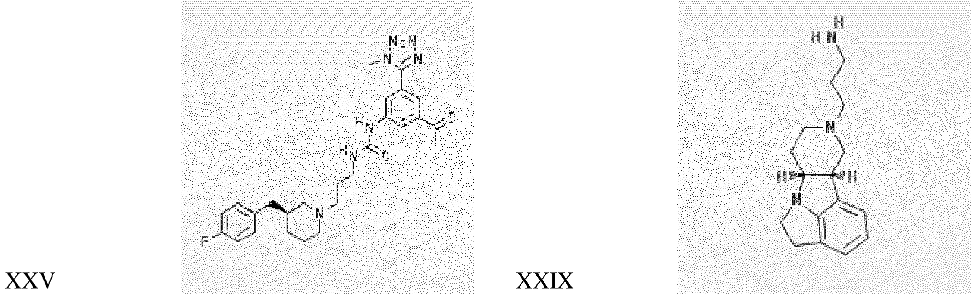

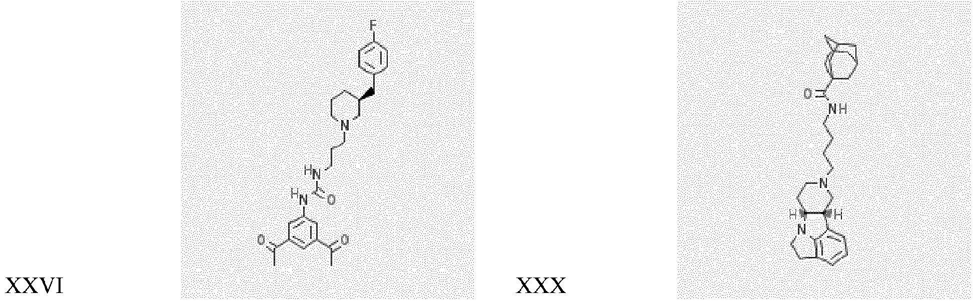

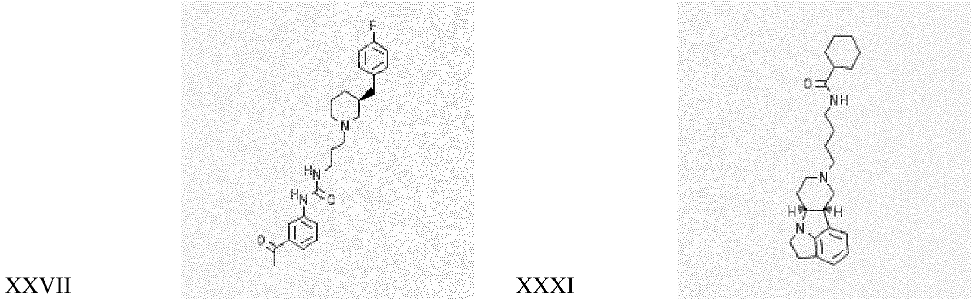

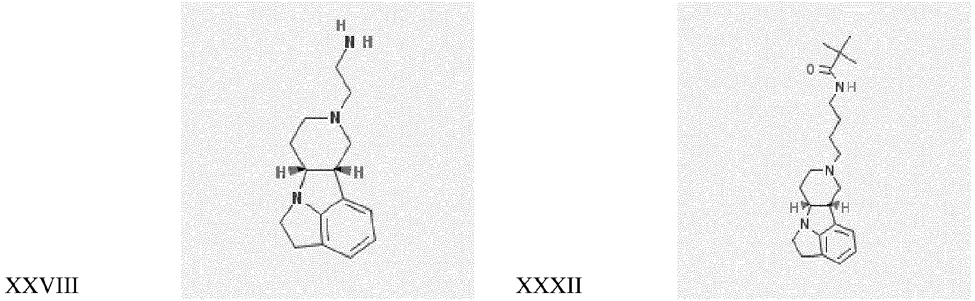

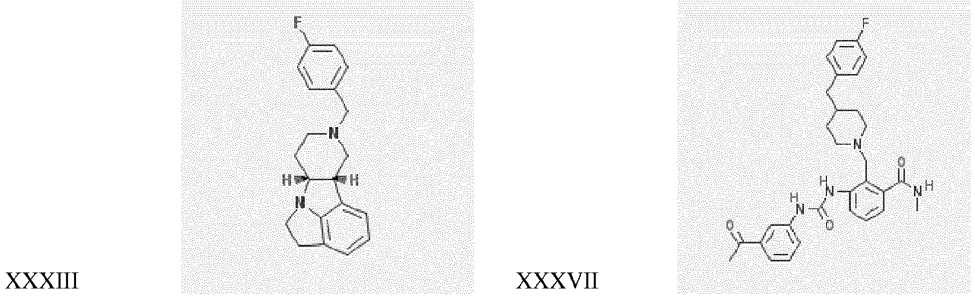

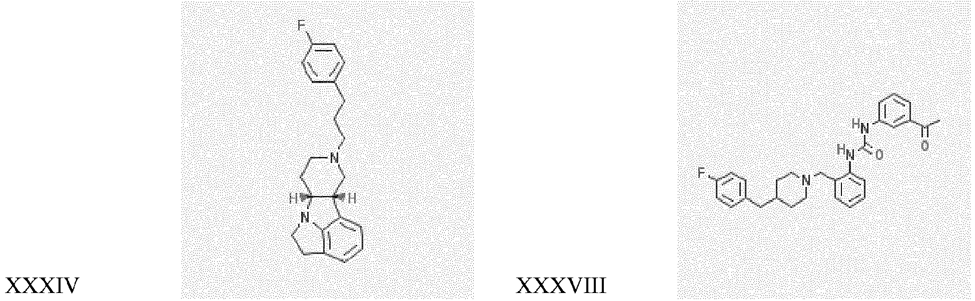

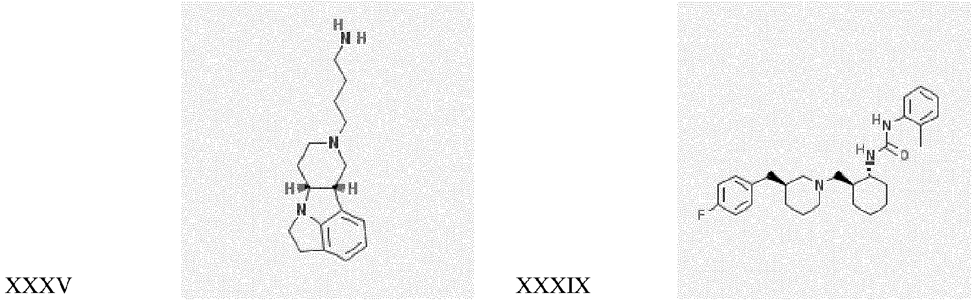

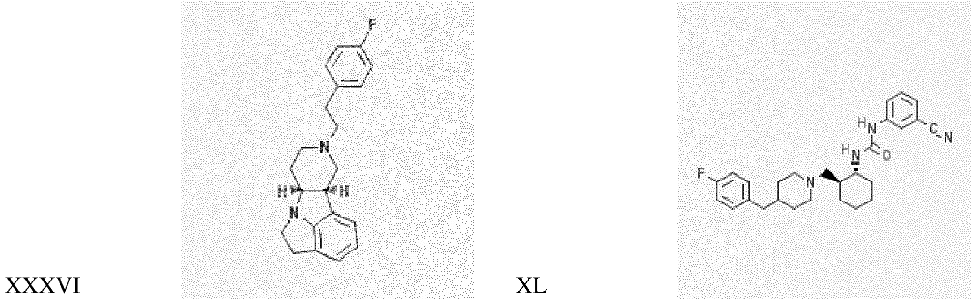

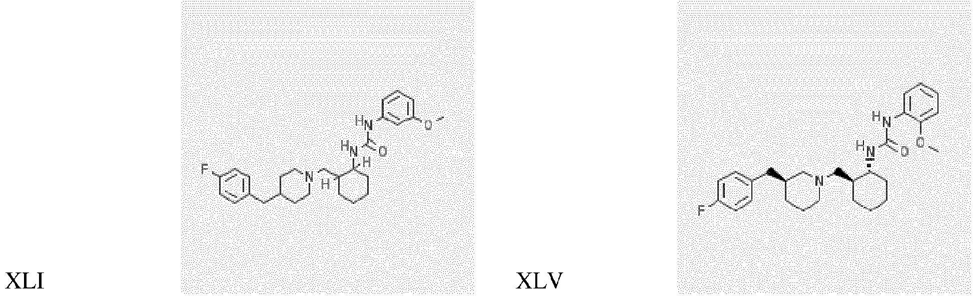

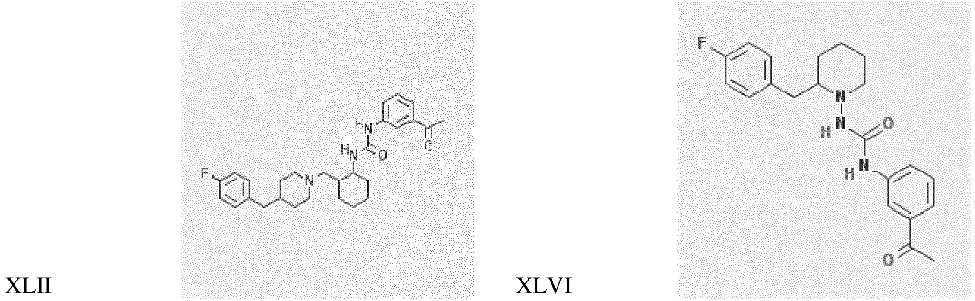

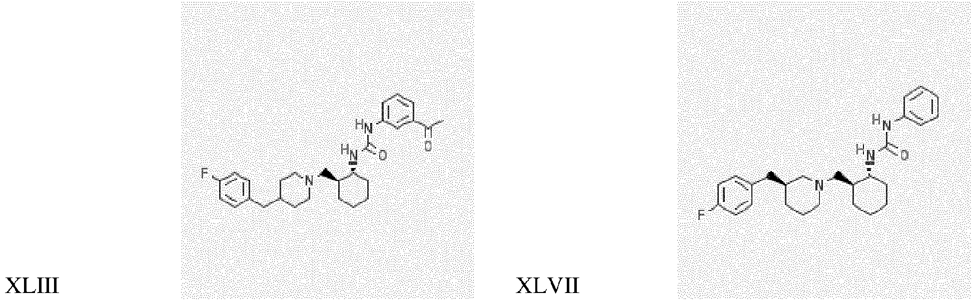

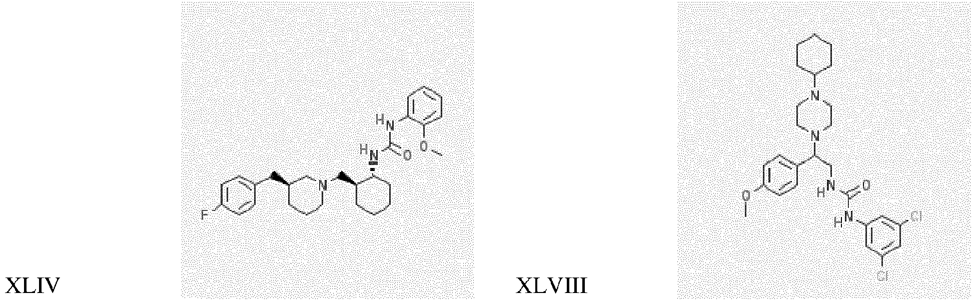

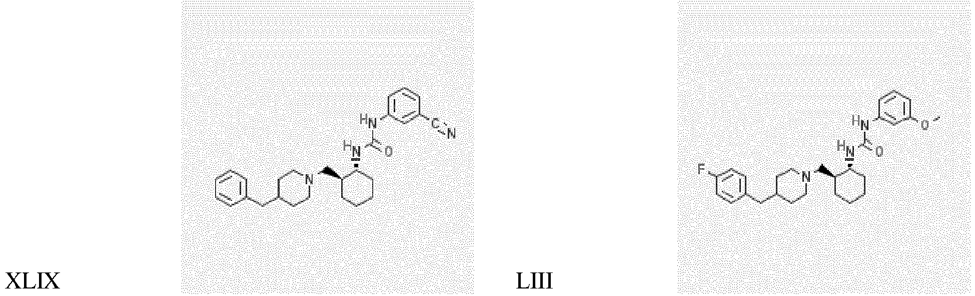

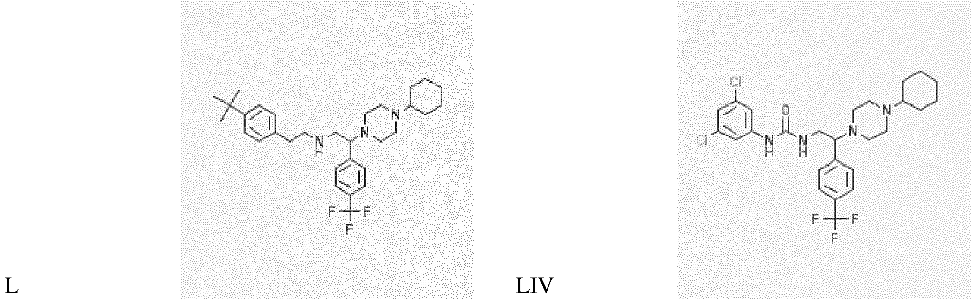

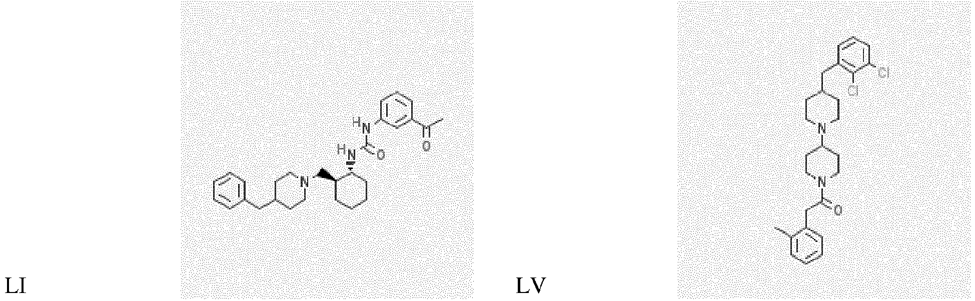

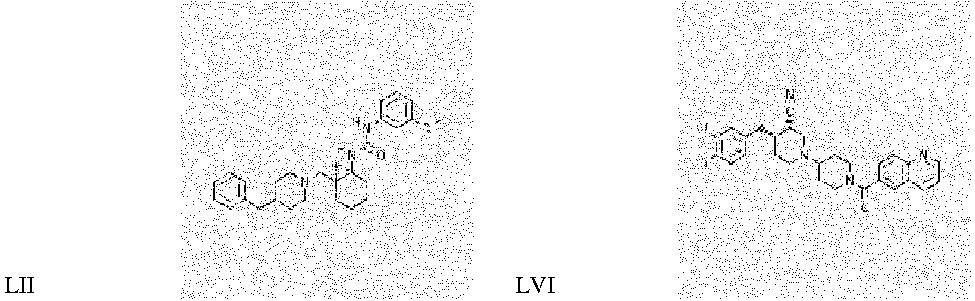

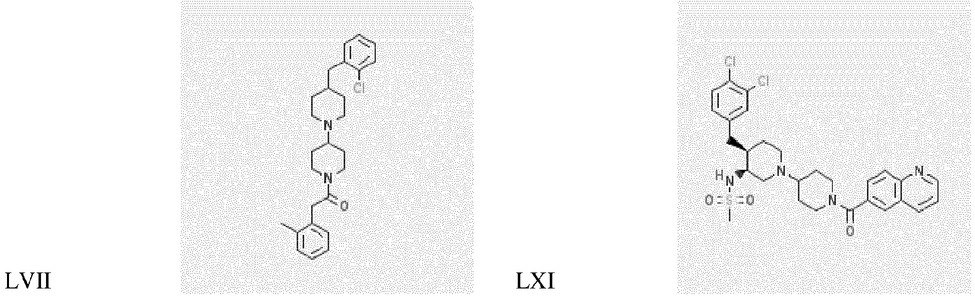

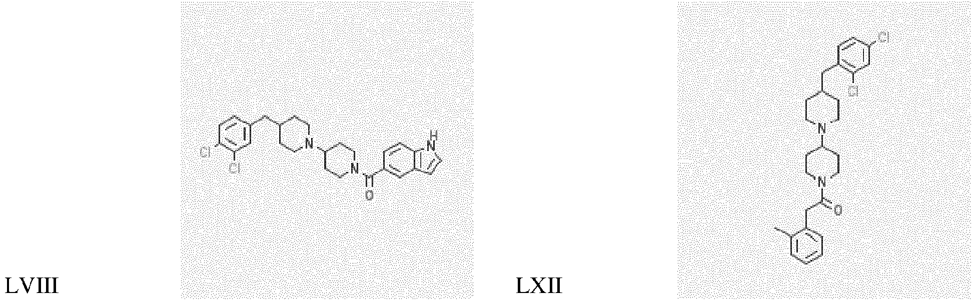

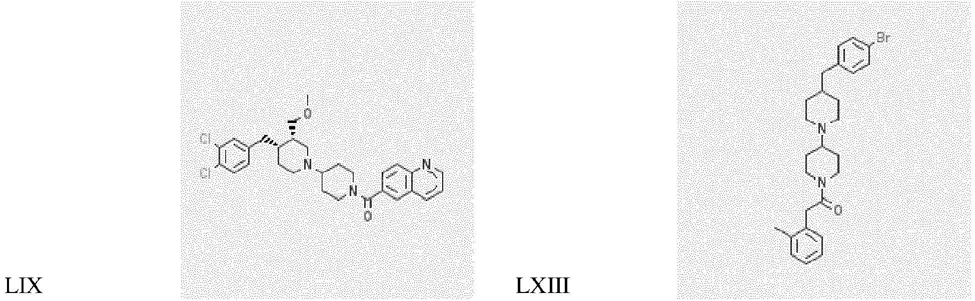

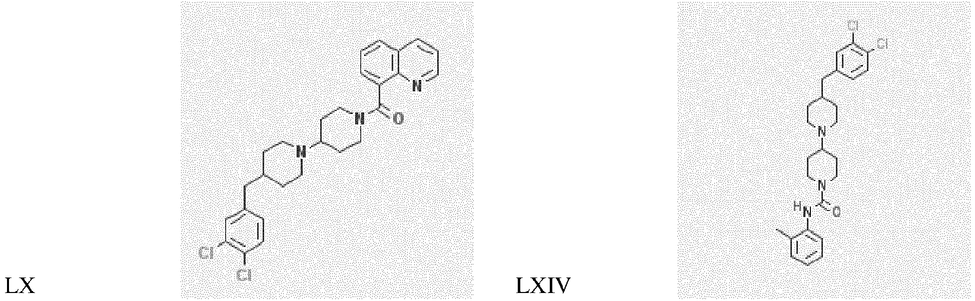

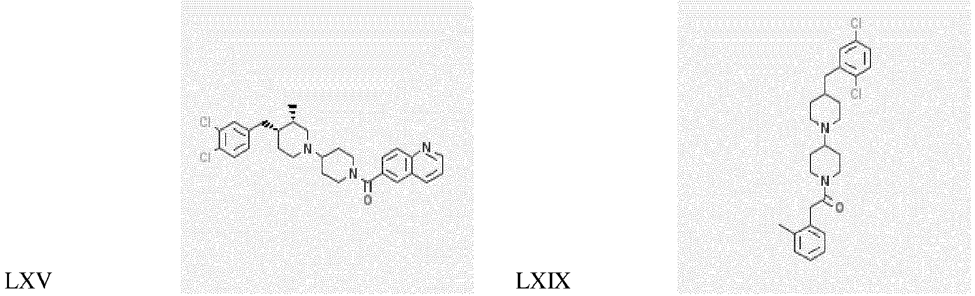

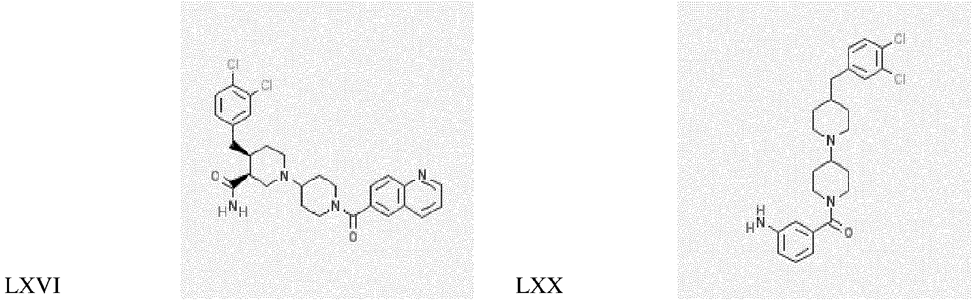

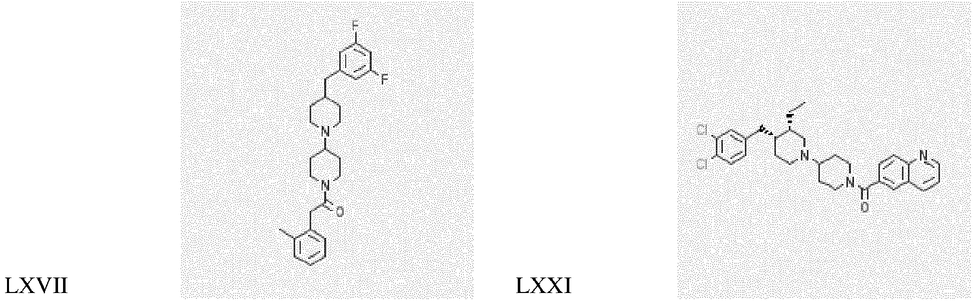

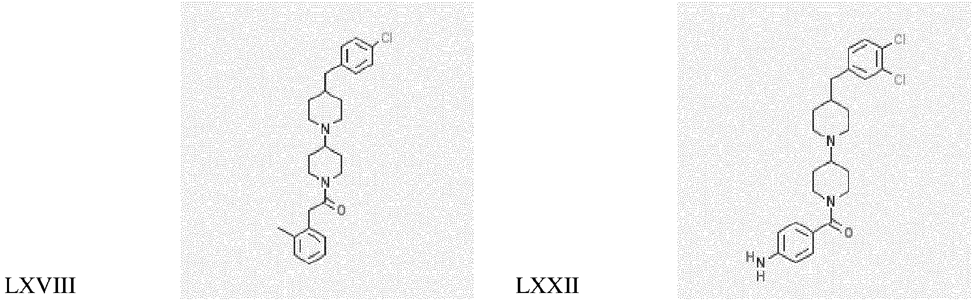

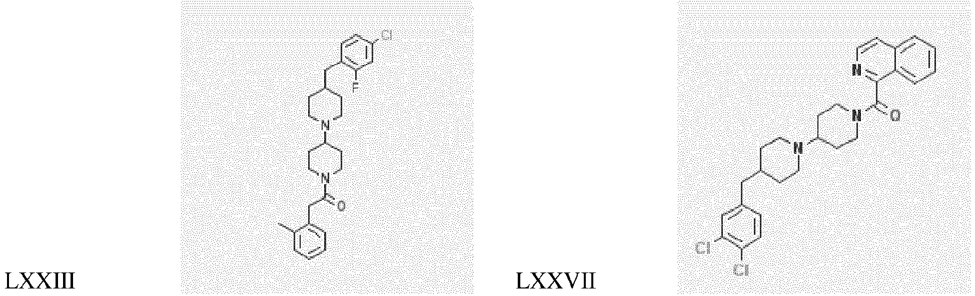

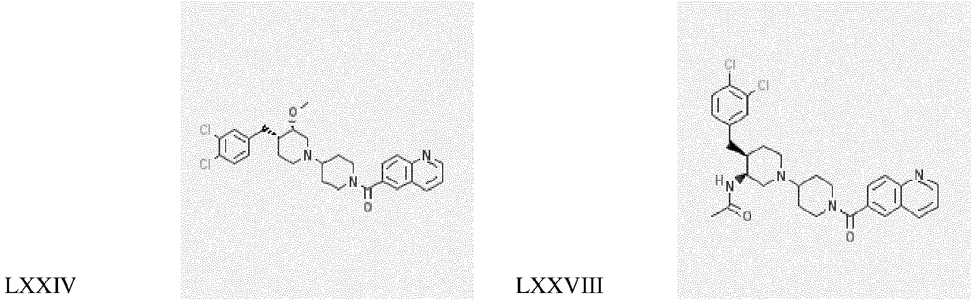

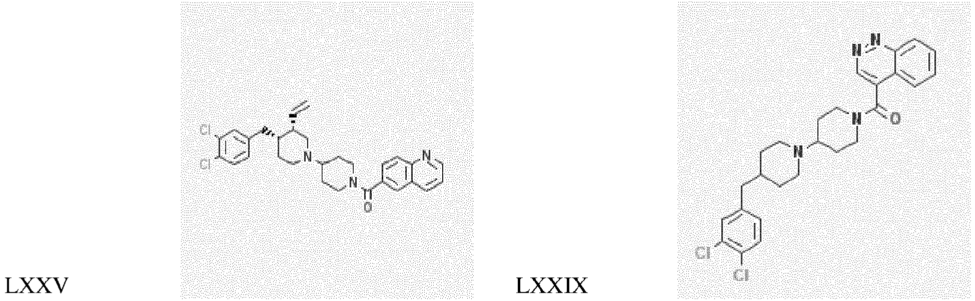

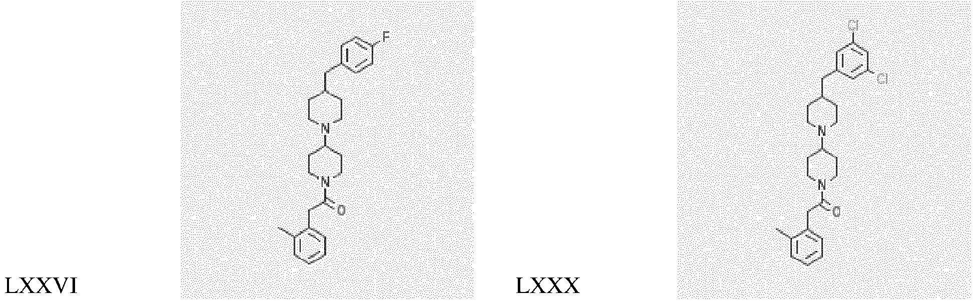

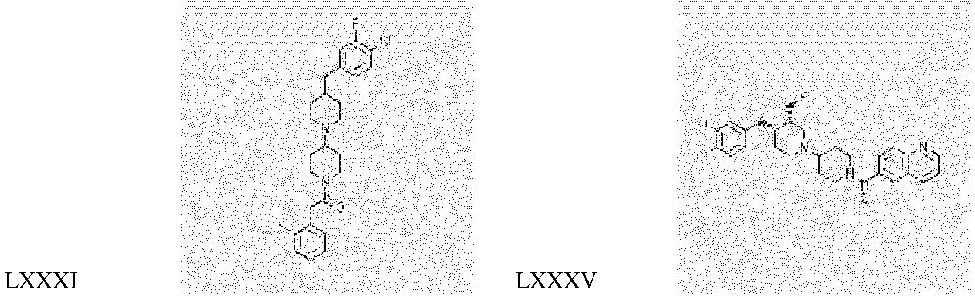

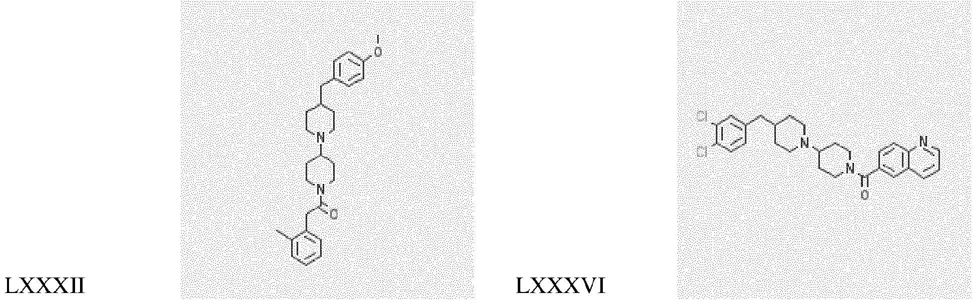

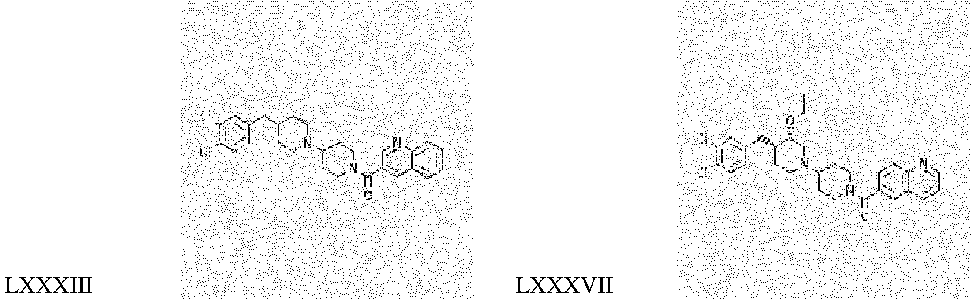

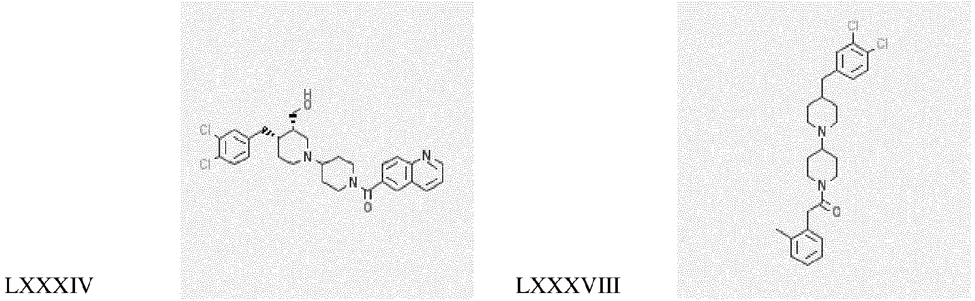

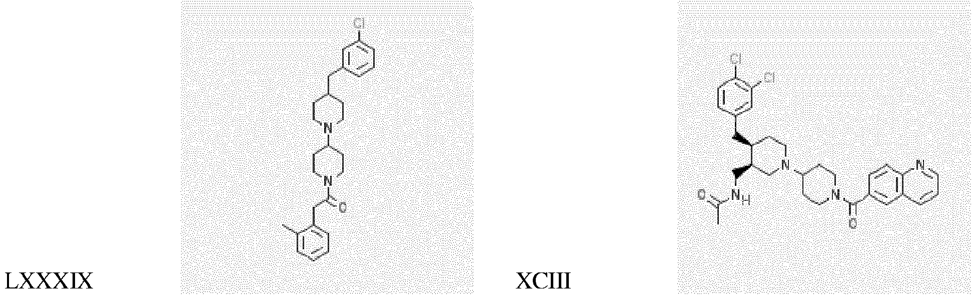

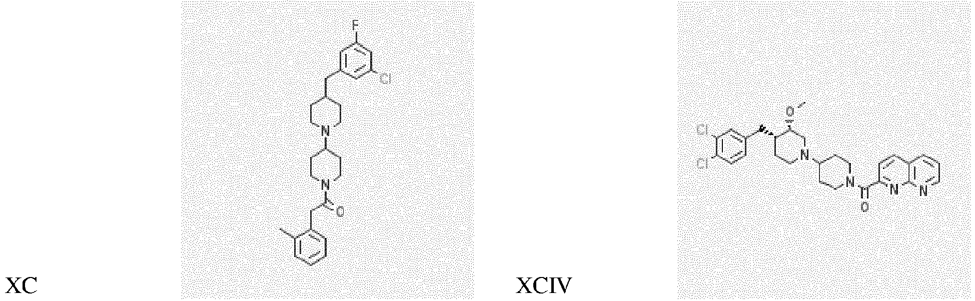

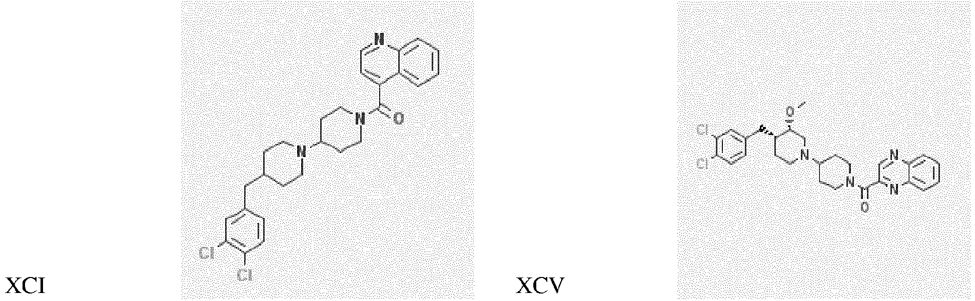

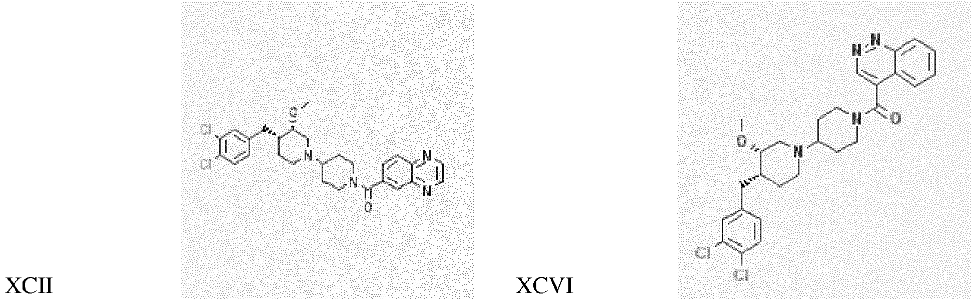

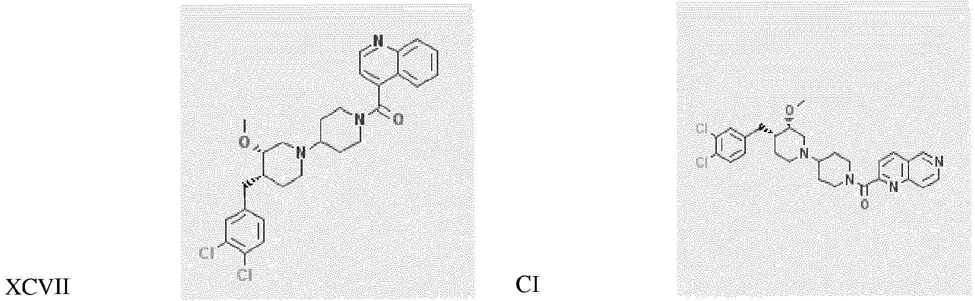

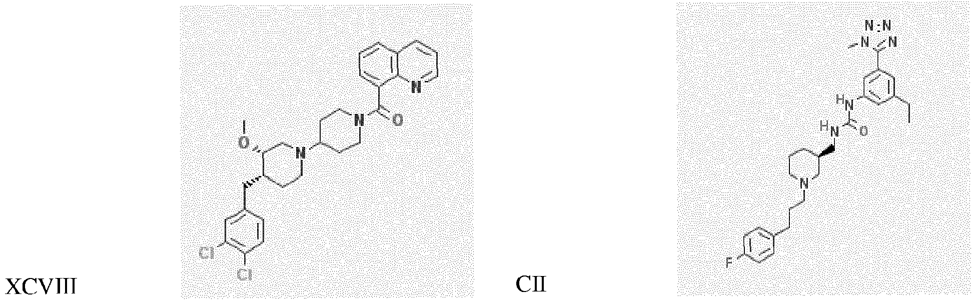

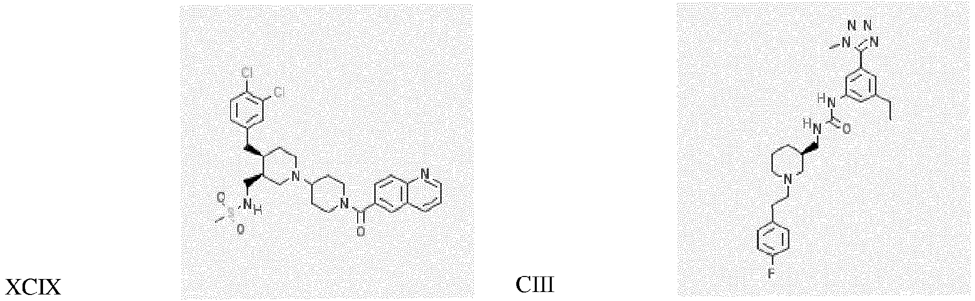

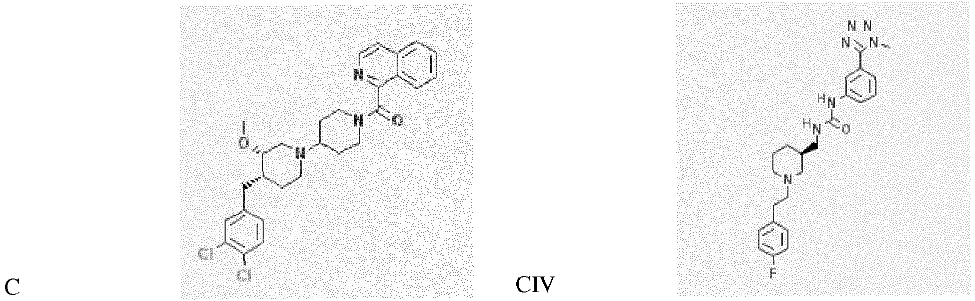

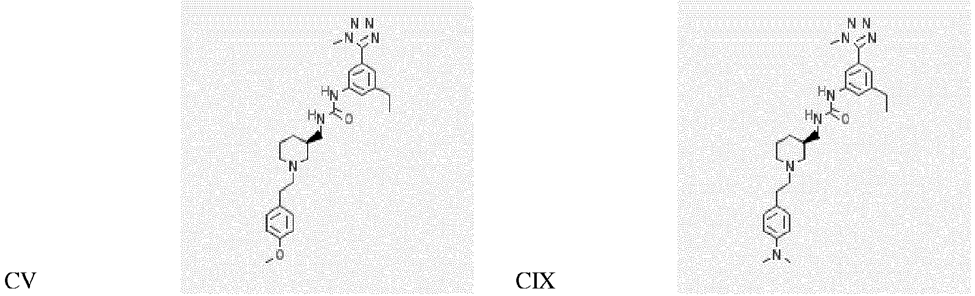

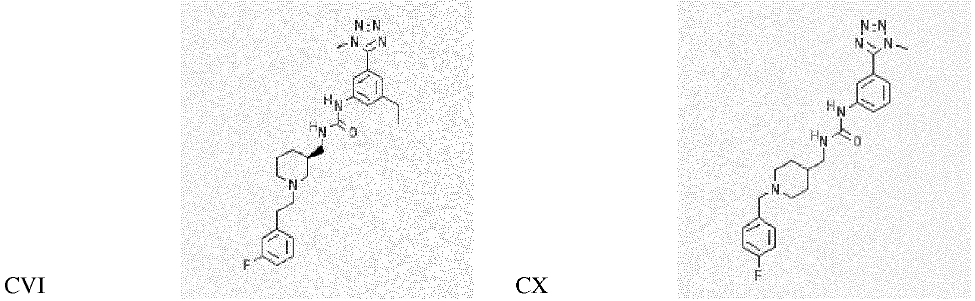

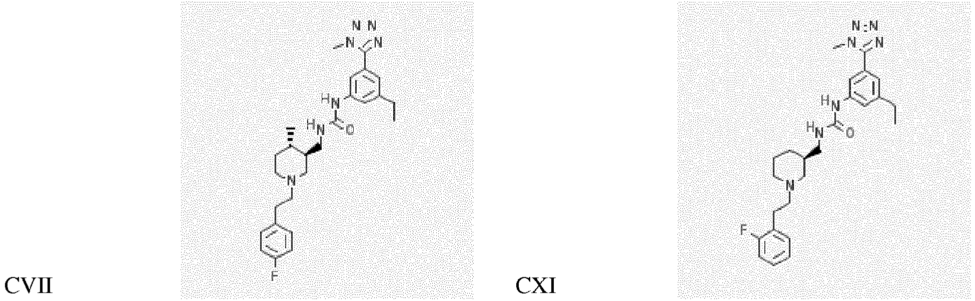

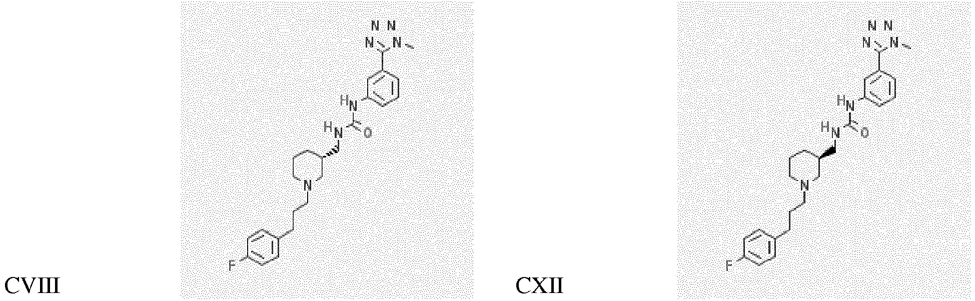

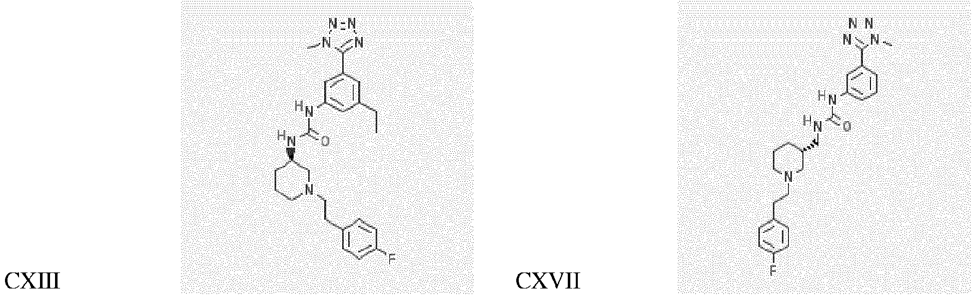

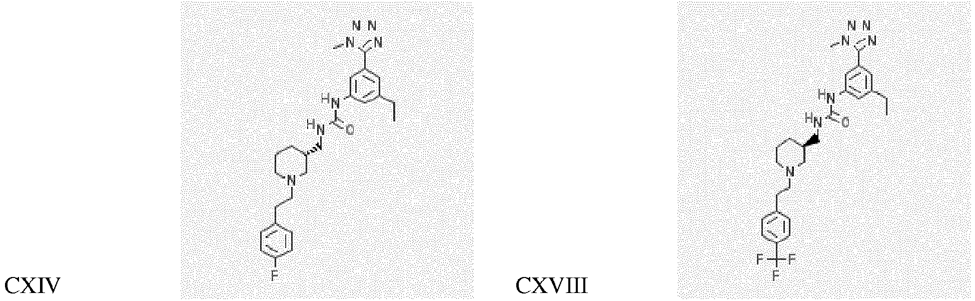

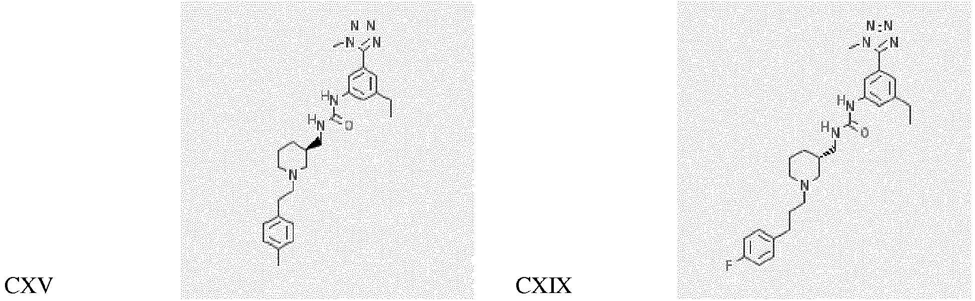

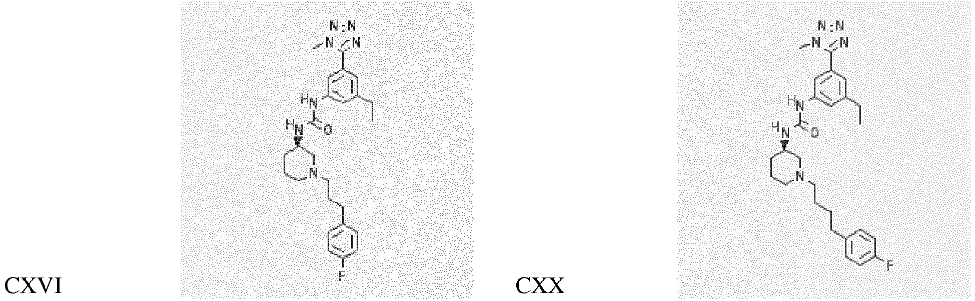

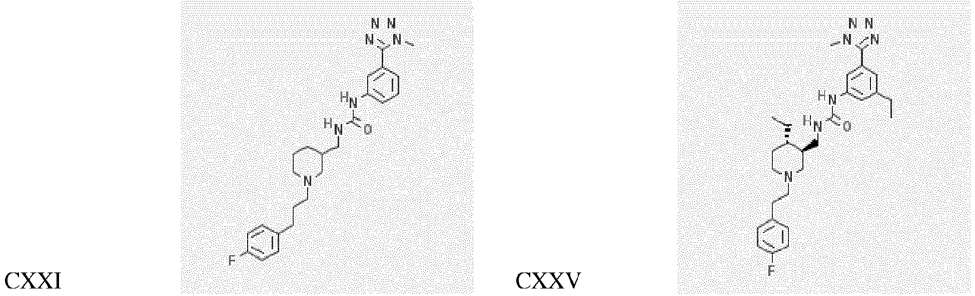

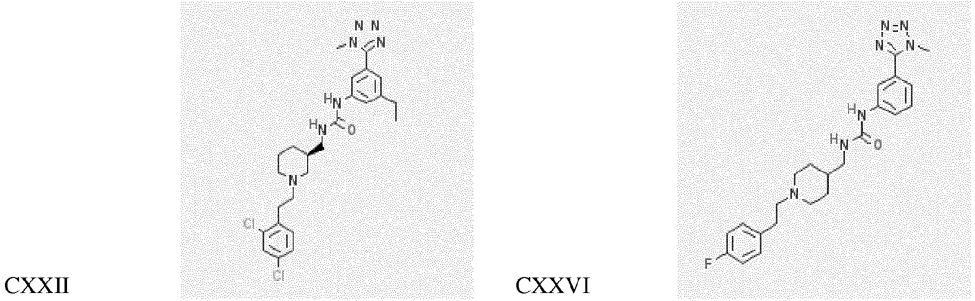

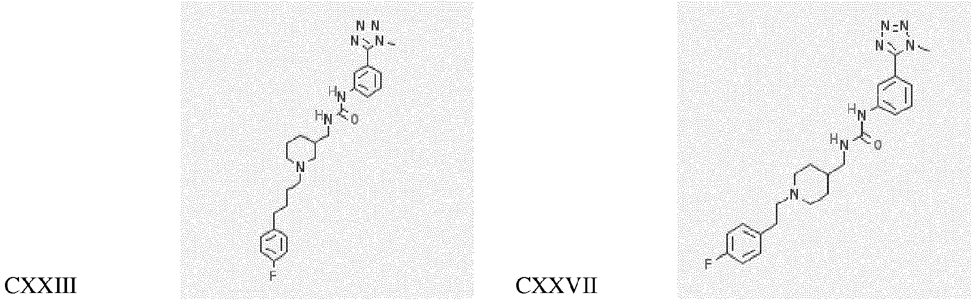

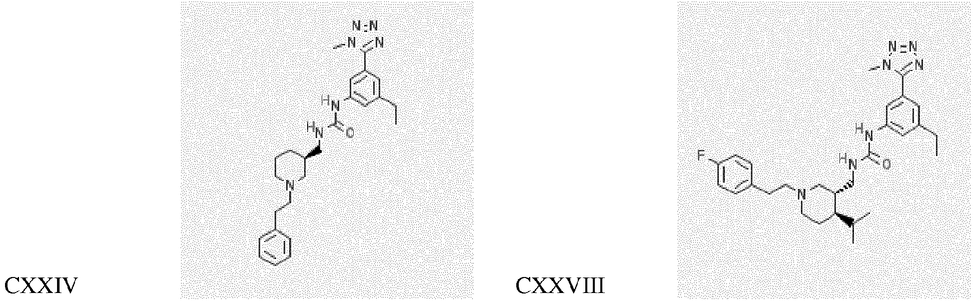

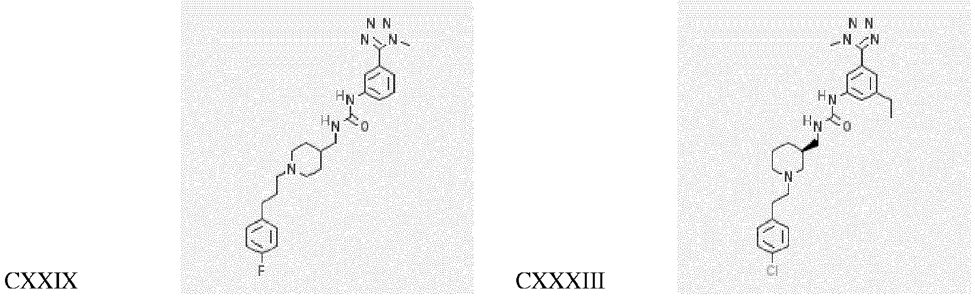

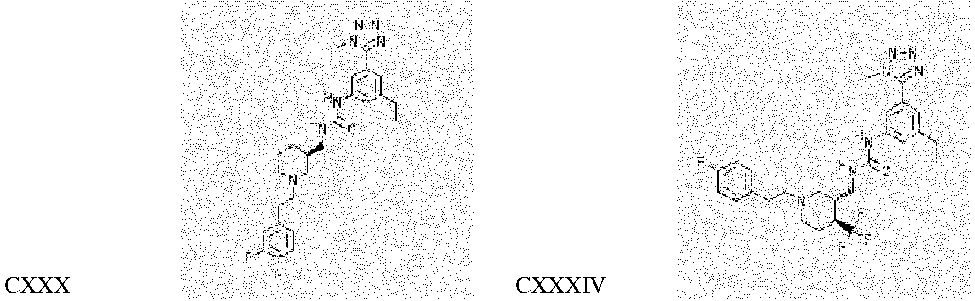

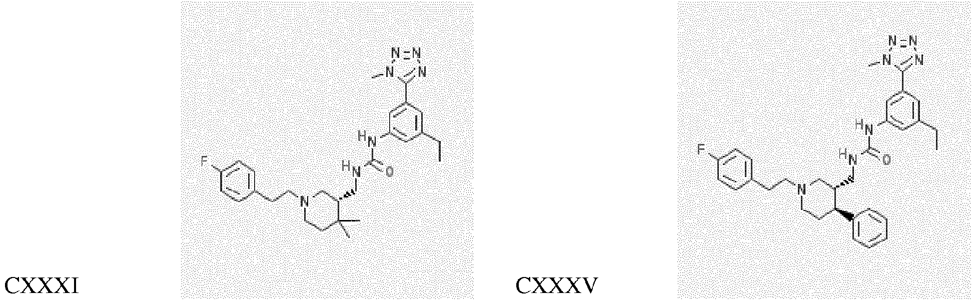

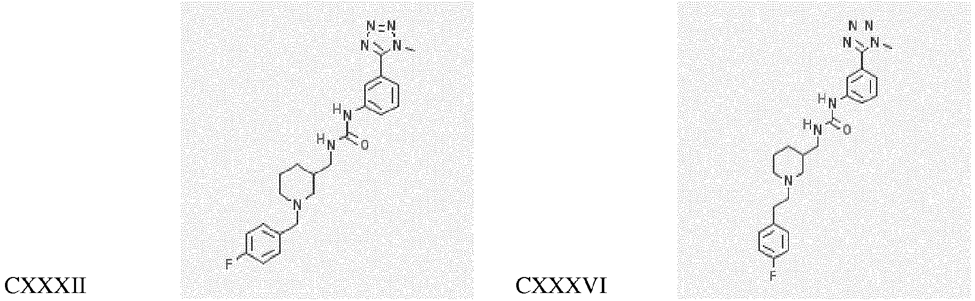

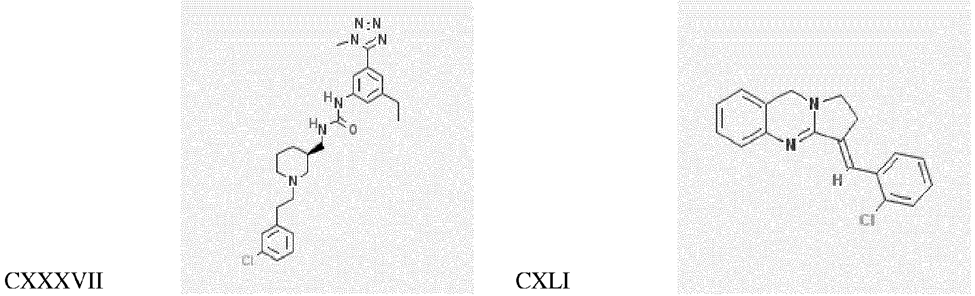

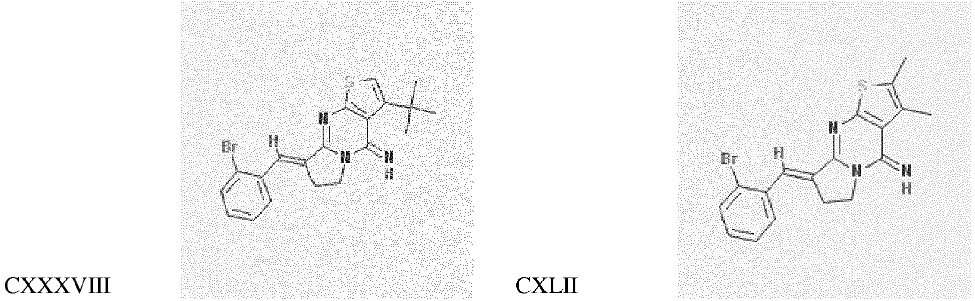

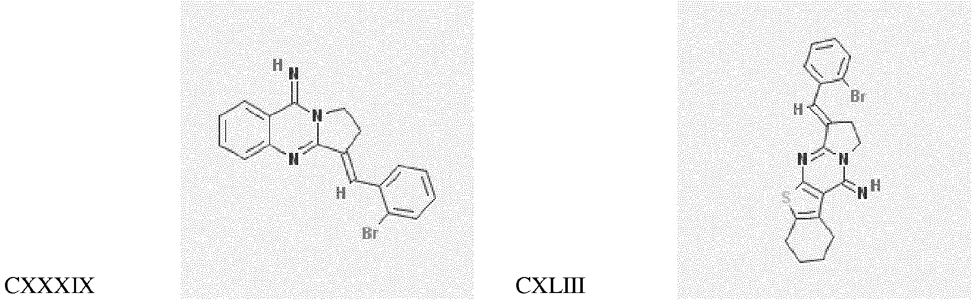

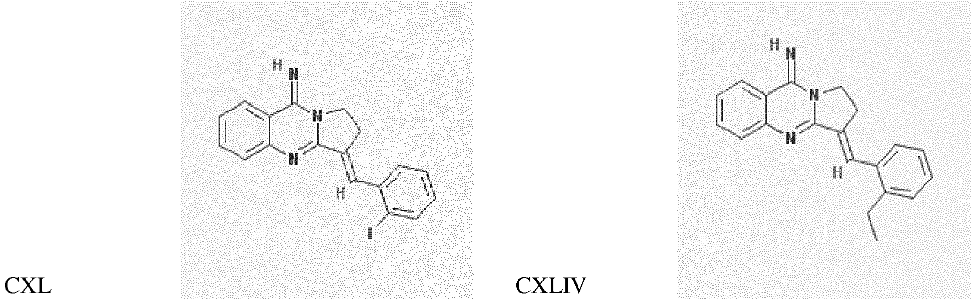

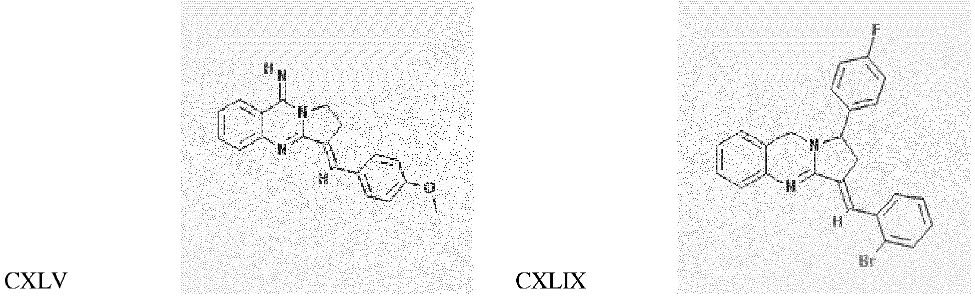

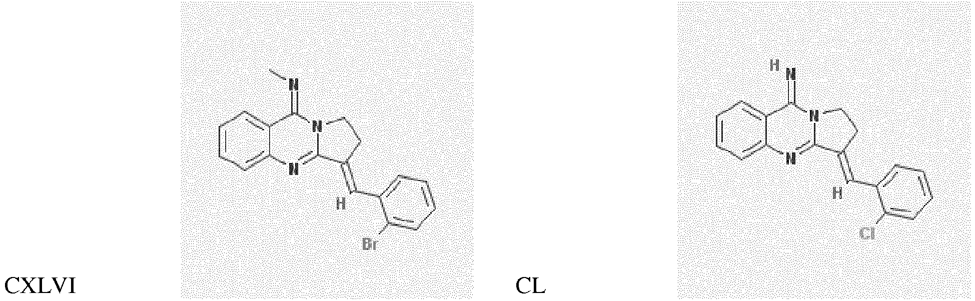

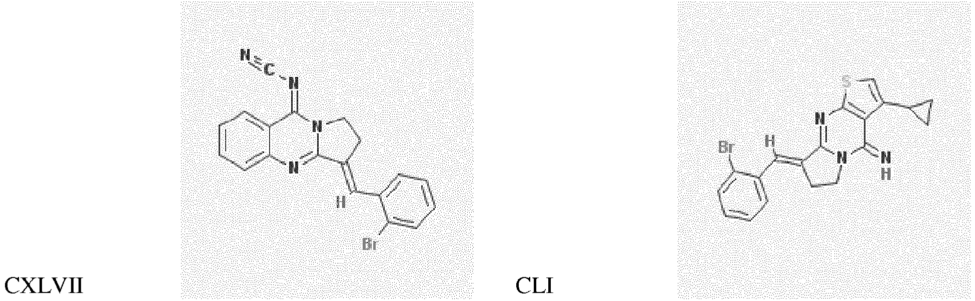

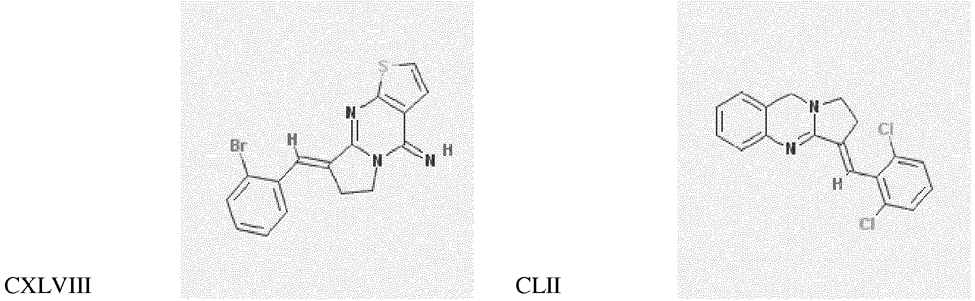

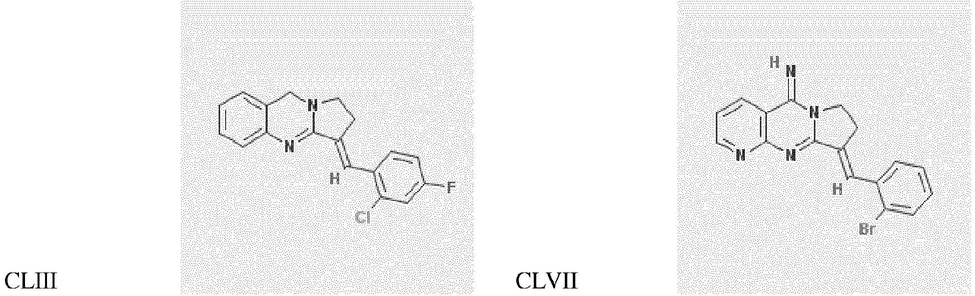

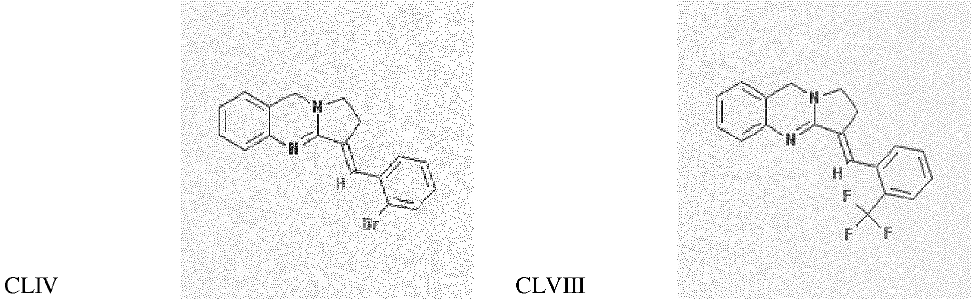

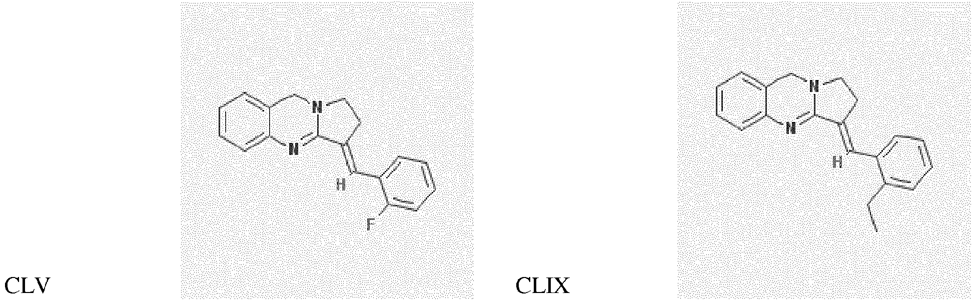

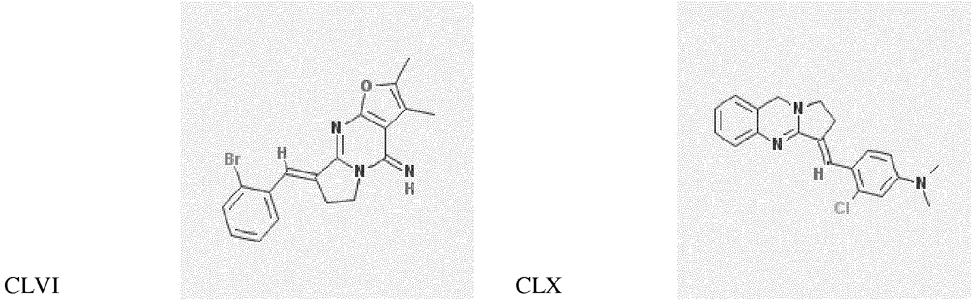

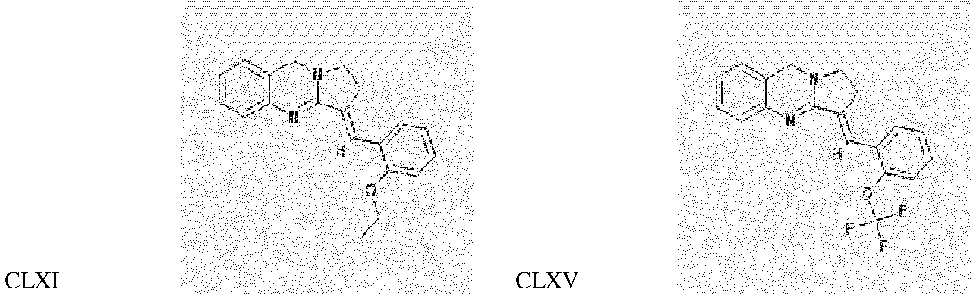

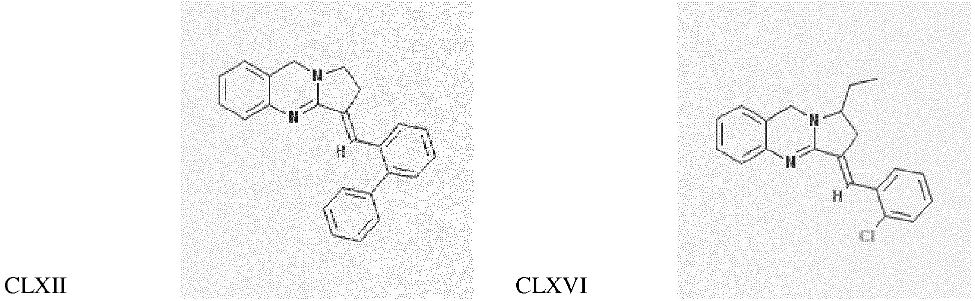

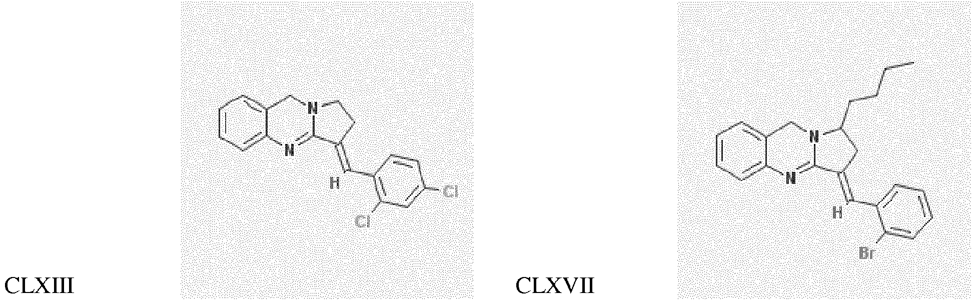

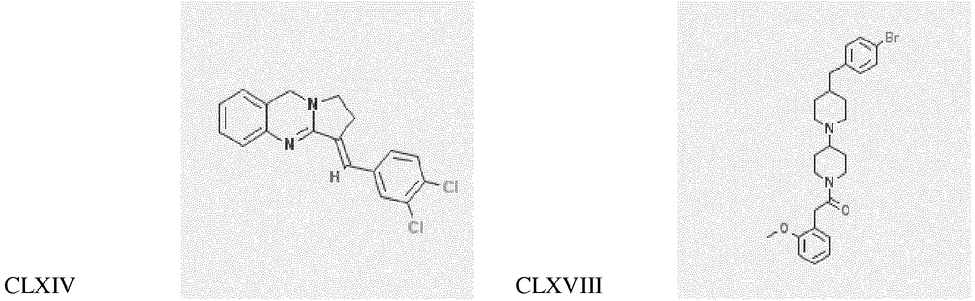

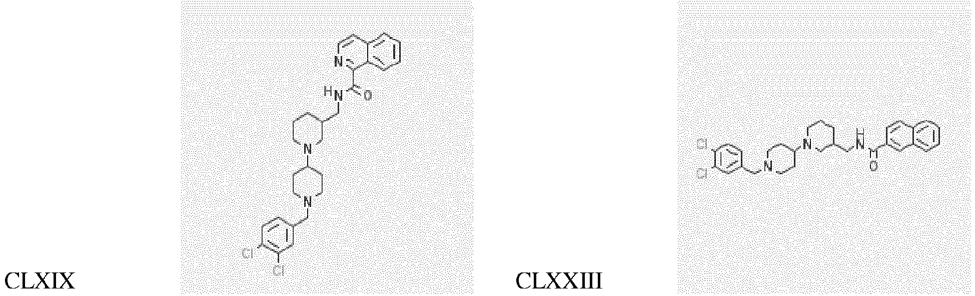

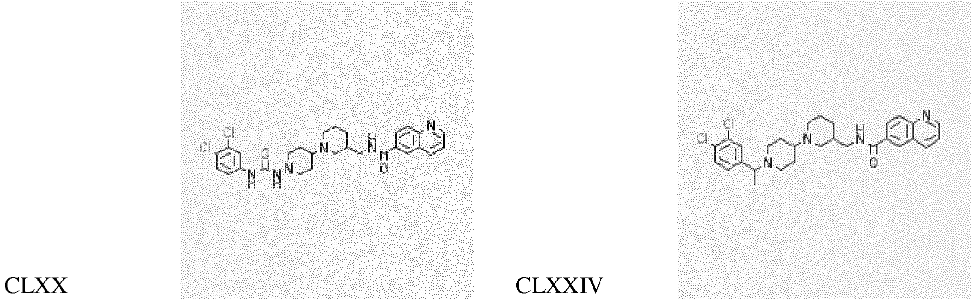

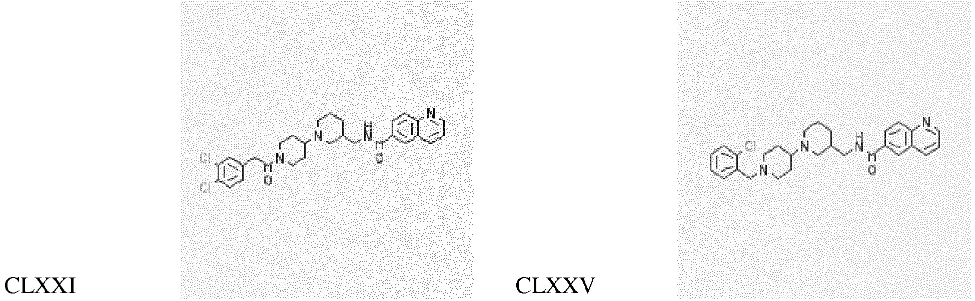

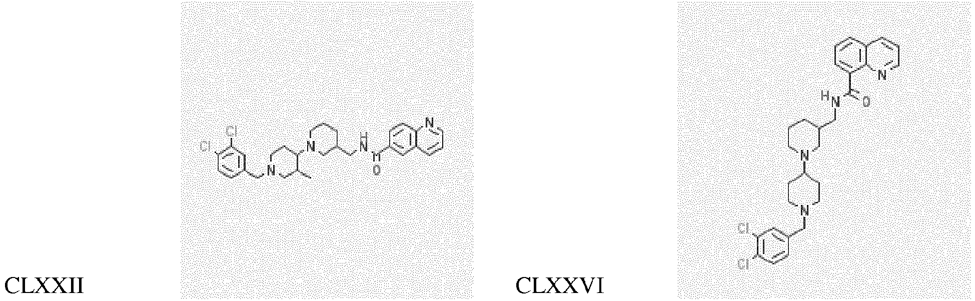

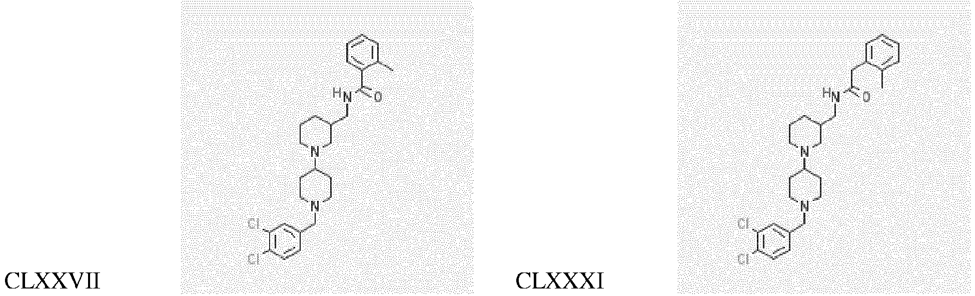

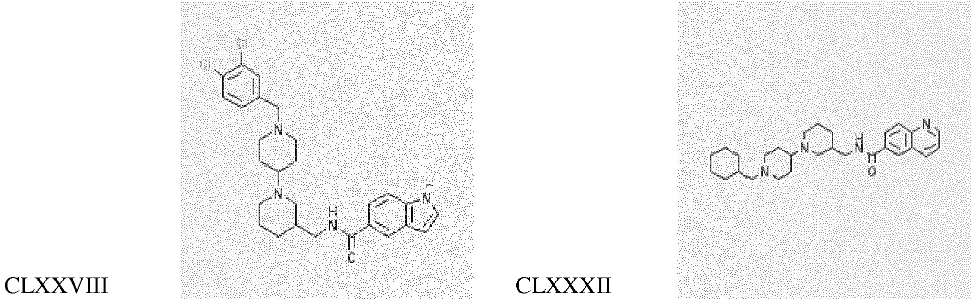

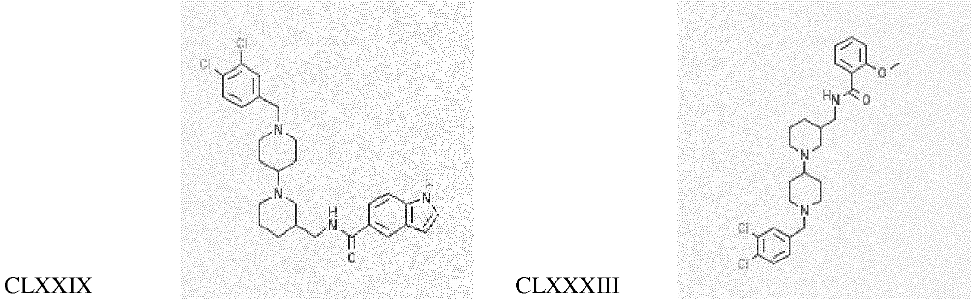

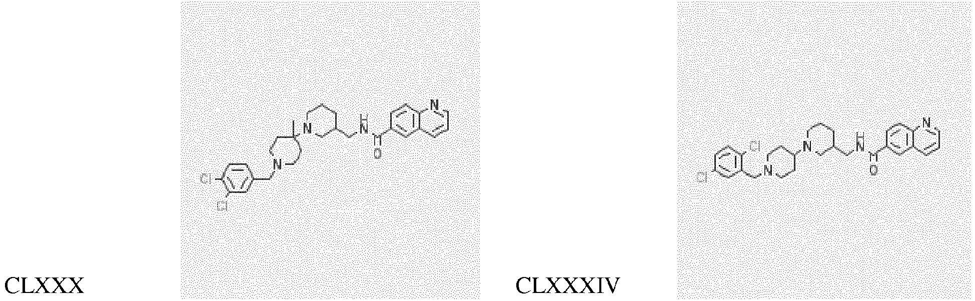

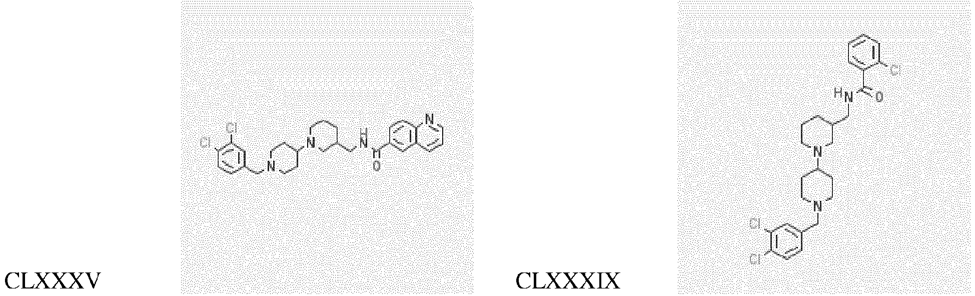

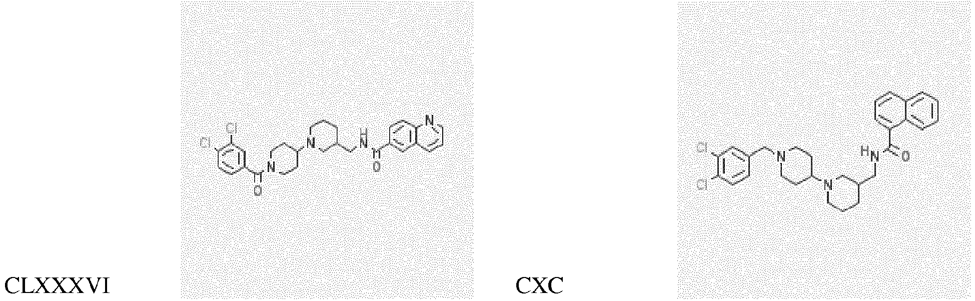

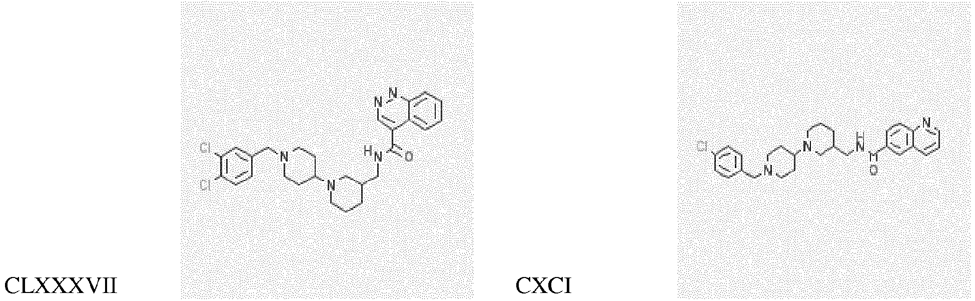

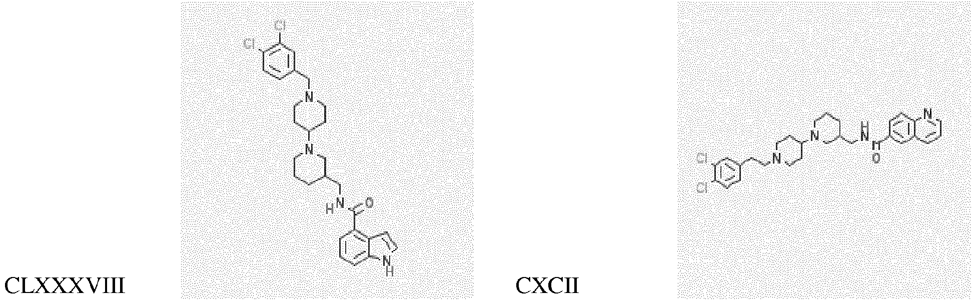

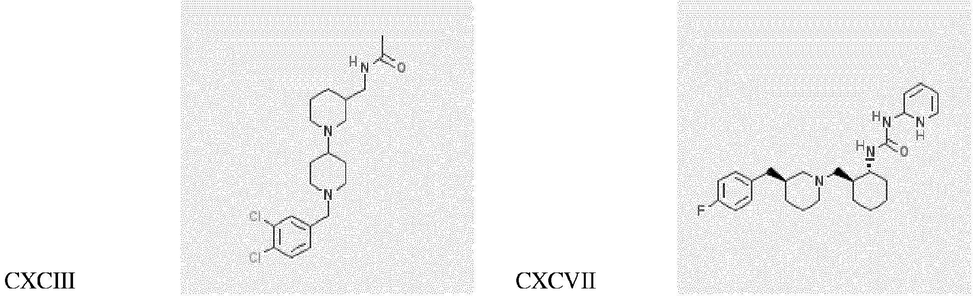

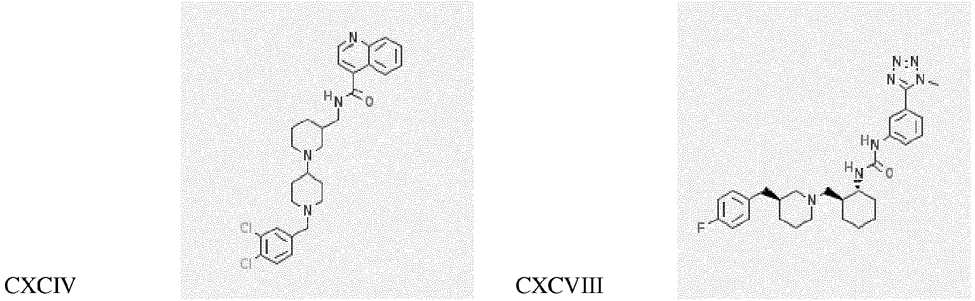

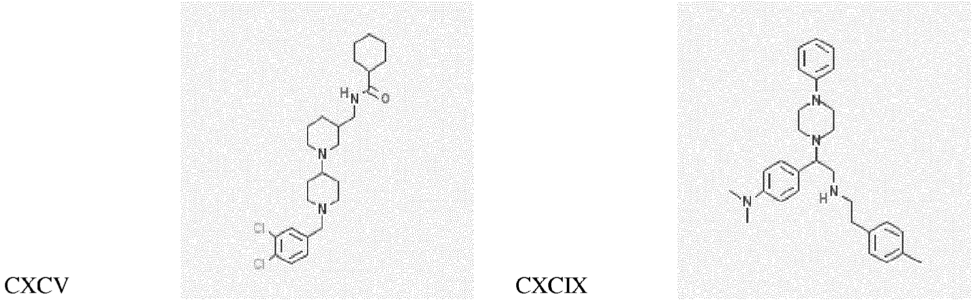

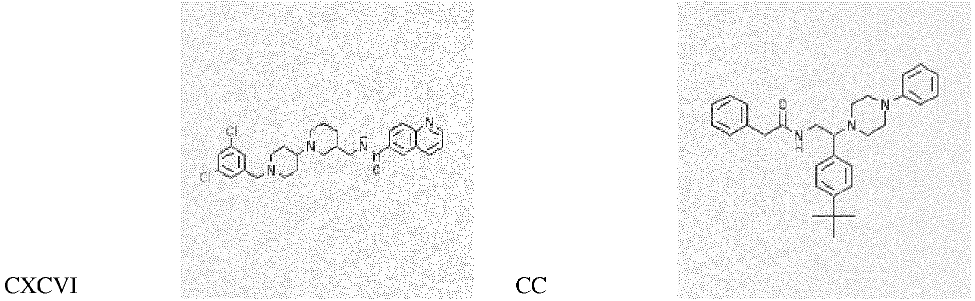

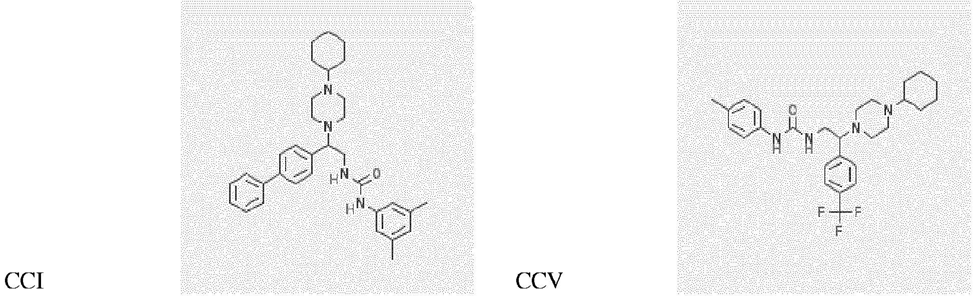

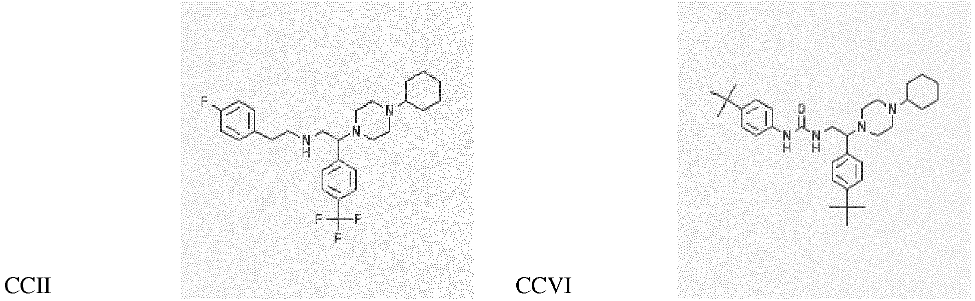

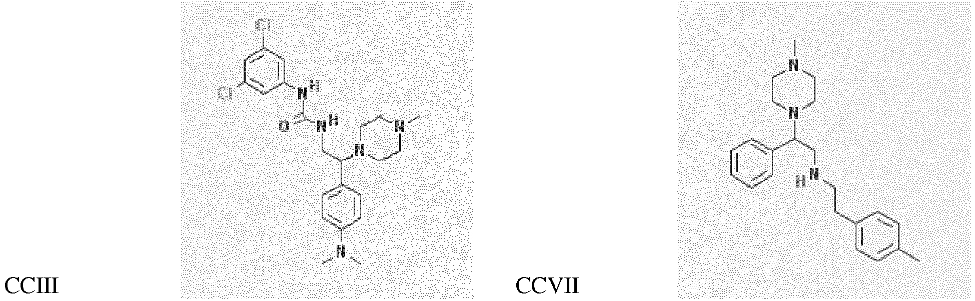

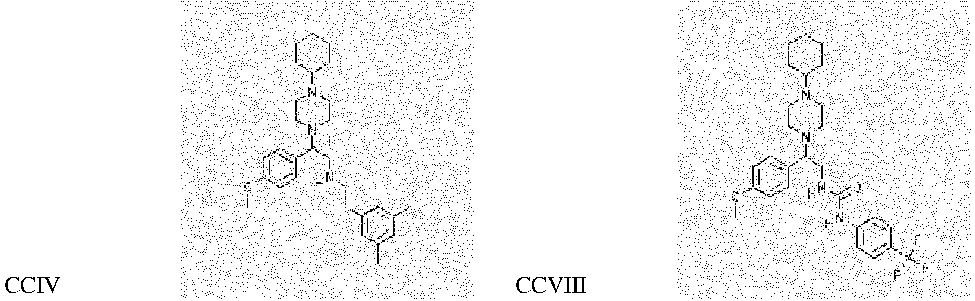

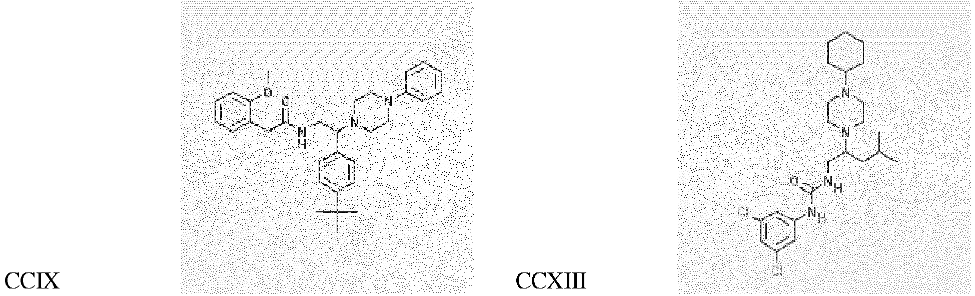

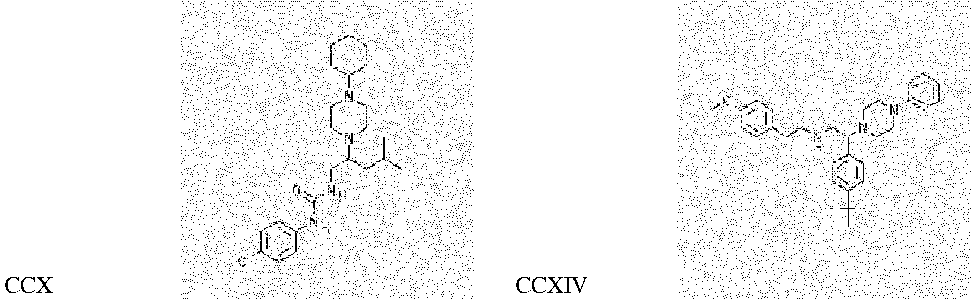

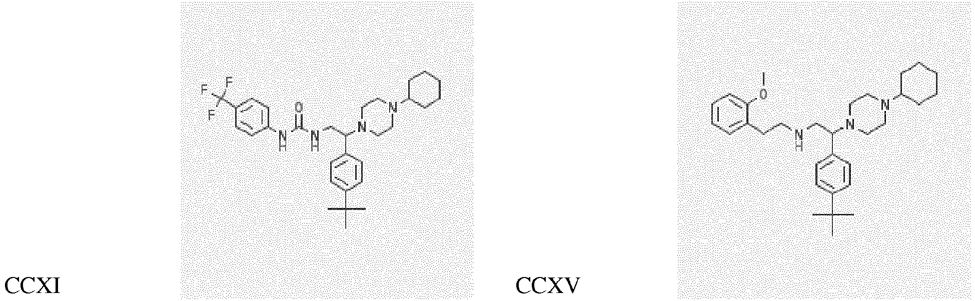

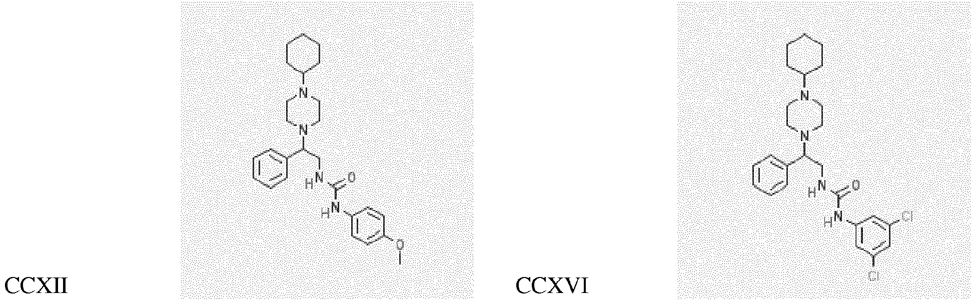

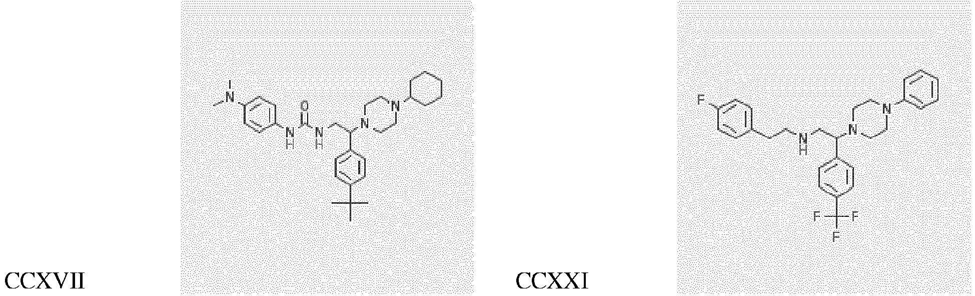

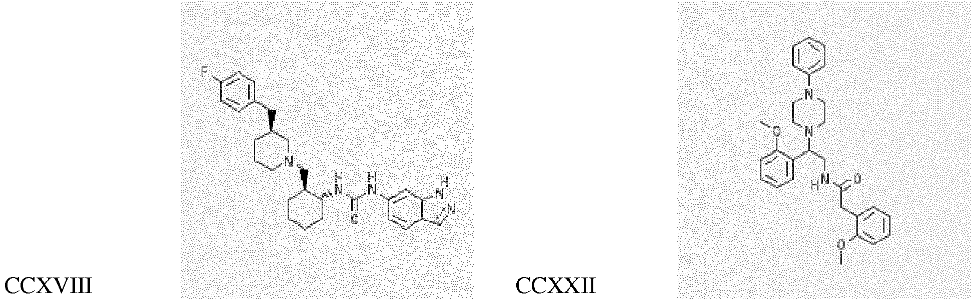

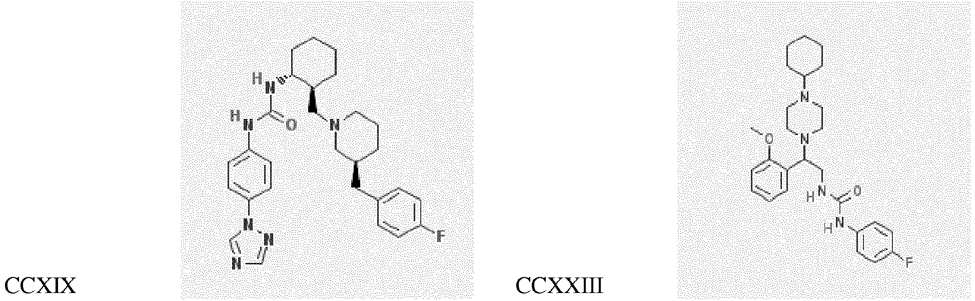

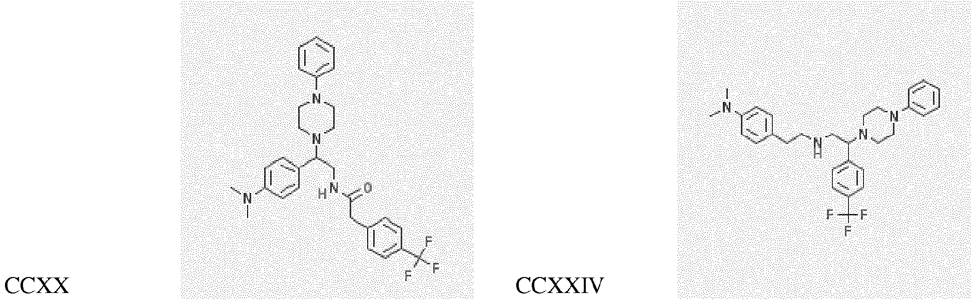

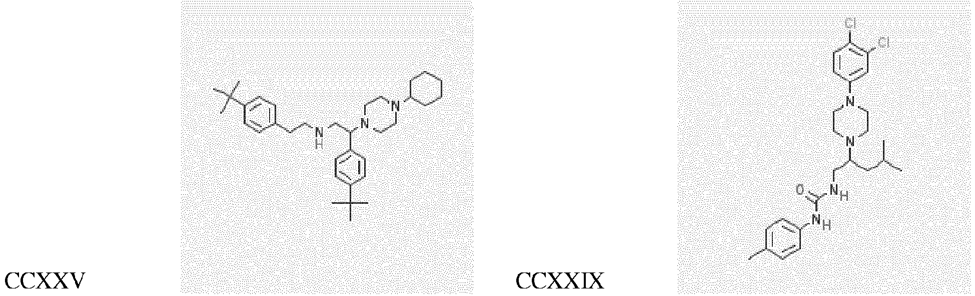

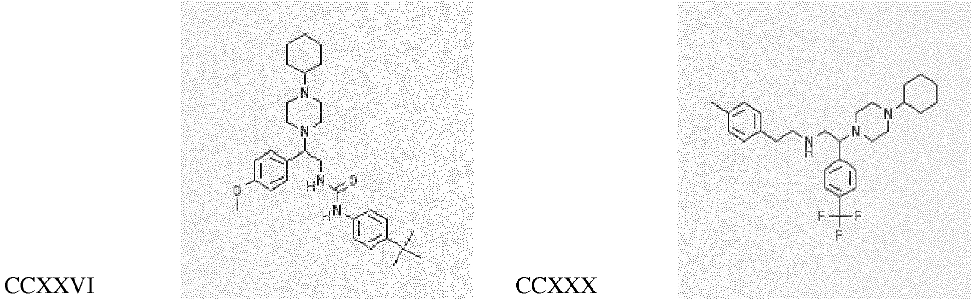

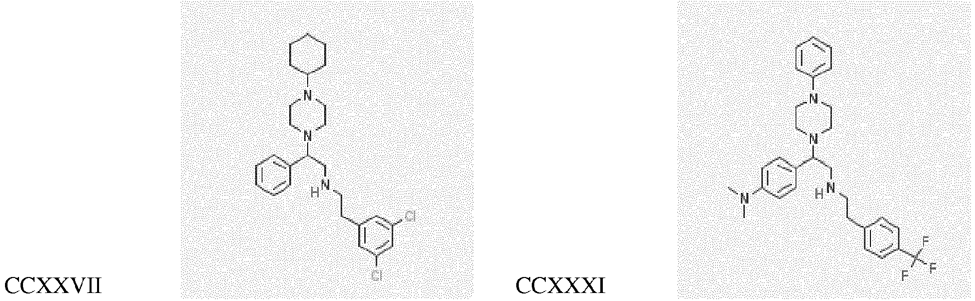

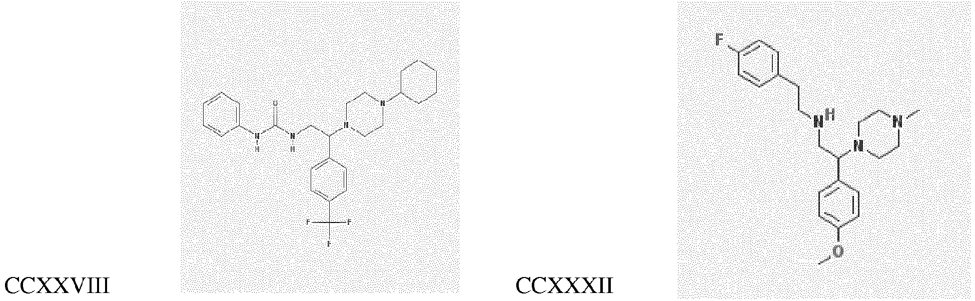

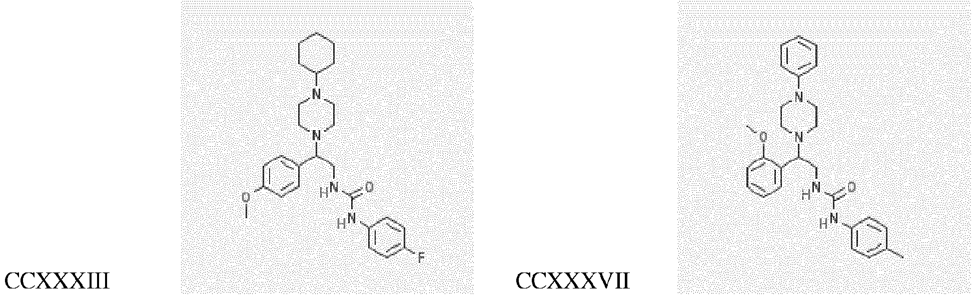

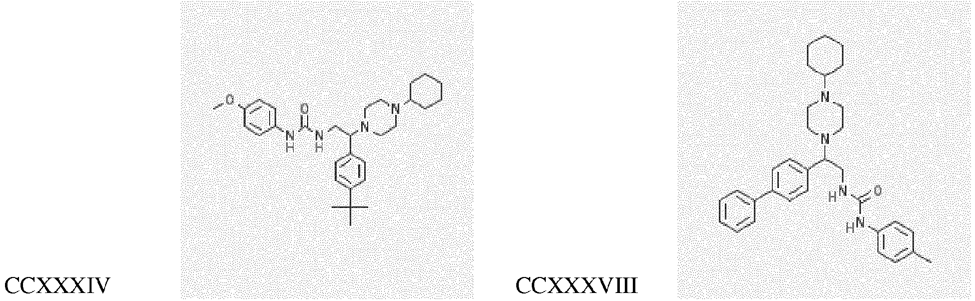

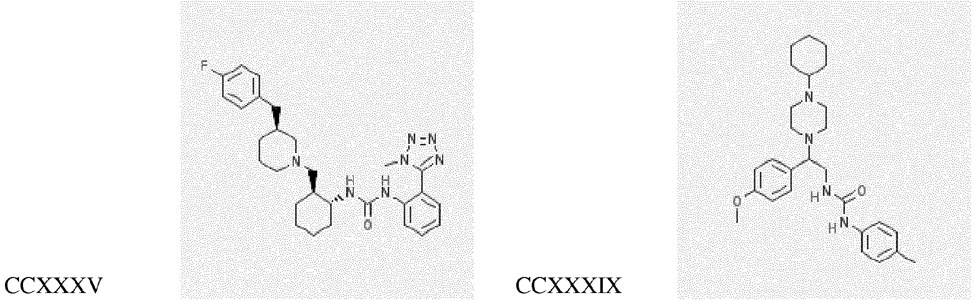

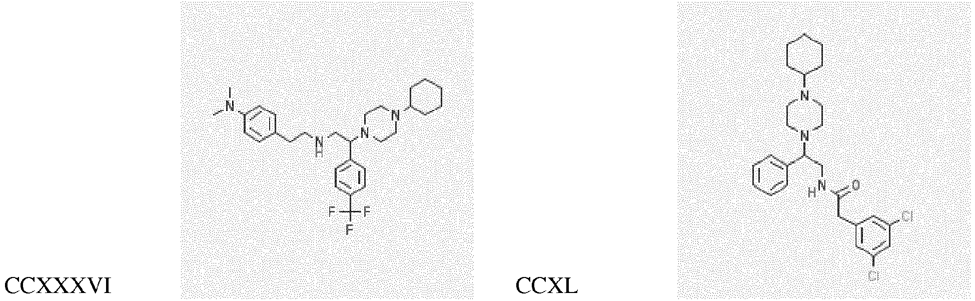

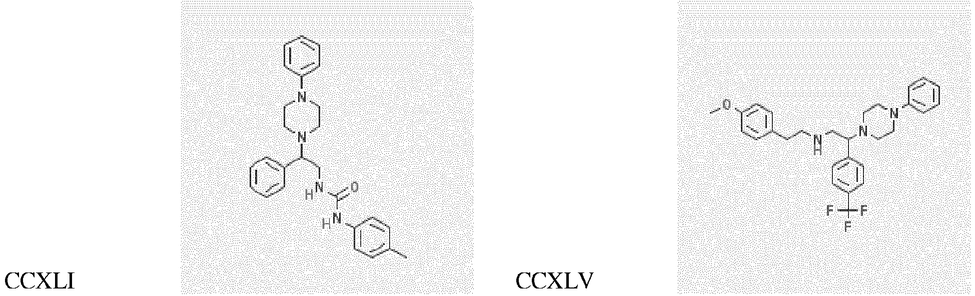

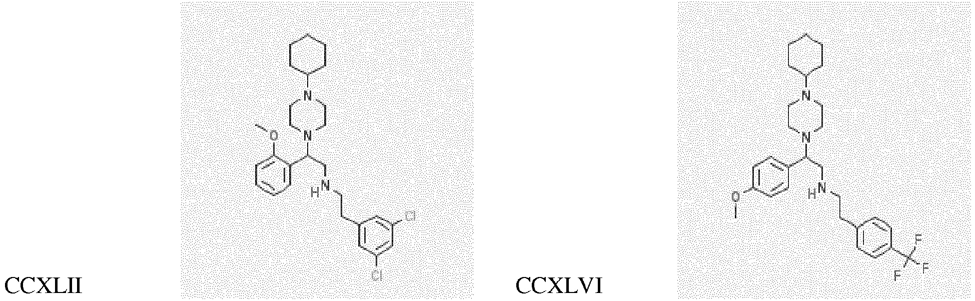

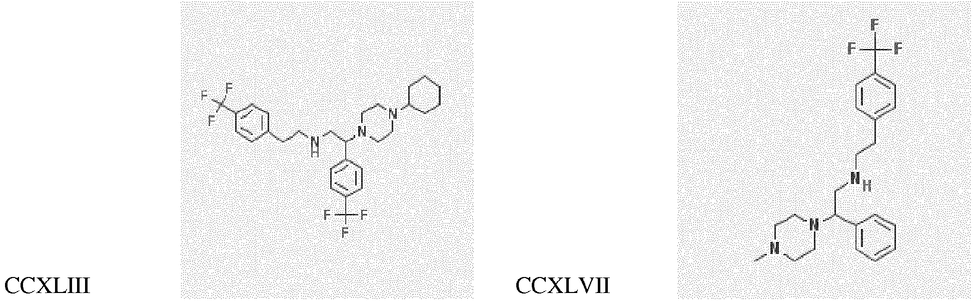

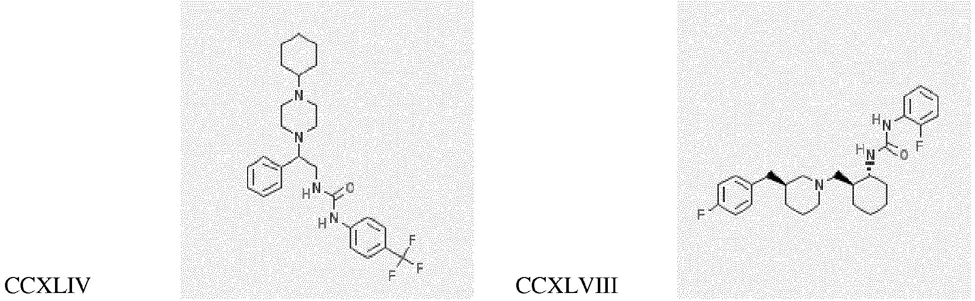

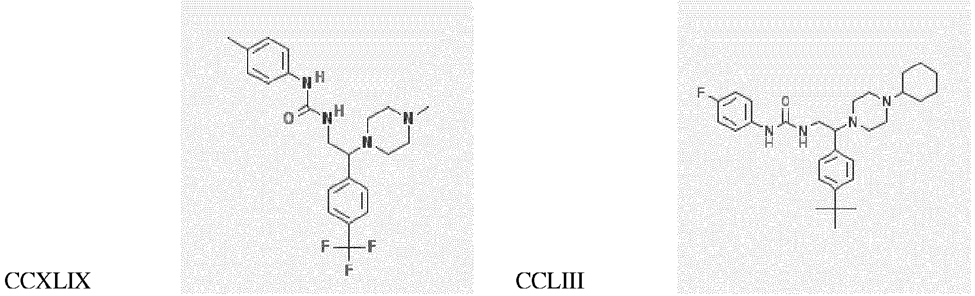

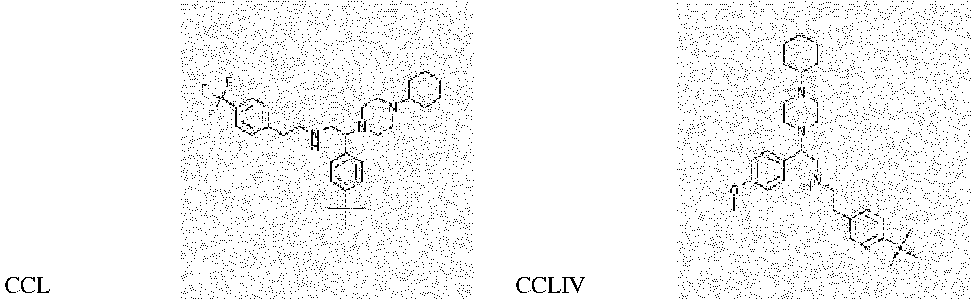

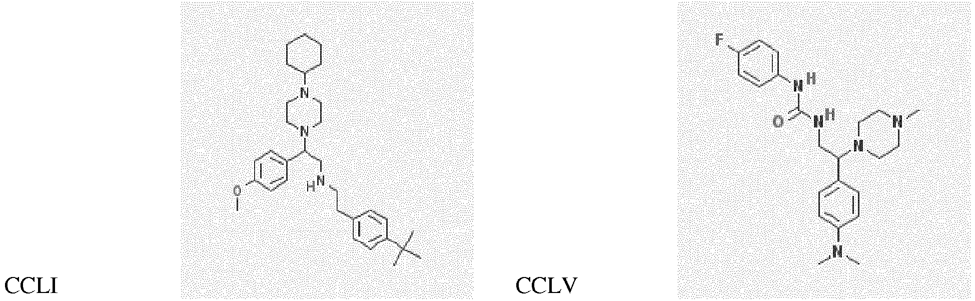

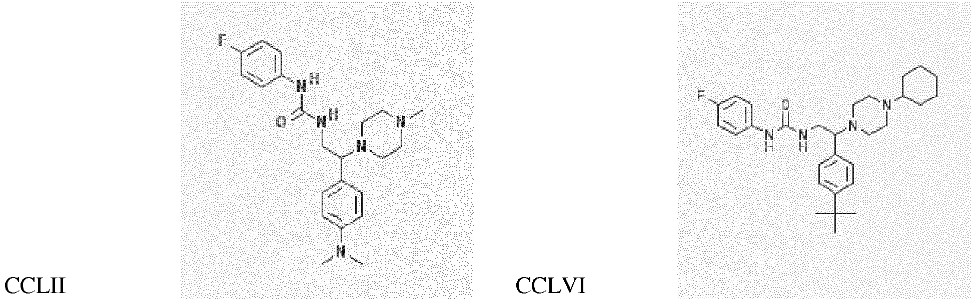

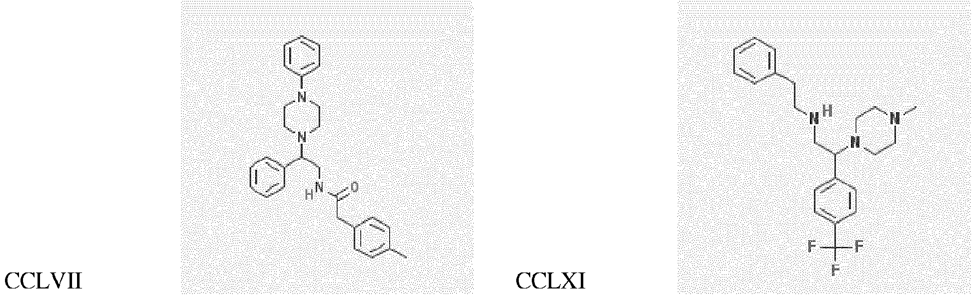

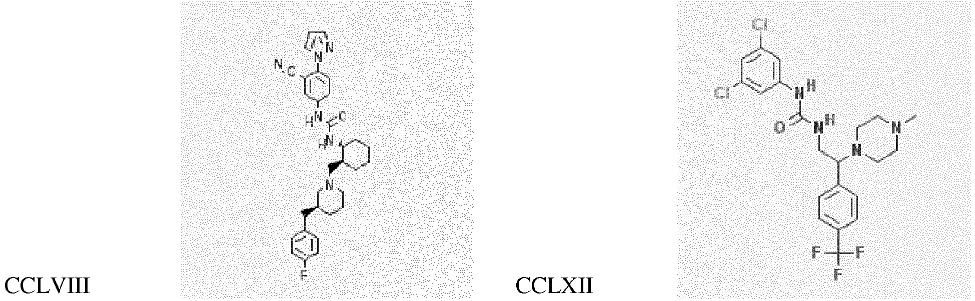

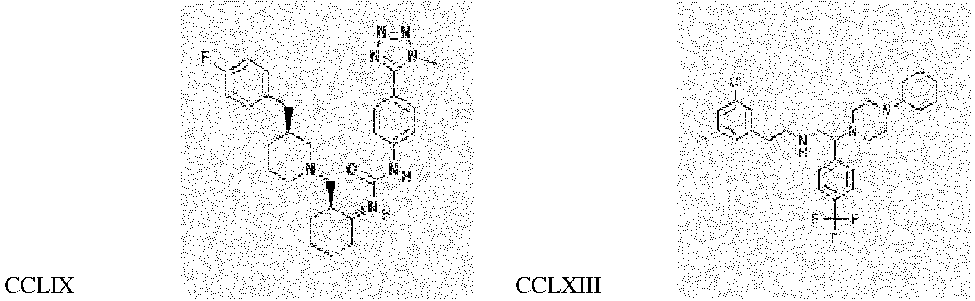

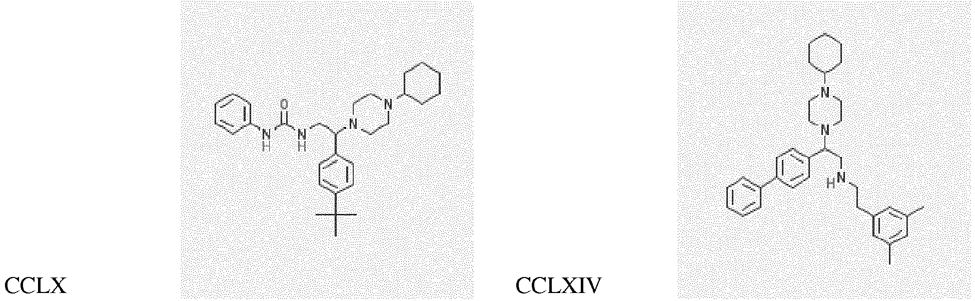

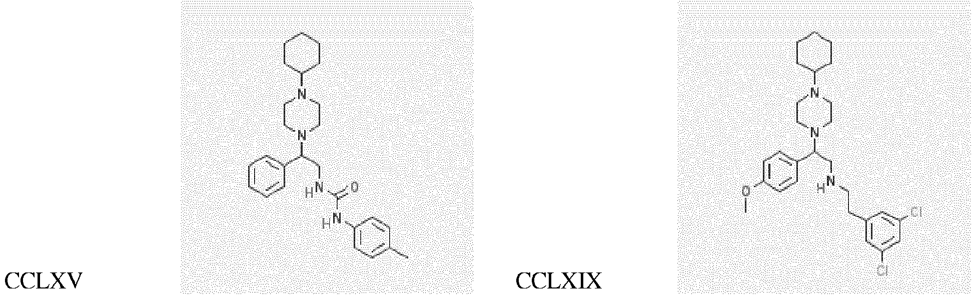

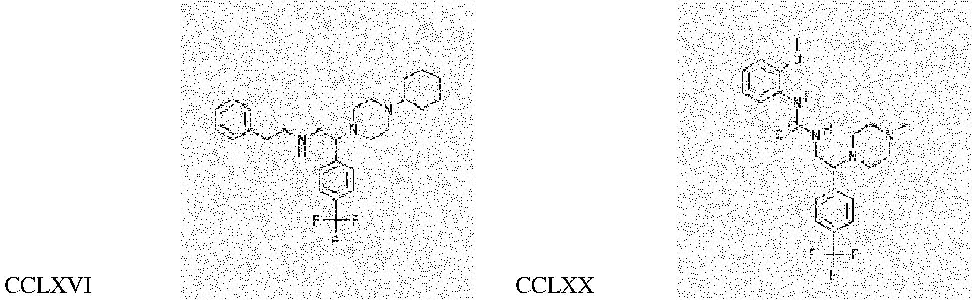

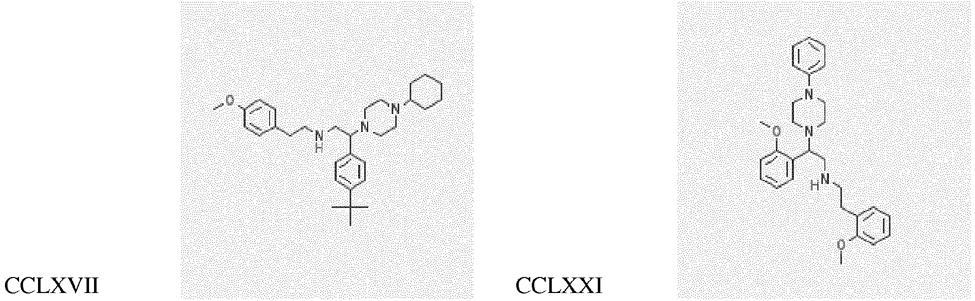

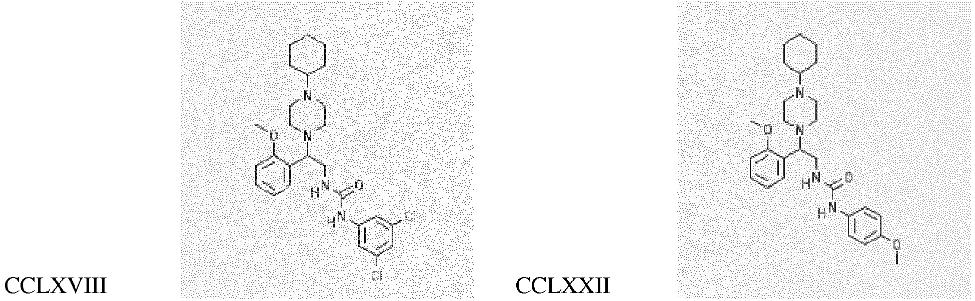

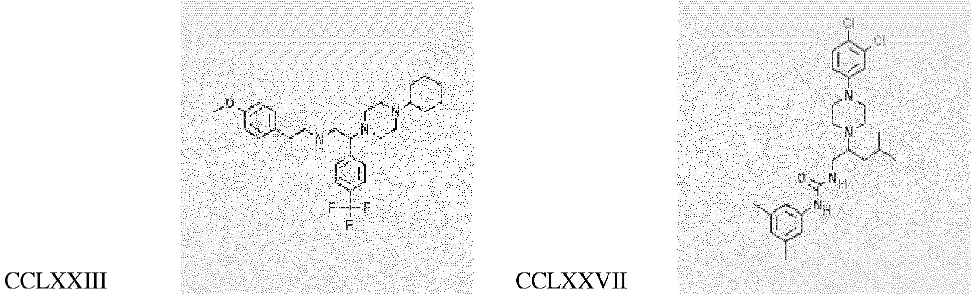

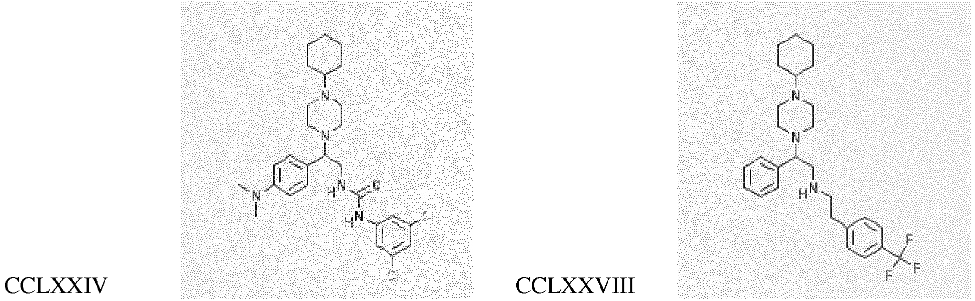

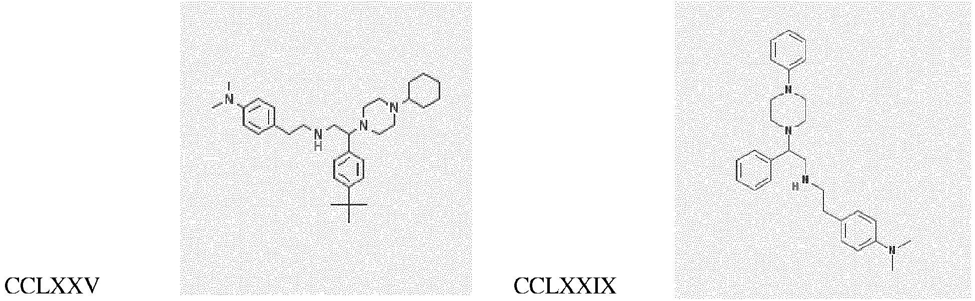

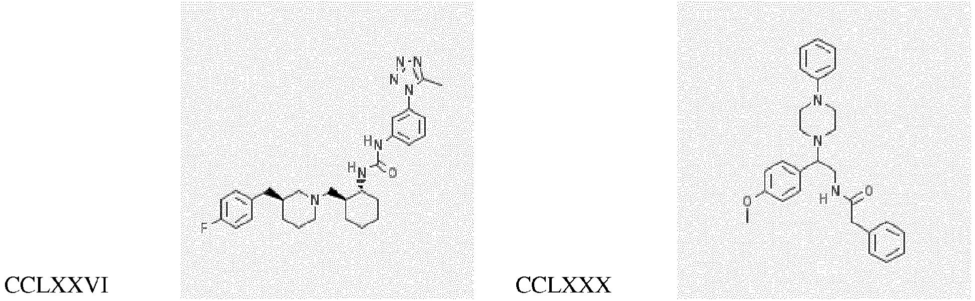

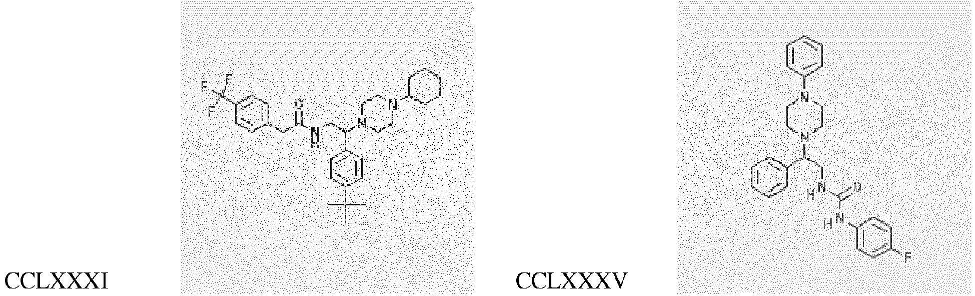

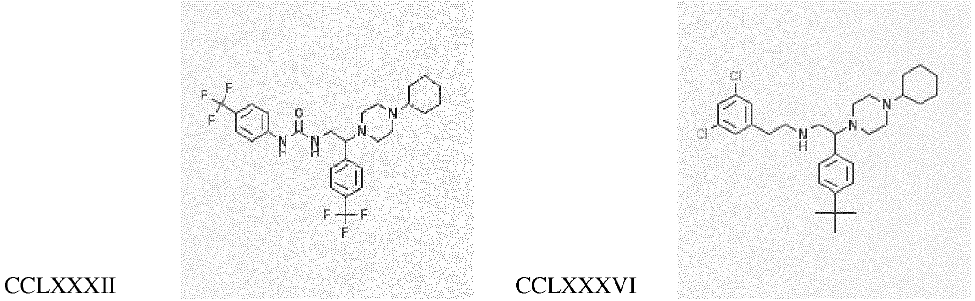

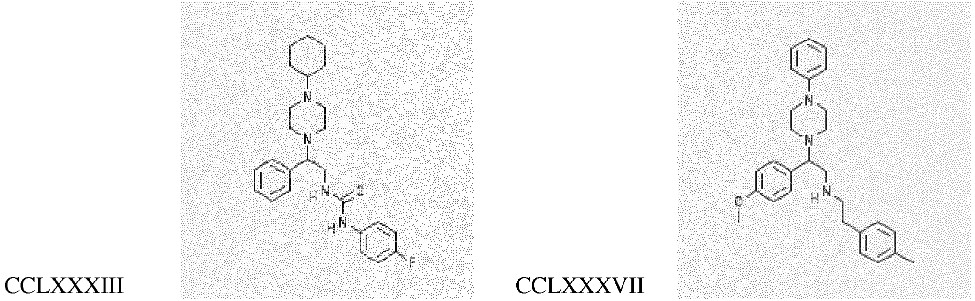

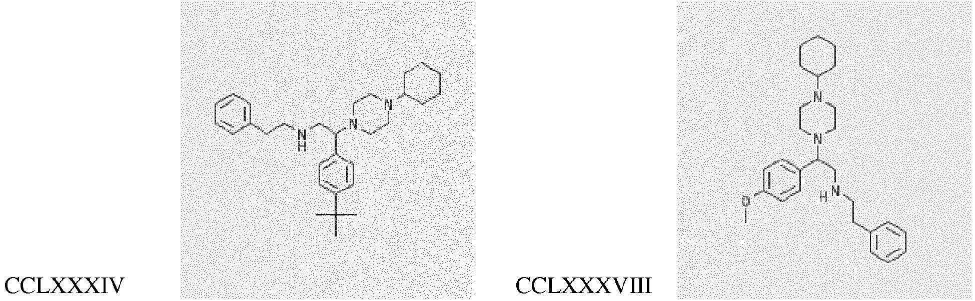

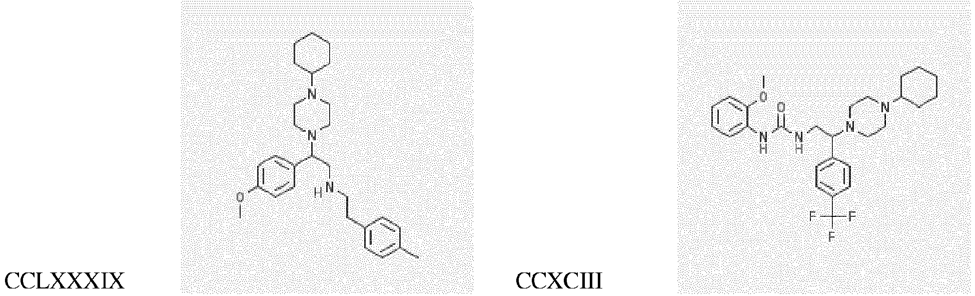

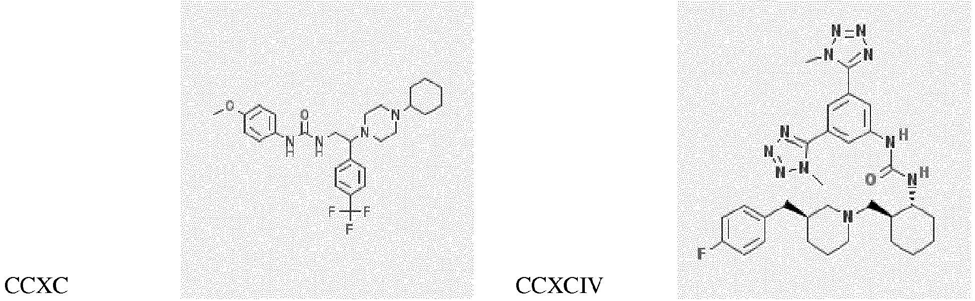

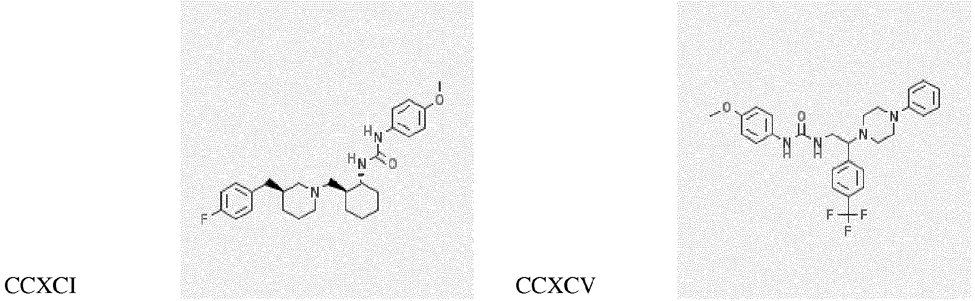

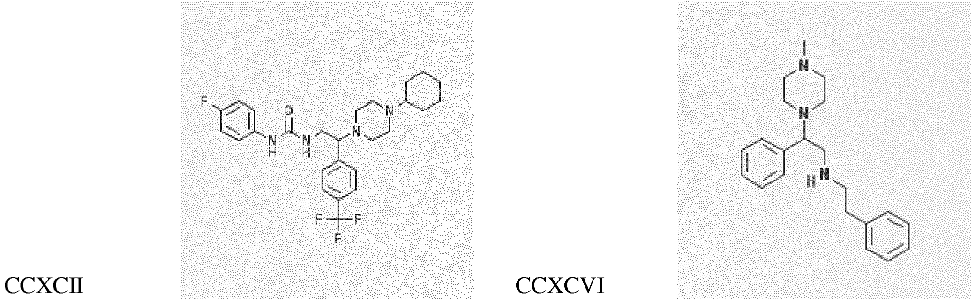

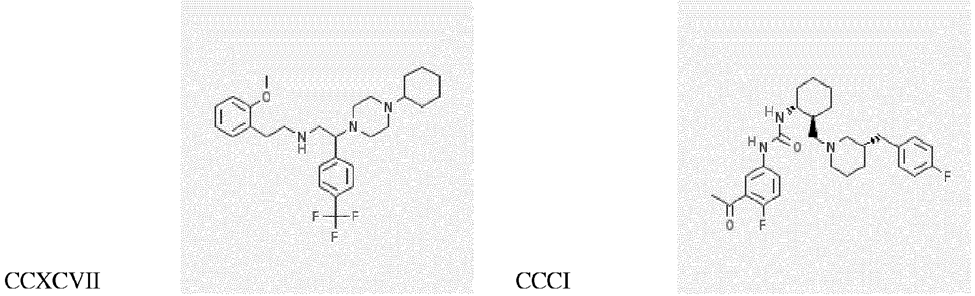

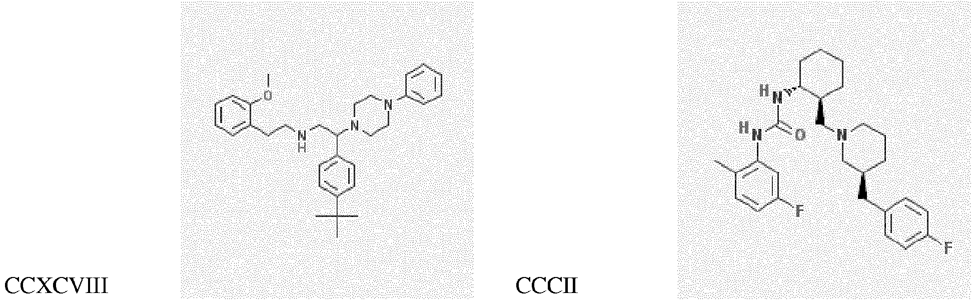

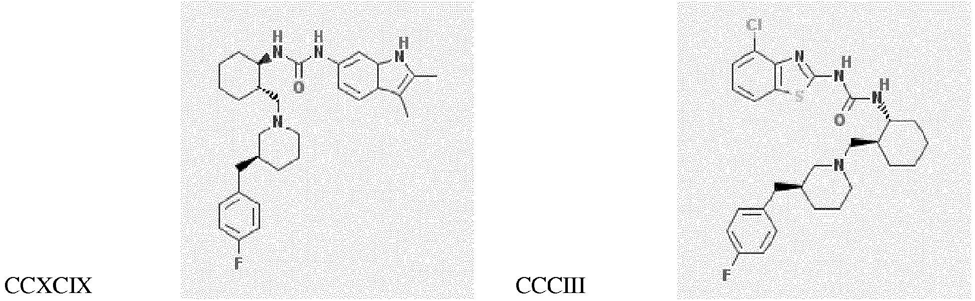

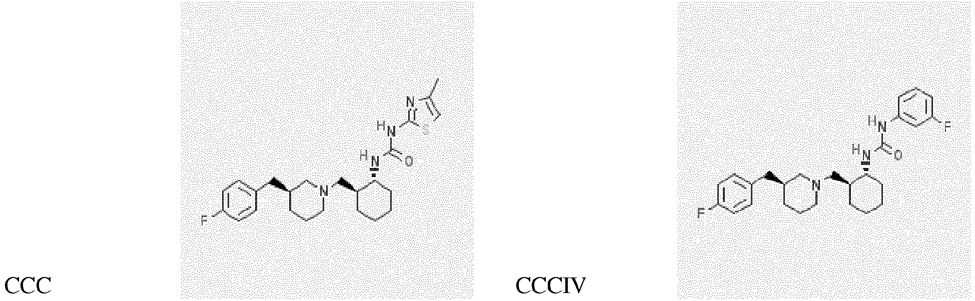

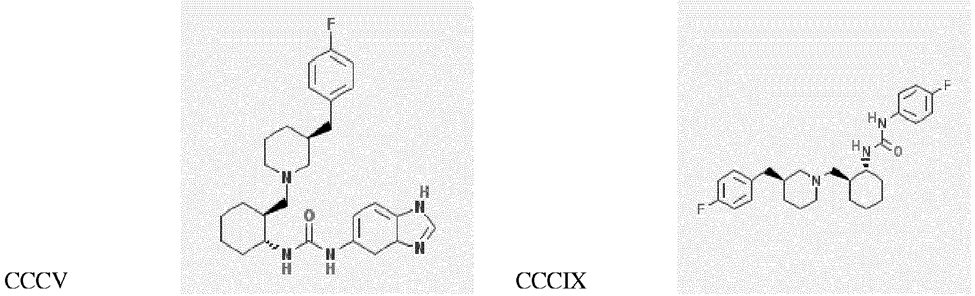

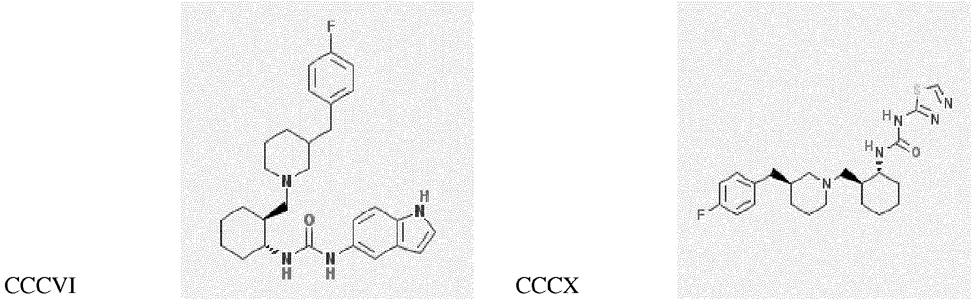

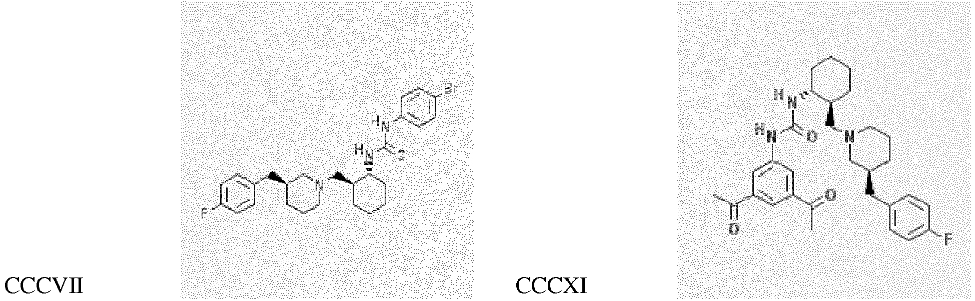

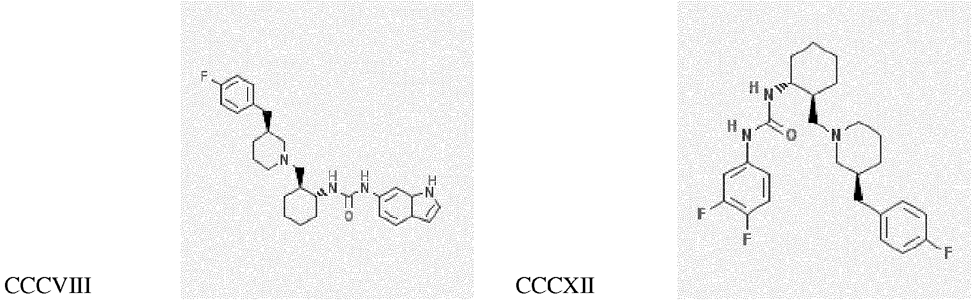

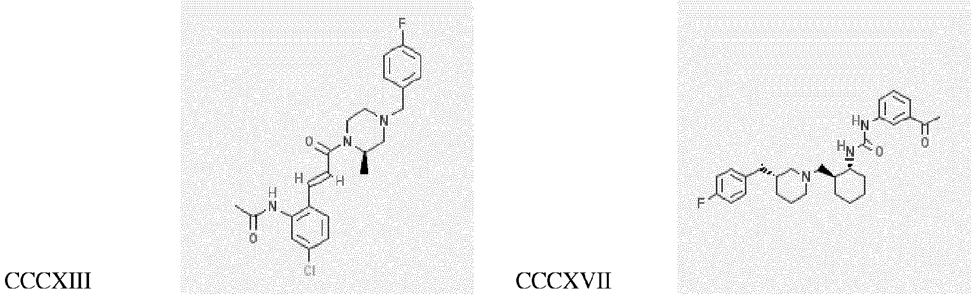

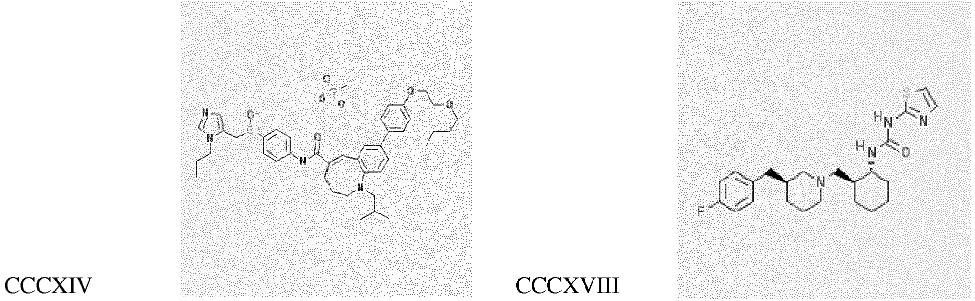

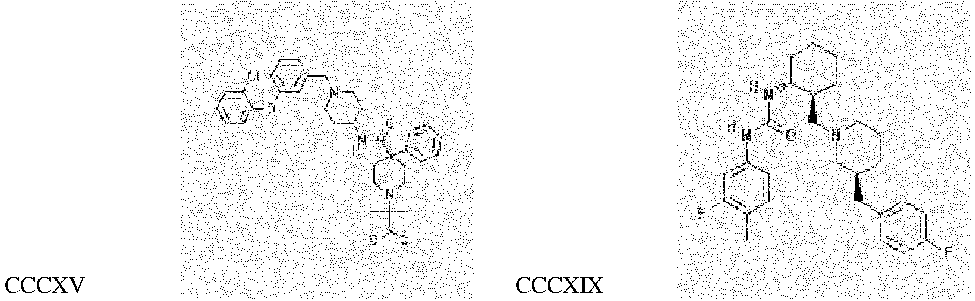

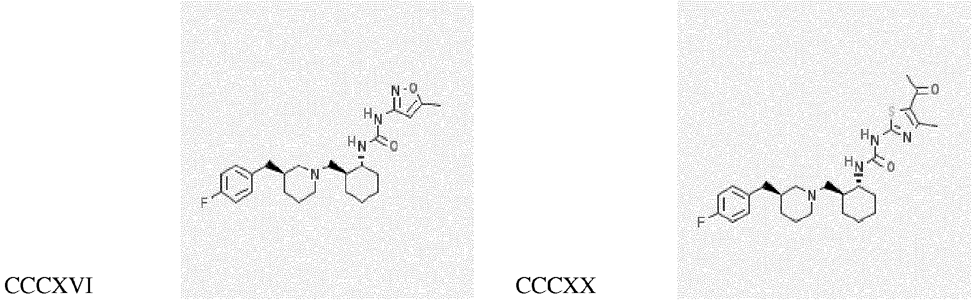

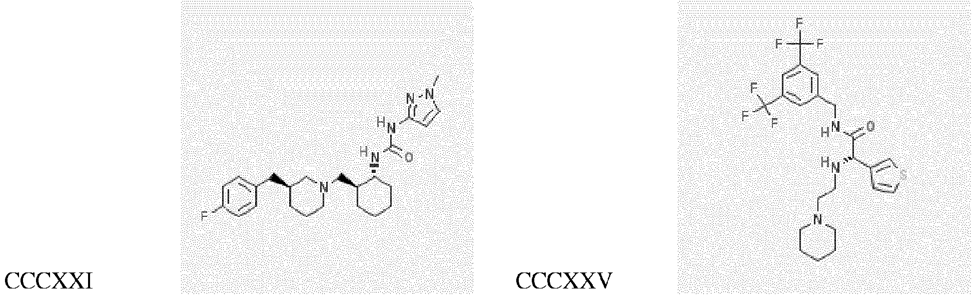

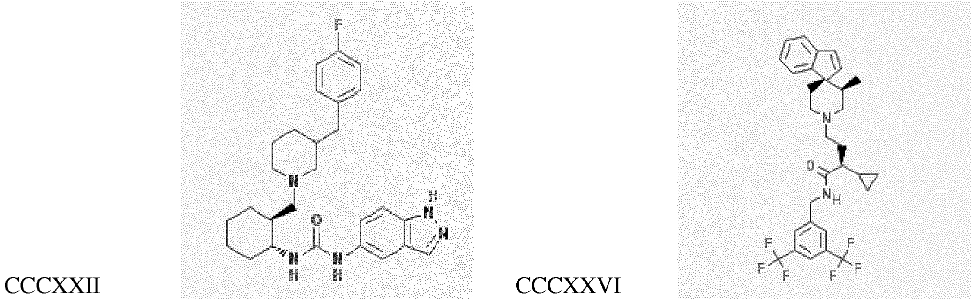

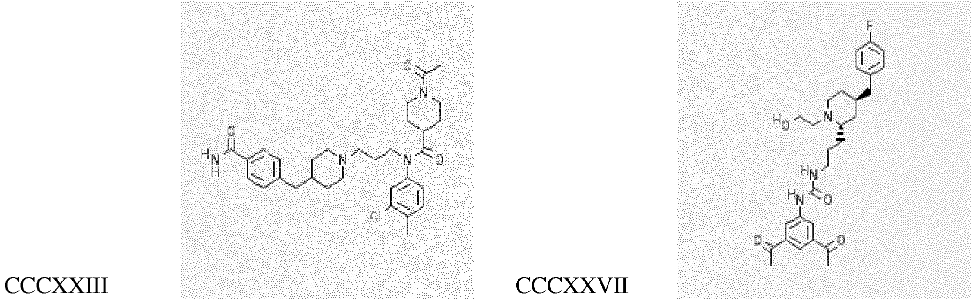

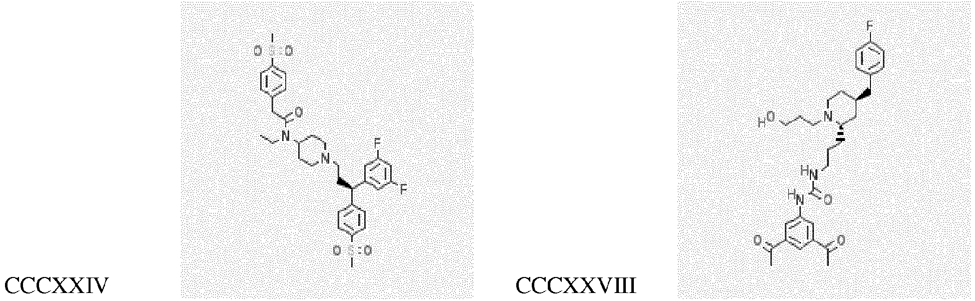

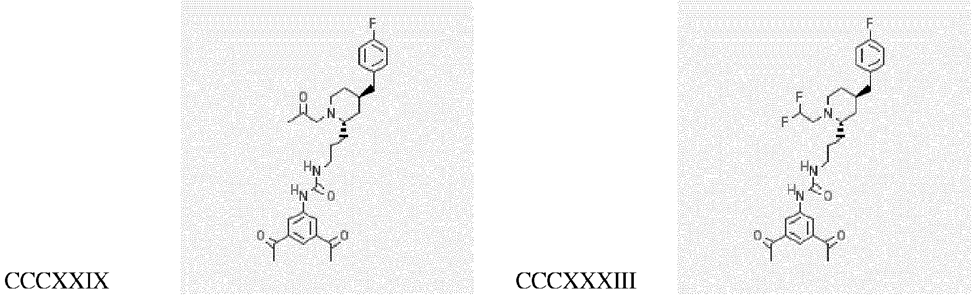

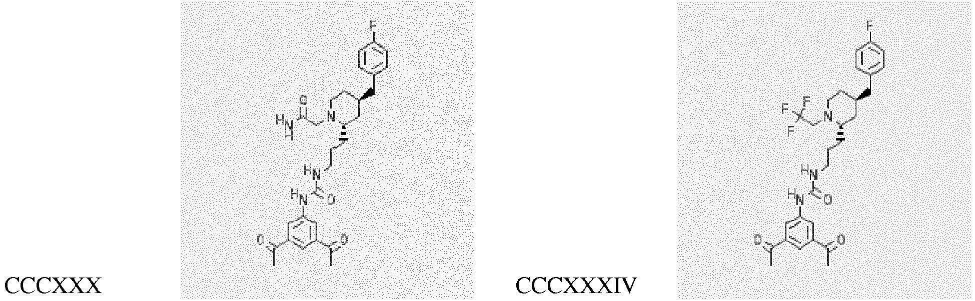

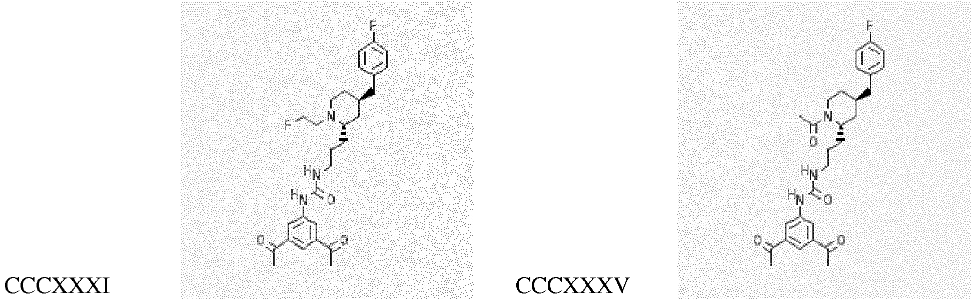

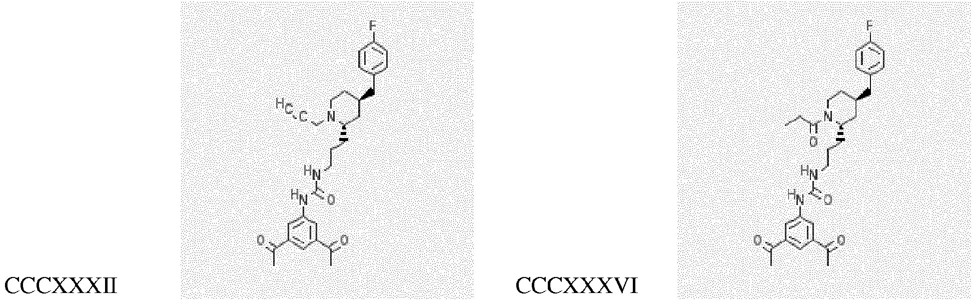

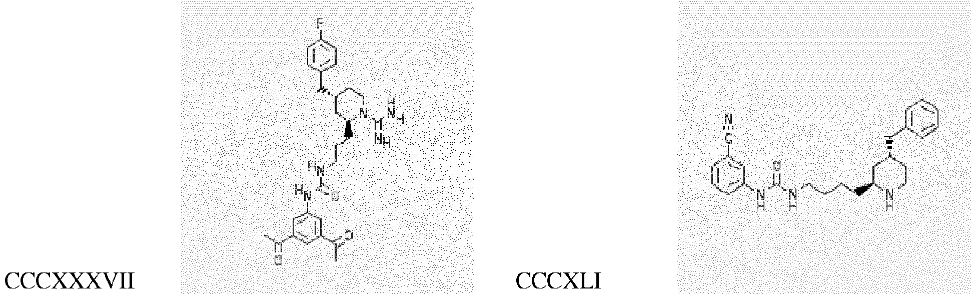

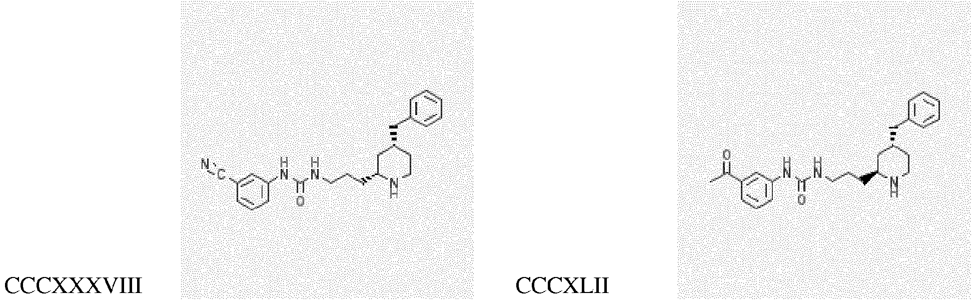

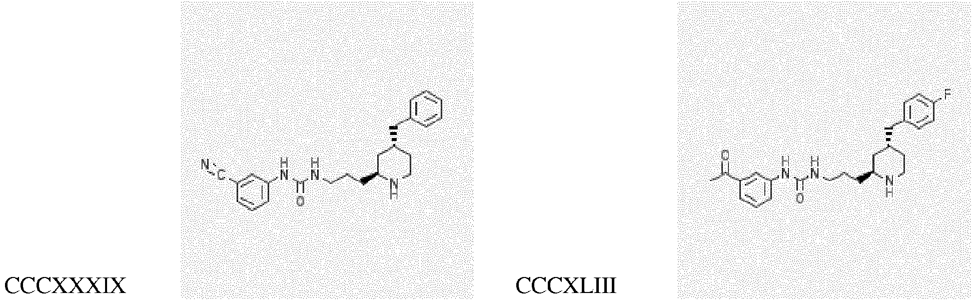

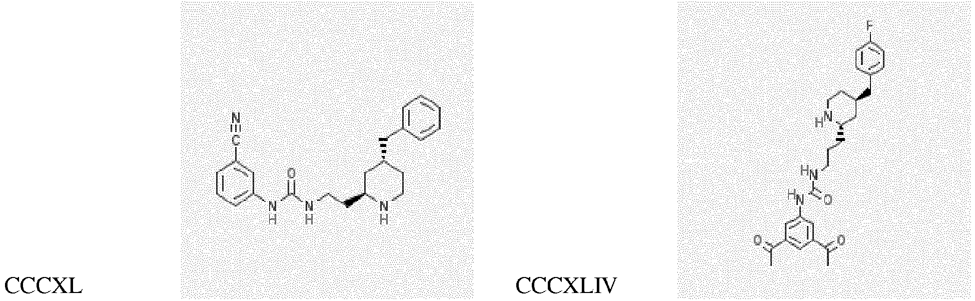

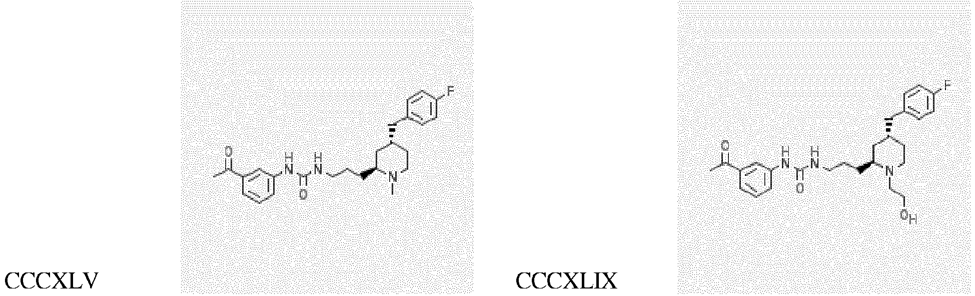

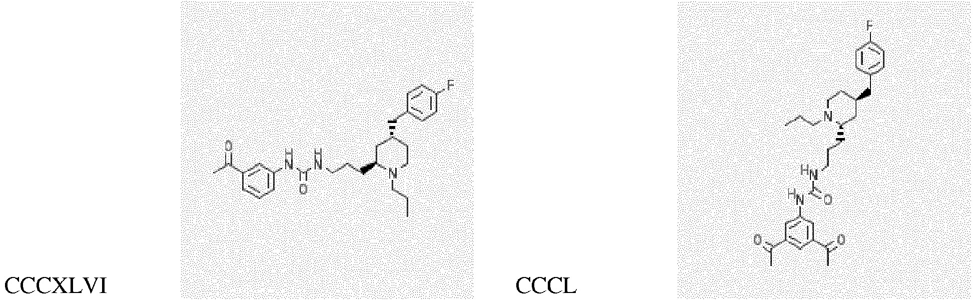

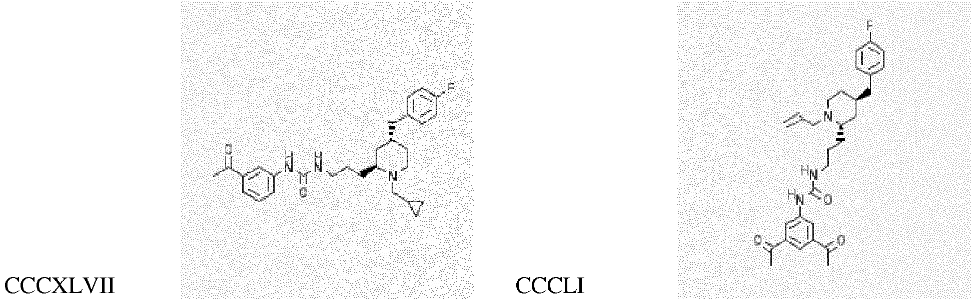

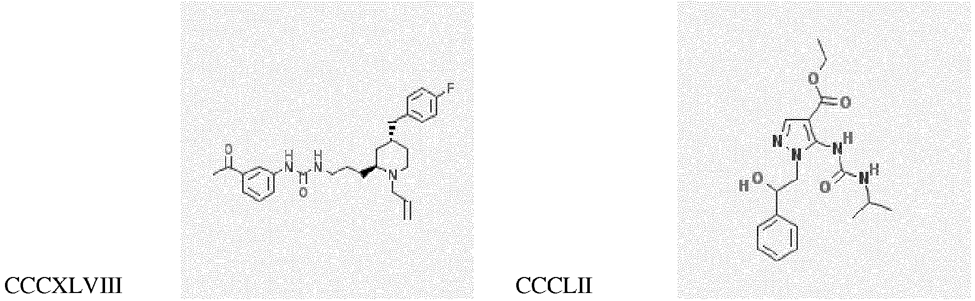

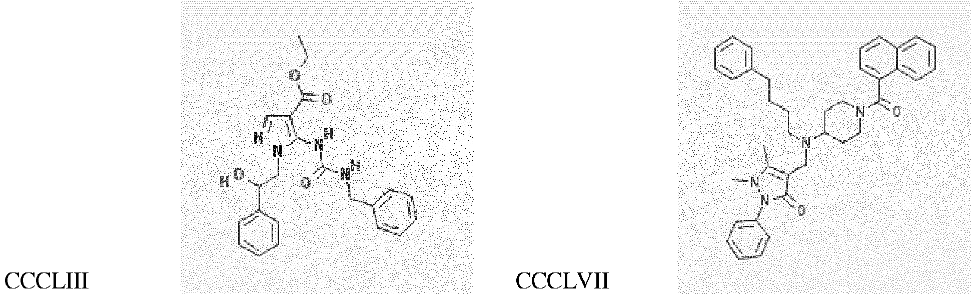

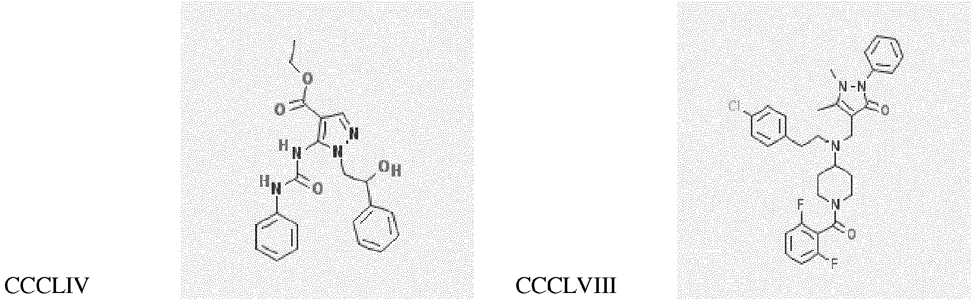

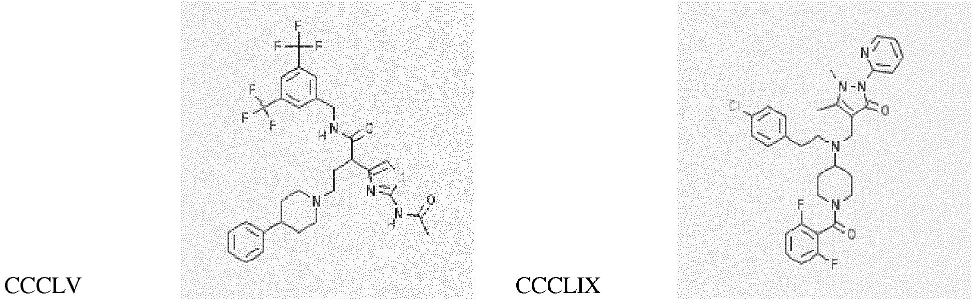

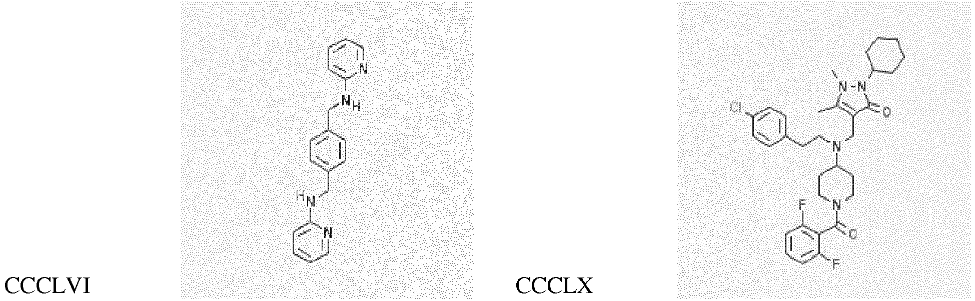

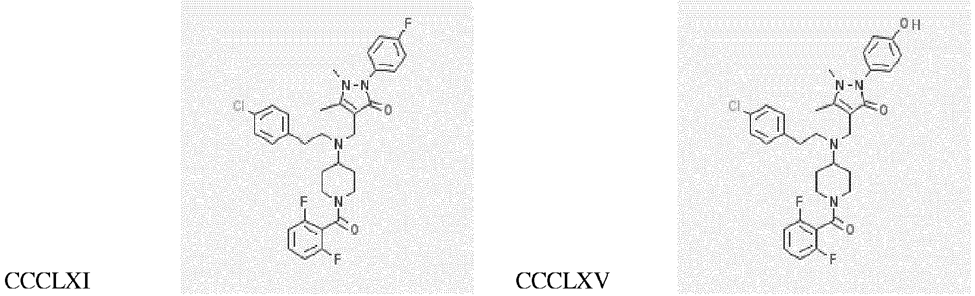

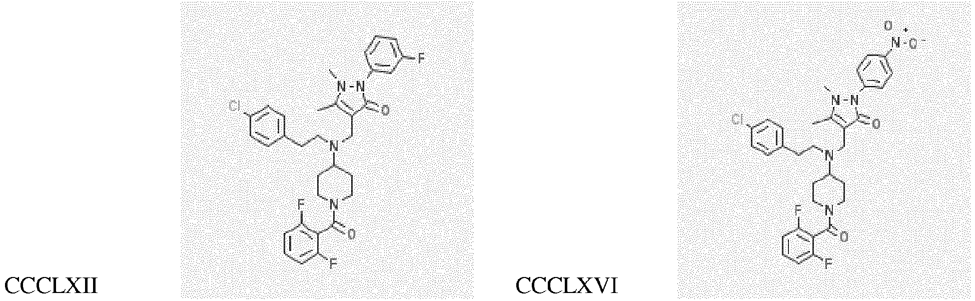

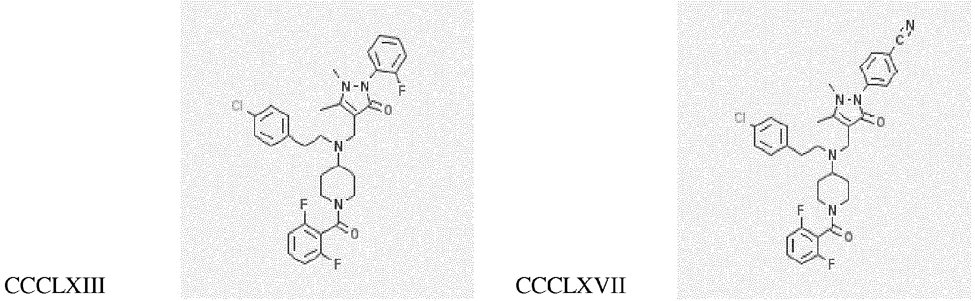

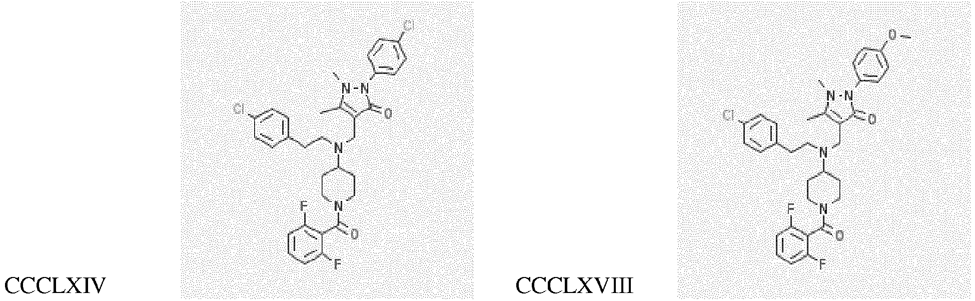

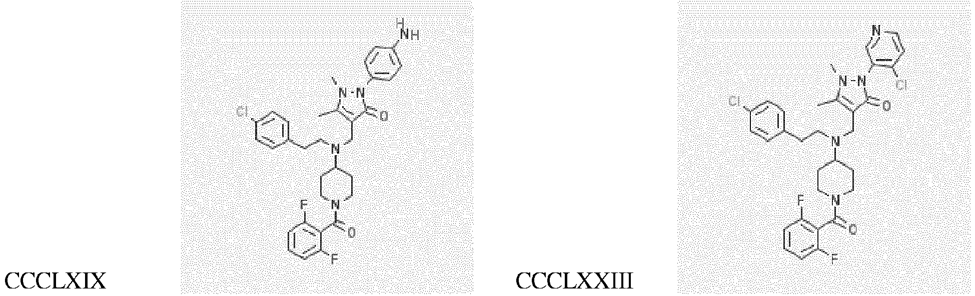

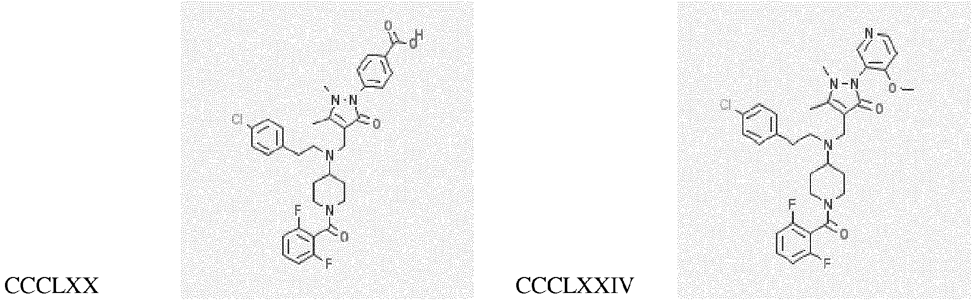

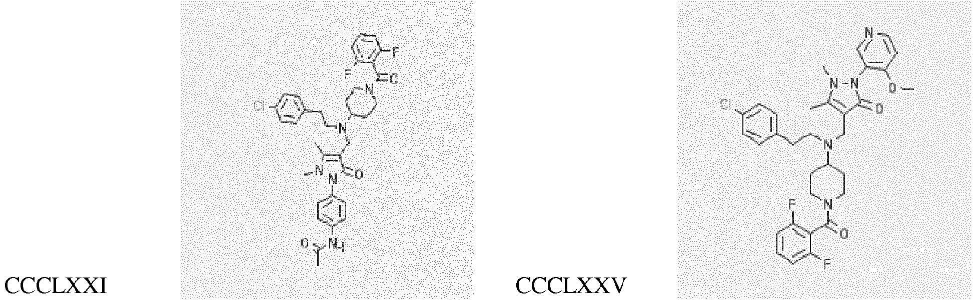

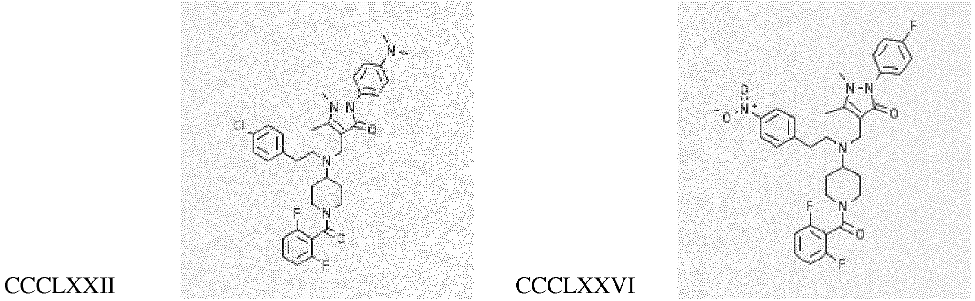

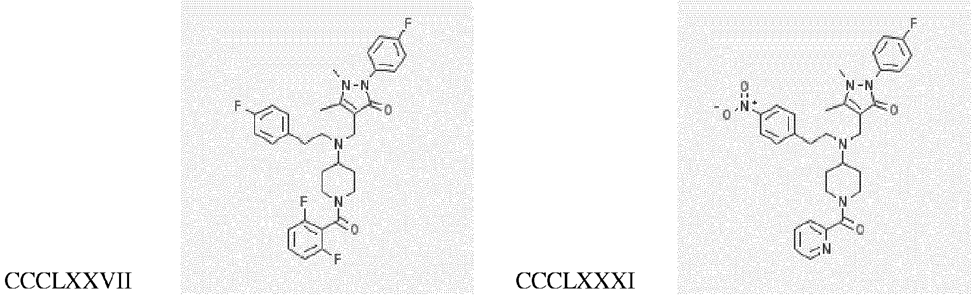

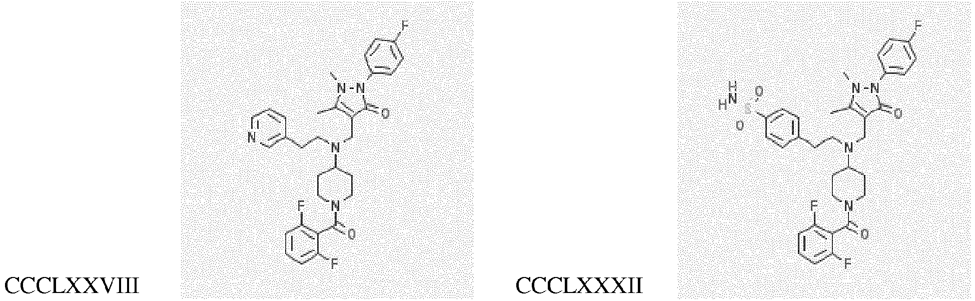

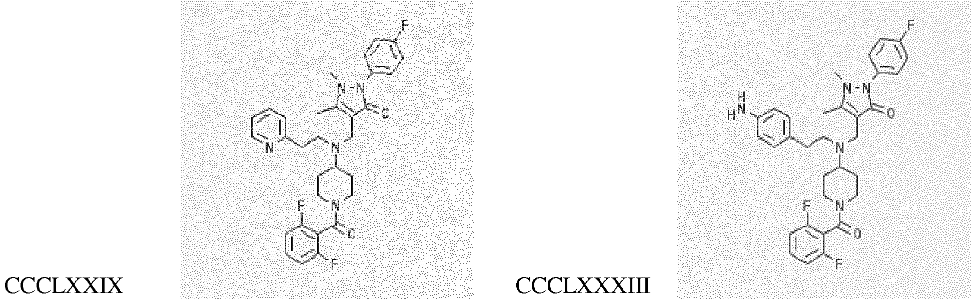

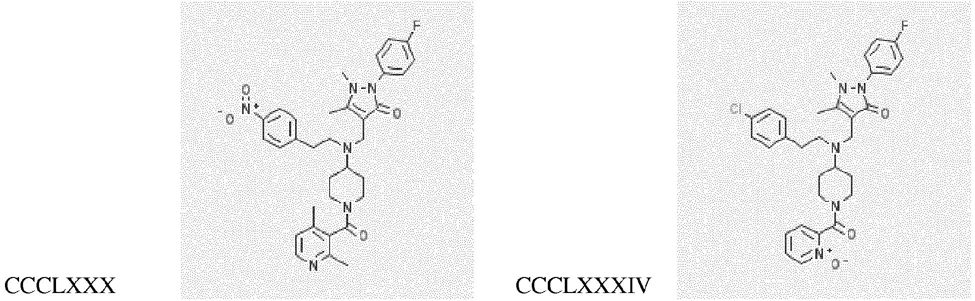

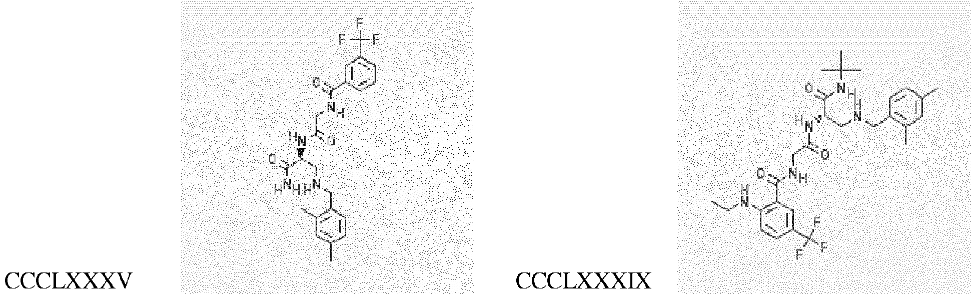

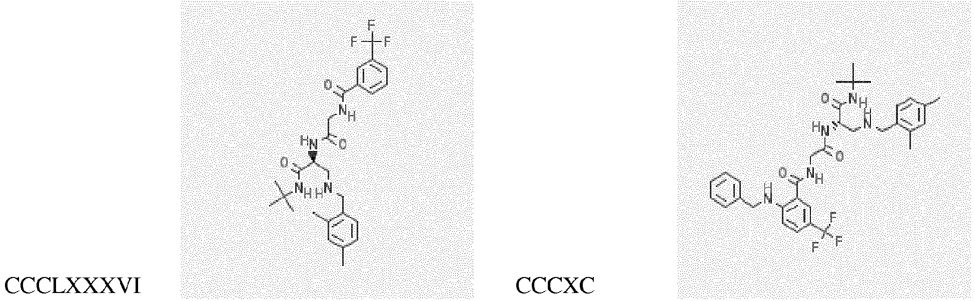

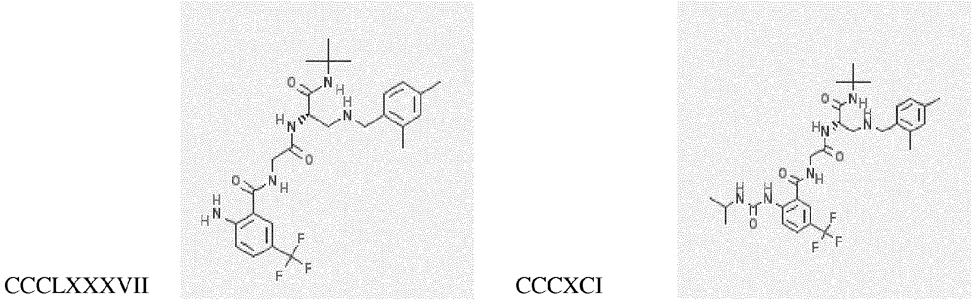

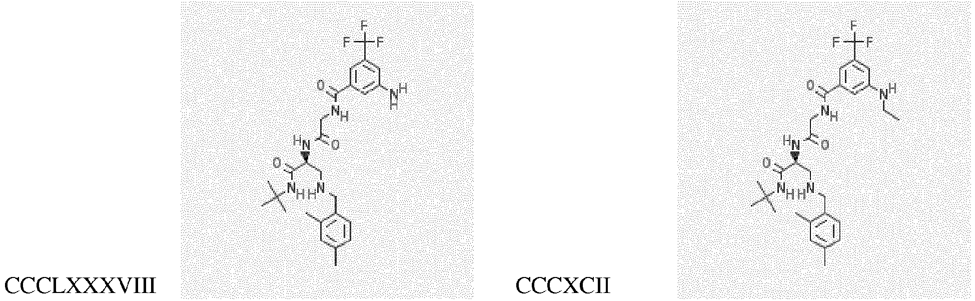

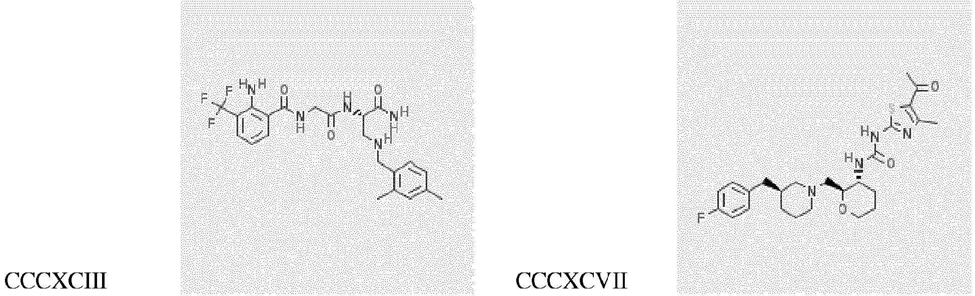

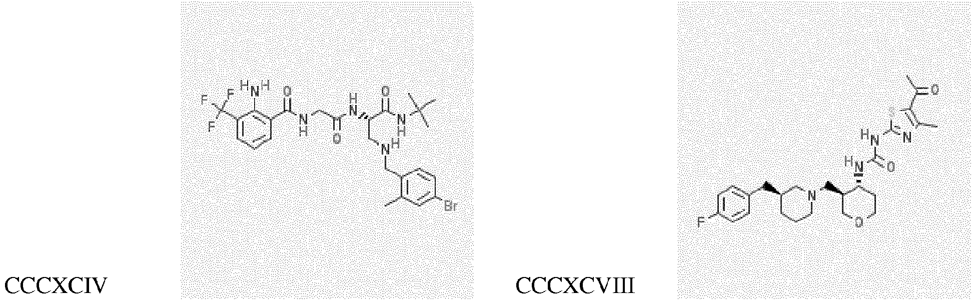

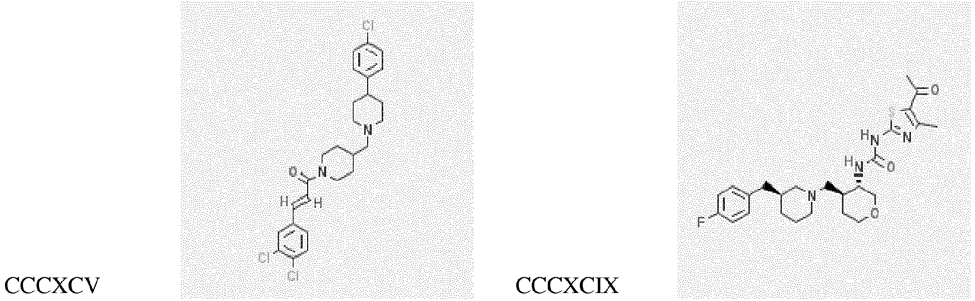

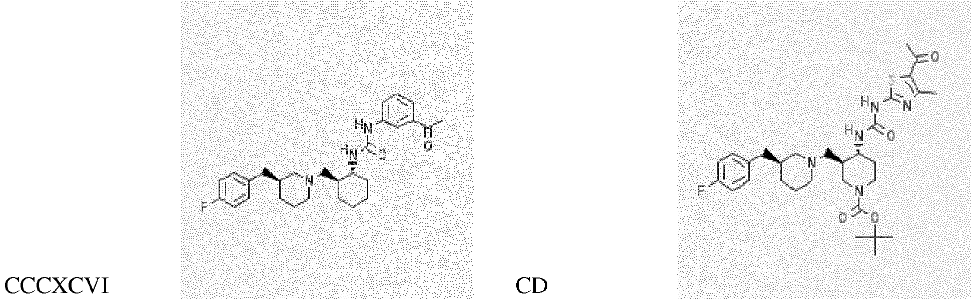

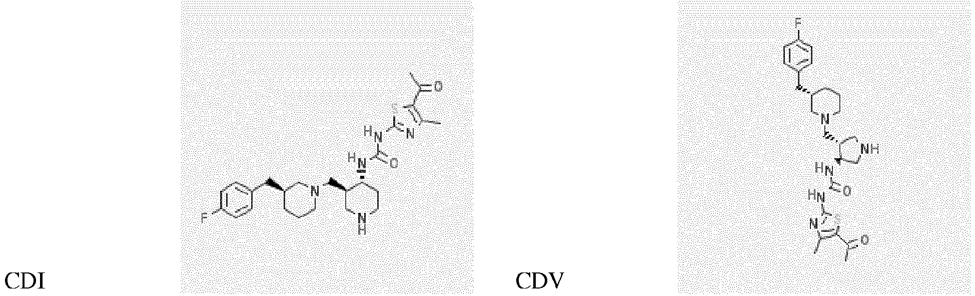

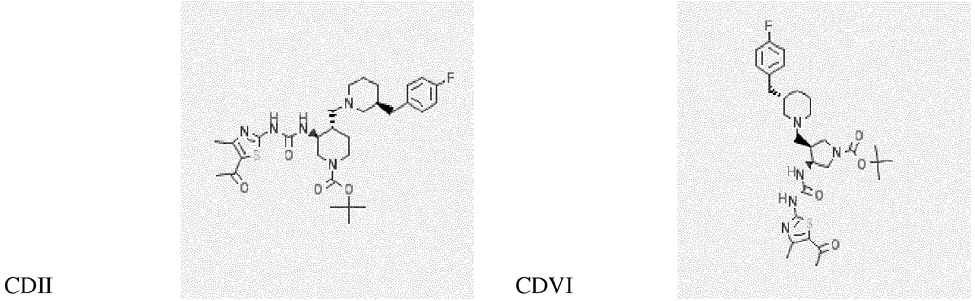

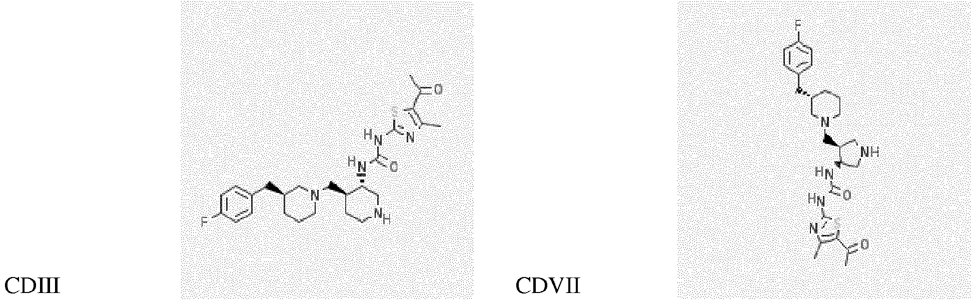

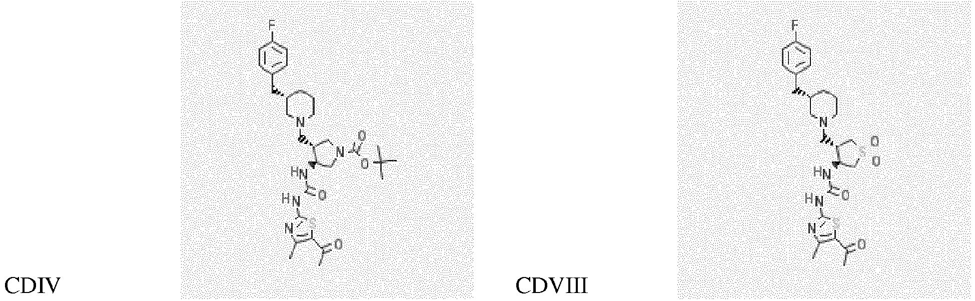

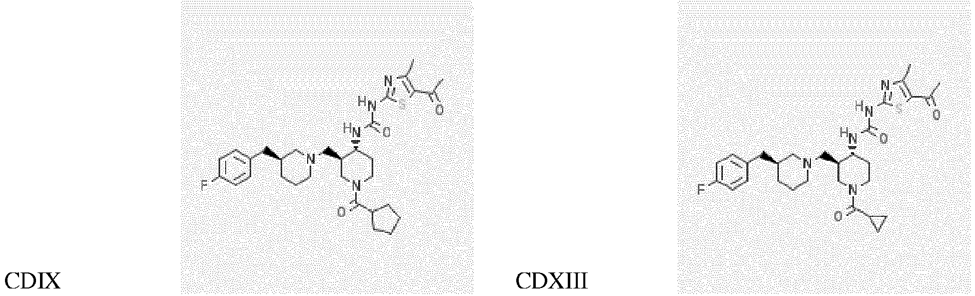

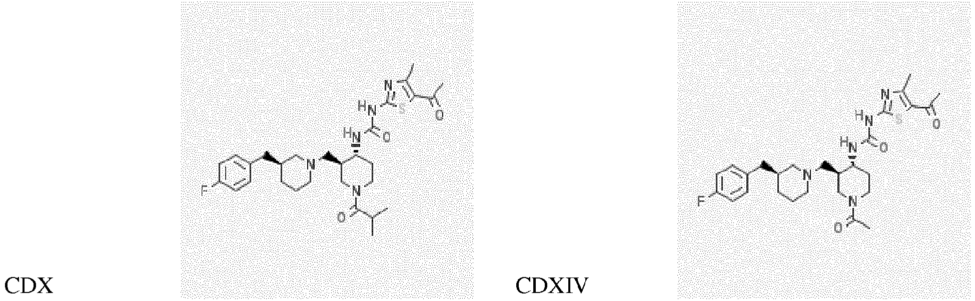

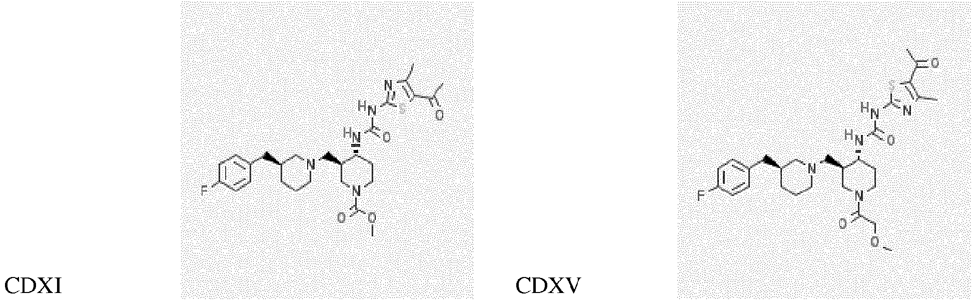

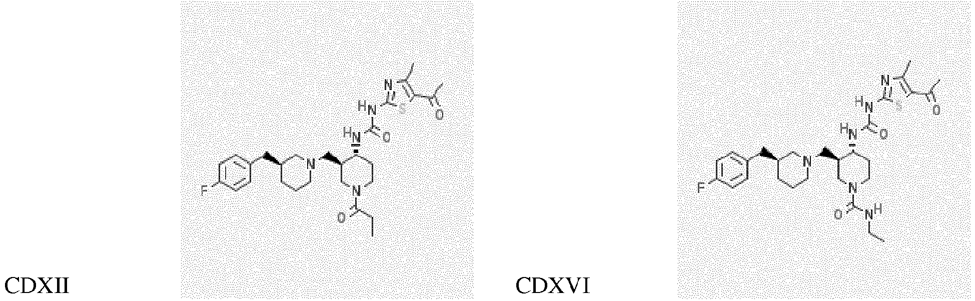

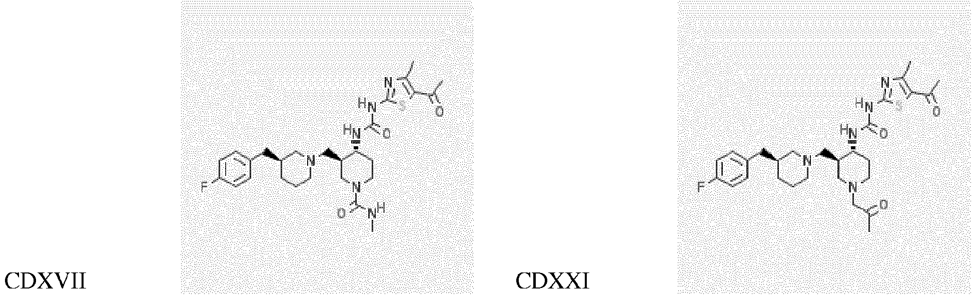

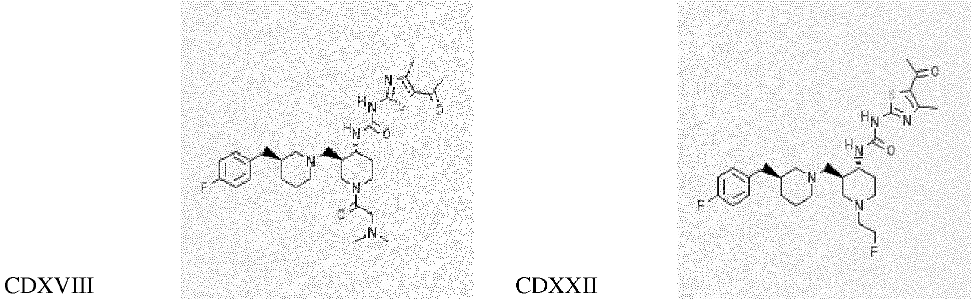

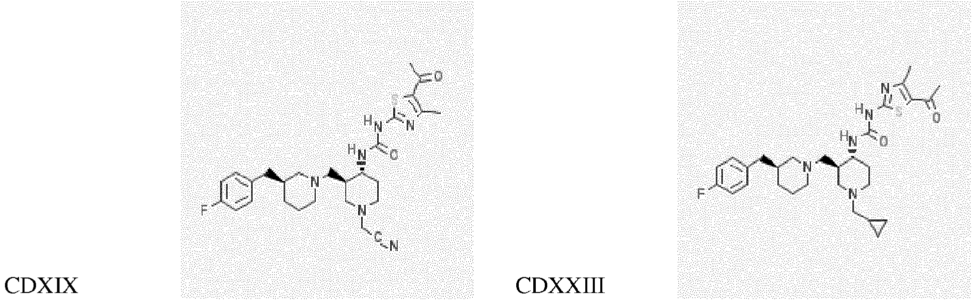

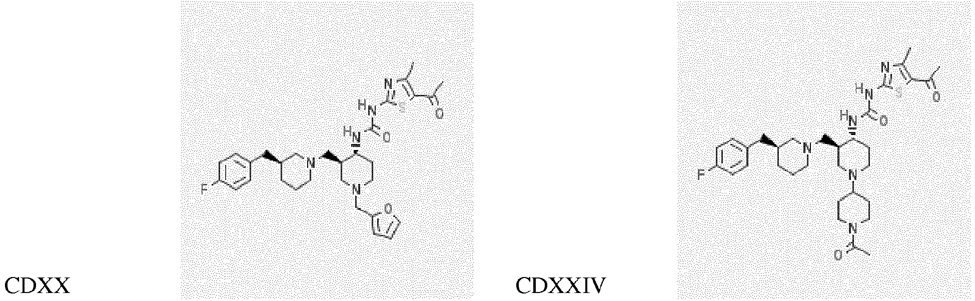

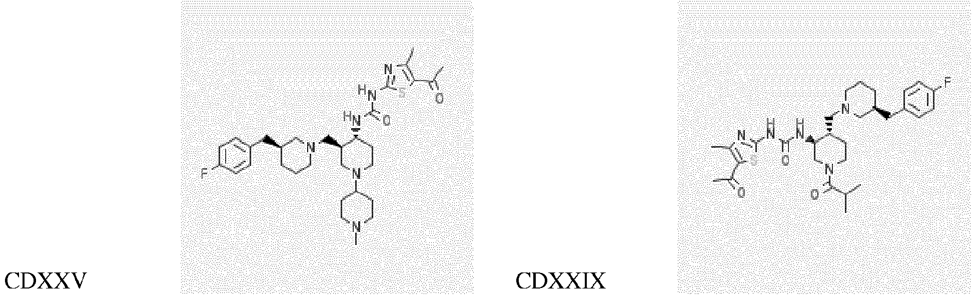

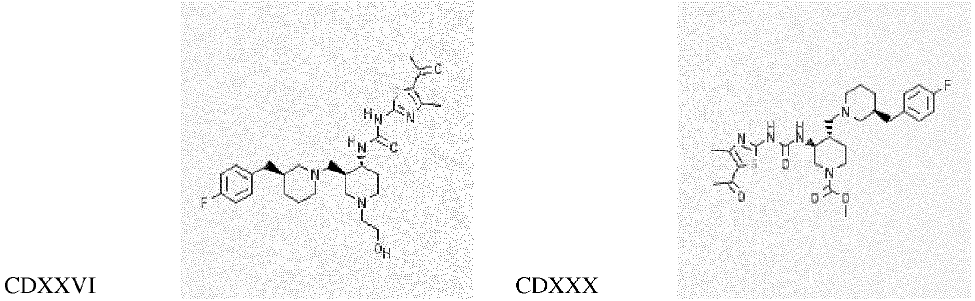

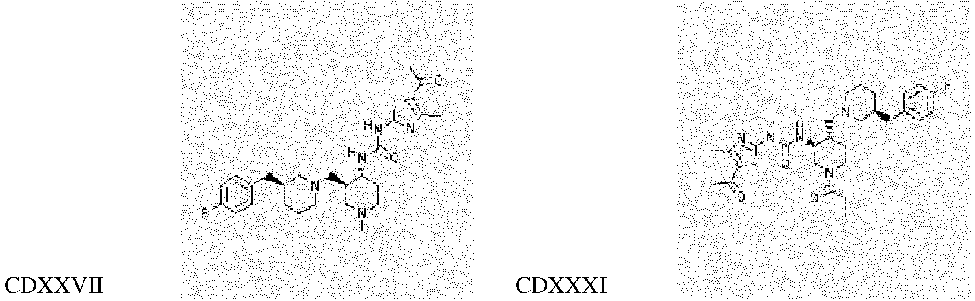

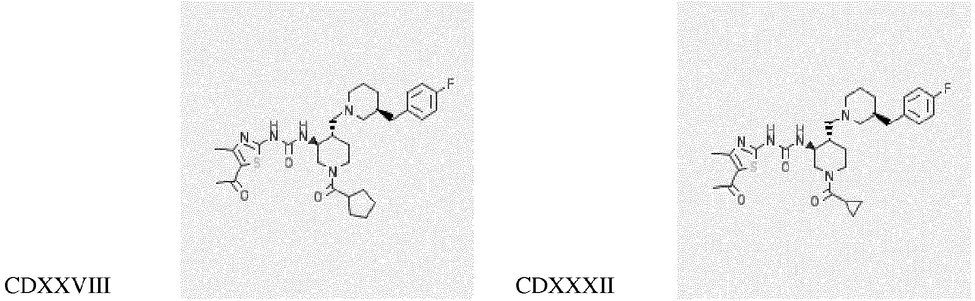

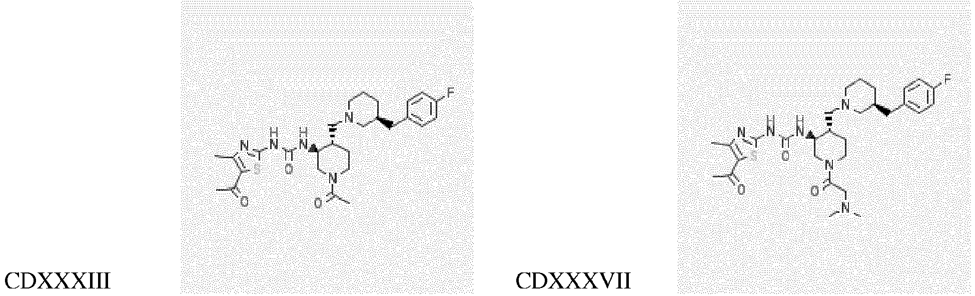

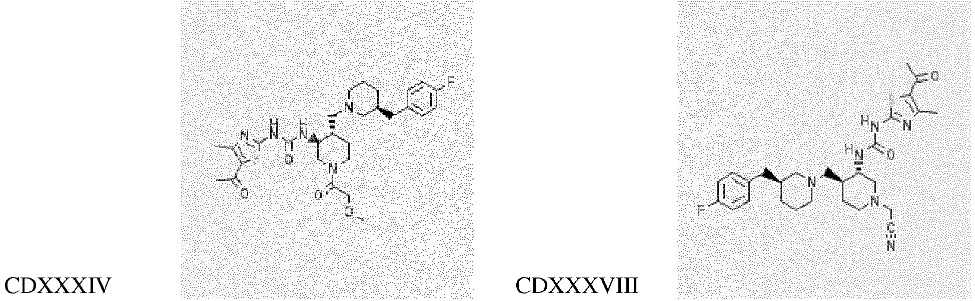

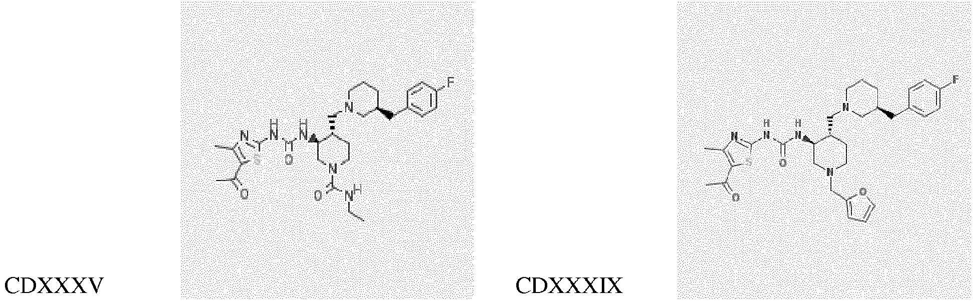

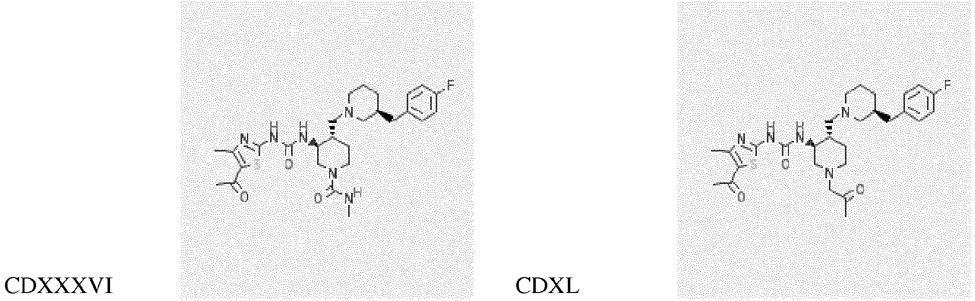

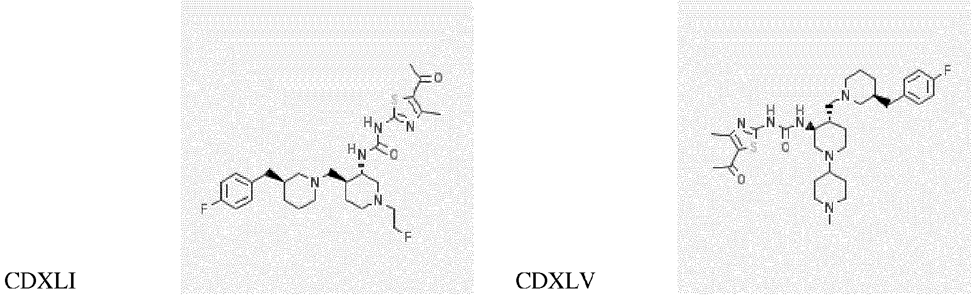

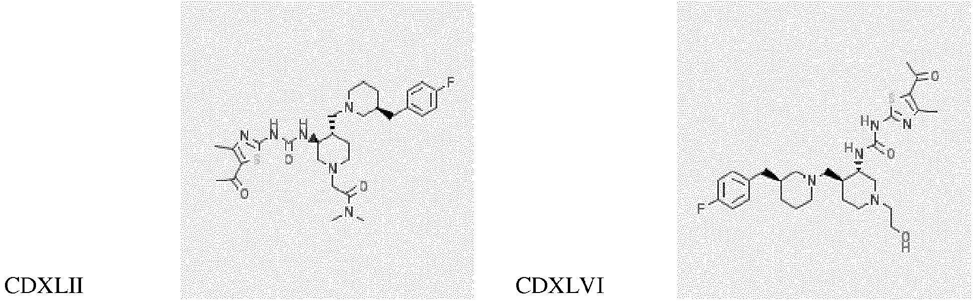

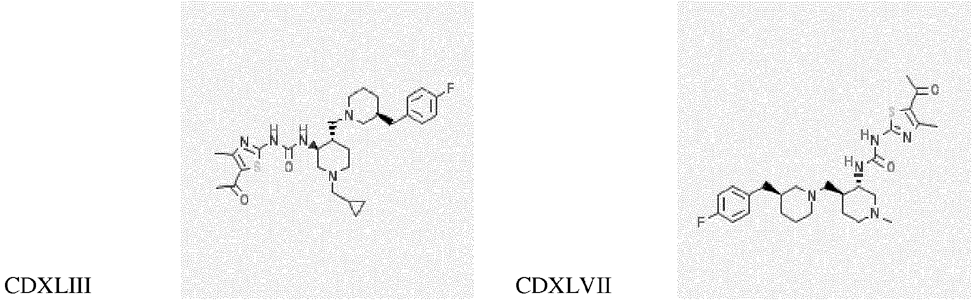

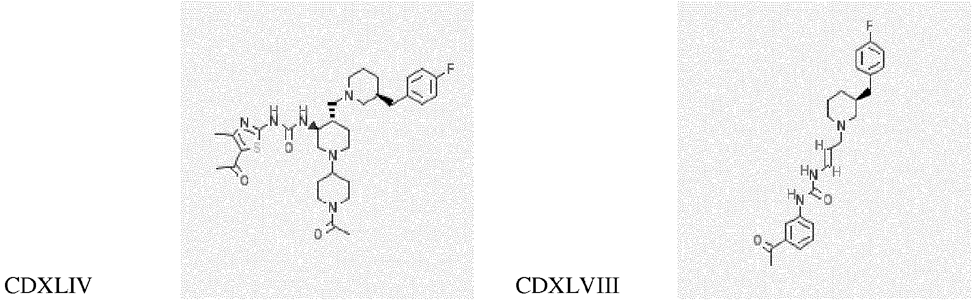

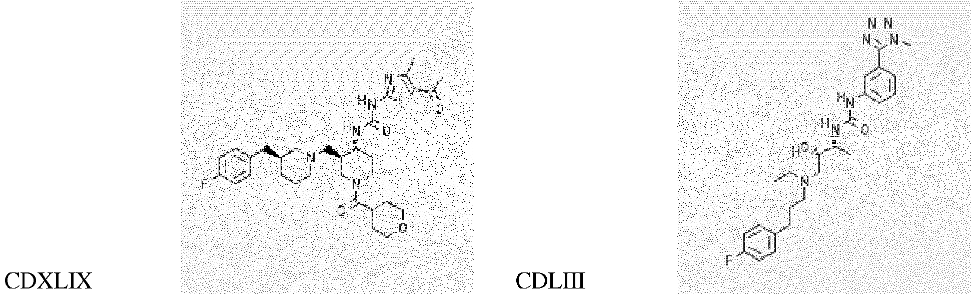

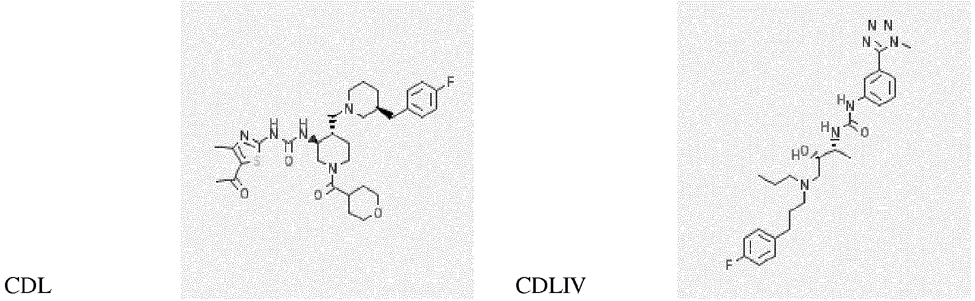

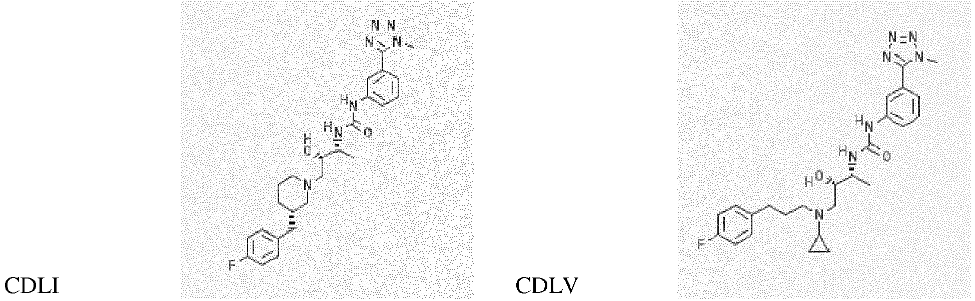

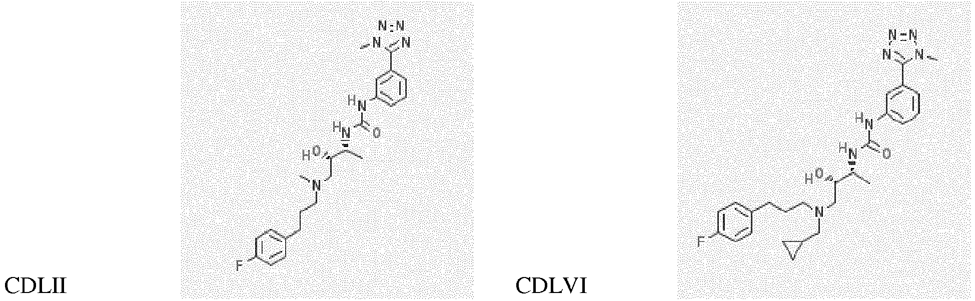

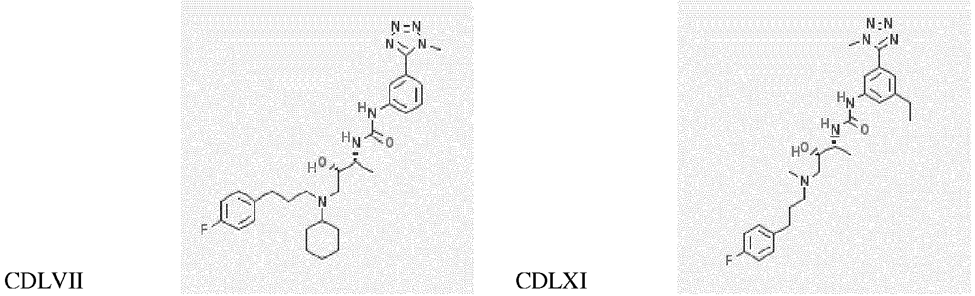

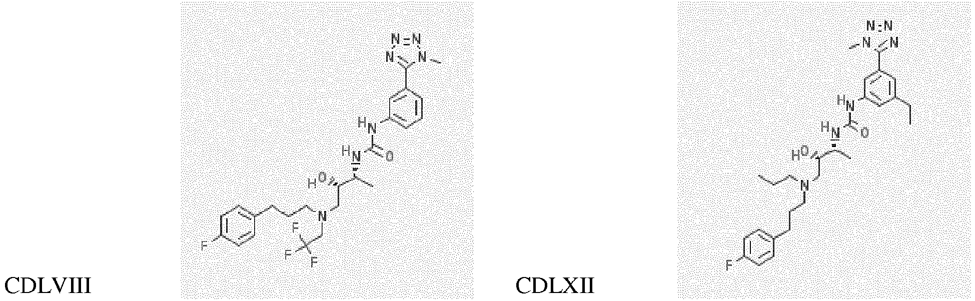

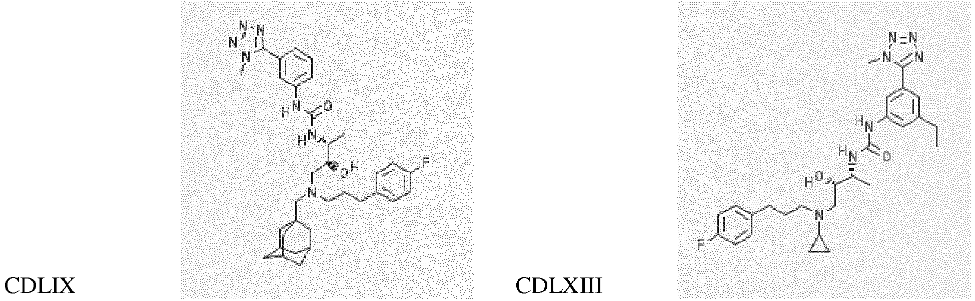

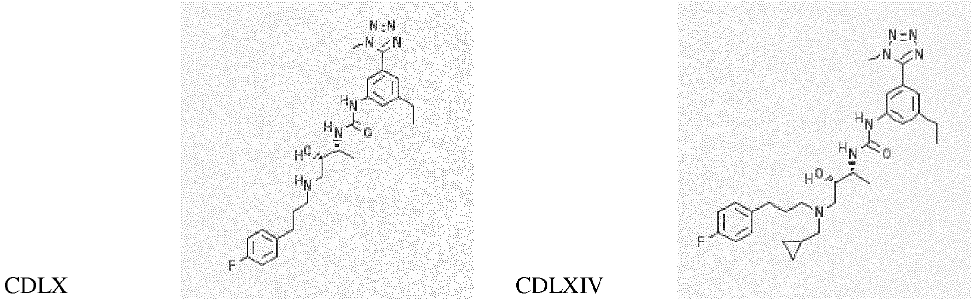

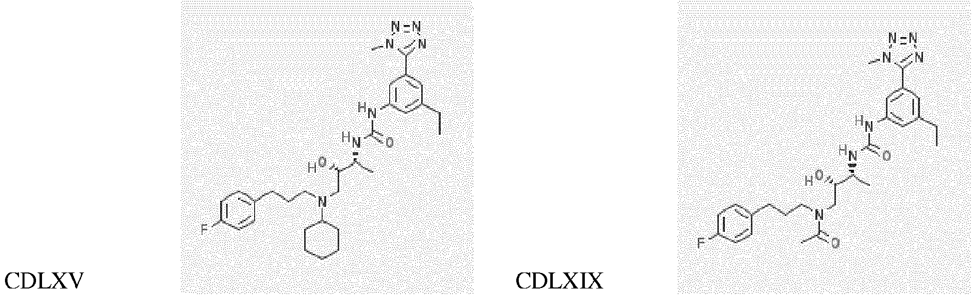

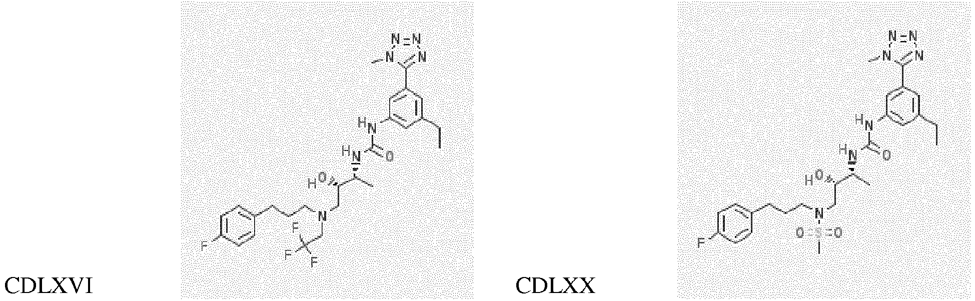

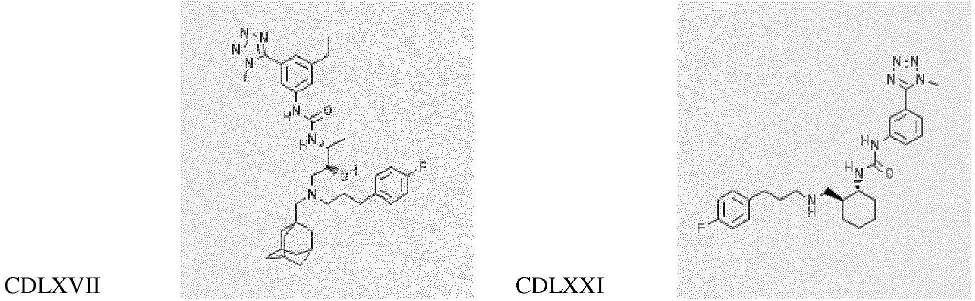

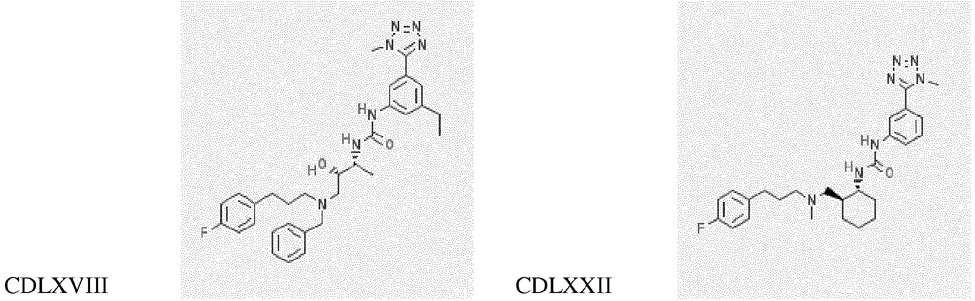

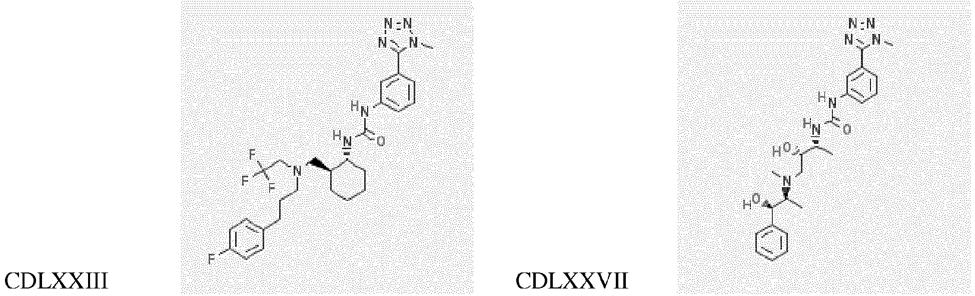

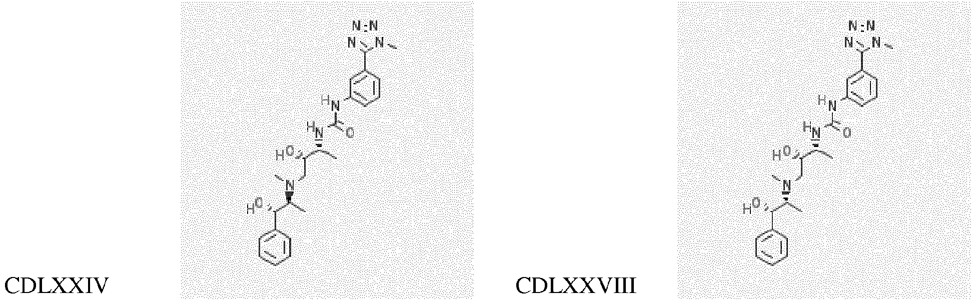

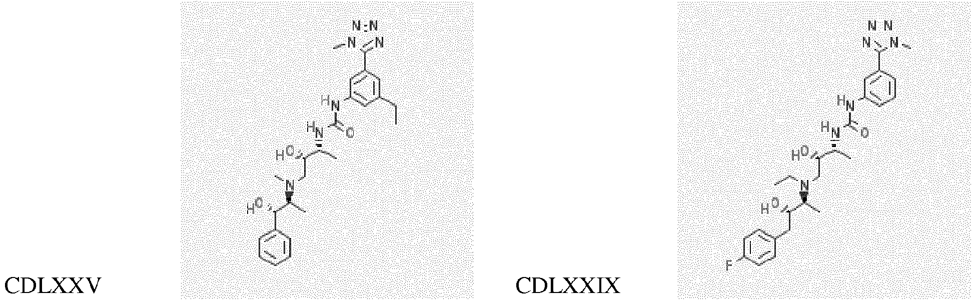

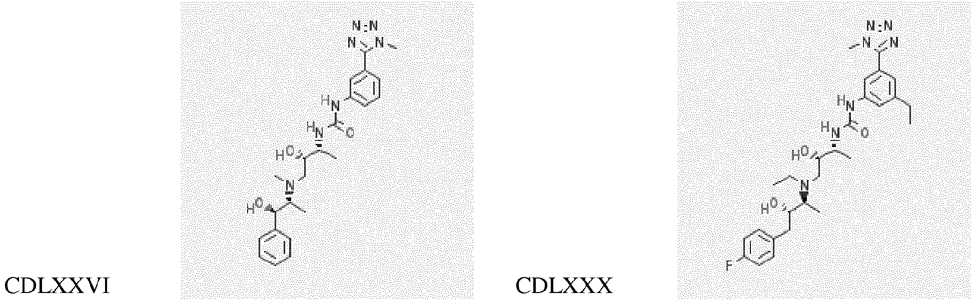

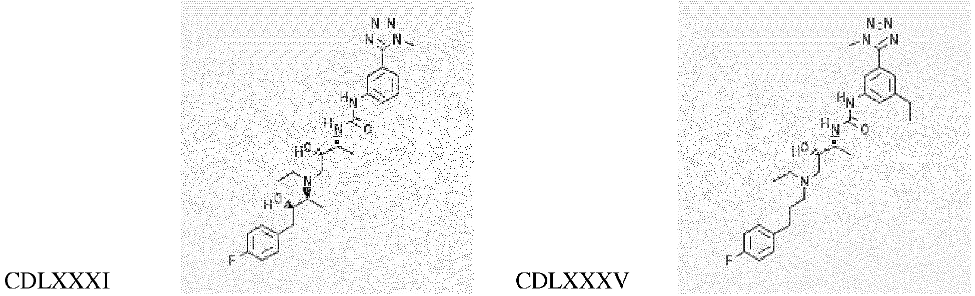

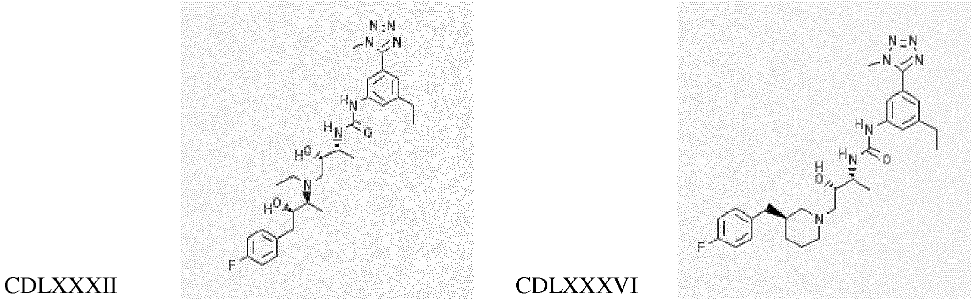

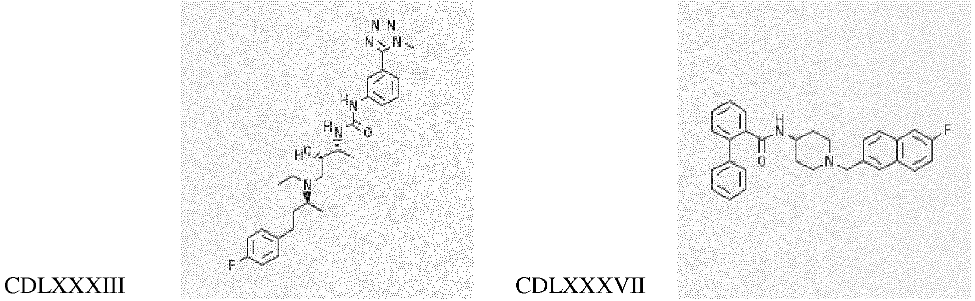

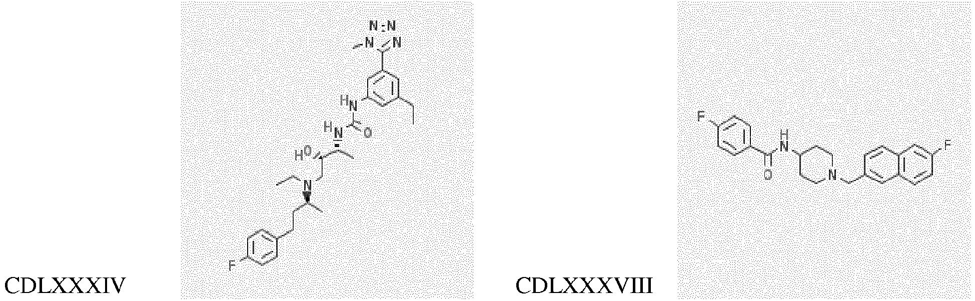

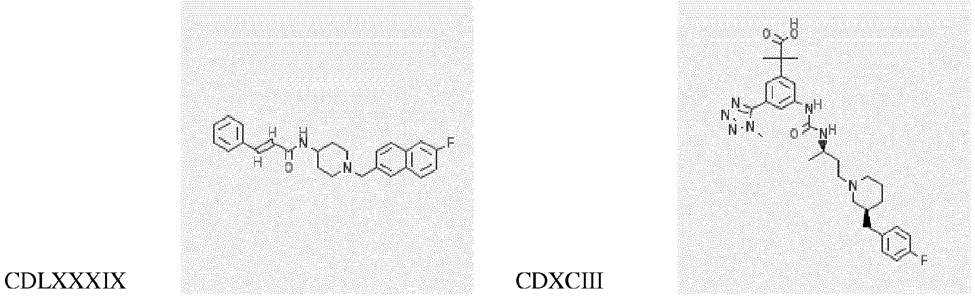

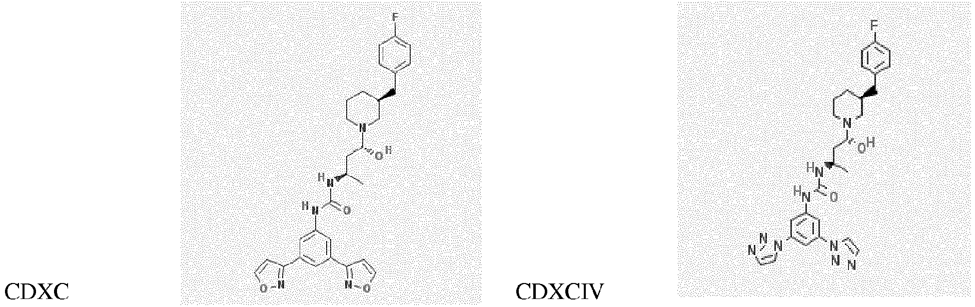

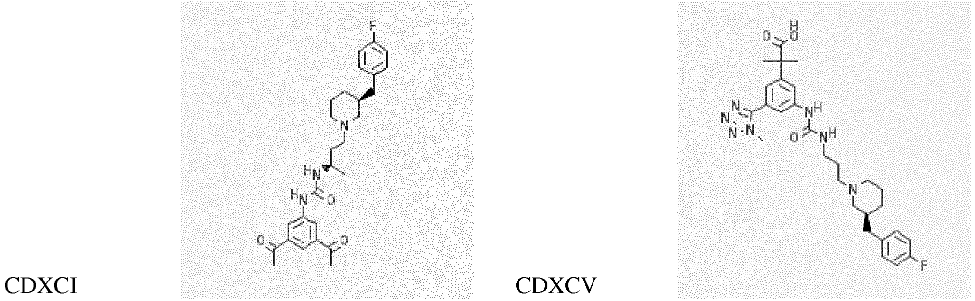

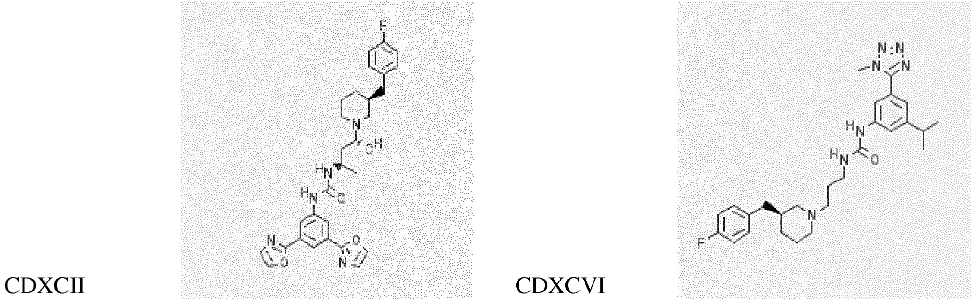

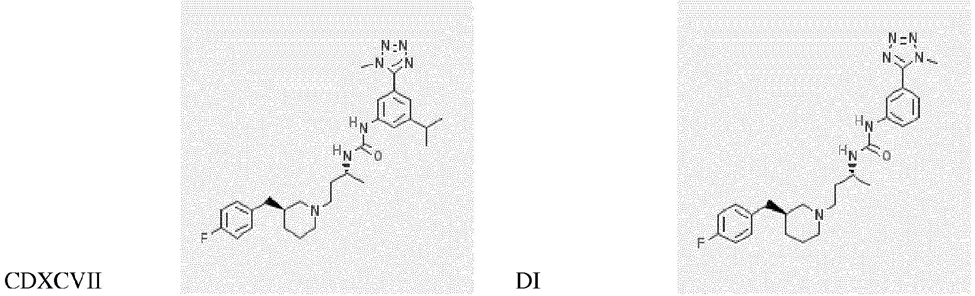

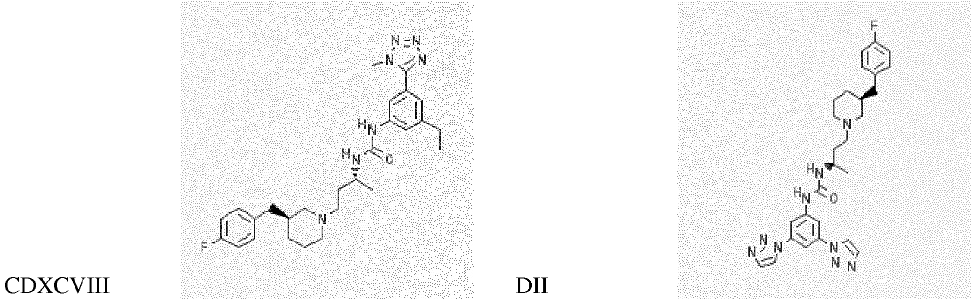

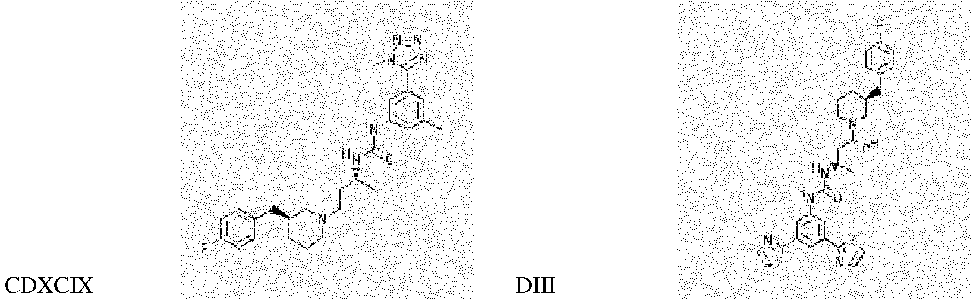

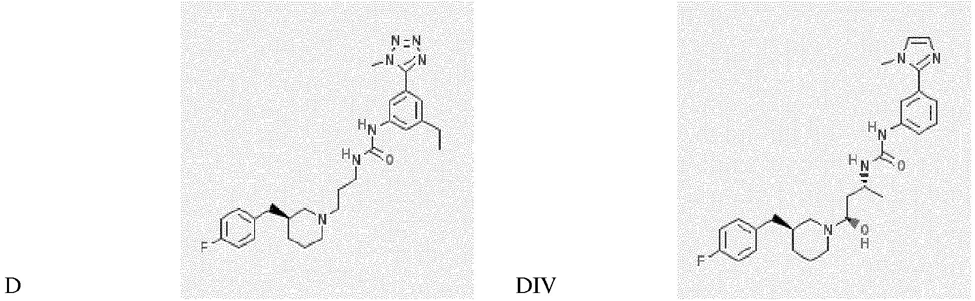

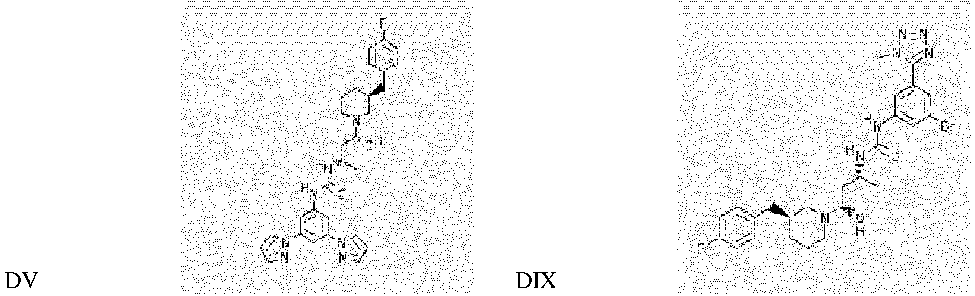

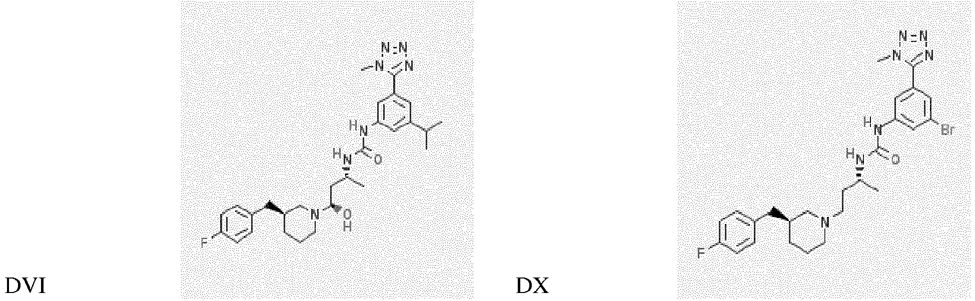

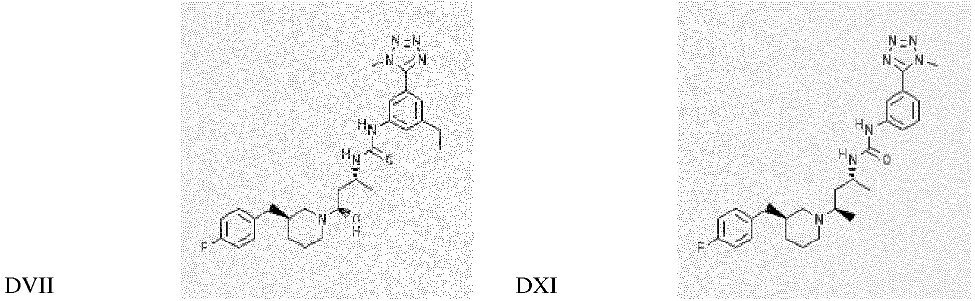

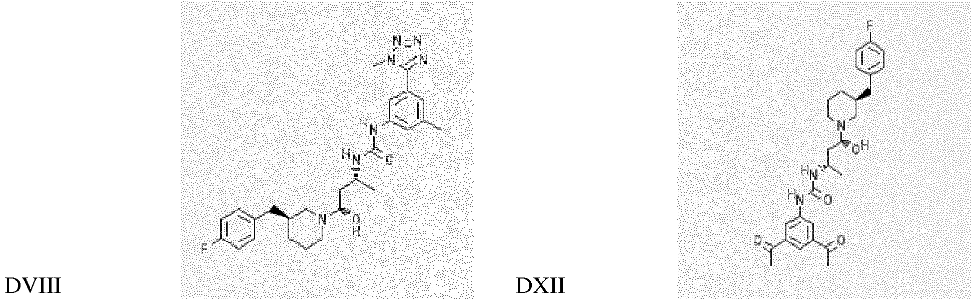

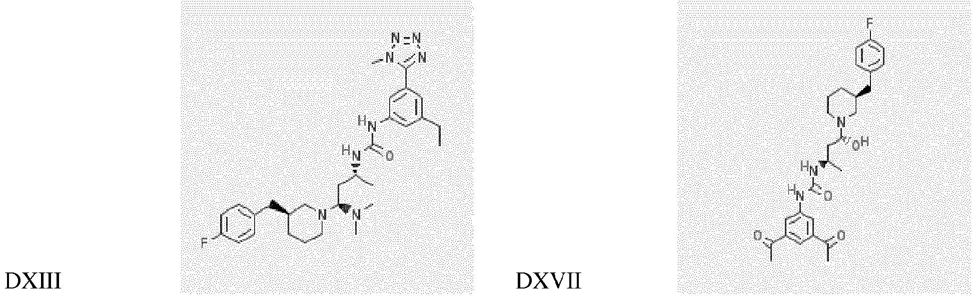

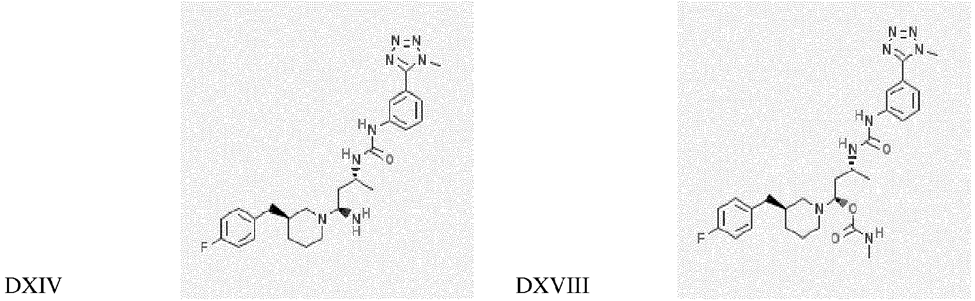

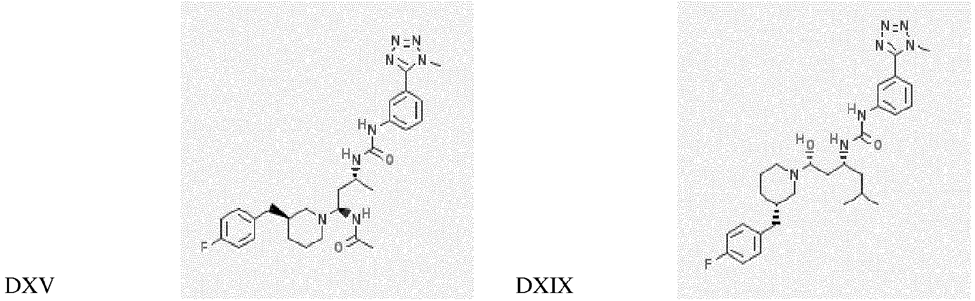

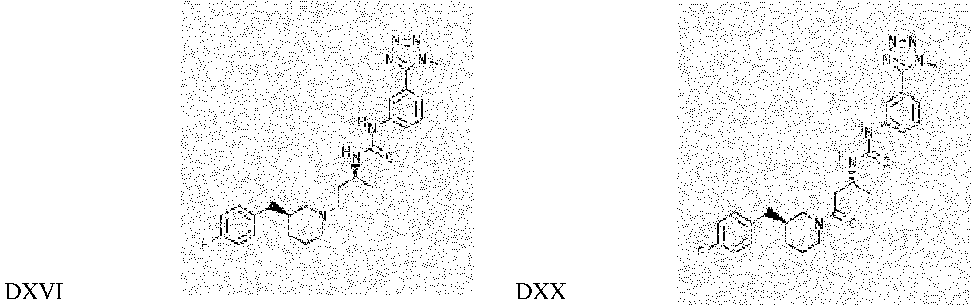

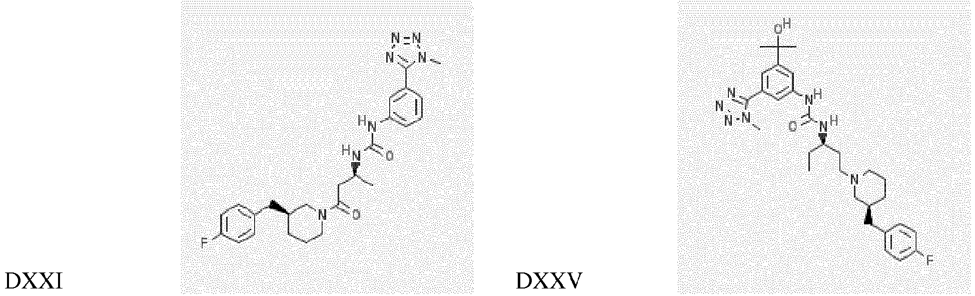

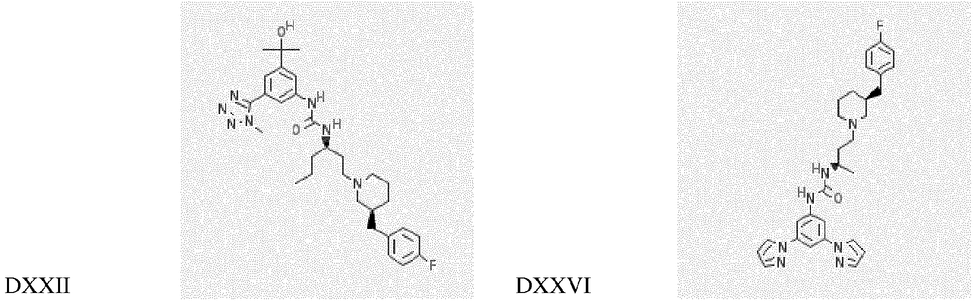

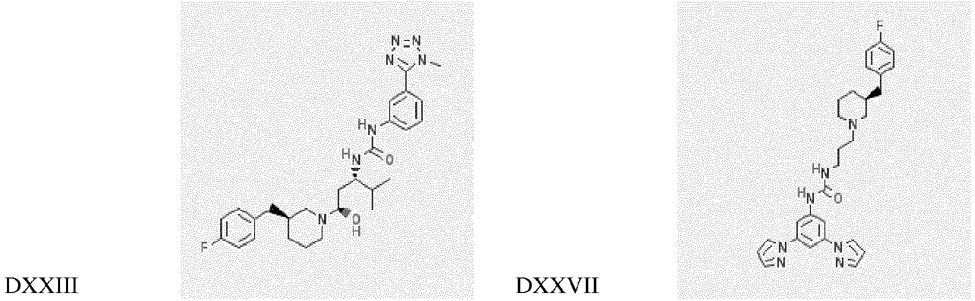

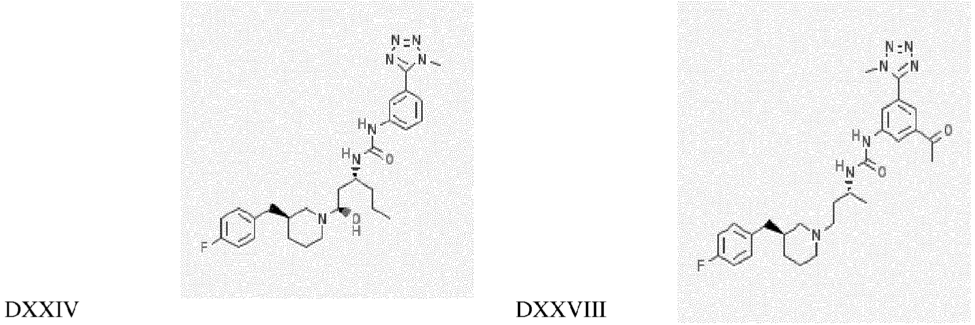

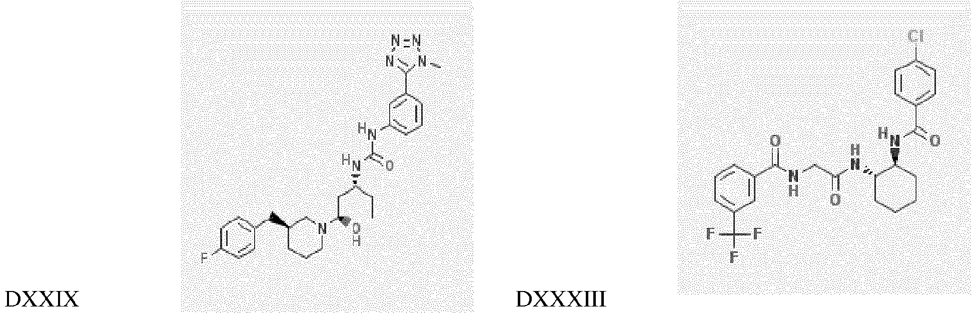

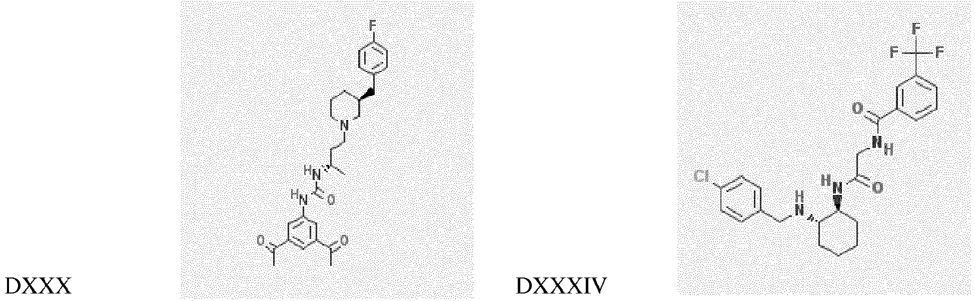

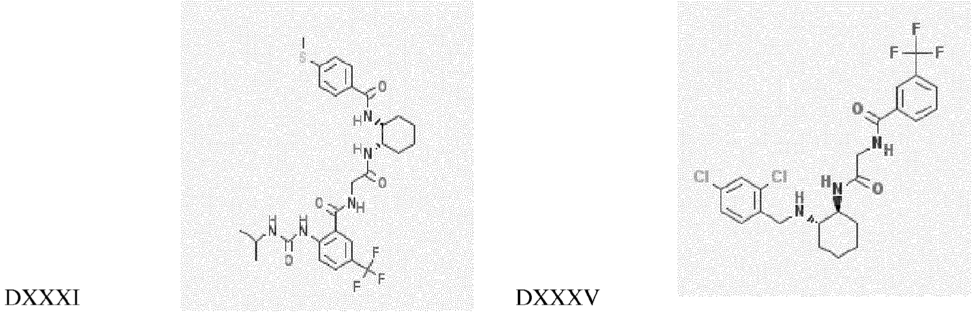

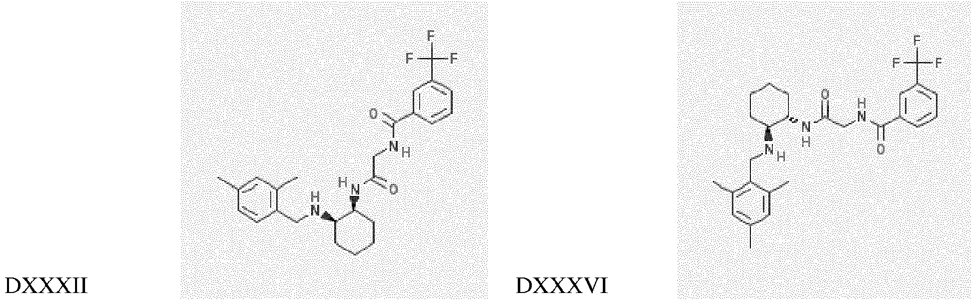

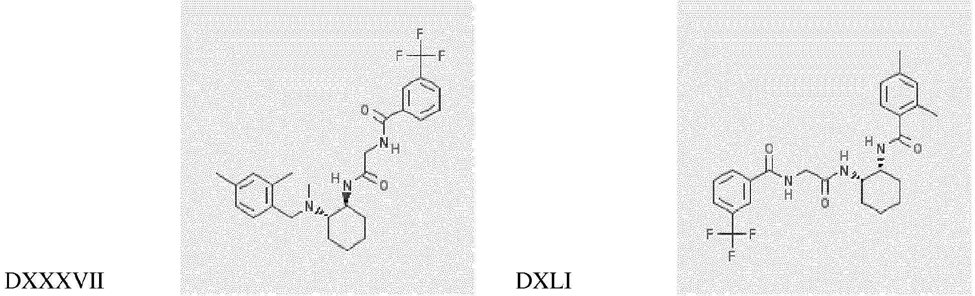

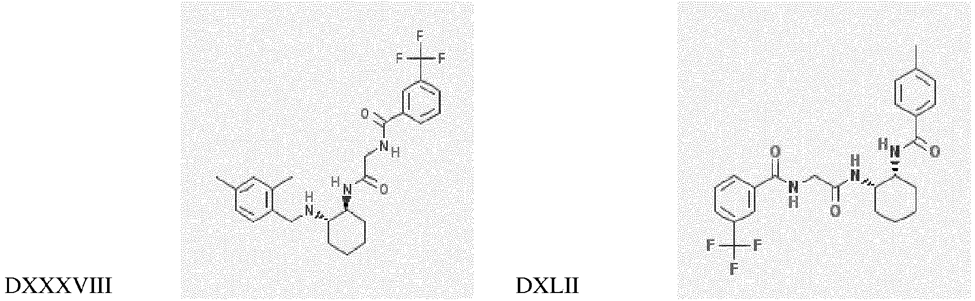

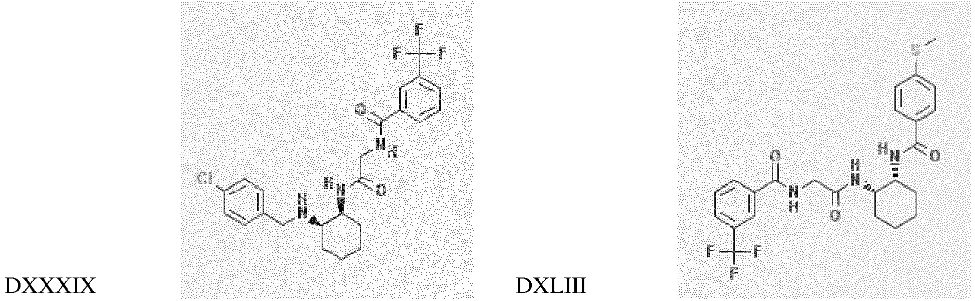

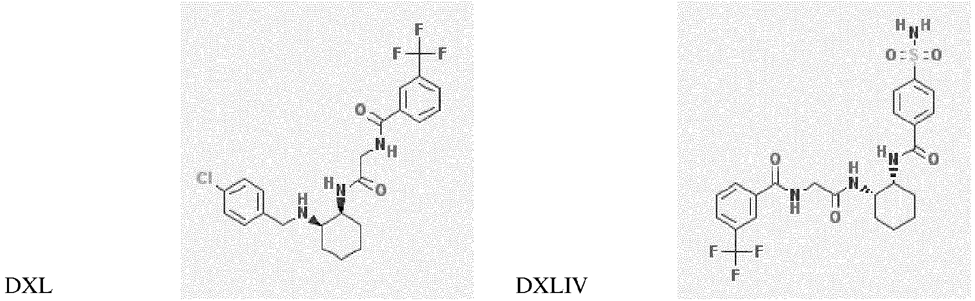

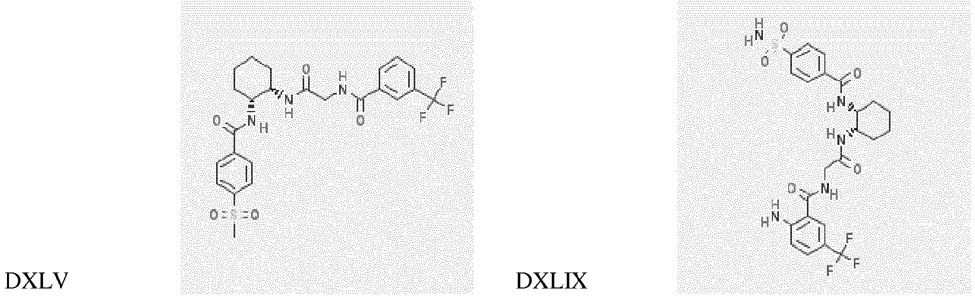

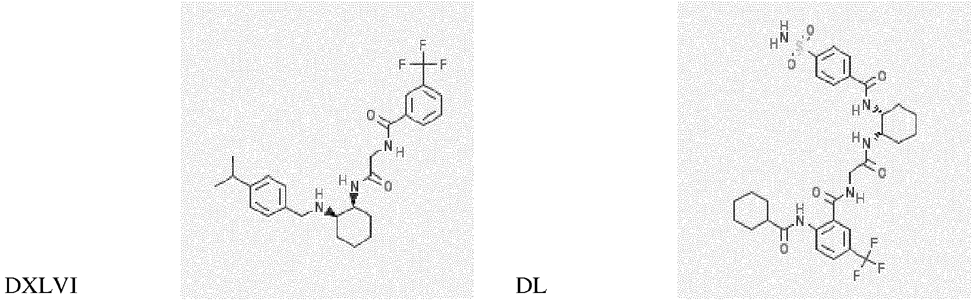

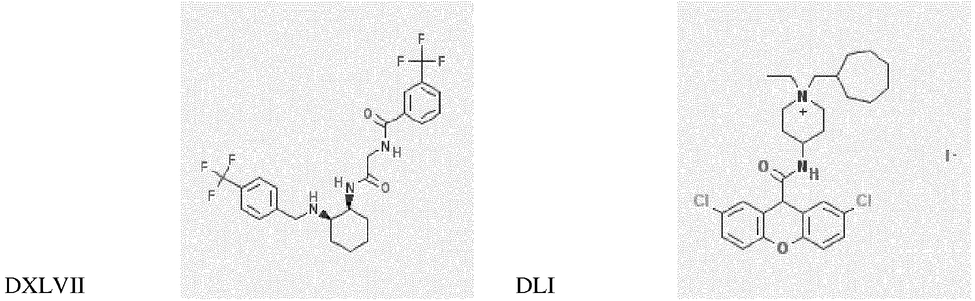

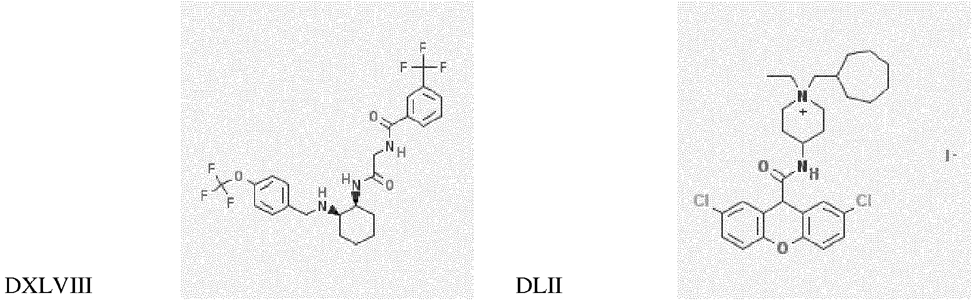

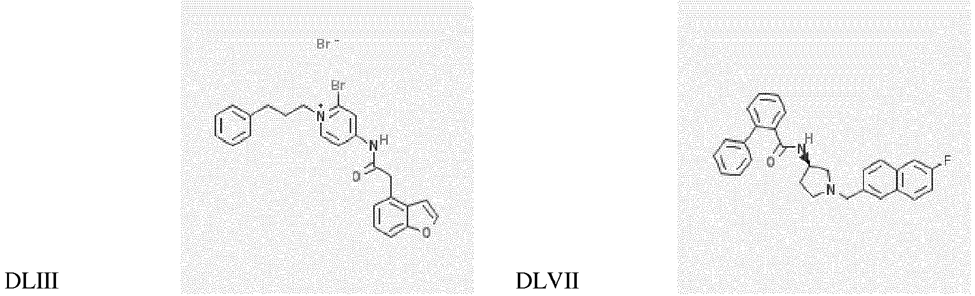

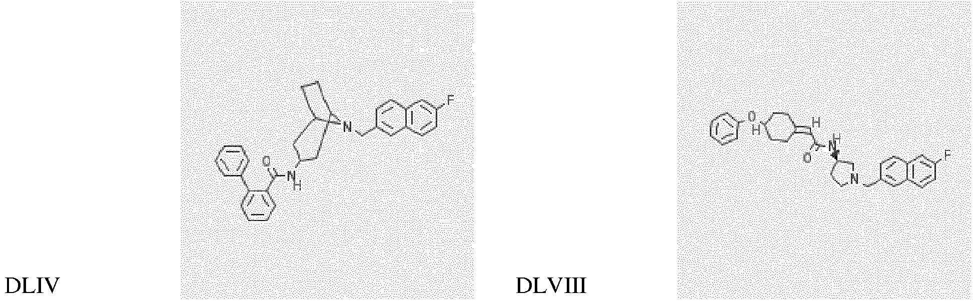

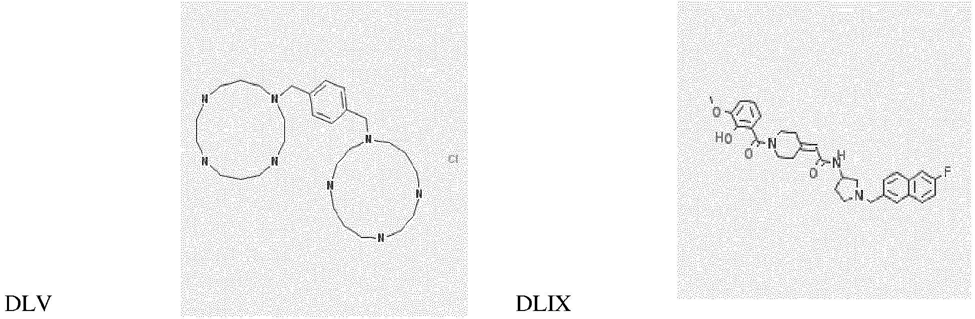

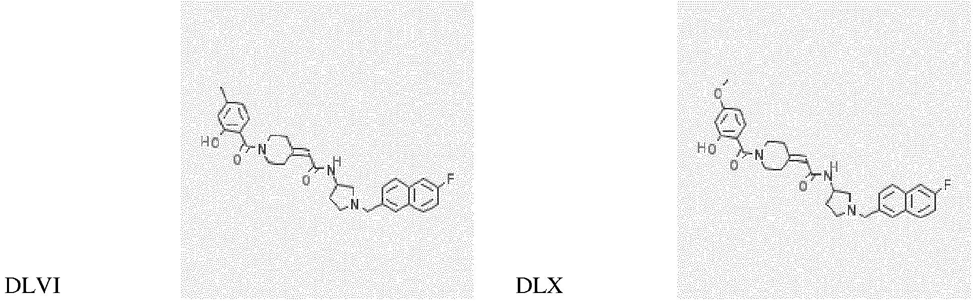

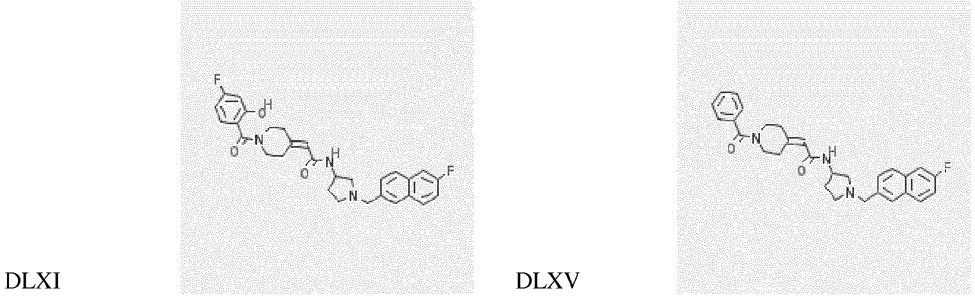

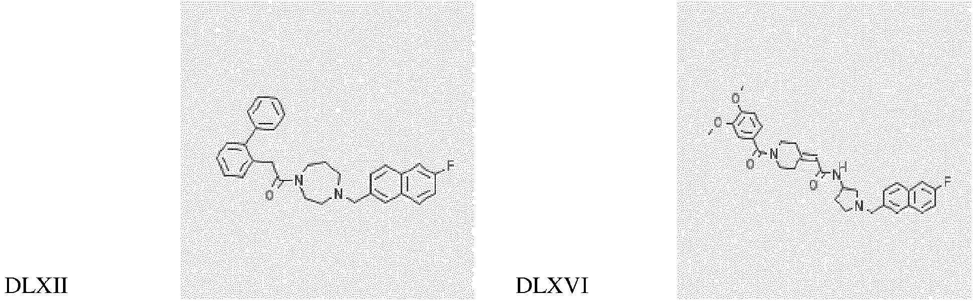

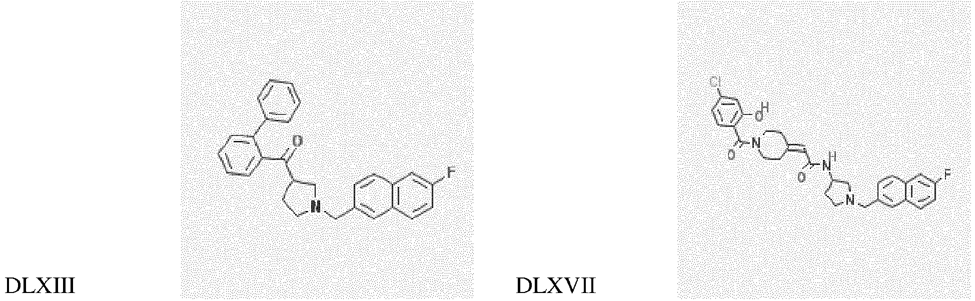

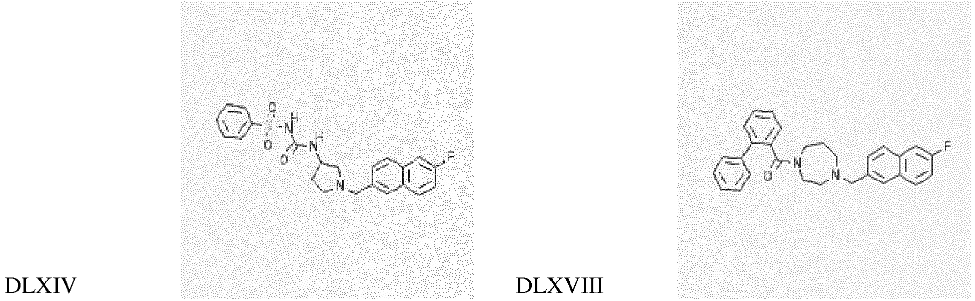

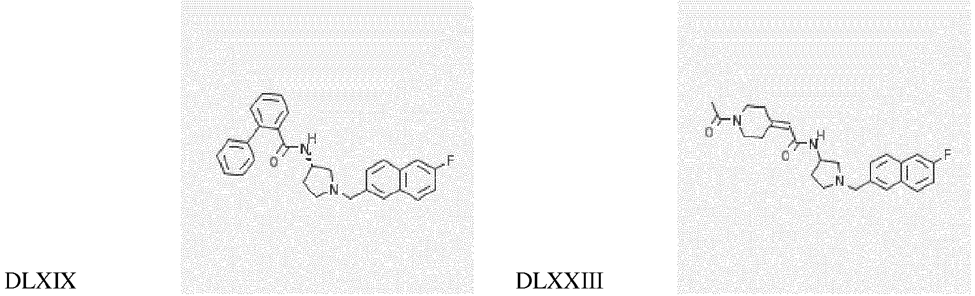

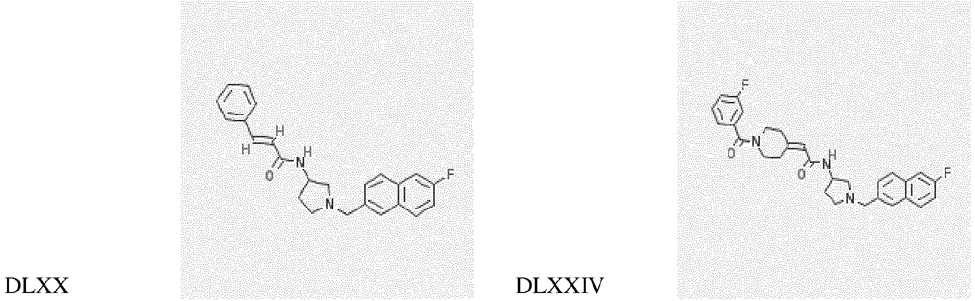

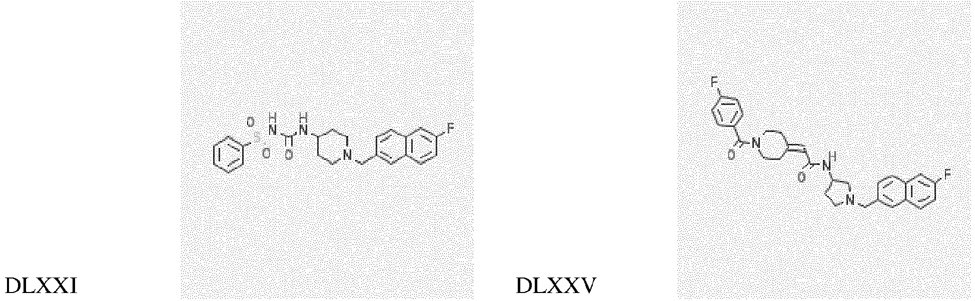

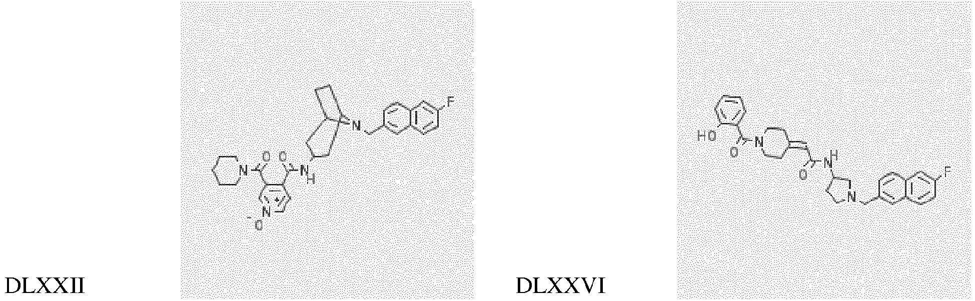

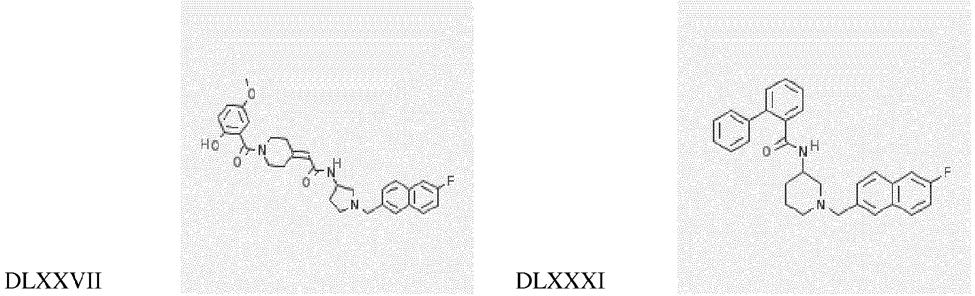

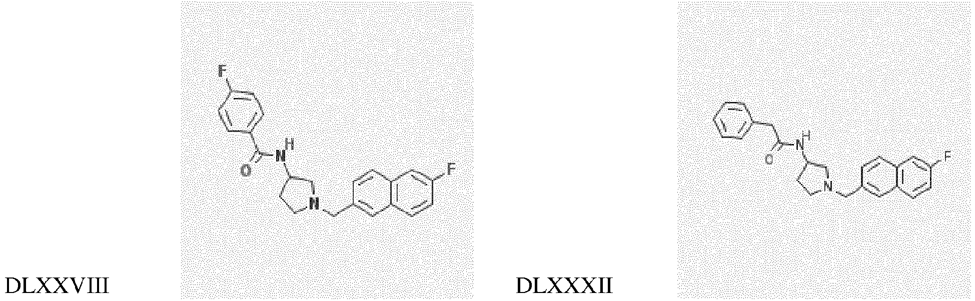

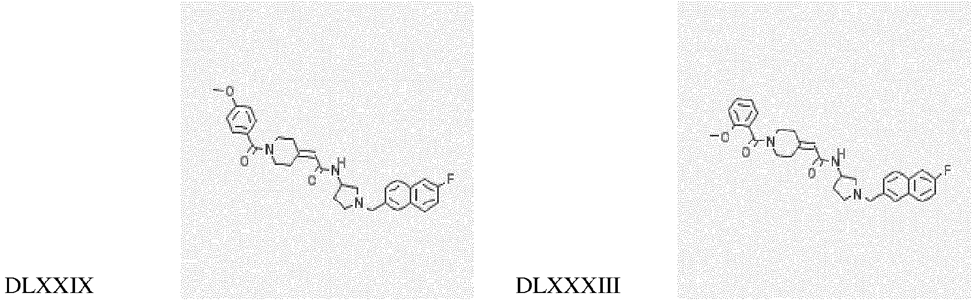

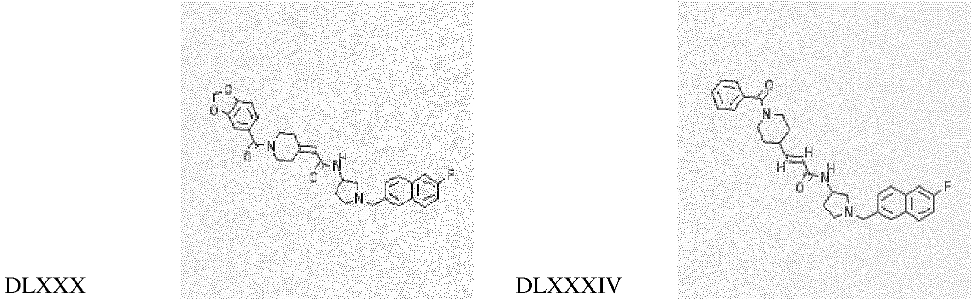

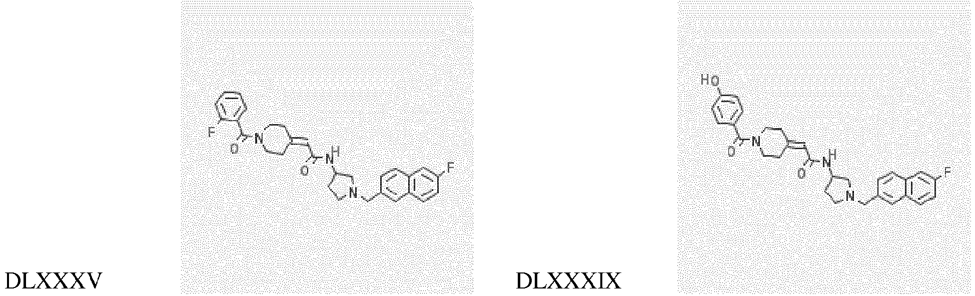

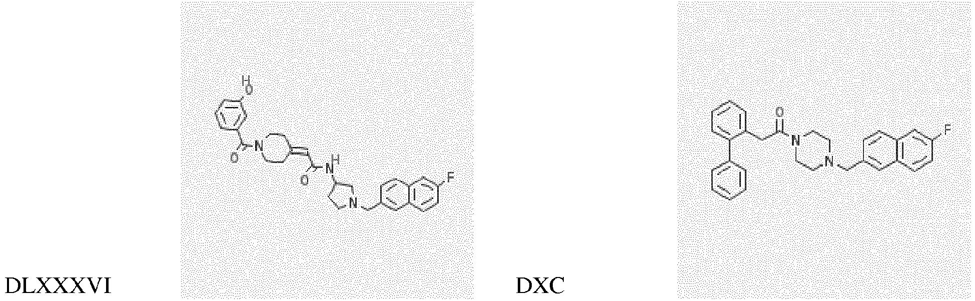

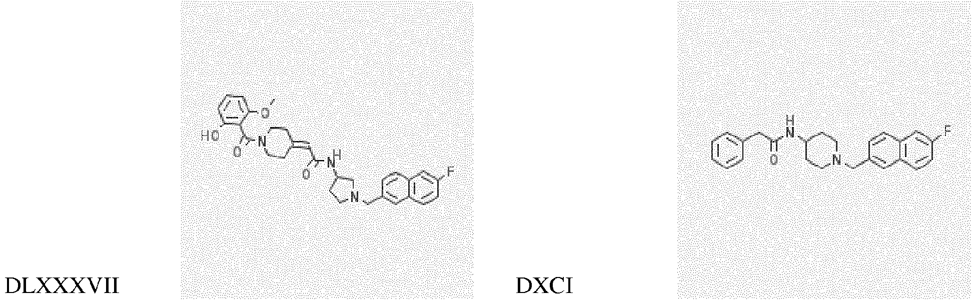

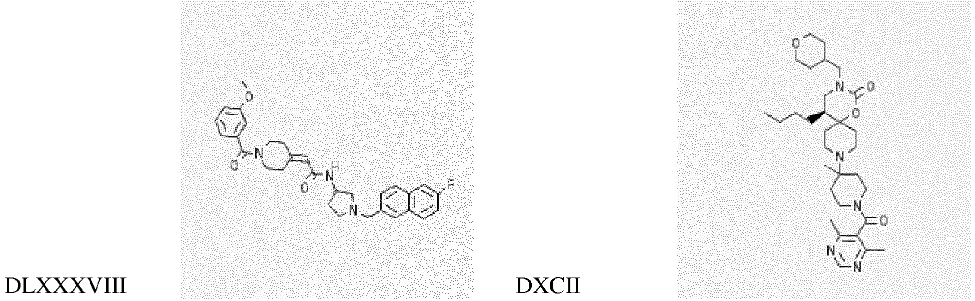

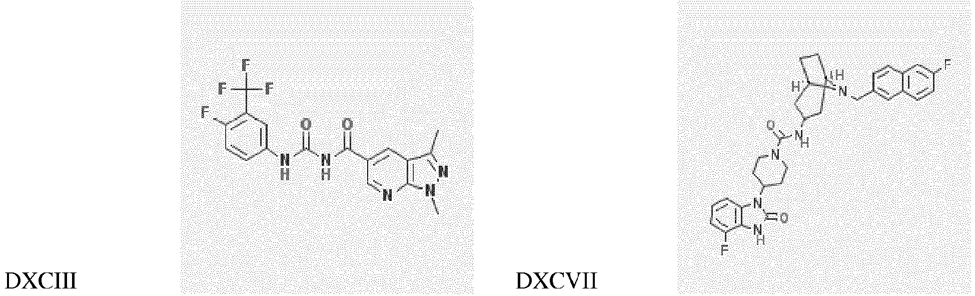

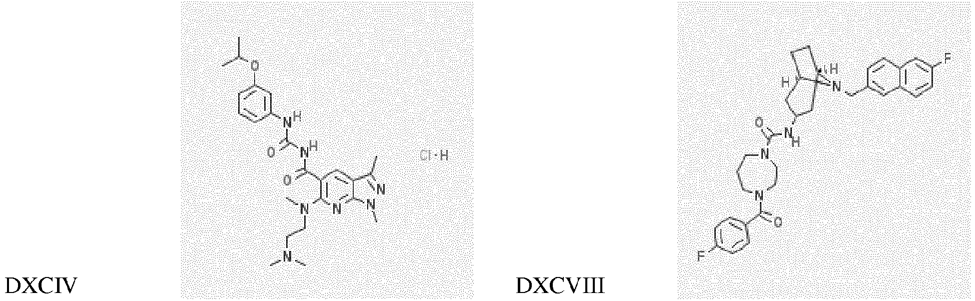

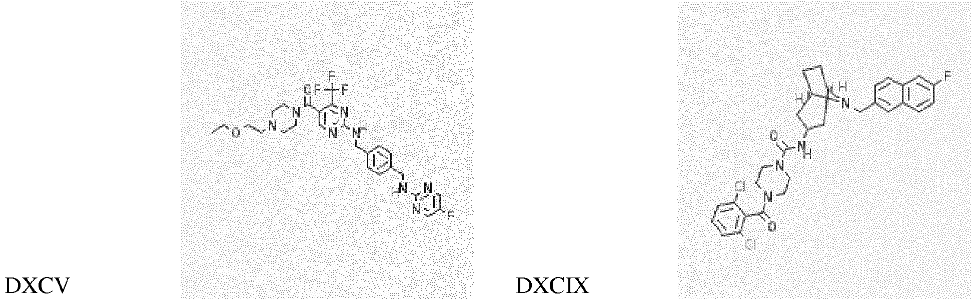

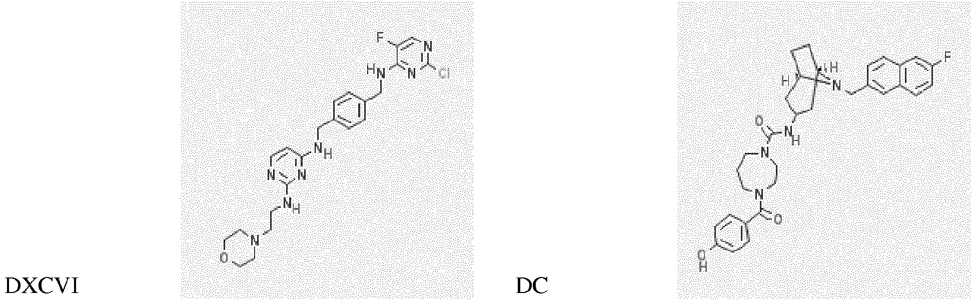

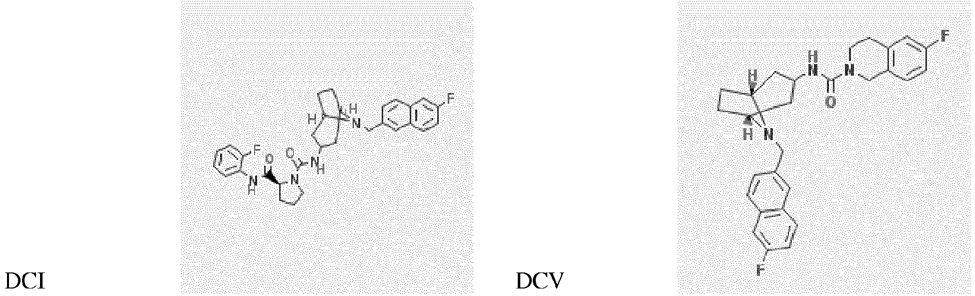

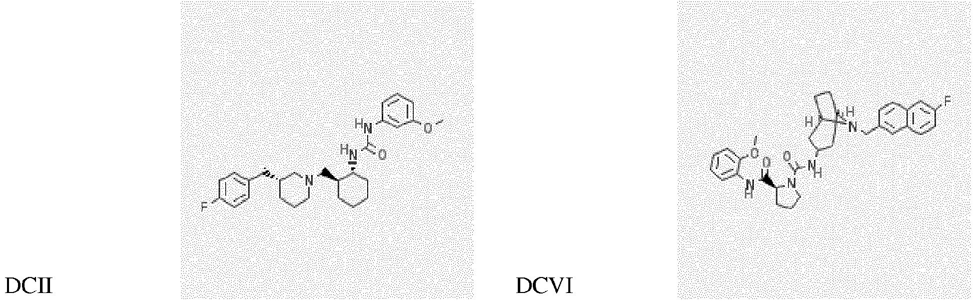

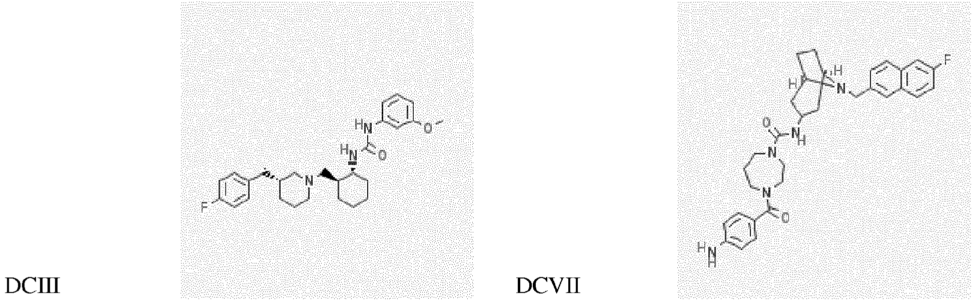

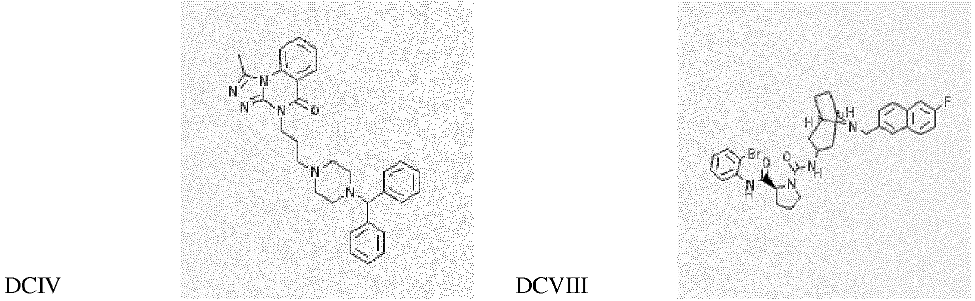

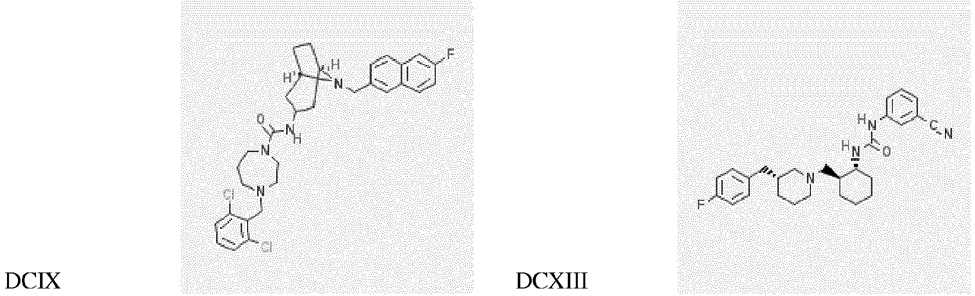

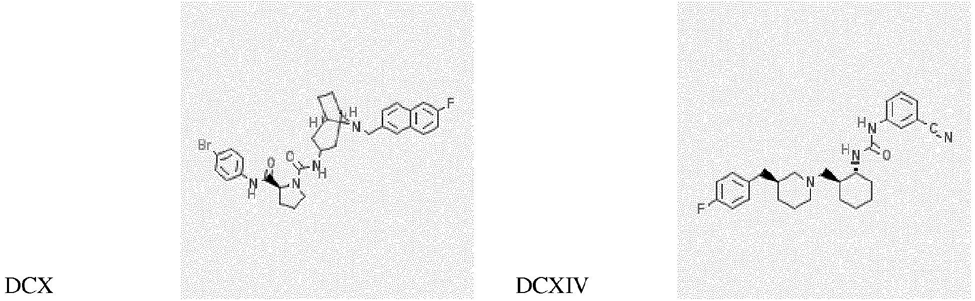

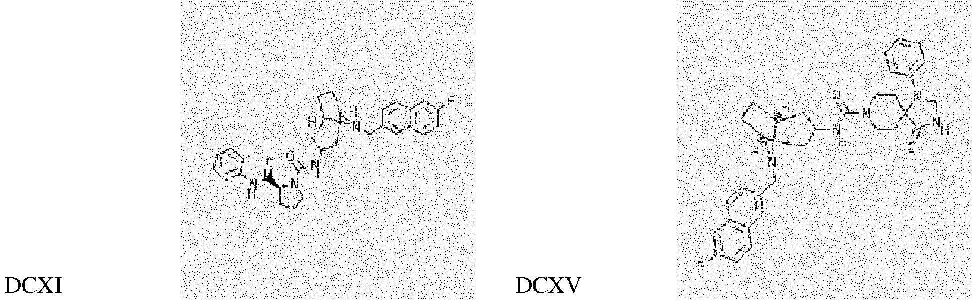

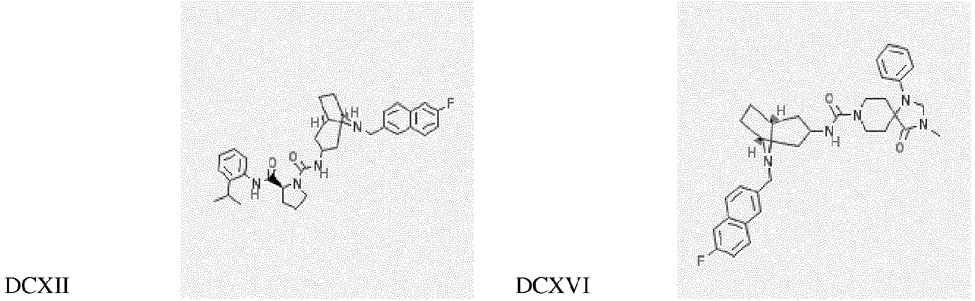

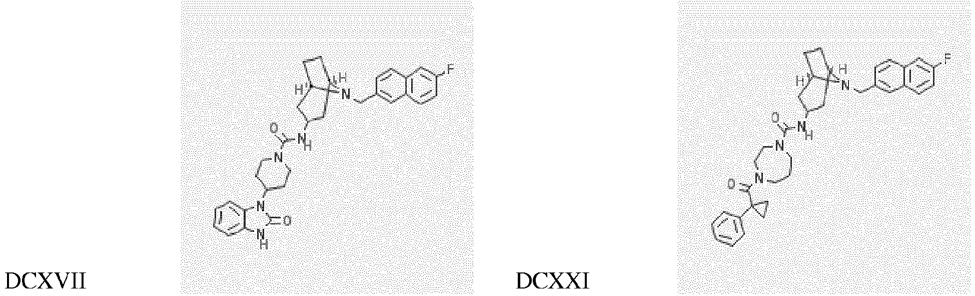

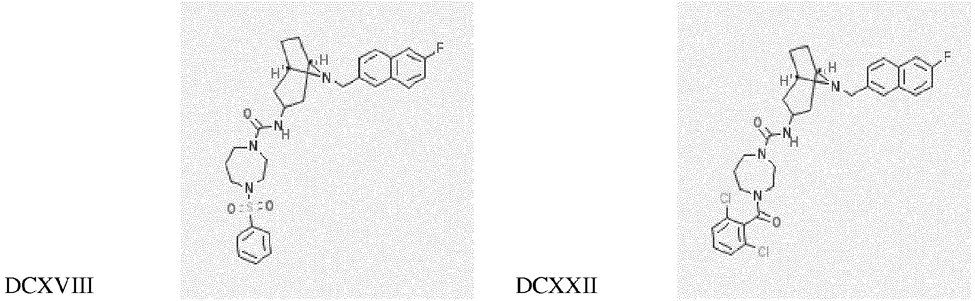

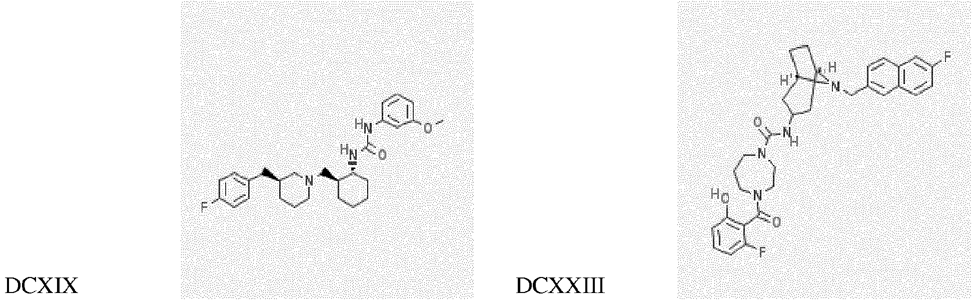

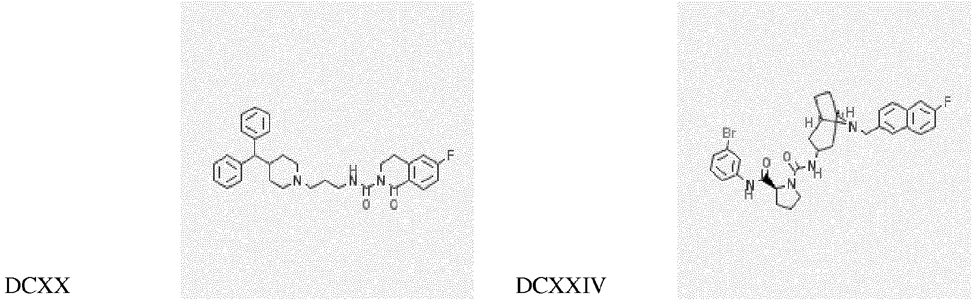

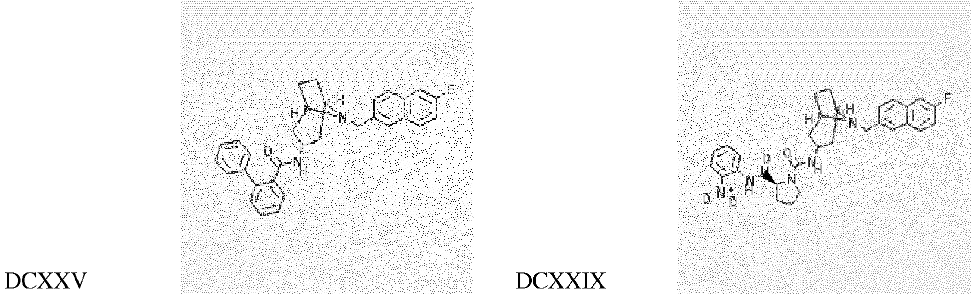

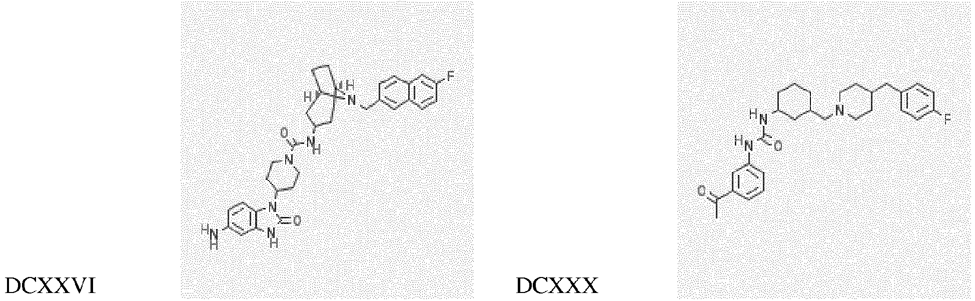

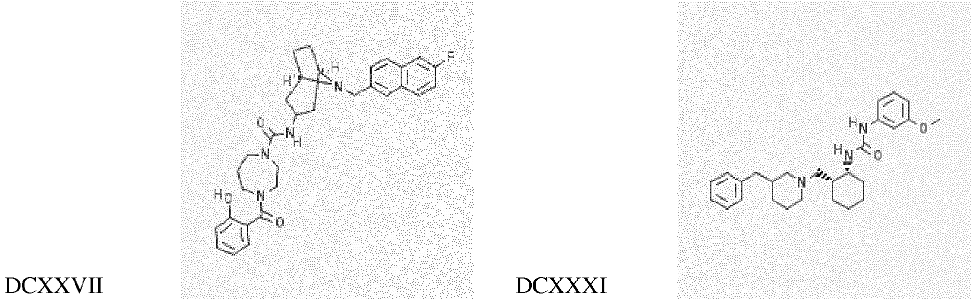

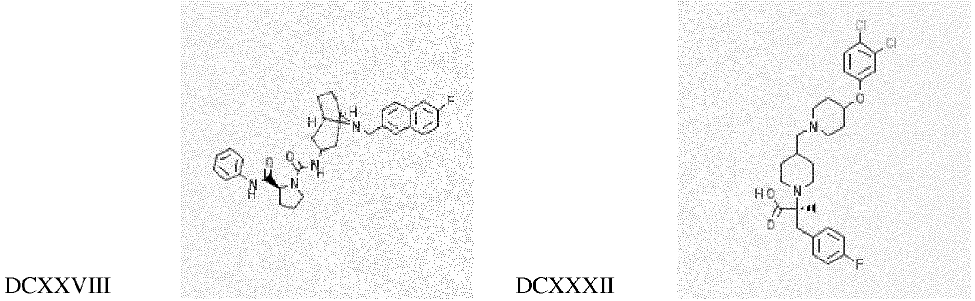

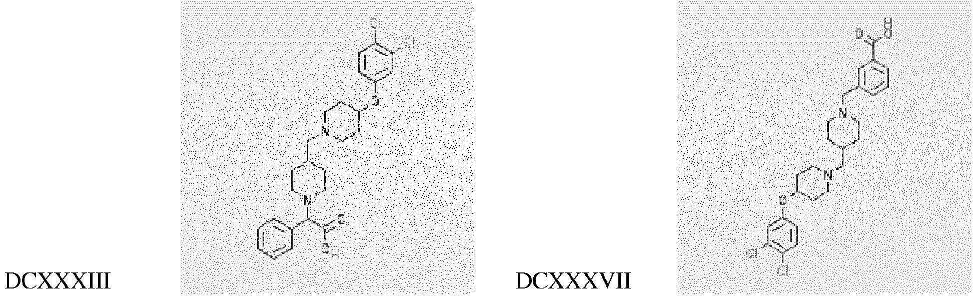

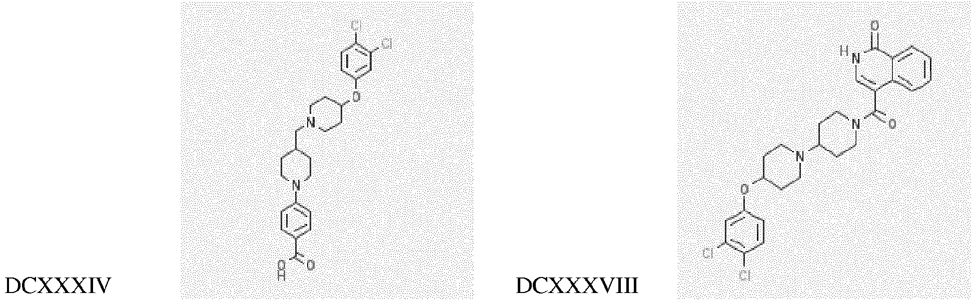

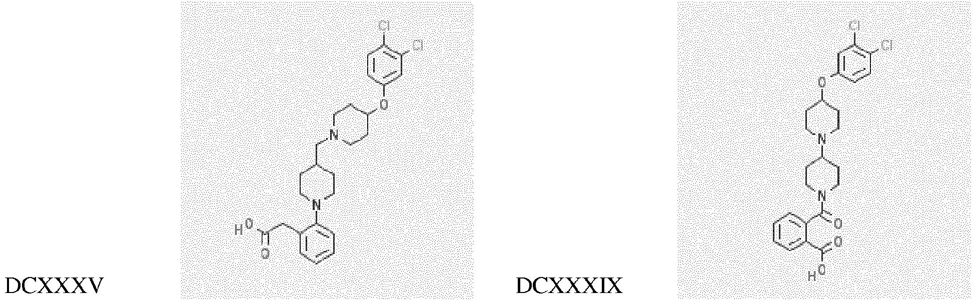

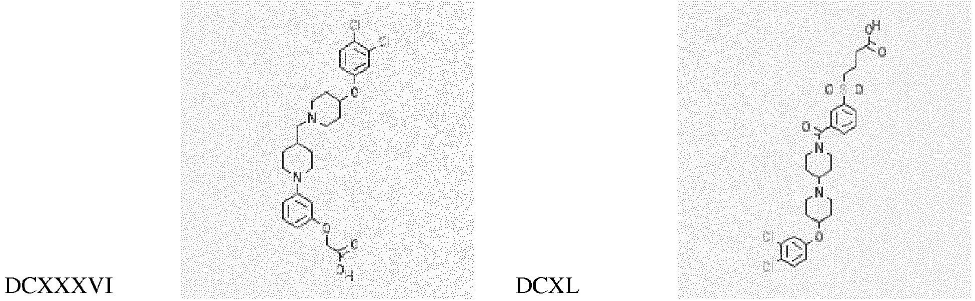

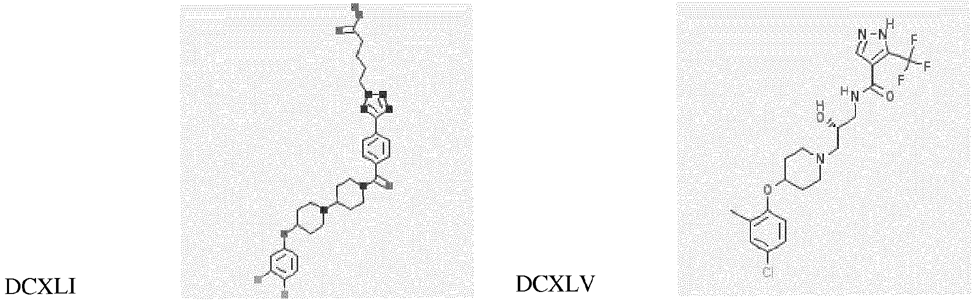

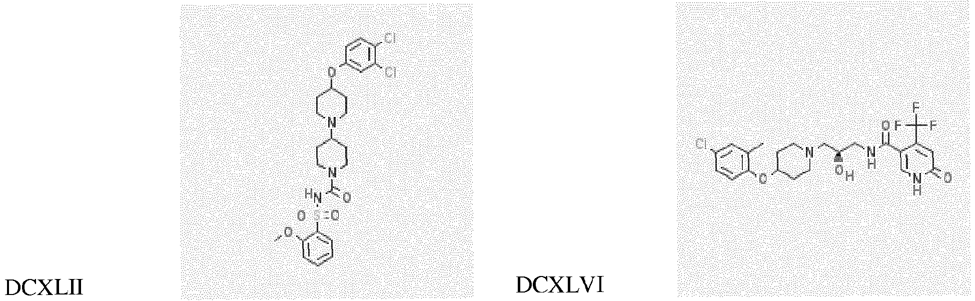

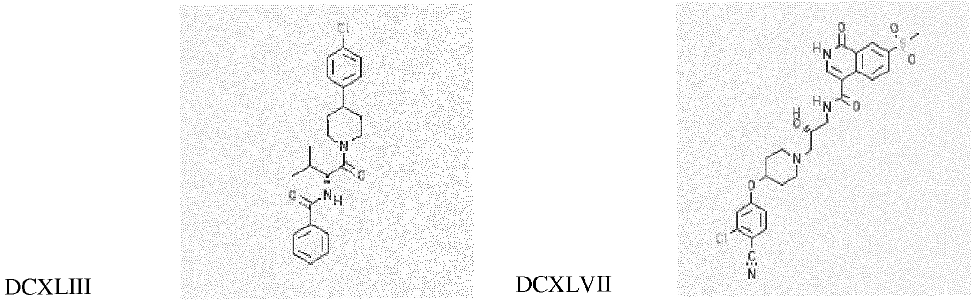

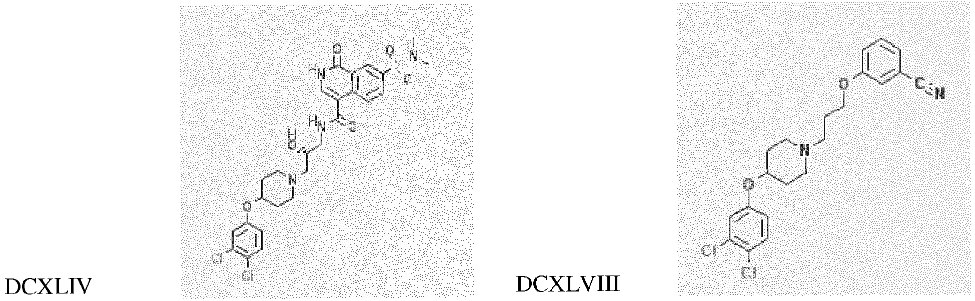

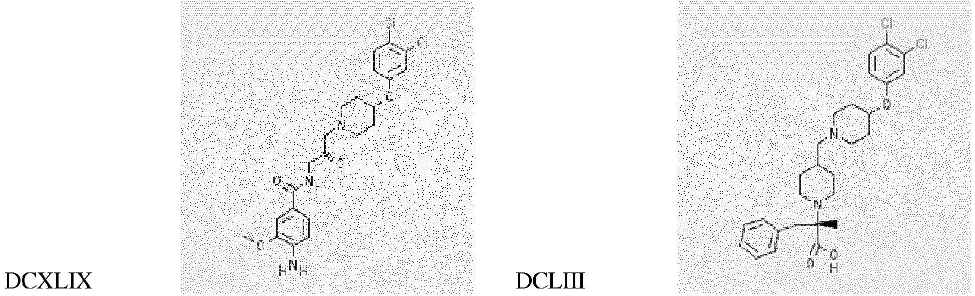

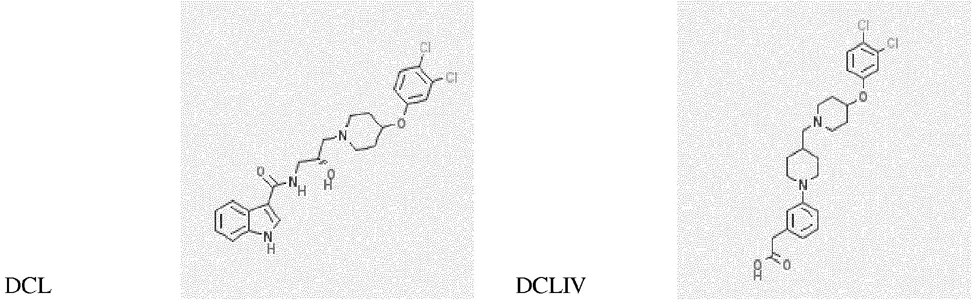

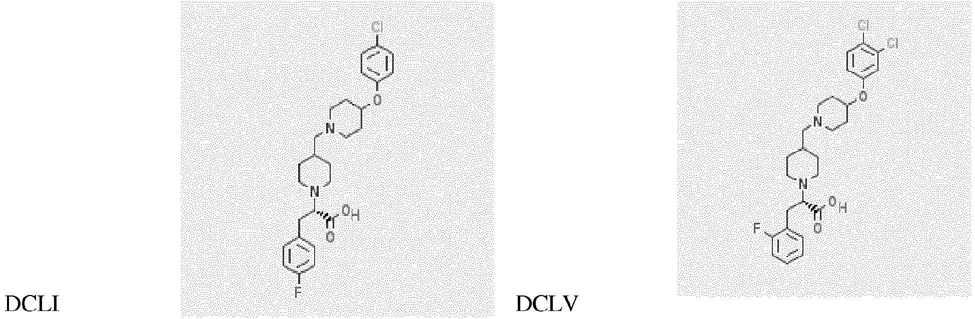

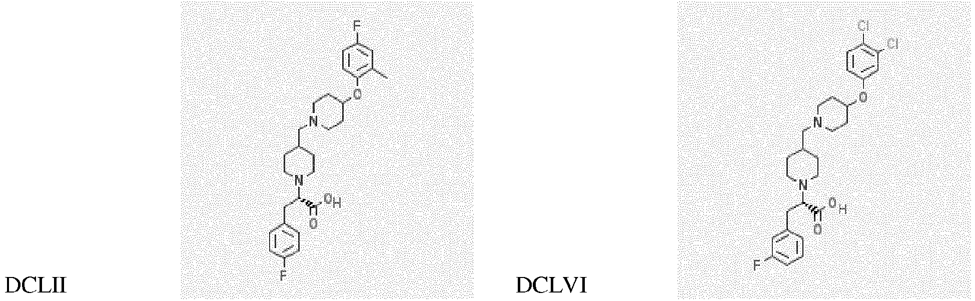

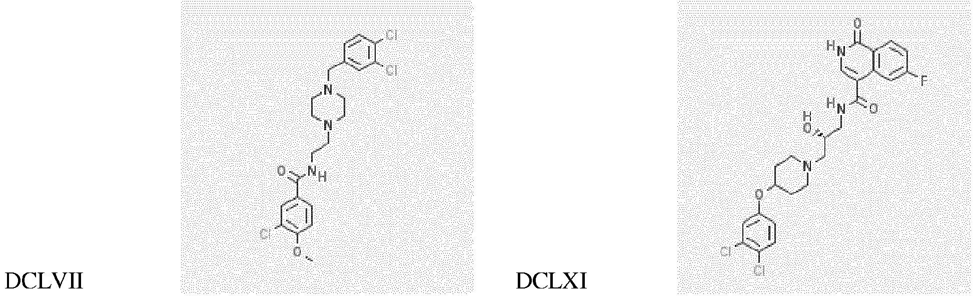

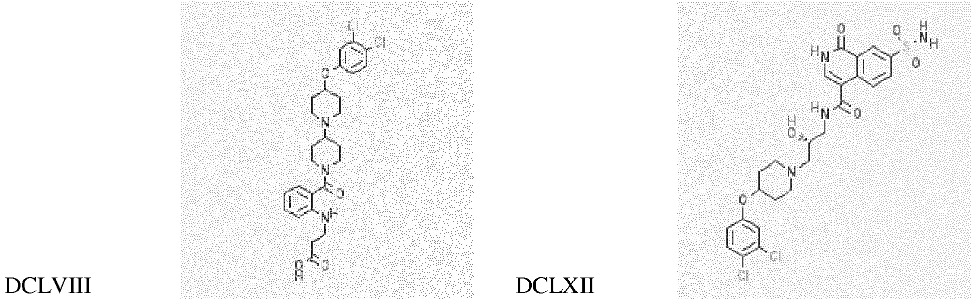

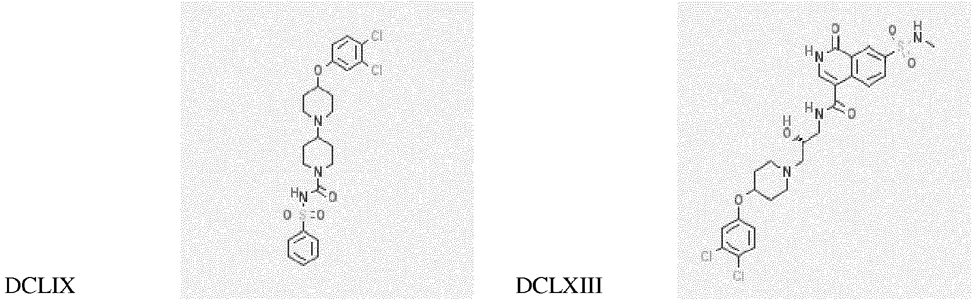

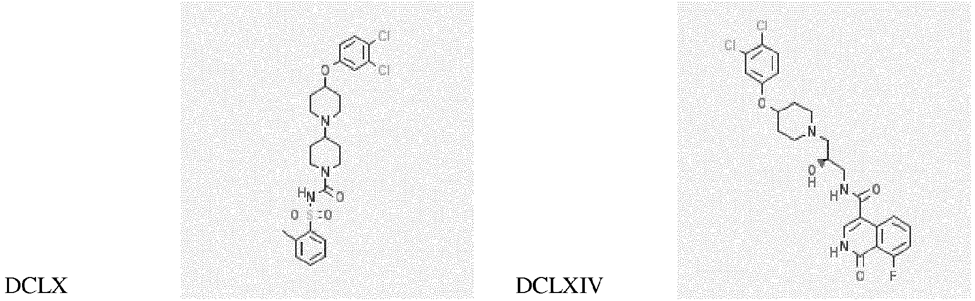

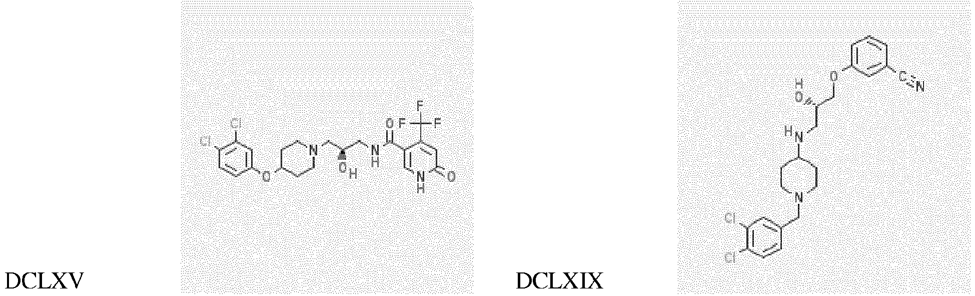

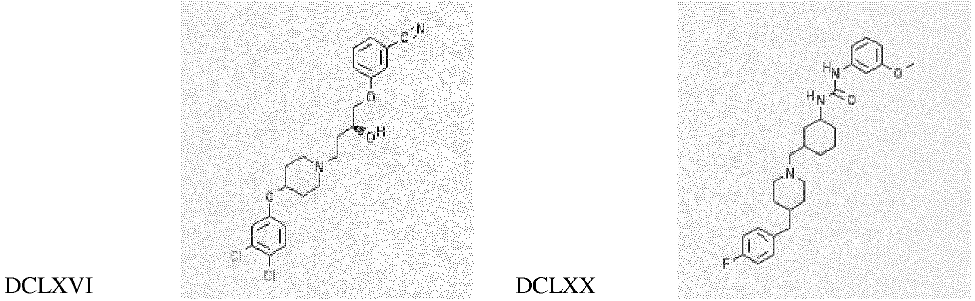

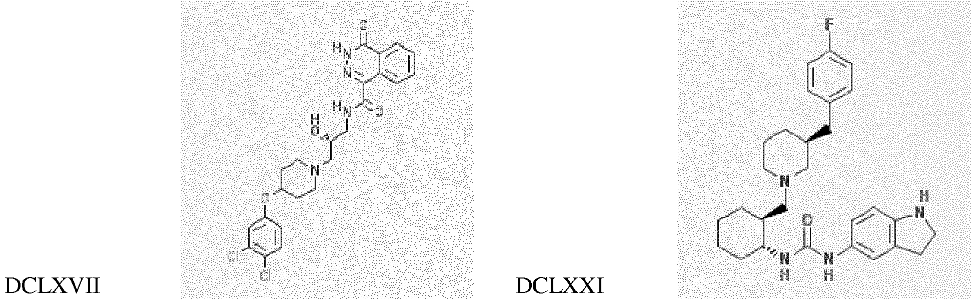

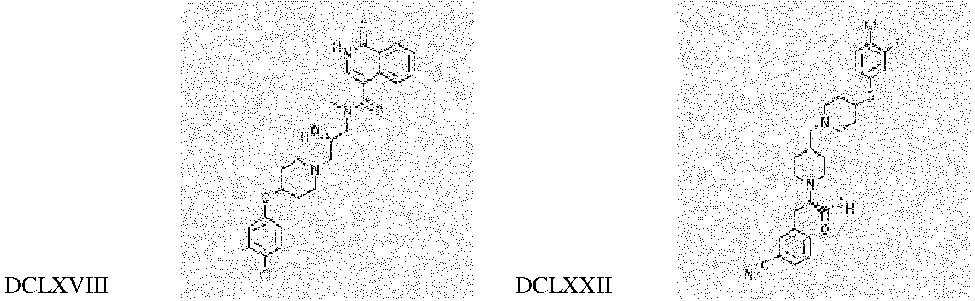

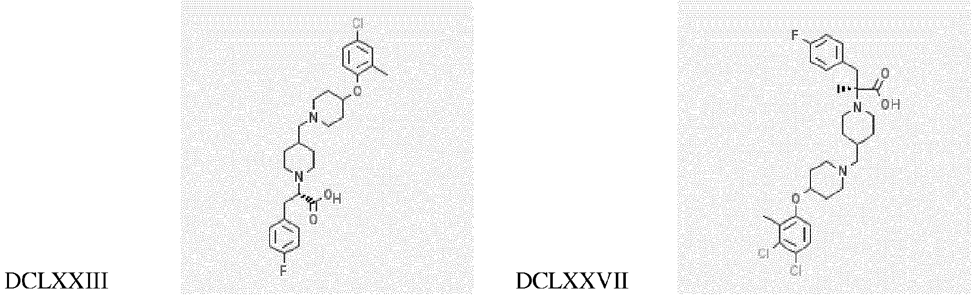

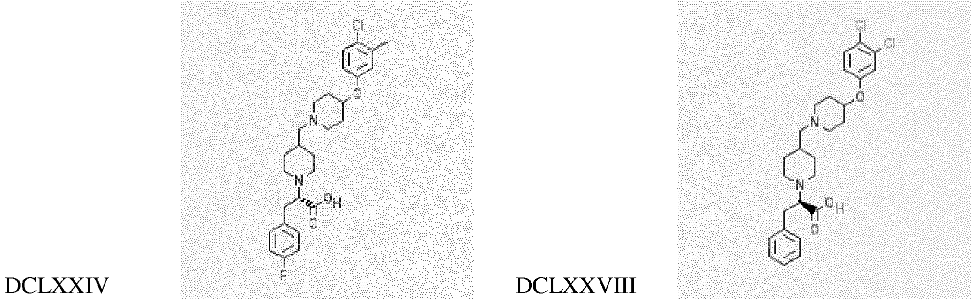

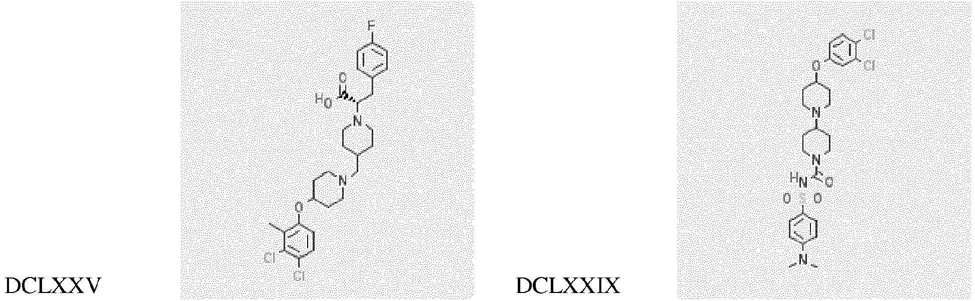

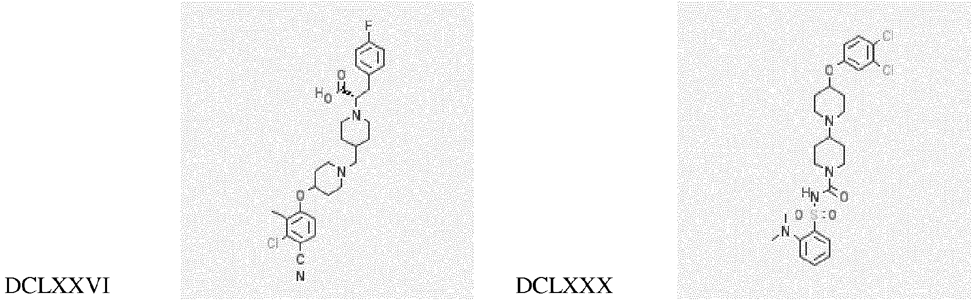

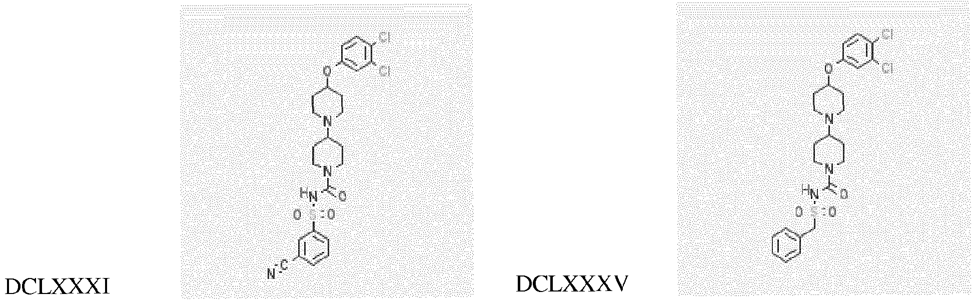

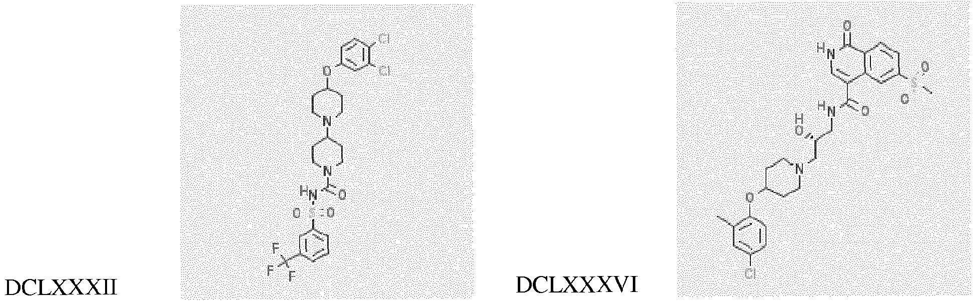

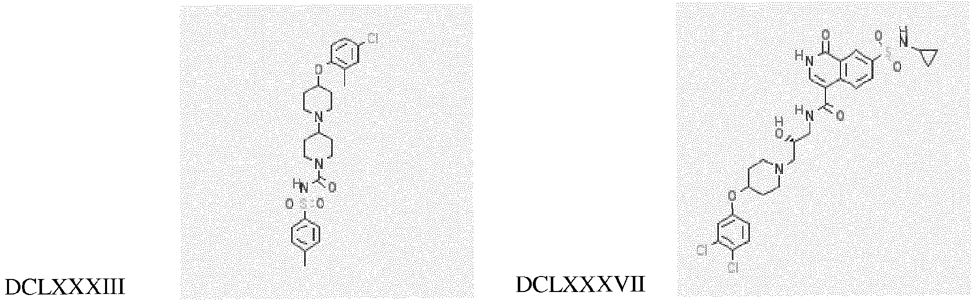

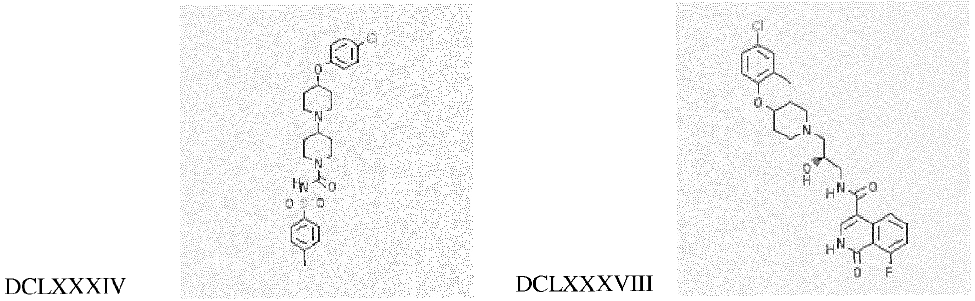

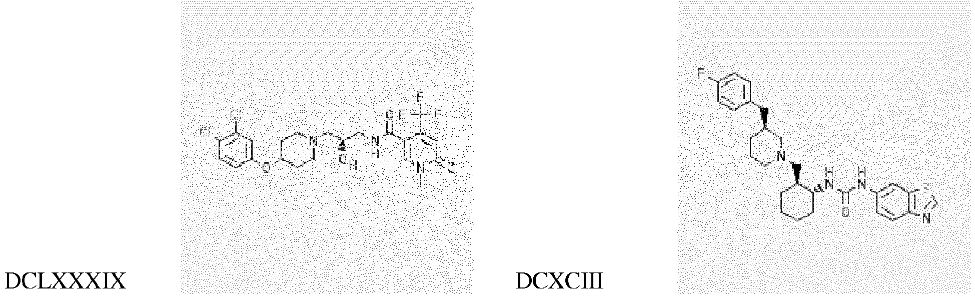

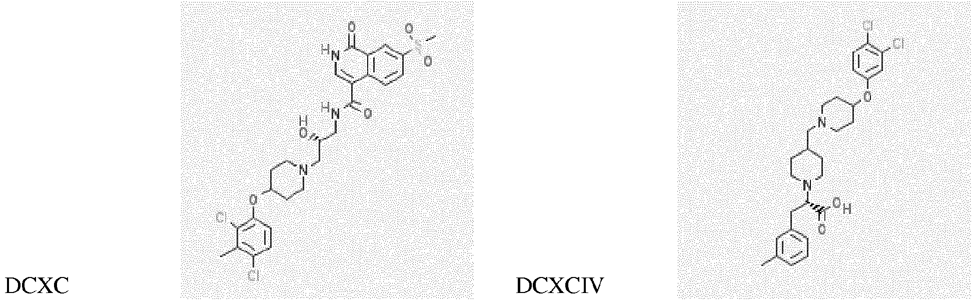

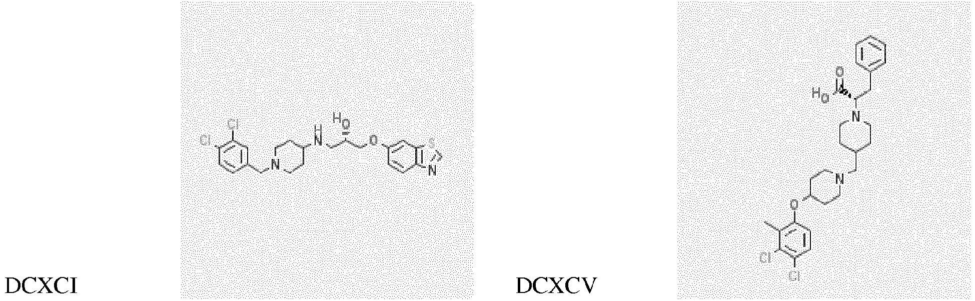

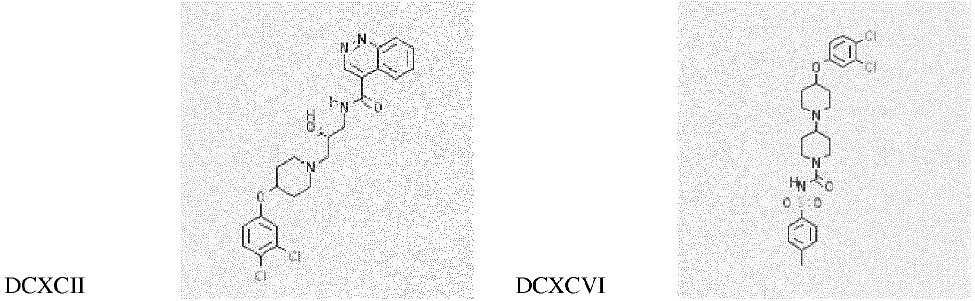

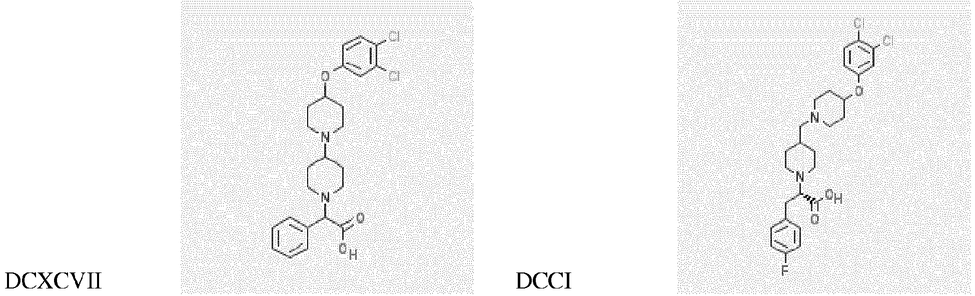

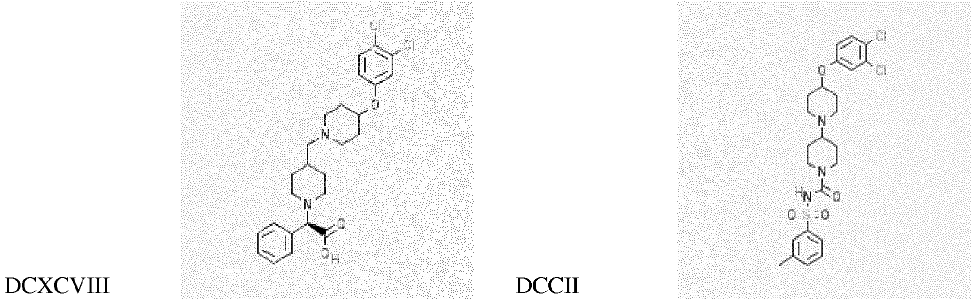

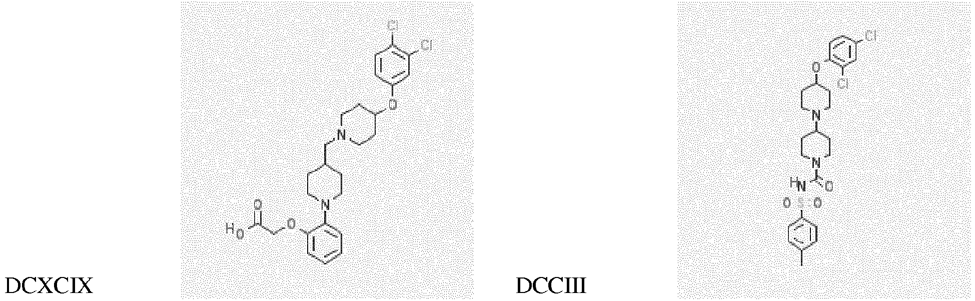

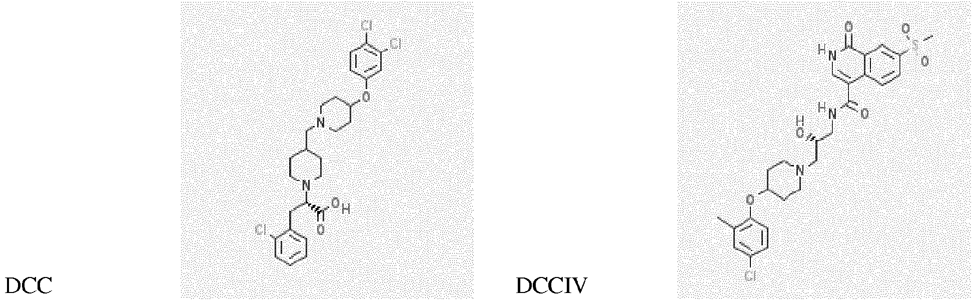

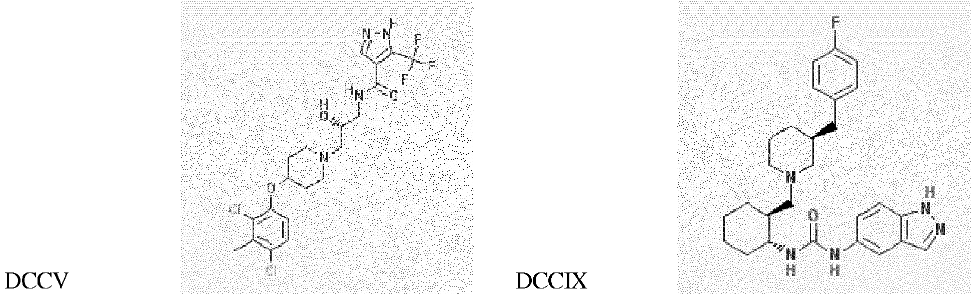

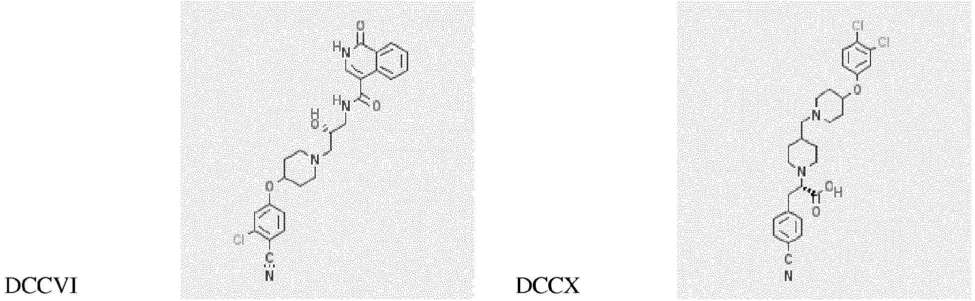

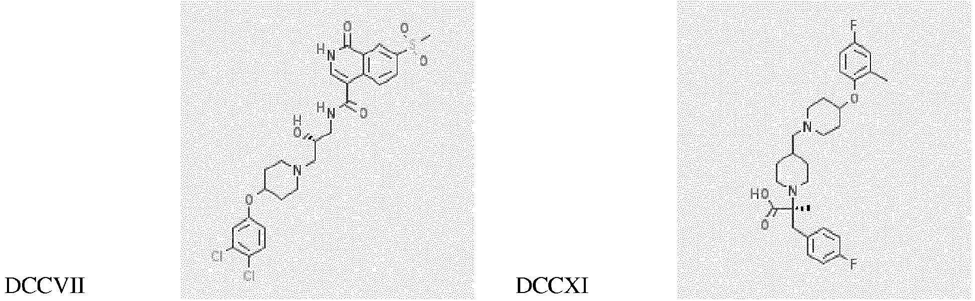

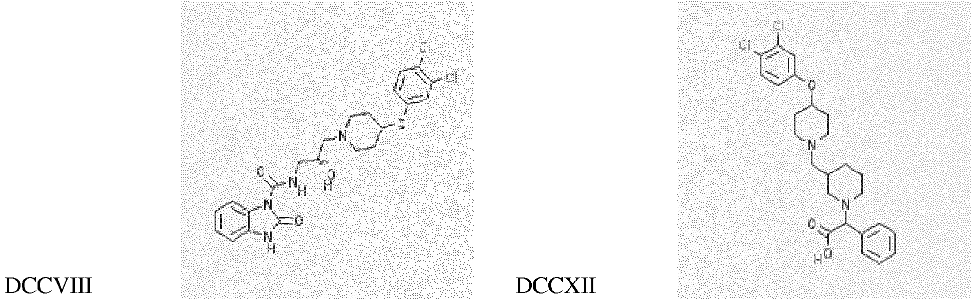

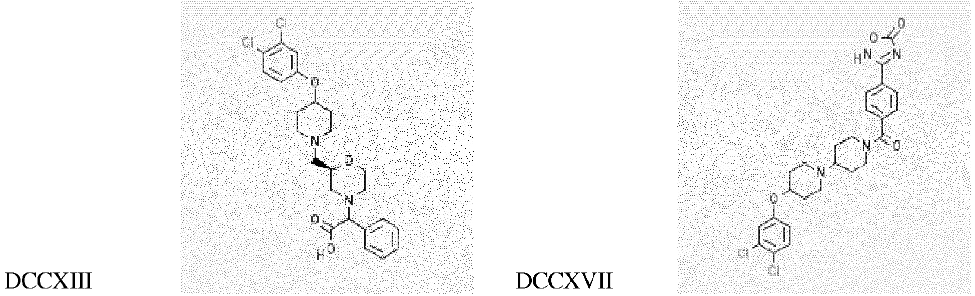

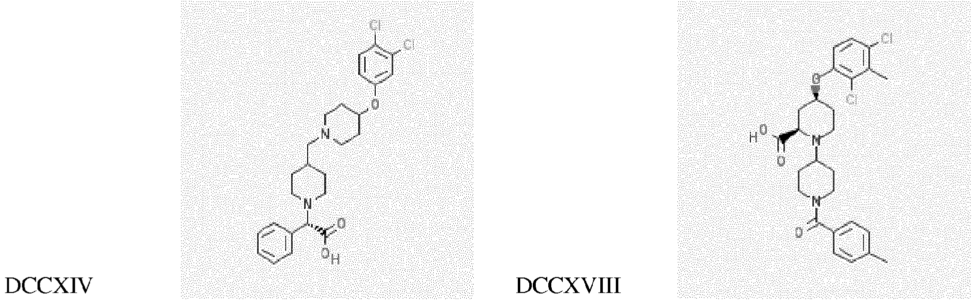

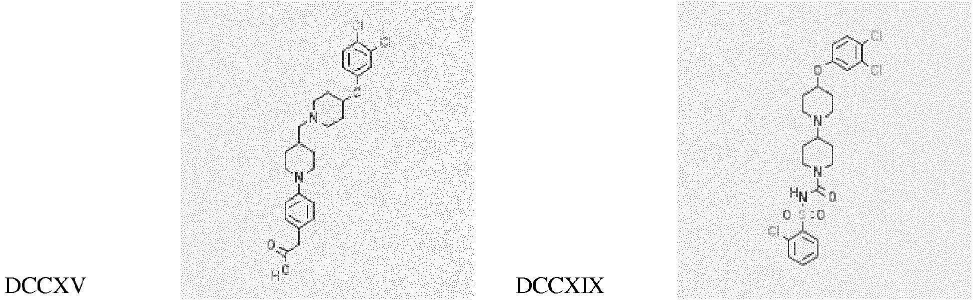

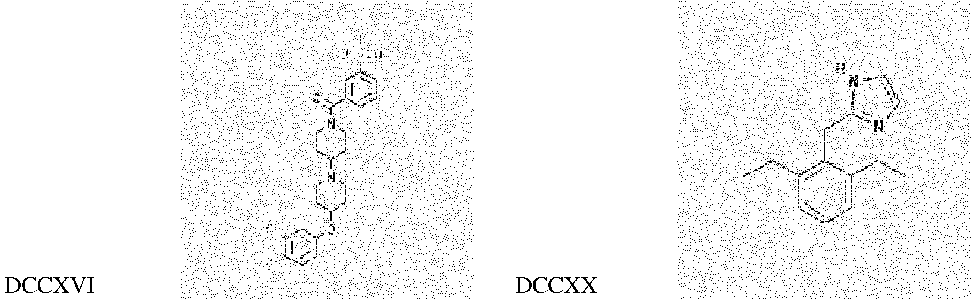

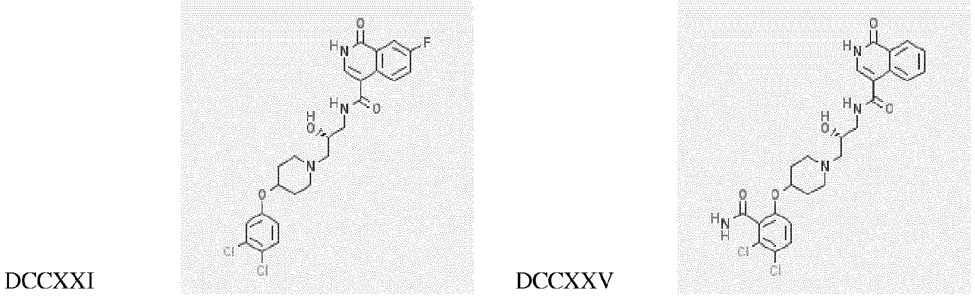

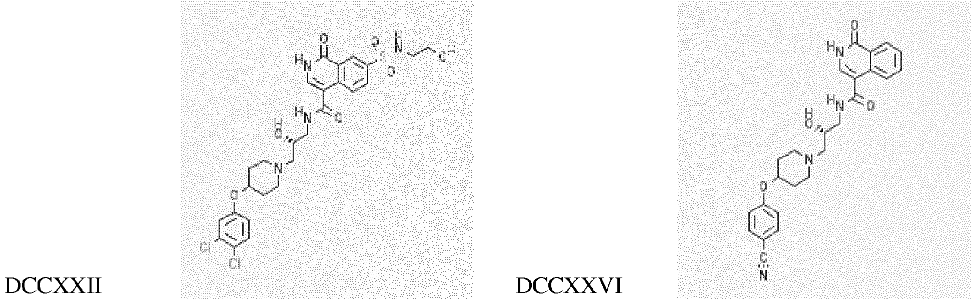

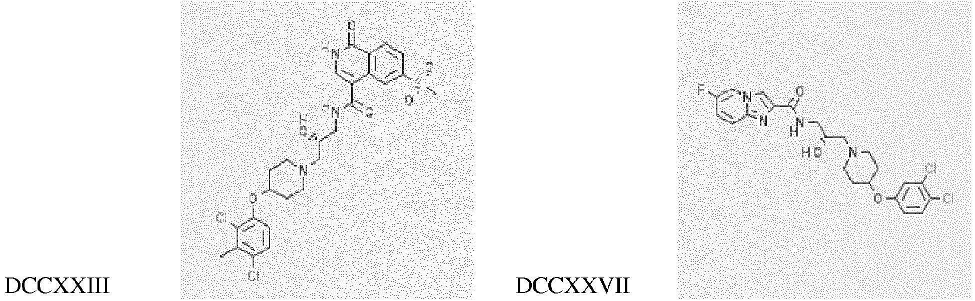

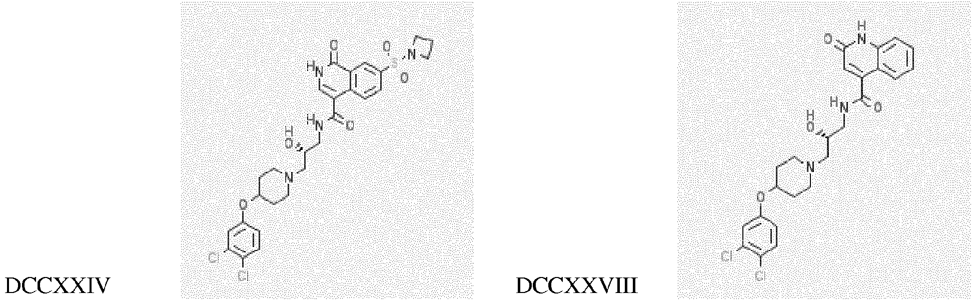

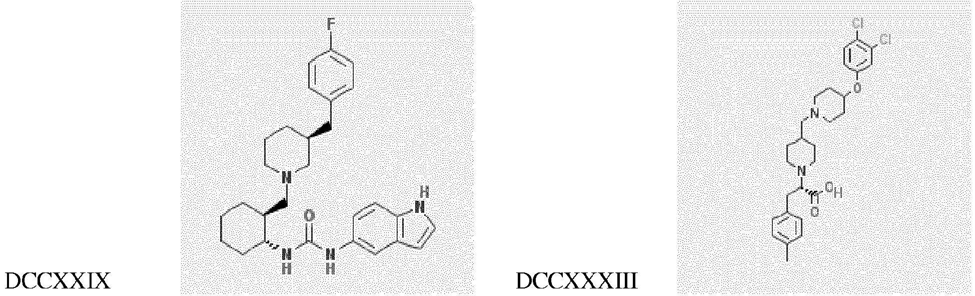

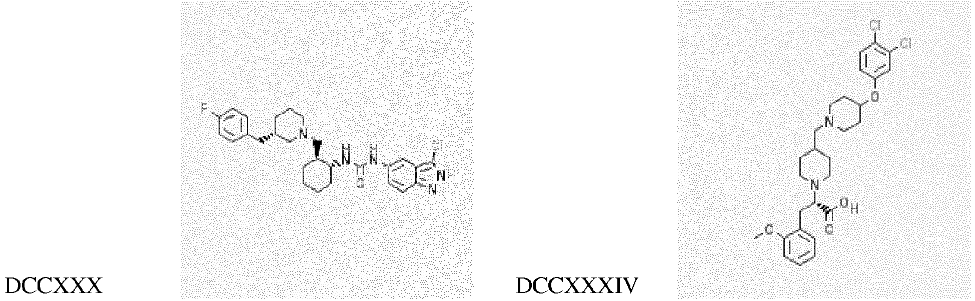

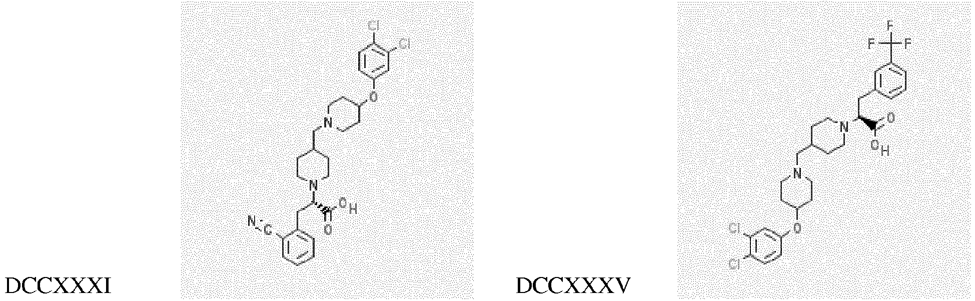

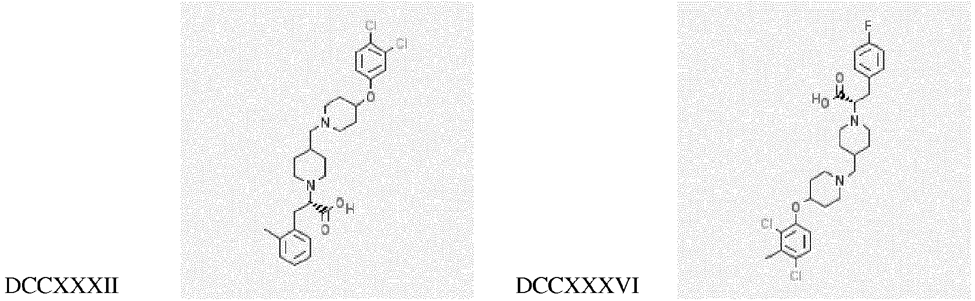

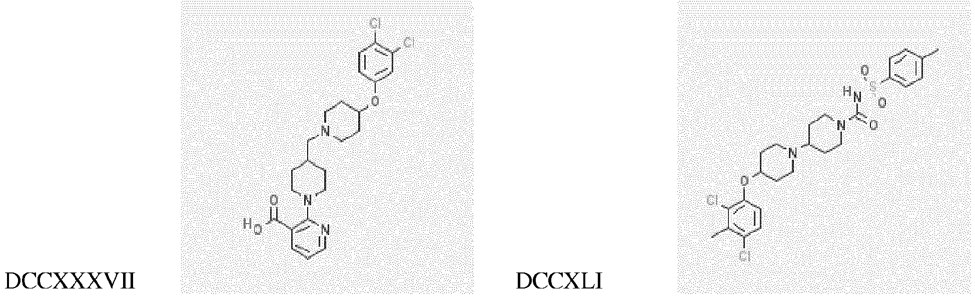

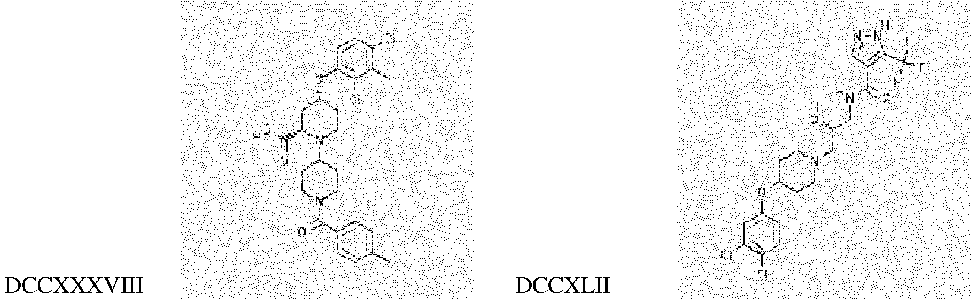

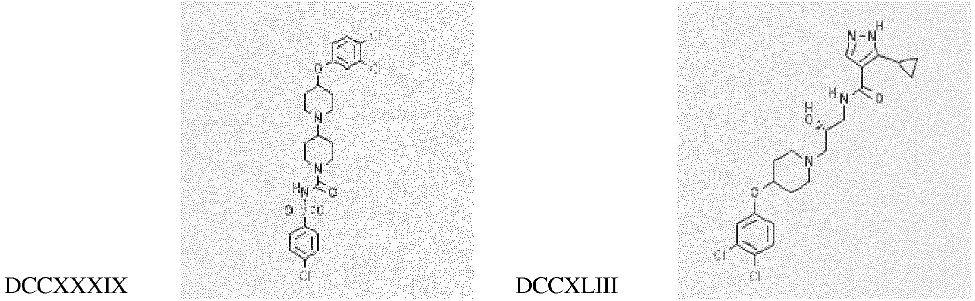

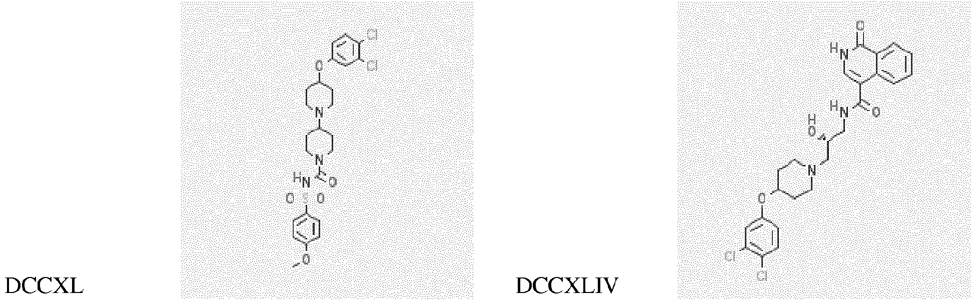

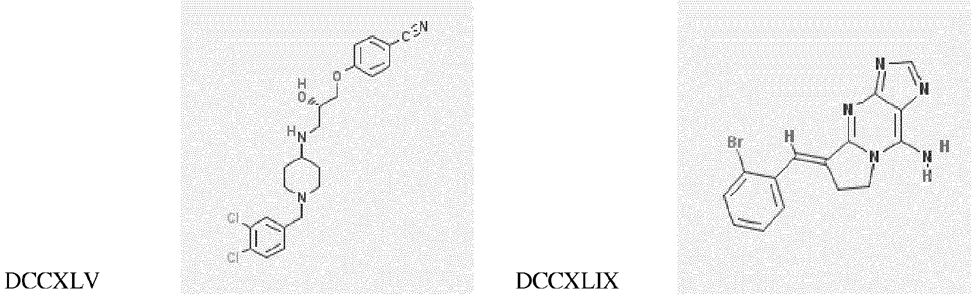

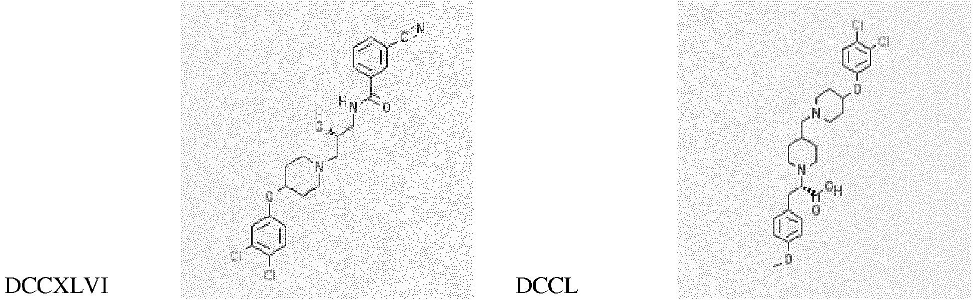

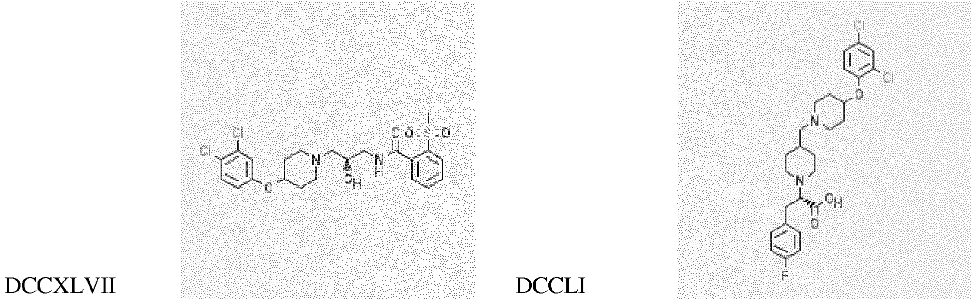

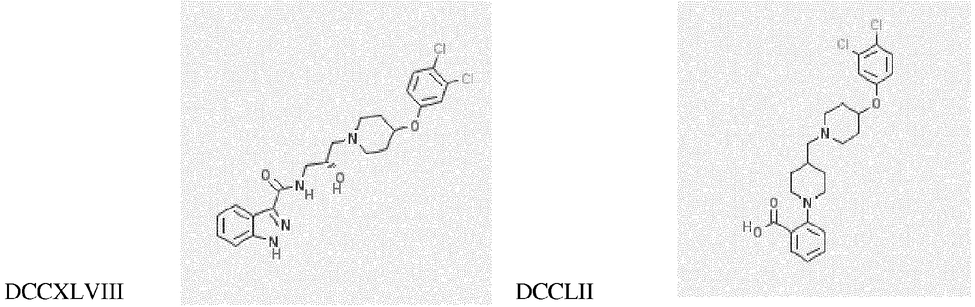

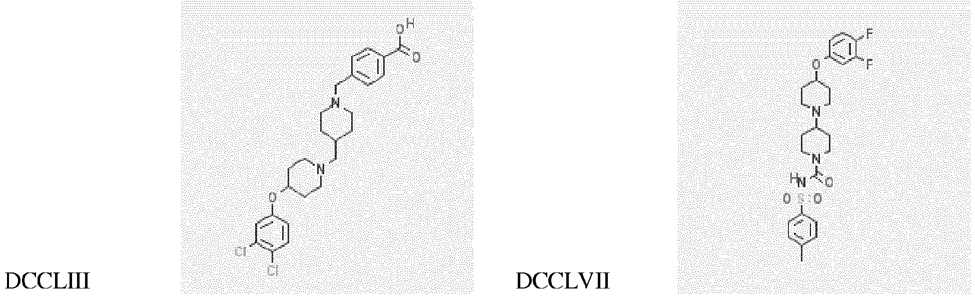

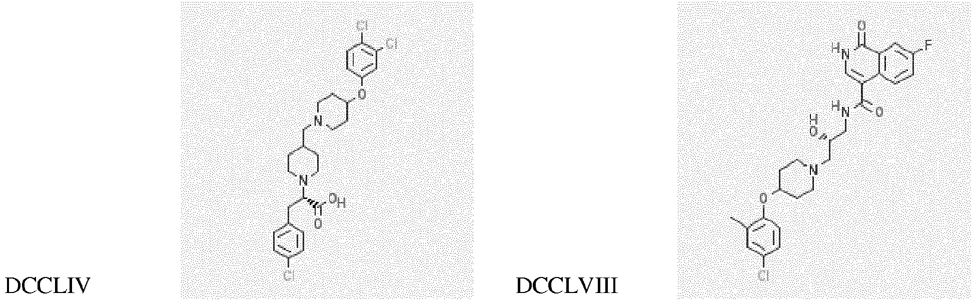

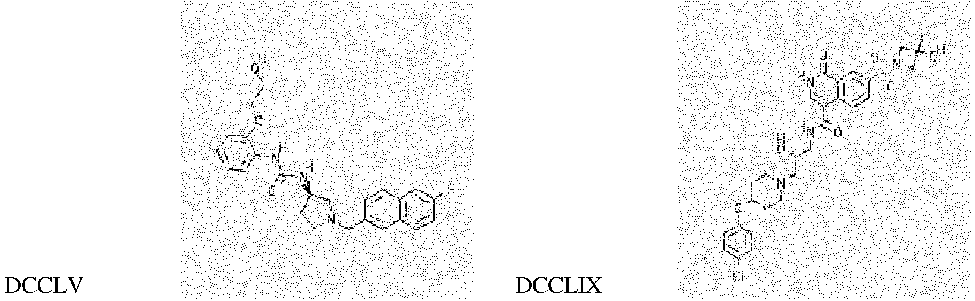

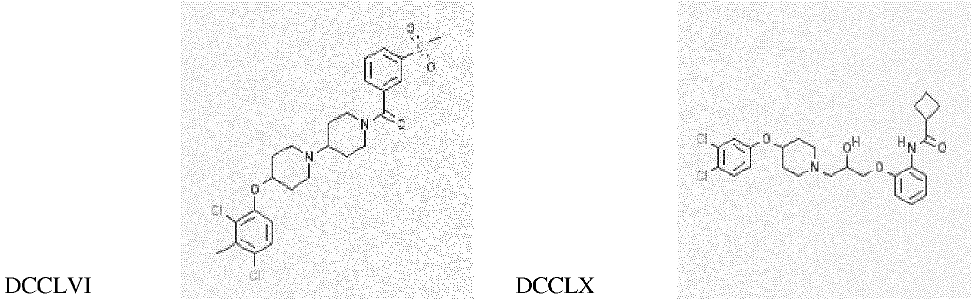

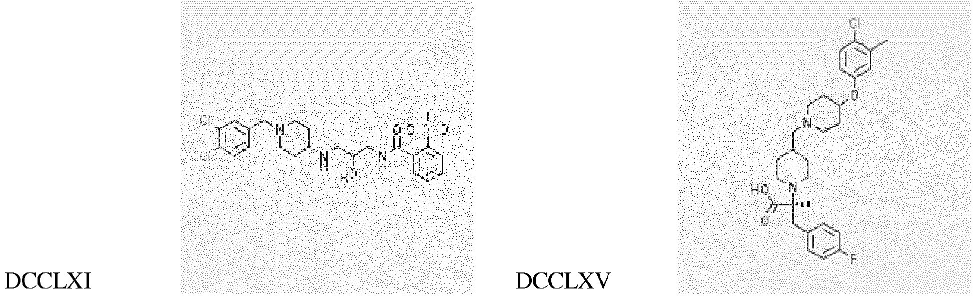

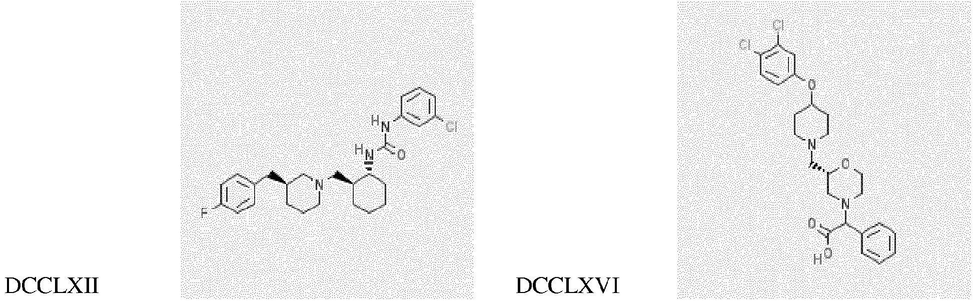

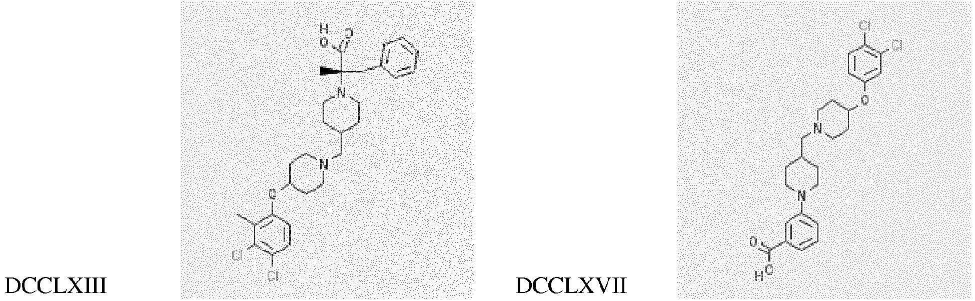

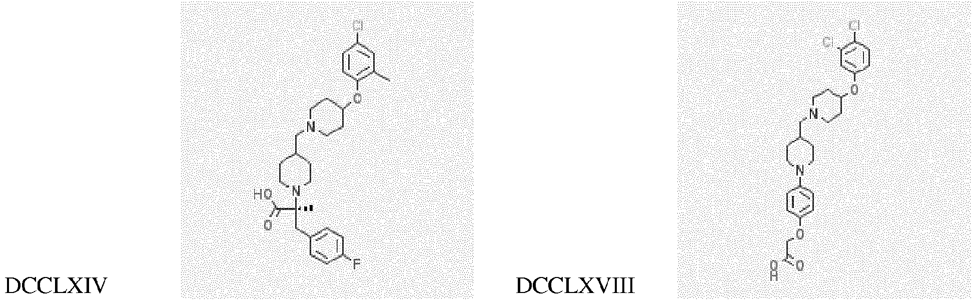

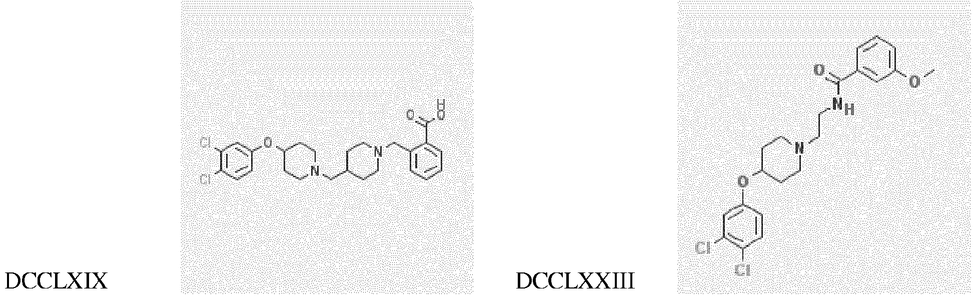

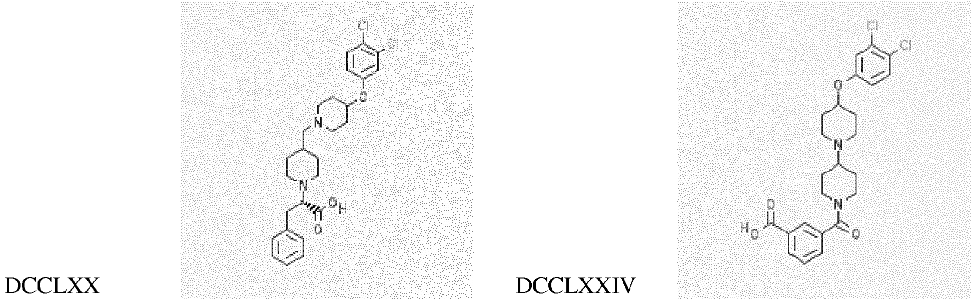

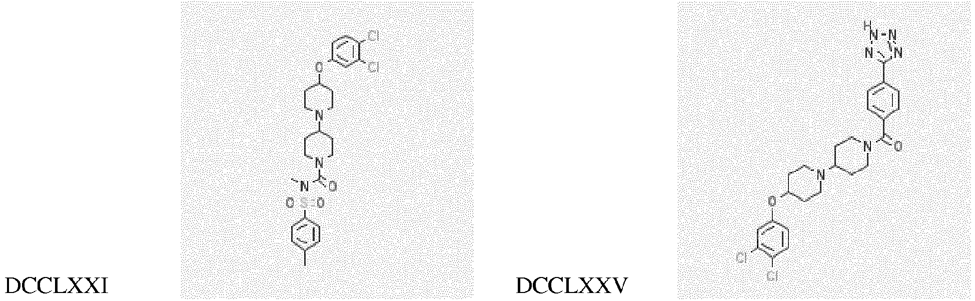

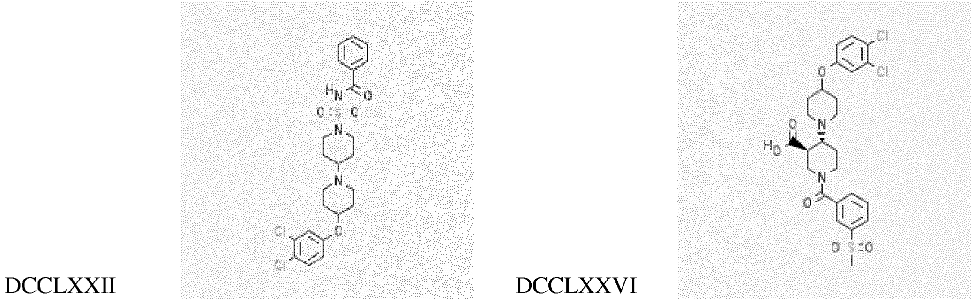

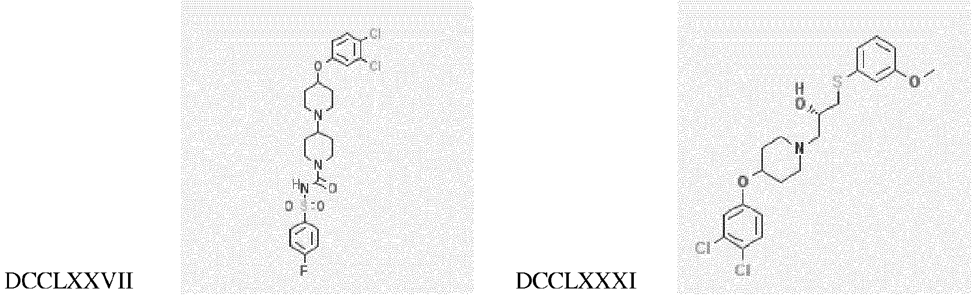

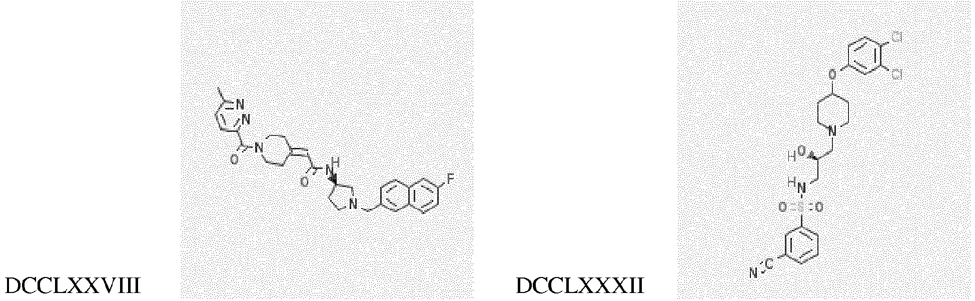

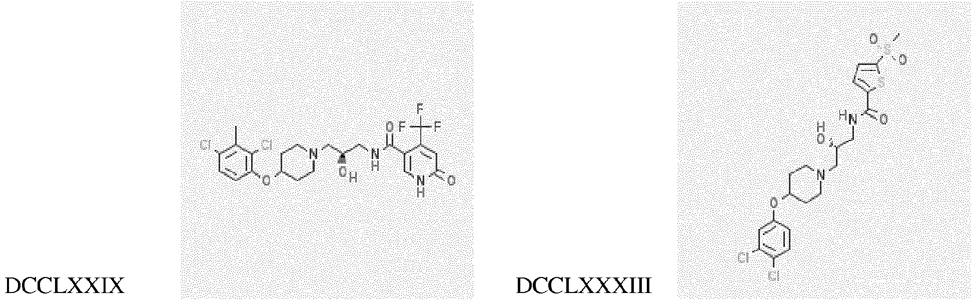

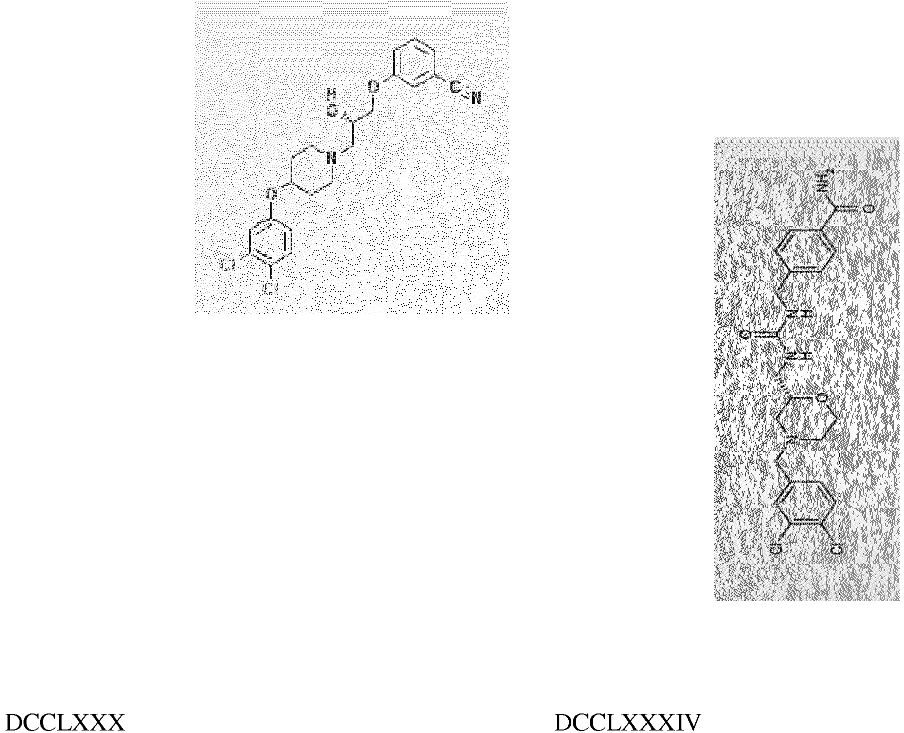

A titre d'exemples de molécules organiques inhibitrices de l'interaction CCL7/CCR3 on peut également citer les inhibiteurs irréversibles de CCR3 tels que les composés morpholine-acétamide et morpholine urée, en particulier ceux décrits dans les Demandes WO 2002/26723 et WO 03/082293, et les Brevets U.S. Nos. 7,101,882, 7,157,457, 7,531,651 et 7,560,548.

Les inhibiteurs de l'interaction CCL7/CCR3 incluent également des peptides. A titre d'exemple de peptides inhibiteurs de l'interaction CCL7/CCR3 on peut citer le peptide met-RANTES (Kiss, Longden et al. 2009), ou d'autres peptides décrits dans Houimel and Mazzucchelli 2013 ; Elsner, Escher et al. 2004 ; Kiss, Longden et al. 2009 ; Elsner, Petering et al. 1997 ; Loetscher, Pellegrino et al. 2001 et Demande Internationale WO2000073327.

Les inhibiteurs de l'interaction CCL7/CCR3 incluent également les anticorps dirigés contre CCR3 ou CCL7, de préférence des anticorps bloquant, qui peuvent être polyclonaux ou monoclonaux.

Le terme "anticorps" englobe également des fragments fonctionnels d'anticorps, y compris des fragments d'anticorps chimériques, humanisés, à chaîne unique ou des fragments de ceux-ci (*e.g.*, des fragments Fv, Fab, Fab' et F(ab')₂). Des anticorps préférés sont ceux qui sont dirigés contre CCL7 ou CCR3. Avantageusement, l'anticorps est un anticorps monoclonal ou un fragment de celui-ci. Les méthodes pour la préparation d'anticorps monoclonaux sont bien connues de l'homme du métier. Les anticorps monoclonaux peuvent être préparés en immunisant un mammifère (par exemple une souris, un rat, un lapin, un camélidé ou l'homme) avec la chimiokine CCL7 ou le récepteur CCR3 ou des fragments de ceux-ci purifiés. A titre d'exemple d'anticorps inhibiteurs de l'interaction CCL7/CCR3 comme à titre d'exemple l'anticorps bloquant monoclonal anti-CCR3 (référence D083-3, clone 444-11, MBL international, Houimel and Mazzucchelli 2013) ou l'anticorps bloquant polyclonal anti-CCL7 (référence AF-282-NA, R&D systems, Szymczak and Deepe 2009).

Les inhibiteurs de l'interaction CCL7/CCR3 incluent également les peptidomimétiques.

Le terme "peptidomimétique" désigne une molécule qui n'est pas un peptide mais qui imite les aspects de la structure des peptides (Elsner, Petering et al. 1997). A titre d'exemple de peptidomimétiques inhibiteurs de l'interaction CCL7/CCR3 on peut citer ceux décrits dans le Brevet US 7,488,717.

Les inhibiteurs de l'interaction CCL7/CCR3 incluent également les aptamères reconnaissant CCL7 ou CCR3. Les aptamères sont des molécules à un seul brin d'acide nucléique (ADN ou ARN) qui sont sélectionnés pour leur capacité à se lier à une molécule cible.

D'autres inhibiteurs de l'interaction CCL7/CCR3 peuvent être identifiés, par exemple, par criblage d'une collection de composés candidats pour leur capacité à inhiber l'activation de CCR3 en présence de CCL7. Des méthodes de mesure de l'activation de récepteurs RCPG qui sont connues en elles-mêmes, et qui sont par exemple couramment utilisées dans des tests de criblage à haut débit, peuvent être utilisées pour évaluer l'activation de CCR3. A titre d'exemple de méthodes d'identification d'inhibiteurs de l'interaction CCL7/CCR3, on peut citer des tests *in vitro* d'interactions ligands/récepteurs (Loetscher, Pellegrino et al. 2001), des dosages fluorimétriques en temps résolus (Kuri-Harcuch and Green 1978) ou encore du criblage par résonnance plasmique de surface (Ohori, Iacono et al. 1994).

Les inhibiteurs utilisés décrits ici peuvent être administrés à un sujet seul, ou en mélange avec au moins un excipient acceptable d'un point de vue pharmaceutique qui peut être tout excipient connu de l'homme du métier. Les excipients acceptables d'un point de vue pharmaceutique varient en fonction de l'inhibiteur utilisé et du mode d'administration choisi.

Les modes et voies d'administration des inhibiteurs utilisés décrits ici peuvent être adaptés par l'homme du métier en fonction du sujet et de l'inhibiteur utilisé. A titre d'exemple, les inhibiteurs peuvent être formulés pour une administration par voie orale ou nasale, ou par injection par voie intraveineuse, intramusculaire ou sous-cutanée.

La détermination de la dose à laquelle ledit inhibiteur est utilisé selon l'invention peut s'effectuer par des techniques connues de l'homme du métier, par exemple lors d'essais cliniques. Cette dose dépendra de divers facteurs comprenant notamment l'activité de l'inhibiteur, du mode d'administration, de la durée de l'administration, de la durée du traitement, d'autres médicaments ou composés utilisés en combinaison avec l'inhibiteur, de l'âge, du sexe, du poids, de l'état de santé générale et de l'histoire médicale antérieure du sujet qui est traité.

Il est également décrit une méthode pour prévenir ou traiter l'extension du cancer de la prostate hors de la capsule prostatique, comprenant l'administration d'une quantité efficace d'au moins un inhibiteur de l'expression de la chimiokine CCL7 ou un inhibiteur de l'expression du récepteur CCR3 ou un inhibiteur de l'interaction CCL7/CCR3, à un sujet ayant besoin de ce traitement. Bien entendu, les spécifications décrites plus haut s'appliquent également à cet aspect de l'invention.

On entend par une quantité efficace, une quantité d'inhibiteur utilisée selon l'invention pour produire le résultat biologique désiré.

La présente invention a également pour objet une méthode *ex vivo* et/ou *in vitro* de détermination du degré d'agressivité d'une tumeur du cancer de la prostate chez un sujet atteint d'un cancer de la prostate, ou de détermination du risque de rechute biologique d'un cancer de la prostate chez un sujet, comprenant les étapes suivantes :
a) déterminer la concentration ou le niveau d'expression du récepteur CCR3 dans un échantillon de cellules prostatiques tumorales obtenues chez ledit sujet,
b) comparer la concentration ou le niveau d'expression du récepteur CCR3 déterminée à l'étape a) avec la concentration ou le niveau d'expression de référence du récepteur CCR3 dans l'épithelium prostatique sain,
une concentration ou un niveau d'expression du récepteur CCR3, dans ledit échantillon de cellules prostatiques tumorales dudit sujet, supérieure à ladite concentration ou dudit niveau d'expression de référence étant l'indication d'une tumeur agressive du cancer de la prostate à haut potentiel de dissémination extraprostatique ou d'un risque de rechute biologique d'un cancer de la prostate.

Un échantillon de cellules prostatiques tumorales peut être obtenu selon des méthodes connues de l'homme du métier. Par exemple, les biopsies prostatiques, dirigées ou non par un examen radiologique sont l'examen standard qui permet d'établir le diagnostic de cancer de la prostate (Ohori, Wheeler et al. 1994). Ces échantillons peuvent également être obtenus à partir de résection endoscopique de la prostate ou de pièces de prostatectomie totale (Ohori, Wheeler et al. 1994).

Un échantillon d'épithelium prostatique sain peut être obtenu selon des méthodes connues de l'homme du métier. Par exemple, il peut s'agir d'epithélium prostatique normal présent en dehors des foyers cancéreux dans les biopsies prostatiques, du tissu prostatique sain présent à côté des foyers cancéreux sur les pièces de résection endoscopique et/ou de prostatectomie totale (Ohori, Wheeler et al. 1994).

On entend par « tumeur agressive du cancer de la prostate à haut potentiel de dissémination extraprostatique » les cancers localisés dont l'évolution est marquée rapidement par une dissémination locale. Ces cancers à risque élevé de progression ont été définis par la classification d'Amico, comme des tumeurs ayant un TNM ≥ T2c (atteinte des deux lobes) ou un Score de Gleason ≥ 8 ou valeur du PSA (ng/ml) > 20.

Ledit sujet est de préférence un homme obèse et/ou présentant un TAPP abondant.

La mesure de la concentration du récepteur CCR3 dans un échantillon de cellules (cellules prostatiques ou cellules de l'épithélium prostatique) peut être effectuée en mettant en oeuvre une méthode immunologique appropriée (*e.g.*, Immunofluorescence, Immunohistochimie) au moyen d'au moins un anticorps spécifique du récepteur CCR3.

L'anticorps spécifique du récepteur CCR3 peut être polyclonal ou monoclonal. Il peut être d'origine humaine ou non humaine (par exemple murin), humanisé, chimérique. Il peut être recombinant ou synthétique. Ce peut également être un fragment d'anticorps (par exemple les fragments Fab'₂ ou Fab) comprenant un domaine de l'anticorps initial reconnaissant l'antigène cible dudit anticorps initial. Ce peut être par exemple le clone Y31, (Abcam Cambridge, MA, USA ; Lee, Kim et al. 2010).

La concentration de référence du récepteur CCR3 dans l'épithélium prostatique sain est fonction de la méthode utilisée pour la mesure de la concentration.

A titre d'exemple, la concentration de référence du récepteur CCR3 dans l'épithélium prostatique sain peut être obtenue par analyse immunohistochimique d'épithélium prostatiques. L'immunomarquage peut faire l'objet d'une évaluation semi-quantitative visuelle. Quatre niveaux d'intensité peuvent être distingués: aucun marquage (0), marquage faible (1+), marquage intermédiaire (1++) et marquage fort (1+++), comme décrit dans les publications (Ohori and Scardino 1994) et (Saitoh, Miura et al. 1994). La concentration de référence du récepteur CCR3 dans l'épithélium prostatique sain est alors compris entre zéro et 1+.

La méthode selon la présente invention peut comprendre une étape supplémentaire (c) après l'étape de comparaison (b), comprenant la classification dudit sujet atteint d'un cancer de la prostate selon le degré d'agressivité de la tumeur du cancer de la prostate, basé sur le niveau de concentration ou d'expression du récepteur CCR3.

La présente invention a également pour objet l'utilisation *ex vivo* et/ou *in vitro* d'un anticorps spécifique du récepteur CCR3, tel que défini ci-dessus, pour déterminer le degré d'agressivité d'une tumeur du cancer de la prostate chez un sujet atteint d'un cancer de la prostate.

Outre les dispositions qui précèdent, l'invention comprend encore d'autres dispositions non-limitatives, qui ressortiront des exemples expérimentaux ci-dessous, ainsi qu'aux figures annexées :

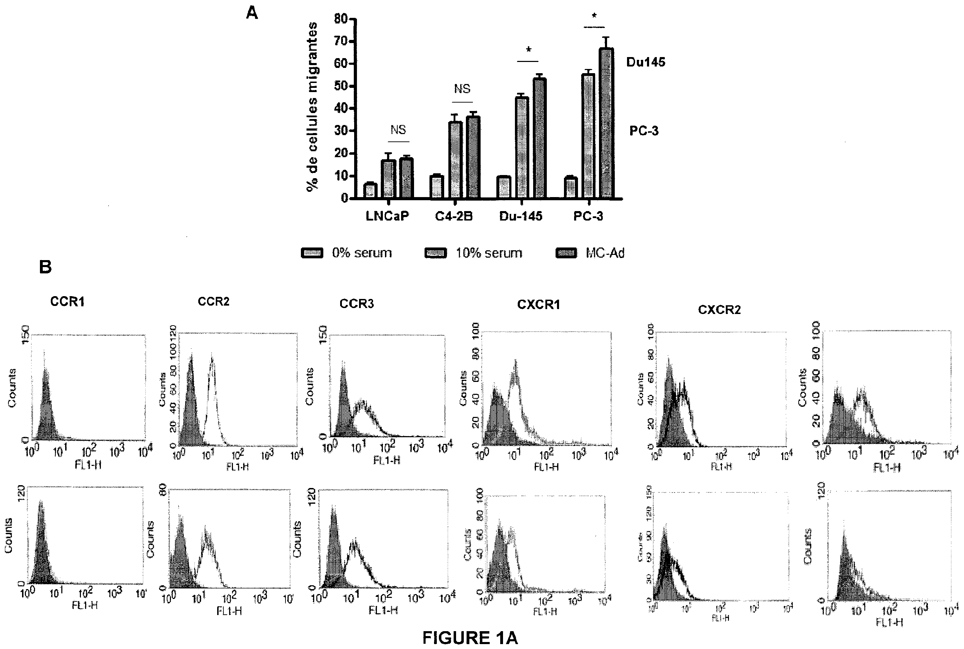

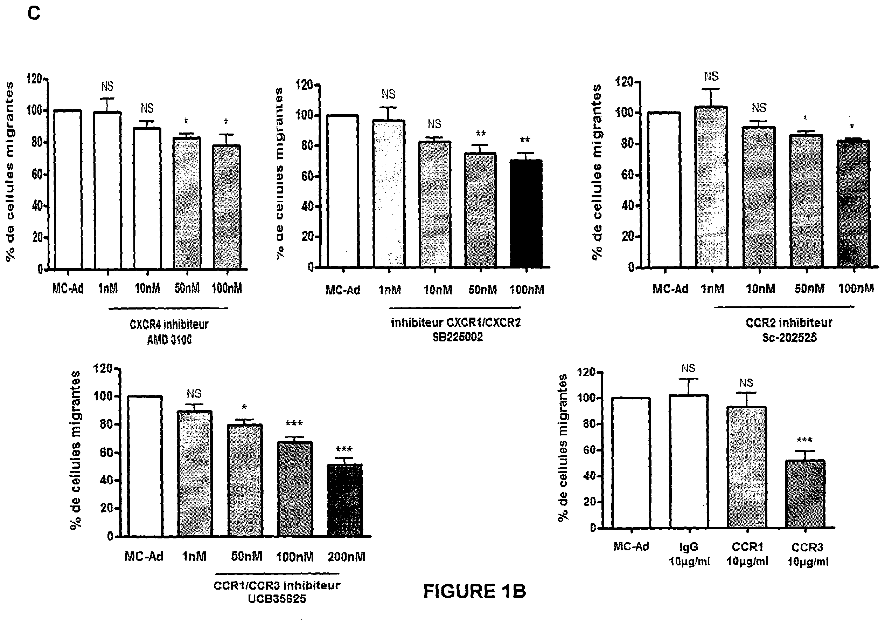

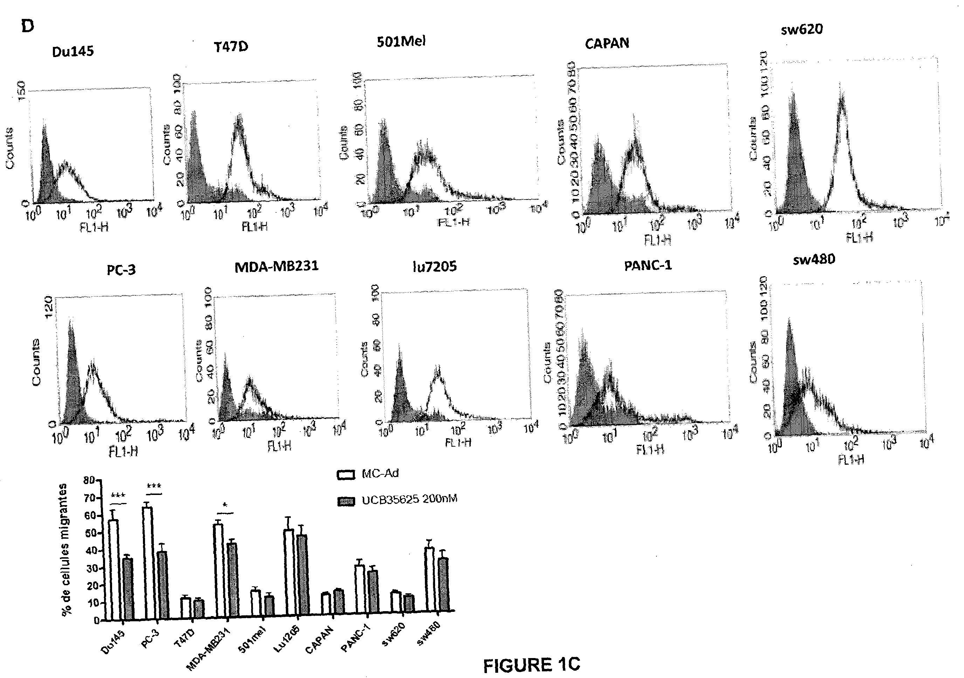

**Figure 1** **:** Identification des récepteurs aux chimiokines et rôle dans la migration des cellules tumorales prostatiques. (**A**) Migration des cellules tumorales prostatiques humaines LNCaP, C4-2B, Du-145 et PC-3 contre milieu sans sérum, contenant 10% de sérum, ou du milieu conditionné d'adipocyte mature (MC-Ad). (**B**) Expression des récepteurs aux chimiokines CXCR1, CXCR2, CXCR4, CCR1, CCR2 et CCR3 par cytométrie en flux dans les cellules Du-145 et PC-3. (C) Migration des PC-3 contre du milieu conditionné d'adipocytes (MC-Ad) en présence d'inhibiteur de CXCR4 (AMD3100), d'inhibiteur de CXCR1 et CXCR2 (SB25002), d'inhibiteur de CCR2 (SB202525), d'inhibiteur de CCR1 et CCR3 (UCB35625) et de 10µg/µl d'anticorps contrôle IgG1, anti-CCR1 ou anti-CCR3. (**D**) Expression du récepteur CCR3 par cytométrie en flux dans les cellules tumorales humaines de la prostate (Du-145 et PC-3), du sein (T47D et MDA-MB231), de mélanome (501Mel et Lu1205), du pancréas (CAPAN et PANC-1) et du colon (sw620 et sw480). Migration des cellules tumorales humaines de la prostate (Du-145 et PC-3), du sein (T47D et MDA-MB231), de mélanome (501Mel et Lu1205), du pancréas (CAPAN et PANC-1) et du colon (sw620 et sw480) contre du milieu conditionné d'adipocyte mature (MC-Ad) en présence d'inhibiteur de CCR1 et CCR3 (UCB35625).

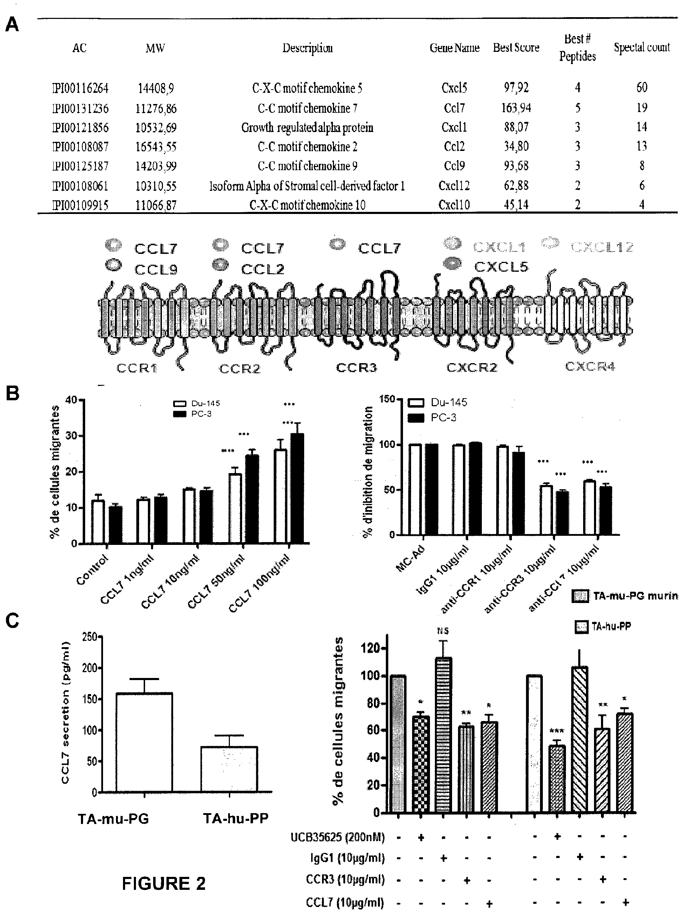

**Figure 2** **:** Identification des chimiokines adipocytaires et rôle dans la migration des cellules tumorales prostatiques. (**A**) analyse du sécrétome adipocytaire (adipocytes 3T3-F442A) par spectrométrie de masse et identification des chimiokines adipocytaires. (**B**) Migration des cellules Du-145 et PC-3 contre du milieu (sans sérum) supplémenté ou non par la chimiokine CCL7 recombinante humaine (1-100ng/ml). Migration des cellules Du-145 et PC-3 contre du Mc-Ad après traitement par des anticorps bloquants anti-CCL7, CCR1 et CCR3. (C) Analyse de la sécrétion de CCL7 dans les milieux conditionnés de tissus adipeux périgonadiques murins (TA-mu-PG) et périprostatiques humains (TA-hu-PP) par ELISA. Migration des cellules PC-3 contre du milieu conditionné de tissus adipeux périgonadiques murins (TA-mu-PG) et périprostatiques humains (TA-hu-PP) après traitement par l'inhibiteur de CCR1/CCR3 (UCB35625 à 200nM) ou par 10µg/ml d'anticorps contrôle IgG1, anti-CCR3 ou anti-CCL7.

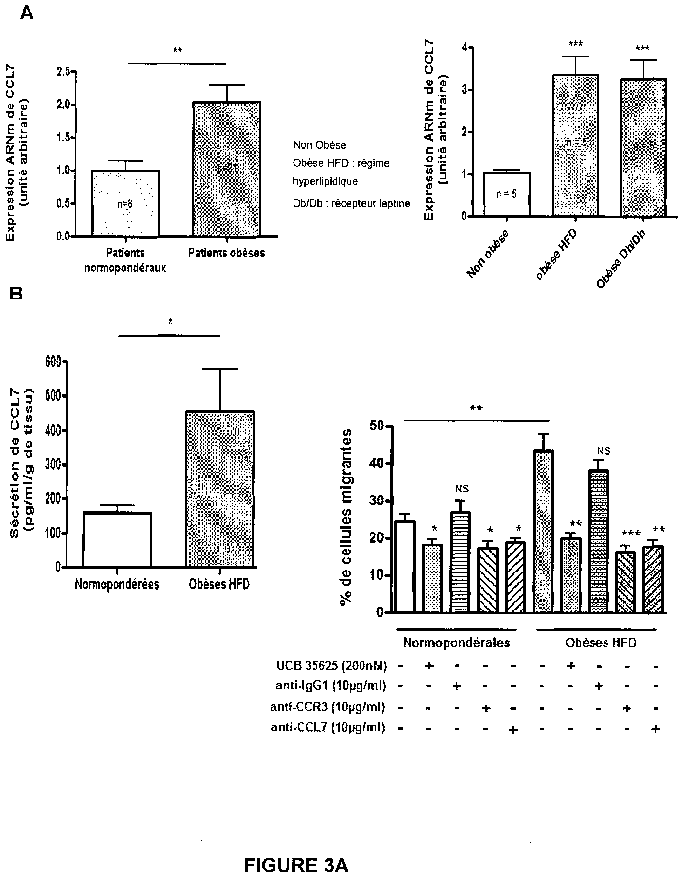

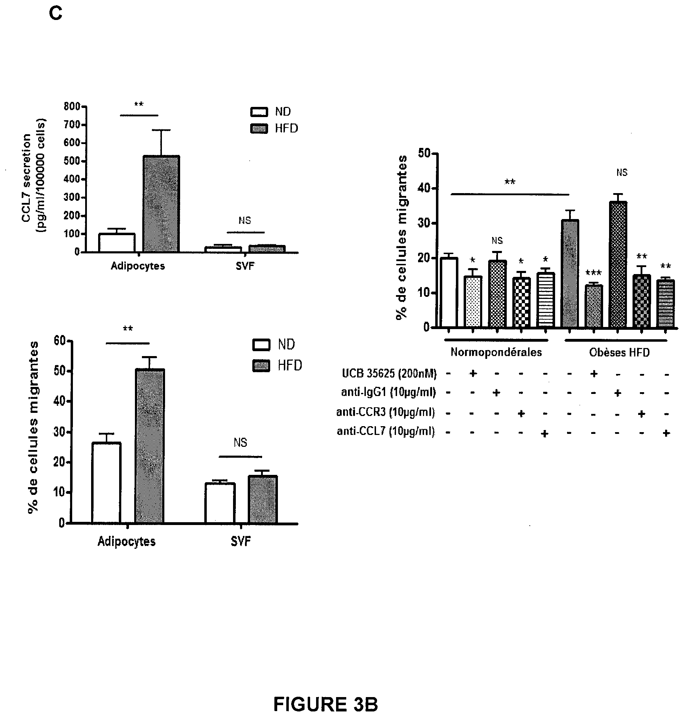

**Figure 3** : L'expression de la chimiokine CCL7 est régulée positivement dans le tissu adipeux et les adipocytes des sujets obèses. (**A**) Analyse de l'ARNm de CCL7 par qPCR dans du tissu adipeux viscéral humain de patients normopondéraux ou obèses. Analyse de l'ARNm de CCL7 par qPCR dans du tissu adipeux périgonadiques de souris normopondérales, en régime hyperlipidique ainsi que de souris obèses Db/Db. (**B**) Mesure par dosage ELISA de la quantité de CCL7 sécrété par des explants de tissus adipeux périgonadiques de souris C57BL/6 normopondérales ou obèses (régime hyperlipidique). Migration de cellules PC-3 contre le milieu conditionné d'explants de tissu adipeux de souris normopondérales ou obèses en présence ou non d'inhibiteur de CCR1/CCR3 (UCB35625 à 200nM) ou de 10µg/ml d'anticorps contrôle IgG1, anti-CCR1 ou anti-CCR3. (**C**) Mesure par dosage ELISA de la quantité de CCL7 sécrété par les adipocytes primaires ou par les cellules de la Fraction Stroma Vasculaire (SVF) isolés du tissu adipeux périgonadique de souris C57BL/6 normopondérales ou obèses (régime hyperlipidique). Migration de cellules PC-3 contre du milieu conditionné d'adipocytes primaires ou de cellules de la SVF isolés de tissus adipeux de souris obèses ou normopondérales. Migration de cellules PC-3 contre du MC-Ad des adipocytes primaires isolés en présence ou non d'inhibiteur de CCR1/CCR3 (UCB35625 à 200nM) ou de 10µg/ml d'anticorps contrôle IgG1, anti-CCR1 ou anti-CCR3.

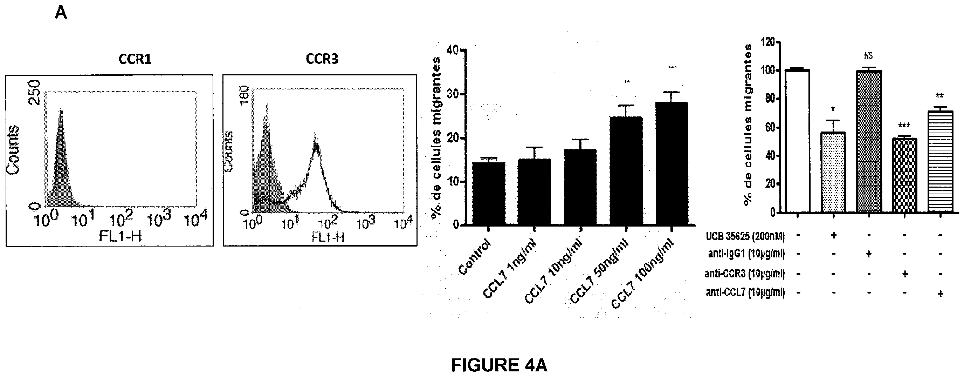

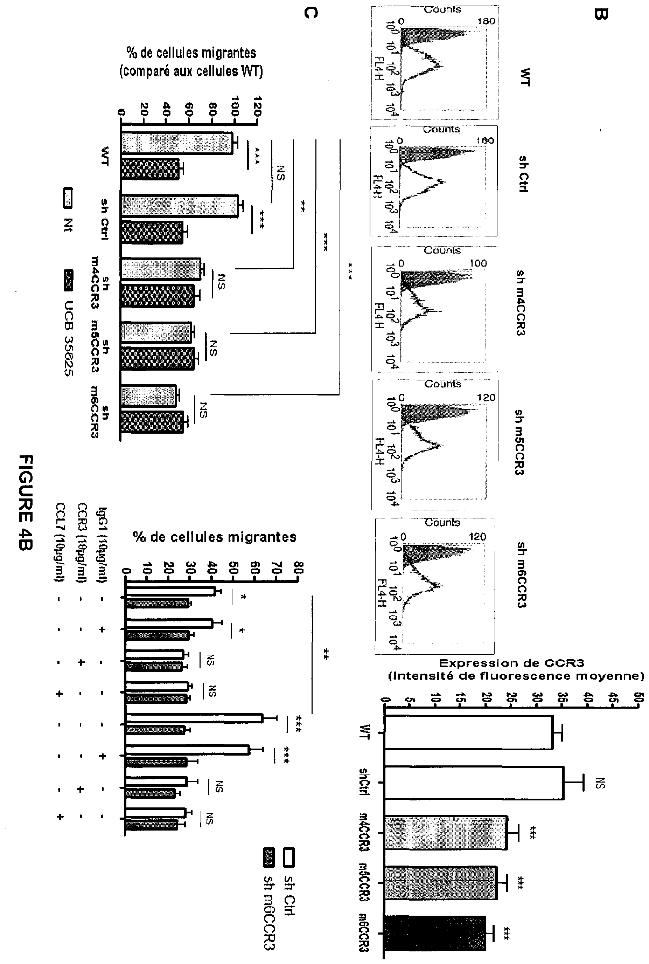

**Figure 4** **:** Implication du récepteur CCR3 dans la migration des cellules TRAMP-C1P3. (**A**) Expression des récepteurs CCR1 et CCR3 par les cellules TRAMP-C1P3 par cytométrie en flux. Migration des cellules TRAMP-C1P3 contre du milieu (sans sérum) supplémenté ou non par la chimiokine CCL7 recombinante humaine (1-100ng/ml). Migration des cellules TRAMP-C1P3 contre du Mc-Ad (3T3-F442A) après traitement par l'inhibiteur de CCR1 et CCR3 (UCB35625 à 200nM) et par 10µg/ml d'anticorps contrôle IgG1, anti-CCR3 et anti-CCL7. (**B**) Expression du récepteur CCR3 par les cellules TRAMP-C1P3 non transfectées (WT) ou transfectées avec un plasmide contrôle (sh Ctrl), ou avec 3 shRNA indépendants ciblant le récepteur CCR3 (m4, m5 et m6CCR3) par cytométrie en flux (avec quantification de l'intensité moyenne de fluorescence). (**C**) Migration des cellules TRAMP-C1P3 transfectées (shCtrl, shRNA m4CCR3, shRNA m5CCR3, shRNA m6CCR3) contre du MC-Ad (3T3-F442A) en présence ou non de l'UCB35625 (200nM). Migration des cellules TRAMP-C1P3 transfectées (shCtrl, shRNA m6CCR3) contre du milieu conditionné de tissu adipeux de souris obèses ou normopondérales en présence ou non de 10µg/µl d'anticorps contrôle IgG1, anti-CCR1 ou anti-CCR3.

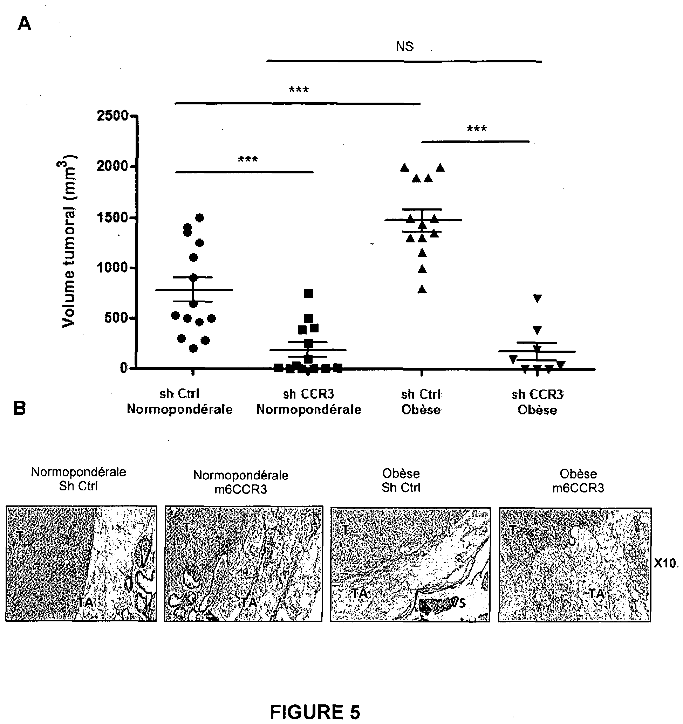

**Figure 5** **:** Implication du récepteur CCR3 dans la progression tumorale *in vivo.* (**A**) Mesure de la croissance tumorale des cellules TRAMP-C1P3 (shCtrl et shCCR3) 20 jours après injection de 2 millions de cellules en intraprostatique chez la souris C57BL/6 normopondérales et obèses. (**B**) Observations de coupes transversales de ces tumeurs après marquage à l'hématoxyline/éosine (H&E) à différents grossissement (zoom 10X).

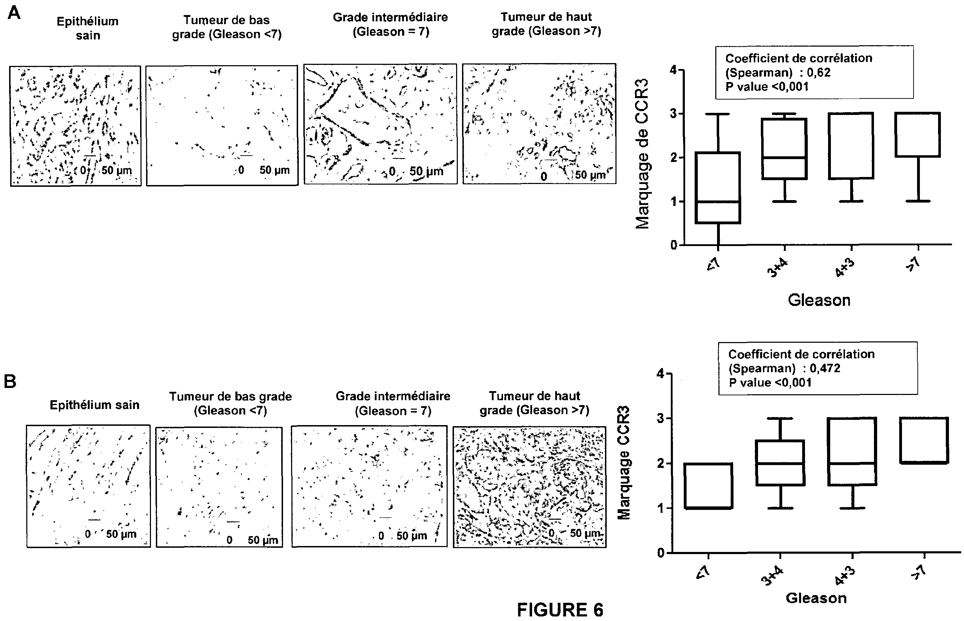

**Figure 6** **:** L'expression du récepteur CCR3 est corrélée à l'agressivité des tumeurs prostatiques humaines. Etude réalisée à partir de deux séries de TMA de cancer de la prostate, une première collection incluant 92 tumeurs (A) et une deuxième collection annotée contenant 101 tumeurs (B). L'expression de CCR3 a été mesurée par immunohistochimie et un résultat représentatif pour l'épithélium sain et chaque groupe de tumeurs : tumeur de bas grade (Gleason <7), grade intermédiaire (Gleason 3+4 et 4+3) et haut grade (Gleason >7) est montrée (A et B, panneaux de gauche). Dans les deux séries, l'expression de CCR3 est fortement corrélée aux scores de Gleason qui reflète l'agressivité du cancer de la prostate. Pour chaque cohorte analysée figure une représentation sous forme de quartile ainsi qu'un test de corrélation (test de Spearman).

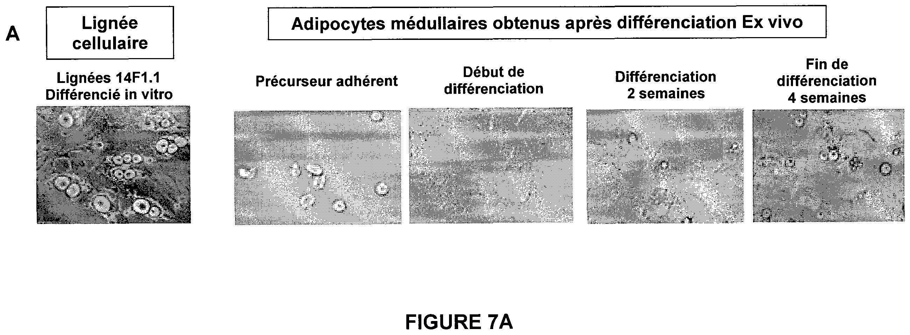

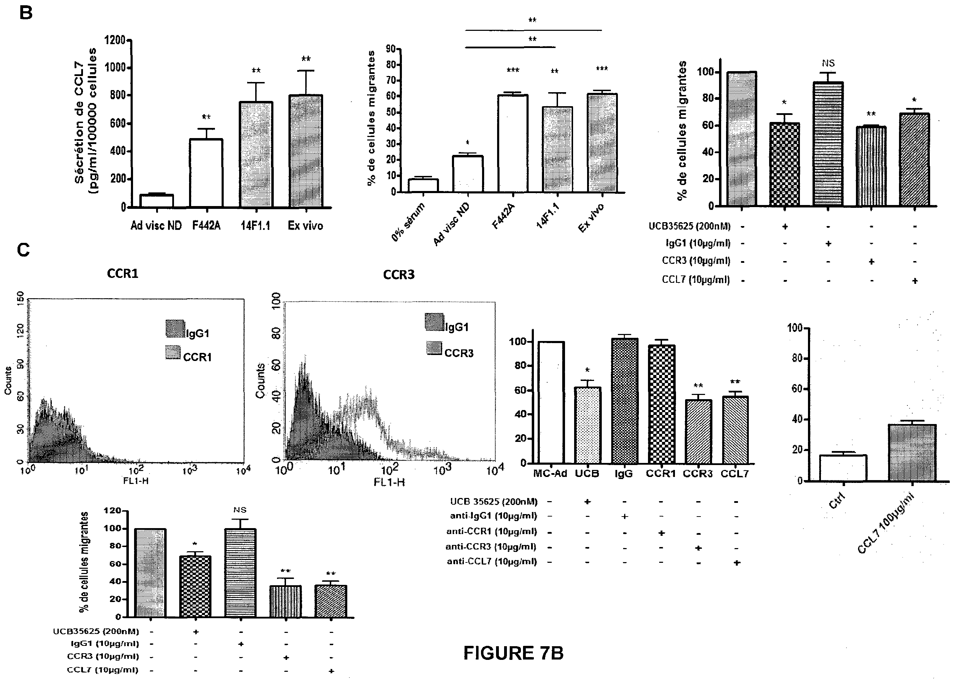

**Figure 7** **:** Rôle de l'axe CCR3/CCL7 dans la migration des cellules tumorales prostatiques en réponse aux sécrétions des adipocytes médullaires murins (contexte de métastase osseuse). **(A)** Mise au point de deux modèles d'adipocytes médullaires : un modèle *in vitro* avec la lignée 14F1.1 et un modèle *ex vivo* issu de la différenciation de cellules progénitrices médullaires mésenchymateuses murines. Au cours du traitement par le milieu de sélection et de différenciation ces cellules changent d'aspect et se chargent en lipides jusqu'à obtenir des adipocytes médullaires après 6 semaines de différenciation *ex vivo.* **(B)** Comparaison de la sécrétion de CCL7 entre des adipocytes primaires viscéraux de souris C57B16, des adipocytes 3T3-F442A différenciés *in vitro,* des cellules 14F1.1 différenciés *in vitro* et des adipocytes médullaires murins différenciés *ex vivo.* Migration des cellules PC-3 contre du milieu conditionné d'adipocytes issues des différents modèles : F442A différenciés, 14F1.1 différenciés et adipocytes médullaires murins matures (*ex vivo*). Migration des PC-3 contre du milieu conditionné d'adipocytes issu d'adipocytes médullaires murins matures (*ex vivo*) en présence ou non d'inhibiteur de CCR1/CCR3 (UCB35625 à 200nM) ou de 10µg/µl d'anticorps contrôle IgG1, anti-CCR1 ou anti-CCR3. **(C)** Expression des récepteurs aux chimiokines CCR1 et CCR3 par cytométrie en flux dans les cellules RM1-BM. Migration des RM1-BM contre du MC-Ad de F442A en présence ou non d'inhibiteur de CCR1/CCR3 (UCB35625 à 200nM) ou de 10µg/µl d'anticorps contrôle IgG1, anti-CCR1 ou anti-CCR3. Migration de cellules RM1-BM contre du milieu sans sérum complémenté ou non par de la chimiokine recombinante CCL7 (100µg/ml). Migration des RM1-BM contre du milieu conditionné d'adipocytes médullaires murins différenciés *ex vivo* (*ex vivo*) en présence ou non d'inhibiteur de CCR1/CCR3 (UCB35625 à 200nM) ou de 10µg/µl d'anticorps contrôle IgG1, anti-CCR1 ou anti-CCR3.

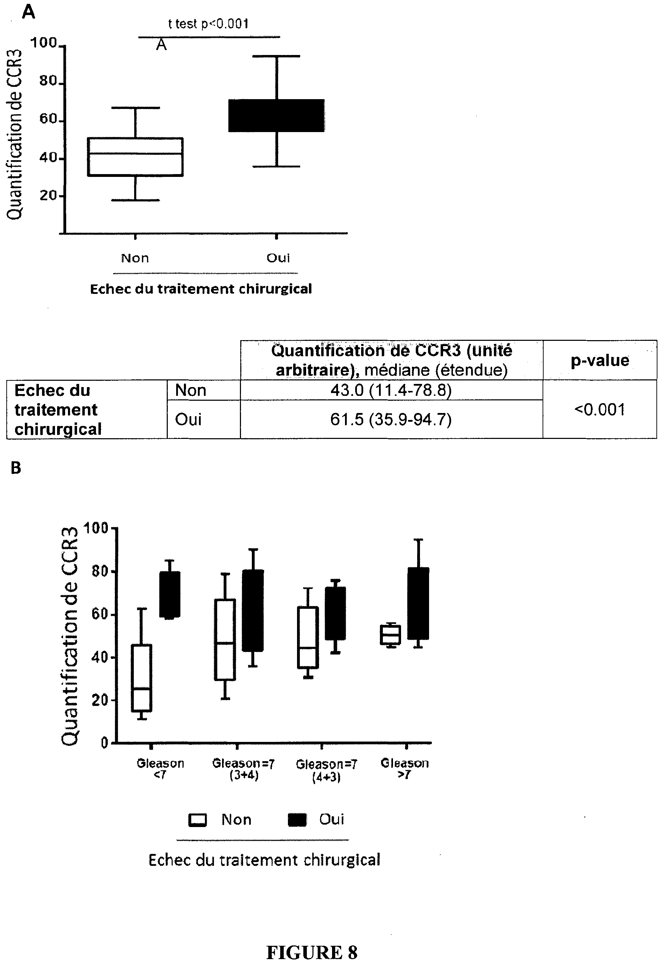

**Figure 8** **:** L'expression de CCR3 est plus élevée dans les tumeurs de patients présentant un échec du traitement par chirurgie quel que soit le score de Gleason. **(A)** Diagramme de Scatchard comparant les valeurs d'expression de CCR3 dans des tumeurs issues de patients présentant ou non un échec du traitement par chirurgie. Nous avons utilisé un test de Student pour corréler l'expression de CCR3 avec la survenue ou non d'un échec du traitement par chirurgie (qui est une variable non ordonnée représentant une catégorie). Les valeurs médianes de l'expression de CCR3 pour les deux catégories sont montrées. **(B)** Diagramme de Scatchard comparant les valeurs d'expression de CCR3, après un an de suivi, avec les 4 classes du score de Gleason. Les valeurs de CCR3 sont systématiquement plus élevées chez les patients présentant un échec du traitement par chirurgie indépendamment de la classe du score de Gleason. L'écart tend à être plus grand pour des tumeurs présentant un score de Gleason bas (<7).

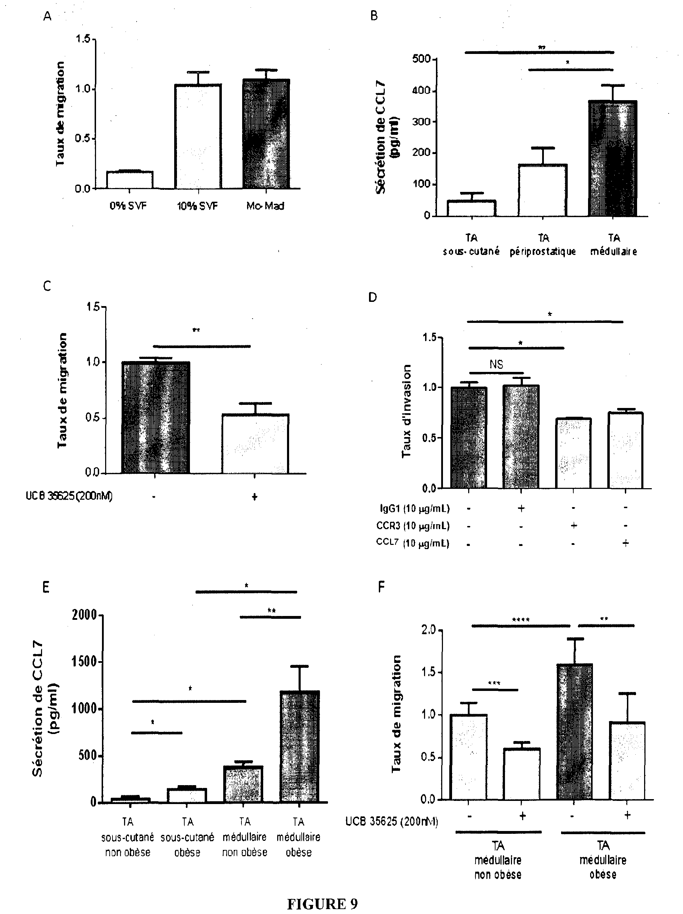

**Figure 9** **:** Les adipocytes médullaires humains possèdent la capacité de chemottracter les cellules tumorales prostatiques humaines, cet effet est dépendant de l'axe CCR3/CCL7 et est régulée par l'obésité. **(A)** Migration des cellules tumorales prostatiques humaines PC-3 contre milieu sans sérum, contenant 10% de sérum, ou du milieu conditionné d'adipocytes médullaires humains obtenu à partir d'échantillons de moelle jaune (MC-MAd) . **(B)** Analyse de la sécrétion de CCL7 dans les milieux conditionnés de tissus adipeux sous-cutané, périprostatique et médullaires humains par ELISA (1g de tissu dans 8ml de milieu durant 24h). **(C)** Migration des cellules PC-3 contre du milieu conditionné d'adipocytes médullaires humains obtenu à partir d'échantillons de moelle jaune (MC-MAd) en présence de l'inhibiteur de CCR1/CCR3 (UCB35625 à 200nM). **(D)** Migration des cellules PC-3 contre du milieu conditionné d'adipocytes médullaires humains obtenu à partir d'échantillons de moelle jaune en présence d'IgG1 et d'anticorps bloquants dirigés contre CCR3 et CCL7 **(E)** Analyse de la sécrétion de CCL7 dans les milieux conditionnés de tissus adipeux sous-cutané et médullaires humains obtenus chez des patients non obèses (IMC < 25mg/m²) ou obèses (IMC> 30 mg/m2) par ELISA (1g de tissu dans 8ml de milieu durant 24h). **(F)** Migration des cellules PC-3 contre du milieu conditionné d'adipocytes médullaires humains obtenu à partir d'échantillons de moelle jaune (MC-MAd) chez des patients obèses ou de poids normal en présence de l'inhibiteur de CCR1/CCR3 (UCB35625 à 200nM).

### EXEMPLE : INHIBITION DU RÉCEPTEUR AUX CHIMIOKINES CCR3 DANS LA DISSÉMINATION DU CANCER DE LA PROSTATE

### MATERIEL ET METHODES

### Anticorps, inhibiteurs et produit utilisés

Les anticorps monoclonaux dirigés contre CCR1 (D063-3 clone 141-2) et CCR3 (D083-3, clone 444-11) proviennent de MBL International (Worburn, MA, USA). Les anticorps monoclonaux anti-CCR2 (clone E68), anti-CCR3 (clone Y31) proviennent de la société Abcam (Cambridge, MA, USA). Les anticorps monoclonaux contre CXCR2 (clone 48311), CXCR4 (clone 44716) et CCR1 (clone 53504) proviennent de R&D Systems (Mineapolis, MN, USA).

L'inhibiteur ciblant le récepteur CXCR4, AMD3100 (Rosenkilde, Gerlach et al. 2004) provient de Sigma Aldrich et est utilisé à des concentrations finales de 1, 10, 50 et 100 nM. L'inhibiteur ciblant les récepteurs CXCR1 et CXCR2, SB225002 (White, Lee et al. 1998)) provident de chez Tocris (Bristol, Royaume-Uni) et est utilisé à des concentrations finales de 1, 10 et 100nM L'inhibiteur ciblant le récepteur CCR2, sc-202525 (Cherney, Mo et al. 2008) provient de Santa Cruz Biotechnology (Dallas, TE, USA) et est utilisé à des concentrations finales de 1, 10 et 50 et 100 nM. L'inhibiteur ciblant le récepteur CCR1 et CCR3, UCB35625 (Sabroe, Peck et al. 2000) provient de Tocris (Bristol, Royaume-Uni) et est utilisé à des concentrations finales de 1, 50, 100 et 200 nM.

### Culture cellulaire

Les lignées tumorales prostatiques LNCaP (ATCC® CRL-1740™), DU-145 (ATCC® HTB-81™), PC-3 (ATCC® CRL-1435™), ainsi que les lignées cellulaires humaines de carcinomes du colon sw480 (ATCC® CCL-228™) et sw620 (ATCC® CCL-227™), du pancréas CAPAN (ATCC® HTB-79™) et PANC-I (ATCC® CRL-2547 ™), du sein ZR-75-1 (ATCC® CRL-1500™) et MDA-MB321 (ATCC® CRM-HTB-26™) et de mélanomes SK-Mel-28 (ATCC® HTB-72™) et Lu1205 (don du Dr. Herlyn, Wistar Institute, Philadelphie, USA) sont cultivées dans du milieu de culture RPMI (Invitrogen, Auckland, NZ) supplémenté avec 10 % de SVF (Sérum de Veau Foetal), 2 mM L-glutamine, 125 µg/ml de streptomycine, 125 U/ml de pénicilline. Toutes les lignées utilisées dans cette étude sont cultivées dans une atmosphère humide à 5 % de CO₂.

La lignée tumorale murine TRAMP-C1P3 dérivée de la lignée TRAMP-C1 (ATCC® CRL-2730™) est cultivée dans du milieu DMEM supplémenté avec 10% de SVF, 125 µg/ml de streptomycine, 125 U/ml de pénicilline, 5mg/ml d'insuline et 10nM de dihydrotestostérone (18).

La lignée RM1-BM est cultivée dans du milieu DMEM (Invitrogen, Auckland, NZ) à 4,5 g/l de glucose, supplémenté avec 10 % de SVF, 125 µg/ml de streptomycine et 125 U/ml de pénicilline. La lignée RM1-BM a été fournie par le Dr. Carl Power (Eastern Virginia MedicalSchool). Il s'agit d'une lignée cellulaire syngénique des souris de fond génétique C57BL/6 et dérivée de la lignée RM1 et qui est caractérisée par sa forte capacité métastatique osseuse. En effet suite à une injection intracardiaque chez la souris C57BL/6 des métastases osseuses sont ainsi obtenues dans 86% des cas (Kanno, Amakasu et al. 1994).

Les adipocytes matures sont obtenus à partir de la différenciation *in vitro* de la lignée murine de pré-adipocytes 3T3-F442A (00070654 Sigma Aldrich) (Kuri-Harcuch and Green 1978). Ces cellules sont cultivées dans du milieu de culture DMEM (Invitrogen, Auckland, NZ) contenant 10% de SVF, 125 µg/ml de streptomycine et 125 U/ml de pénicilline (milieu DMEM complet). Pour induire la différenciation adipocytaire, 6x10⁴ cellules sont ensemencées dans une plaque 6 puits. Après 3 jours de culture, les cellules confluentes sont incubées en présence de milieu DMEM complet contenant 50 mM d'insuline (appelé milieu de différenciation des adipocytes) pour une période de 10 à 14 jours. Après 10 jours de culture dans ces conditions, 80% des cellules présentent des gouttelettes lipidiques spécifiques de la différenciation adipocytaire. Le milieu conditionné d'adipocyte (MC-Ad) (1% BSA, 0% SVF) est obtenu au cours de la culture des adipocytes murins différenciés *in vitro* dans les conditions décrites précédemment.

Au cours de cette étude il a été utilisé un modèle d'adipocytes médullaires *in vitro* : la lignée 14F1.1. Cette lignée dérive de cellules de la moelle osseuse immortalisées et sélectionnées pour leur capacité à se différencier soit en cellules endothéliales, soit en adipocytes médullaires (Yamamura, Salomon et al. 2011) et (Menzies and Salomon 2011). Ces cellules sont cultivées dans du milieu de culture DMEM (Invitrogen, Auckland, NZ) contenant 10% de SVF, 125 µg/ml de streptomycine et 125 U/ml de pénicilline (milieu DMEM complet). La différenciation adipocytaire est obtenue 28 jours après que les cellules soient entrées en confluence.

### Modèle d'adipocytes médullairesdifférenciés ex vivo

Afin d'obtenir des adipocytes médullaires *ex vivo* il a été mis au point un protocole de différenciation des cellules de la fraction stroma vasculaire de la moelle osseuse en adipocytes médullaires chez la souris C57BL/6. Afin d'isoler ces cellules, les tibias et fémurs de souris C57BL/6 sont isolés puis sont placés dans du DMEM (1% BSA) à 4°C. Les os sont ensuite coupés aux deux extrémités puis, à l'aide d'une seringue, les cellules contenues dans la moelle sont récupérés par injection de milieu froid à l'intérieur de l'os. La solution cellulaire ainsi obtenue est traitée par une solution de libérase à 2,5 µg/mL à raison de 5µL pour 2 mL de suspension cellulaire. Le mélange est laissé à incuber au bain marie à 37°C pendant 5 minutes. Cette étape permet d'éliminer toutes traces de matrice extracellulaire. La libérase est ensuite neutralisée par un ajout volume à volume de DMEM (10% SVF), puis la solution cellulaire est filtrée pour éliminer les débris et les caillots sanguins. La suspension cellulaire est centrifugée à 2000 RPM pendant 20 minutes et le culot contenant les cellules de la fraction stromale est resuspendu dans du milieu DMEM contenant 10% de SVF, 125 µg/ml de streptomycine et 125 U/ml de pénicilline. Les cellules sont ensemencées dans des plaques 24 puits à une densité de 1-2x 10⁷ cellules/mL et mis en culture à 37°C avec 5% de CO₂. Les cellules sont laissées en culture de cette manière 4 jours puis le milieu de culture est changé pour ne conserver que les cellules ayant adhérées. Une fois les cellules arrivées à confluence, le milieu est remplacé par un milieu de différenciation. Ce milieu est composé de DMEM (10% SVF) complémenté avec de la dexaméthasone à 1µM, de l'IBMX 0,45mM, de l'indométacine à 60µM et de l'insuline à 10µg/mL. Le milieu est changé tous les 4 jours durant 30 à 35 jours. Au bout de cette période les précurseurs mésenchymateux se sont différenciés en adipocytes médullaires et contiennent des gouttelettes lipidiques visibles en microscopie. Le milieu conditionné d'adipocyte médullaires *ex vivo* (MC-Ad méd) (1% BSA, 0% SVF) est obtenu de la manière décrite précédemment.

### Echantillons d'adipocytes médullaires humains

Les échantillons de moelles osseuses jaunes humaines et de tissus adipeux sous-cutanés humains ont été collectés lors de la pose de prothèse de hanche et de genoux réalisées dans le service de Traumatologie de l'Hôpital Pierre Paul Riquet (CHU de Toulouse, France). Ce recueil d'échantillon a été réalisé en accord avec les recommandations du comité d'éthique et de protection des personnes de Midi-Pyrénées (France). La non-opposition au prélèvement a été recueillie pour chaque patient, au préalable de l'opération, en accord avec l'article L. 1211-2 du Code de la Santé Publique. Ces tissu adipeux sont immédiatement placés à 37°C dans du milieu DMEM (sans sérum) supplémenté par 1% d'Albumine de Sérum Bovin, à raison de 1g de tissu pour 8mL de milieu. Les tissus sont laissés à sécréter durant 24 heures puis le milieu contenant les sécrétions est récupéré et congelé.

### Echantillons de tissus adipeux viscéraux humains pour analyse de l'expression de CCL7 par Q-PCR

Les tissus adipeux viscéraux (TA-Visc) humains ont été collectés selon les directives du Comité d'éthique des hopitaux Toulouse-Rangueil et Nancy-J. d'Arc (France). Tous les patients ont donné leur consentement éclairé pour participer à l'étude et les recherches ont été effectuées en conformité avec la déclaration d'Helsinki révisée en 2000 (http://www.wma.net/e/policy/b3.htm). Les échantillons humains de TA-Visc (intra-abdominal) de sujets normopondéraux ont été obtenus à partir de 8 patients (âgés de 40,3 ± 2,2 ans et d'indice de masse corporelle (IMC) de 23,3 ± 1,2 kg/m2) ayant subi une intervention chirurgicale intra-abdominale. Les échantillons de TA-Visc de sujets obèses ont été obtenus à partir de 21 patients souffrant d'obésité morbide de grade III (âgés de 41,3 ± 2,2 ans et d'IMC de 45,7 ± 2,9 kg/m2) avant une chirurgie bariatrique. Les échantillons de tissus ont été immédiatement congelés dans de l'azote liquide après prélèvement et stockés à -80 °C.

### Echantillons de tissus adipeux périprostatiques humains

Les échantillons de tissu adipeux périprostatique ont été collectés à partir de prostatectomie radicale en accord avec les recommandations du comité d'éthique du CHU de Rangueil (Toulouse, France). Tous les patients ont donné leur consentement éclairé pour participer à cette étude qui a été menée en conformité avec la Déclaration d'Helsinki révisée en 2000. Les échantillons de tissu adipeux périprostatiques sont prélevés à proximité de la tumeur et ne présentent pas de fibrose apparente. Ce tissu adipeux est immédiatement placé à 37°C dans du milieu DMEM (sans sérum) supplémenté par 1% d'Albumine de Sérum Bovin.

### Echantillons de moelle jaune humaine et de tissu adipeux sous-cutanés :

Les échantillons de moelles osseuses jaunes humaines et de tissus adipeux sous-cutanés humains ont été collectés lors de la pose de prothèse de hanche et de genoux réalisées dans le service de Traumatologie de l'Hôpital Pierre Paul Riquet (CHU de Toulouse, France). Ce recueil d'échantillon a été réalisé en accord avec les recommandations du comité d'éthique et de protection des personnes de Midi-Pyrénées (France). La non-opposition au prélèvement a été recueillie pour chaque patient, au préalable de l'opération, en accord avec l'article L. 1211-2 du Code de la Santé Publique. Ces tissu adipeux sont immédiatement placés à 37°C dans du milieu DMEM (sans sérum) supplémenté par 1% d'Albumine de Sérum Bovin, à raison de 1g de tissu pour 8mL de milieu. Les tissus sont laissés à sécréter durant 24 heures puis le milieu contenant les sécrétions est récupéré et congelé.

### Echantillons de tissus adipeux périgonadiques murins

Il a été utilisé des souris males C57BL/6 provenant de chez Janvier (Le Genest St-Isle, France) âgées de 8 semaines et hébergées dans des cages Plexiglas ventilées (3 souris par cages). Ces souris C57BL/6 sont soit placées sous un régime normal constitué de 5% de lipides (PicoLab Rodent Diet 20; Purina Mills Inc., Brentwood, Missouri, USA), ou, sous régime hyperlipidique (60% de lipides, 22% de glucides et 18% protéines) durant 10 semaines. L'ensemble des procédures a été validé par le comité d'éthique animal Midi-Pyrénées (France). A la fin du régime, les animaux sont sacrifiés par dislocation cervical, pesées puis le tissu adipeux périgonadique (TA-PG) est prélevé et immédiatement place à 37°C dans du milieu DMEM (sans sérum) supplémenté par 1% d'Albumine de Sérum Bovin.

### Mesure d'expression génique par Q-PCR

Les ARN totaux sont extraits à l'aide du kit RNeasy® Mini (Qiagen GmbH, Hilden, Germany). Un microgramme d'ARN total est transcrit en ADNc par la reverse transcriptase Superscript II (Invitrogen, Auckland, NZ). L'incubation est réalisée en parallèle dans les mêmes conditions pour chaque échantillon en l'absence de Superscript II pour s'assurer qu'il n'y ait pas de contamination par de l'ADN génomique. La réaction de PCR en temps réel est réalisée avec 50 ng d'ADNc, 5 µl d'une solution d'oligonucléotides ciblant le gène murin de CCL-7 et 10 µl de SYBR Green TaqMan Universal PCR master mix (Applied Biosystems, Foster City, CA). La réaction de PCR est tout d'abord réalisée sur les gènes de mélanges (GAPDH et HPRT) afin de normaliser l'expression génique (utilisation du logiciel GeNorm). Des analyses ont ensuite porté sur l'expression de gène CCL7 (humain ou murin). Les séquences nucléotidiques des amorces et les concentrations optimales d'utilisation sont regroupées dans le tableau 1 ci-dessous.

**Tableau 1 : amorces utilisés pour la Q-PCR**

| Gène étudié | Espèce | Amorce sens (SEQ ID NO:) | Amorce anti-sens (SEQ ID NO :) | Concentration utilisée |
|---|---|---|---|---|
| CCL7 | Humain | | | 900nM |
| CCL7 | Murin | | | 900nM |
| GAPDH | Humain / Murin | | | 500nM |
| HPRT | Humain / Murin | | | 900nM |
| HPRT | Murin | | | 300nM |

### Digestion du tissu adipeux à la collagénase

Le tissu adipeux humain ou murin est digéré à la libérase (Roche Applied Science, Meylan, France) pendant environ 30 minutes à 37°C sous agitation. Après digestion la libérase est inhibée par ajout de sérum dans le milieu contenant les cellules. Les adipocytes, en raison de leur contenu lipidique, flottent tandis que les cellules de la fraction stroma vasculaire (SVF) coulent et se retrouvent dans le fond du tube. Les adipocytes primaires isolés et les cellules de la SVF sont ensuite cultivés dans du milieu DMEM (sans sérum) contenant 1% de BSA pendant 24 heures. Du milieu conditionné est prélevé après 24h de sécrétion. Les cellules sont comptées enfin d'expérience pour rapporter les différentes sécrétions au nombre de cellules.

### Dosage de CCL7 humain et murin par test ELISA

Les dosages de CCL7 humain et murin ont été réalisés à l'aide de kit ELISA provenant respectivement de sociétés Peprotech et R&D system en accord avec les protocoles fournis par ces fabricants.

### Analyse de l'expression des récepteurs aux chimiokines dans les lignées tumorales par cytométrie en flux

Les cellules tumorales (de la prostate, du sein, du côlon, du pancréas, ou de mélanome) ont été lavées trois fois dans du tampon FACS (PBS supplémenté avec 0,5% de BSA et 2% de SVF) et fixées avec une solution de paraformaldéhyde à 3,7% pendant 20 min à 4°C. Les cellules tumorales ont été mises en suspension avec des anticorps anti-CXCR4 (10µg/mL) anti-CXCR1 (10µg/mL), anti-CXCR2 (1/25), anti-CCR1 (10µg/mL), anti-CCR2 (20µg/mL), anti -CCR3 (10µg/mL) ou d'anticorps de contrôle d'isotypique anti-IgG de souris à 4 °C pendant 2 heures. Les cellules tumorales ont été incubées avec un anticorps secondaire anti IgG (dilution au 1/200) de souris couplé à l'Alexa Fluor 488 ou couplé à la Cyanine 5 (pour les cellules exprimant la GFP) pendant 30 minutes à 4 °C. Enfin, 2x10⁴ cellules ont été analysées par cytométrie en flux en utilisant un cytomètre en flux FACScan et FACScalibur (analyse sur logiciel CellQuest™ (BD-PharMingen)).

### Tests de migration en chambre de Boyden

Pour évaluer la capacité des cellules tumorales à migrer en fonction du milieu de culture, des tests de migration en chambre de Boyden ont été réalisés à l'aide d'inserts Greiner bio-one de 8 µm de porosité. Dans le compartiment inférieur est introduit soit du milieu de culture avec ou sans sérum, soit du milieu conditionné d'adipocytes F442A (MC-Ad), de tissu adipeux périprostatique humain (MC-TAPP), de tissu adipeux périgonadique murin (MC-TAPG), d'adipocytes ou de cellules de la fraction vasculaire (SVF) isolés de TAPP ou de TAPG. Ces milieux conditionnés peuvent être prétraités durant 30 minutes par l'ajout d'anticorps bloquants la chimiokine CCL7. Des expériences avec la chimiokine recombinante CCL7 ont également été réalisées, dans ce cas la chimiokine est ajoutée à du milieu de culture sans sérum pour vérifier son potentiel chimioattractant sur une lignée cellulaire. Dans la chambre supérieure 1x10⁵ cellules, préalablement déplétées durant 6 heures en sérum sont déposées dans du milieu de culture sans sérum. Pour les tests de migration en présence d'anticorps bloquants spécifiques de CCR1 ou CCR3 ou d'inhibiteurs de CCR1/CCR3, CCR2, CXCR1/CXCR2 et CXCR4, les cellules tumorales sont préalablement incubées dans un tube avec l'anticorps bloquant ou de l'inhibiteur pendant 30 min à 37°C. La migration des cellules tumorales est évaluée après 12h de migration à 37°C et 5% CO₂ par coloration au bleu de toluidine 1% supplémenté avec du borax 0,1M (Sigma, St Louis, MO, USA) et quantification de l'absorbance (mesurée à 570 nm).

### Analyse du sécrétome adipocytaire (spectrométrie de masse)

Les milieux conditionnés d'adipocytes (MC-Ad) sont prélevés après 24 heures de sécrétion (milieu sans sérum ni rouge de phénol) centrifugé et filtré pour éliminer les débris cellulaires et supplémenté en inhibiteur de protéases. La concentration de 5 ml de milieu de MC-Ad adipeux a été réalisée avec de la résine StrataClean (Agilent Technologies, Santa Clara, CA, USA) selon les instructions du fabricant.

Après réduction et alkylation de la cystéine, l'échantillon a été séparé par SDS-PAGE (12% d'acrylamide). Les protéines ont été visualisées par coloration au bleu de Coomassie et chaque piste a été coupée en 13 bandes homogènes et digéré (digestion du gel par trypsine). Le produit de la digestion a été analysé par nanoLC-MS/MS via le système Ultimate3000 (Dionex, Amsterdam, Pays-Bas) couplé à un spectromètre de masse LTQ-Orbitrap (Thermo Fisher Scientific, Brême, Allemagne) comme décrit précédemment (Contreras, Ferrero Guadagnoli et al. 2010), sauf que les peptides ont été élués en utilisant un gradient de 5 à 50% de solvant B pendant 60 min à un débit de 300 nL / min. Mascot a été utilisé pour extraire automatiquement les listes « peak lists » des fichiers bruts. Les données MS / MS ont été comparées à l'ensemble des entrées dans les bases de données de souris (*Mus musculus*) et l'identification des peptides a été validé par informatique comme décrit précédemment (Santini, Salomon et al. 2010), sauf que 1% FDR a été utilisée pour les peptides et protéines validation.

### Inhibition de l'expression de CCR3 par l'utilisation de shRNA

Pour inhiber de façon stable l'expression du récepteur CCR3 (NM_009914) dans les cellules TRAMP-C1P3, il a été utilisé une approche de shRNA permettant une régulation négative stable même après plusieurs mois de culture. Les séquences interférentes ont été générées à l'aide de l'outil «Center siDesign» de Dharmacon. Les séquences des brins sens des shRNA générées sont les suivantes :
m4CCR3 (SEQ ID NO : 1) : 5'-cgcgtccccAGACCACACCCTATGAATAttcaagagaTATTCATAGGGTGTGGTCTtttttggaaat -3,
m5CCR3 (SEQ ID NO : 2) :5'-cgcgtccccGACCACACCCTATGAATATttcaagagaATATTCATAGGGTGTGGTCtttttggaaat -3 et
m6CCR3 (SEQ ID NO : 3) 5'-cgccgtccccGGTGAGAGGTTCCGGAAACttcaagagaGTTTCCGGAACCTCTCACCtttttggaa at-3.

Les nucléotides ciblant la séquence de CCR3 sont indiqués en majuscules, alors que la séquence responsable de la structure en épingle et les séquences nécessaires pour le clonage directionnel sont représentés en lettres minuscules. Le vecteur lentiviral LVTHM ® est un vecteur lentiviral (LV) codant pour les séquences shRNA CCR3 sous le contrôle du promoteur H1 exprimant également la GFP. Les cellules TRAMP-C1P3 ont ensuite été transfectées en présence de m4, m5 ou m6-LVTHM durant 24 heures à une multiplicité d'Infection (*MOI*) de 1:10. La population transfectée est sélectionnée par expression de la GFP (Tri de cellulaire). Ces cellules ont ensuite été analysées par cytométrie en flux pour valider la perte de l'expression du gène de CCR3. Le contrôle utilisé est le plasmide pLVTHM qui exprime une séquence shRNA inefficace.

### Injection intraprostatique de cellules TRAMP-C1P3 chez la souris C57BL/6

Des souris males C57BL/6 de 18 semaines sous régime normal ou hyperlipidique ont été anesthésiées sous isoflurane. Une incision transversale a été réalisée au niveau de la partie inférieure de l'abdomen. Les muscles de la paroi abdominale ont été incisés, puis la vessie et les vésicules séminales ont été libérés afin d'exposer le lobe dorsal de la prostate. Une suspension de 2x10⁶ cellules (dans 30 µl de PBS) a ensuite été soigneusement injectée sous la capsule de la prostate par l'intermédiaire d'une aiguille de calibre 30. L'incision a été fermée à l'aide de fil de suture 4-0 et d'agrafes. Les animaux sont ensuite placés en cages et un suivi régulier est réalisé afin de suivre macroscopiquement l'état général de la souris. Après 20 jours de prise tumorale, les souris sont sacrifiées, la tumeur primaire est ainsi prélevée, mesurée et pesée, et une acquisition en lumière blanche et GFP est réalisée. Les tumeurs sont ensuite placées dans une solution de paraformaldéhyde (4%) puis inclus en paraffine.

### Prélèvement des échantillons humains de tumeurs prostatiques et Tissue MicroArrays TMA

La première base de données inclut une cohorte de 92 patients ont tous été opérés d'un adénocarcinome prostatique dans le Département d'Urologie du CHU de Toulouse (France).

Pour la seconde base de données, il s'agit d'une étude monocentrique et prospective. Les patients inclus ont tous été opérés d'un adénocarcinome prostatique, entre le 1er Février 2010 et le 1er Décembre 2011, dans le Département d'Urologie du CHU de Toulouse (France). Le traitement chirurgical a consisté en une prostatectomie radicale par voie laparoscopique robot-assistée ou par voie ouverte rétro-pubienne. La prostatectomie était associée dans certains cas à un curage ganglionnaire ilio-obturateur bilatéral (standard ou étendu). L'ensemble des patients inclus dans l'étude présentait une maladie localisée sans localisation métastatique lors de l'intervention. Pour tous les patients, un formulaire de non-opposition à l'utilisation de leurs échantillons tissulaires à des fins scientifiques a été recueilli et signé avant l'intervention afin d'être inclus dans une étude. La pièce fraîche, accompagnée du certificat de non-opposition, est dans les 15 minutes qui suivent l'exérèse chirurgicale prise en charge afin d'avoir un délai entre la dévascularisation et la congélation le plus court possible pour assurer une préservation des molécules labiles. Les échantillons tissulaires prélevés sont placés dans des cryotubes puis congelés dans de l'azote et stockés à -80°C.

Afin de faciliter l'étude, une analyse histologique multiplex a été possible grâce à la réalisation de « tissue microarrays » (TMA) sur des zones d'intérêt sélectionnées sur des lames d'échantillons de prostate. Les zones d'intérêt retenues pour la préparation des TMA ont été sélectionnées par deux pathologistes en double aveugle : le but était de sélectionner une zone riche en tissu carcinomateux. Les zones tumorales sélectionnées correspondaient morphologiquement à des secteurs présentant une différentiation histologique comparable au score de Gleason de la tumeur ou au score de Gleason le plus péjoratif en cas de tumeur plurifocale. Après avoir choisi les blocs à échantillonner, des carottes d'un diamètre de 0,2 mm étaient incluses de manière orthonormée selon un plan préétabli dans un bloc receveur. Le bloc receveur pouvait être ensuite coupé sur un microtome standard.

### Analyse de l'expression de CCR3 par immunohistochimie sur tissu

Des expériences d'immunohistochimie sur des biopsies de tumeurs prostatiques humaines ont été réalisées pour détecter l'expression de CCR3. L'immunomarquage a été réalisé à l'aide du EnVision™ FLEX Mini Kit, High pH (Dako Autostainer/Autostainer Plus) (Dako France, Trappes, FR). Les TMA (Tissue Micro Arrays) sont plongés dans du xylène pour enlever la paraffine puis ensuite réhydratés par des bains successifs d'alcool gradués (100 à 70% puis eau distillée), suivie d'un traitement avec la solution de démasquage antigénique pour la récupération d'antigène (tampon citrate Dako1X pH 6 au bain-marie 95°C). Après une étape de saturation des péroxydases endogènes (Peroxydase blocking, Dako), les échantillons sont incubés avec l'anticorps primaire anti-CCR3 (dilué au 1/100). Les TMA sont ensuite mis à incuber avec l'anticorps secondaire biotinylé, puis avec de la streptavidine conjugué HRP (Dako). Ils sont ensuite rincés, puis traités au liquide DAB (BioGenex, San Ramon, Californie), et lavés avec de l'eau distillée. Enfin la contre coloration a été réalisé à l'hématoxyline (Dako), et les différents TMA sont finalement montés à l'aide du réactif EUKITT. Les lames sont scannées numériquement via l'appareil Hamamatsu Nanozoomer 2.0RS et analysées à l'aide du logiciel fournis par le constructeur. La lecture des TMA a été réalisée par un anatomopathologiste.

### Mesure de la surface des dépôts adipeux périprostatiques

La mesure de la surface des dépôts adipeux périprostatiques a été faite sur 15 patients issus de la cohorte principale utilisée dans notre étude. Les mesures ont été réalisées à partir de donnée d'imagerie de scanner ou d'IRM préopératoires de chacun de ces patients. Pour cela il a été utilisé des images de sections transverses au niveau fémoral, à l'endroit où la fusion de la symphyse pubienne débute. Le dépôt adipeux qui a été étudié se situe entre la partie antérieure de la prostate et la symphyse pubienne. Il est facilement reconnaissable par la différence d'atténuation entre l'os, la prostate et le tissu adipeux. L'épaisseur et la largeur du dépôt ont été mesurées à l'aide de ces images. L'aire de ce dépôt a ensuite été approximé pour chaque patient, par calcul d'une aire elliptique à partir des données récoltées.

### Analyse statistique

La significativité statistique des différences entre les moyennes (au moins trois expériences indépendantes) a été évaluée à l'aide de tests student, effectuée à l'aide du logiciel Prism (GraphPad Inc.). Pour les analyses chez le patient, des tests de corrélations de rang de Spearman ont été réalisés. On parle de corrélations fortes pour des valeurs supérieures à 0,7, de corrélations moyennes pour des valeurs comprises entre 0,3 et 0,7 et de corrélations faibles pour des valeurs inférieures 0,3. Les valeurs de p inférieures à 0,05 (*), <0,01 (**) et <0,001 (***) sont jugées comme significatives alors que «NS» signifie non significatif.

### RESULTATS

### 1) Le récepteur CCR3 contribue à la migration des cellules tumorales prostatiques contre le milieu conditionné d'adipocytes

Des tests de migration en chambre de Boyden ont été effectués avec des lignées de cancer de la prostate d'agressivité croissante (LNCaP, C4-2B, Du-145 et PC-3). La chambre inférieure contient différents milieux : RPMI sans sérum de veau foetal (contrôle négatif), RPMI contenant 10% de sérum de veau foetal (contrôle positif), milieu conditionné d'adipocytes matures (MC-Ad) (obtenus à partir de la différentiation *in vitro* de la lignée de préadipocytes murins 3T3-F442A (Kuri-Harcuch and Green 1978). Le pourcentage de cellules migrantes contre un milieu contenant 10% de sérum est dépendant de l'agressivité tumorale. Le pourcentage de migration en présence de MC-Ad est similaire à la migration induite par du milieu contenant 10% de sérum pour les lignées LNCaP et C4-2B et est plus importante pour les lignées Du-145 et PC-3 (p<0,05), montrant que ce milieu contient des chimiokines (voir Fig. 1A).

Comme décrit précédemment (Vindrieux, Escobar et al. 2009), les récepteurs CCR2, CXCR1, CXCR2 et CXCR4 sont exprimés par les lignées de cancer de la prostate Du-145 et PC-3, expression détectée par cytométrie en flux. Le récepteur CCR3 est exprimé dans les lignées de cancer de la prostate Du-145 et PC3 contrairement aux récepteurs CCR1 (voir Fig. 1B).

La fonctionnalité de ces récepteurs dans la migration des cellules PC-3 contre du MC-Ad a été testée à l'aide d'inhibiteurs spécifiques connus utilisés aux doses inhibitrices décrites dans la littérature. Les résultats (voir Fig. 1C) sont les suivants :
- L'AMD3100 (inhibiteur spécifique de CXCR4; Rosenkilde *et al.*, 2004) diminue significativement d'environ 20% (p<0,05) la migration des cellules PC-3 contre le milieu MC-Ad seulement à la dose maximale de 100nM ;
- Le SB225002 (inhibiteur de CXCR2 et CXCR1 à une moindre affinité ; White *et al.*, 1998) inhibe significativement d'environ 30% la migration induite par le MC-Ad à la dose de 50nM (p<0,01) ;
- Le sc-202525 (antagoniste spécifique de CCR2 ; Cherney *et al.*, 2008) inhibe de seulement de 10% la migration des cellules tumorales (p<0,05) ;
- L'UCB35625 (inhibiteur de CCR1 et CCR3 ; Sabroe *et al.,* 2000) entraine une diminution significative dose dépendante de la migration des cellules tumorales PC-3 induite par le MC-Ad dès 50nM (p<0,05). L'effet maximum est observé à 200nM (environ 50% d'inhibition, p<0,001). Ces résultats ont été confirmés par une approche plus spécifique reposant sur l'utilisation d'anticorps bloquants CCR1 (référence D063-3, clone 141-2, MBL international Corp, (Iacono, Masciangelo et al. 1994)) ou CCR3 (D083-3, clone 444-11, MBL international, (Iacono, Masciangelo et al. 1994)). L'incubation des cellules tumorales avec un anticorps bloquant dirigé contre CCR3 (10µg/ml) entraine une importante inhibition de la migration induite par le MC-Ad (environ 50%, p<0,01) par rapport au contrôle isotypique. En revanche aucun effet d'inhibition n'est observé avec l'utilisation d'anticorps bloquant dirigé contre CCR1, qui n'est pas exprimé par les cellules tumorales.

La spécificité du rôle de CCR3 dans la migration des cellules prostatiques contre du milieu conditionné d'adipocytes matures a été étudiée. Les résultats sont présentés à la figure 1D. Différentes tumeurs épithéliales ont été utilisées, pour lesquelles du tissu adipeux (TA) se trouve anatomiquement à proximité lorsqu'elles deviennent invasives. Il s'agit du cancer du sein (TA mammaire), du cancer du pancréas et du colon (TA viscéral). De plus, des lignées de mélanome ont été inclues ; cette tumeur lorsqu'elle envahit se trouvant au contact du TA de l'hypoderme. Deux couples de lignées ont été choisies pour chaque type tumoral. Comme montré dans la figure 1D (panel de gauche), les lignées de cancers du sein (T47D et MDA-MB231), du colon (sw480 et sw620), du pancréas (CAPAN et PANC-1) et de mélanome (501Mel et SK28) expriment le récepteur CCR3 (cytométrie en flux). Toutes ces lignées migrent contre le MC-Ad, le pourcentage de cellules migrantes étant en rapport avec leur degré d'agressivité. La migration des cellules tumorales de cancer du pancréas, du colon et du mélanome contre du milieu MC-Ad n'est pas affectée par la présence de l'UCB35625 à une dose de 200nM (panel de droite). Seul un effet très modeste (inhibition de l'ordre de 10%, p<0,05) est observé pour la lignée de cancer du sein agressive MDA-MB231, alors que la migration de l'autre lignée de cancer du sein (T47D) n'est pas affectée. Dans cette même série d'expériences, l'inhibition significative de la migration contre le milieu MC-Ad des cellules tumorales prostatiques PC-3 et Du-145 d'environ 50% est retrouvée (p<0,001).

### 2) Le ligand de CCR3, CCL7 contribue à la migration des cellules tumorales prostatiques contre le milieu conditionné d'adipocytes

Une analyse protéomique a été réalisée sur le MC-Ad (Service de Protéomique, IPBS, CNRS UMR5089, Toulouse France). Cette analyse a été réalisée sur 3 milieux indépendants. Six chimiokines ont été détectées de façon reproductible : CXCL1, CXCL5, CXCL12, CCL2, CCL7 et CCL9 (panneau de droite). Les chimiokines, CXCL1 et CXCL5 peuvent interagir avec le récepteur CXCR2, CXCL12 avec CXCR4 et CCL2 avec CCR2 (panneau de gauche). Ces résultats (voir Fig. 2A) pourraient expliquer pourquoi les inhibiteurs de CXCR1/CXCR2, CXCR4 et CCR2 diminuent partiellement le chimiotactisme induit par le MC-Ad (Fig. 1C). Parmi les ligands de CCR3, seul CCL7 est retrouvé. Il est également à noter qu'aucun ligand de CXCR1 n'a été détecté dans les sécrétions adipocytaires analysées.

La chimiokine recombinante CCL7 est capable d'induire la migration des cellules prostatiques PC-3 et Du-145 de manière dose dépendante dans un test de migration en chambre de Boyden. Les résultats (voir Fig. 2B) sont significatifs pour les deux lignées à partir de 50 ng/mL par rapport au milieu seul (panneau de gauche). Dans ces deux mêmes lignées cellulaires, la migration des cellules prostatiques est inhibée d'environ 50% par un anticorps bloquant dirigé contre CCR3 et par un anticorps bloquant dirigé contre CCL7 (AF-456-NA, R&D System) (p<0,001), alors que l'anticorps bloquant CCR1 n'a pas d'effet (panneau de droite).

Du tissu adipeux périgonadique (TA-mu-PG) a été prélevé chez des souris mâle (3 expériences indépendantes réalisées sur 3 lots de souris indépendants, contenant de 3 à 10 souris). Des expériences similaires ont été réalisées avec du tissu adipeux périprostatique chez l'homme (TA-hu-PP). Ce TA est obtenu sur des pièces de prostatectomie, à distance de la tumeur. Ce tissu présente des caractéristiques macroscopiques de TA et ne contient pas de cellules tumorales. Ces expériences ont été réalisées chez 5 patients ayant un IMC compris entre 20 et 25. Pour ces deux types de TA, du milieu conditionné a été préparé à partir d'une culture de 24h. Ces expériences (voir Fig. 2C) montrent que CCL7 est sécrété par ces deux TA avec une moyenne de 155 ± 20 pg /mL /g de TA chez la souris et de 75± 15 pg /mL / g de TA chez l'homme (panneau de gauche). Les milieux conditionnés de TA humains et murins sont capables d'induire de façon similaire la migration des cellules tumorales (environ 20% de cellules migrantes pour les deux milieux). Enfin, cette migration est significativement inhibée par l'utilisation d'inhibiteurs et d'anticorps bloquant l'axe CCR3/CCL7 et par l'inhibiteur de CCR3 (UCB35625 à 200nM) (panneau de droite). L'axe CCR3/CCL7 est donc impliqué dans la migration des cellules prostatiques contre les sécrétions du tissu adipeux viscéral murin et périprostatique humain.

### 3) La sécrétion de CCL7 par le TA est augmentée dans un contexte d'obésité ce qui contribue à amplifier le rôle de l'axe CCR3/CCL7 dans la migration des cellules prostatiques

L'expression génique de CCL7 a été évaluée dans une série d'échantillons de TA viscéraux (TAV) humains (intra-abdominal) de patients soit normopondéraux (Indice de Masse Corporelle (IMC moyen 23,3± 1,2 kg/m², 8 échantillons), soit obèses (IMC moyen 45,7± 2,9 kg/m², 21 échantillons). Les résultats (voir Fig. 3A) montrent une augmentation d'environ 3 fois des ARNm de CCL7 dans les TAV de sujets obèses comparé à des sujets d'IMC normal (p< 0,01) (panneau de gauche). Chez la souris, le niveau d'expression des ARNm de CCL7 est aussi augmenté d'un facteur 3 dans les souris rendues obèses par un régime riche en lipides (« High Fat Diet », HFD) par rapport aux souris de poids normal (« Normal Diet », ND) (p<0,001). Des résultats similaires ont été obtenus avec un modèle d'obésité génétique de souris présentant une invalidation du récepteur à la leptine (souris db/db) par rapport aux souris sauvages.

Du tissu adipeux périgonadique (TA-mu-PG a été prélevé chez des souris soumises pour 8 semaines, soit à un régime normal (souris normopondérales) ou à un régime riches en lipides ce qui conduit à l'apparition d'une obésité (obèses, HFD) (expériences indépendantes réalisées sur 3 lots de souris différents). Les résultats sont présentés à la figure 3B. Dans le milieu conditionné préparé comme précédemment, une augmentation de 2,7 fois du niveau de sécrétion de CCL7 est observée dans les tissus issus de souris obèses par rapport aux souris de poids normal (p<0,05) (panneau de gauche). Ces milieux conditionnés ont été utilisés pour réaliser des expériences de migration en chambre de Boyden. La migration des cellules tumorales PC-3 contre le milieu conditionné est significativement augmentée par l'obésité (28% de cellules migrantes contre le MC de TA de souris normopondérales versus 40% contre le MC du TA de souris obèses HFD, p<0,01) (panneau de droite). Dans les deux conditions, la migration des cellules tumorales contre le MC est inhibée par l'utilisation d'inhibiteurs et d'anticorps bloquant l'axe CCR3/CCL7, l'effet étant toutefois plus prononcé dans des conditions d'obésité. Ainsi, l'UCB35625 ou des anticorps anti-CCR3 ou CCL7 inhibent de 25% la migration contre le MC du TA-mu-PG ND, alors que cet effet est de 60 % pour le MC du TA-mu-PG HFD.

Les TA-mu-PG de souris ND ou HFD ont été digérés à la collagénase afin de permettre de séparer, après une courte centrifugation, les adipocytes matures (cellules flottantes) de la fraction stroma-vasculaire (contenant les cellules endothéliales, les macrophages, les cellules souches adipocytaires et les pré-adipocytes...). Ces adipocytes matures ont ensuite été incubés pour 24 heures dans un milieu sans sérum supplémenté par 1% de BSA afin de préparer du MC. Le dosage de CCL7 dans ces milieux (3 milieux indépendants) a été réalisé par ELISA. Les résultats (voir Fig. 3C) montrent que les adipocytes primaires sécrètent du CCL7 et que cette sécrétion est fortement régulée dans un contexte d'obésité (90 ± 10 pg/mL/10⁵ cellules versus 720 ± 80 pg/mL/10⁵ cellules, p<0,001). La migration des cellules tumorales contre les MC d'adipocytes issus d'animaux obèses est significativement augmentée par rapport aux MC d'animaux de poids normal (p< 0,01). Dans les deux conditions, la migration des cellules tumorales contre le MC est inhibée par l'utilisation d'inhibiteurs et d'anticorps bloquant le CCR3, l'effet étant toutefois plus prononcé dans des conditions d'obésité. Ainsi, l'inhibition de CCR3 par l'UCB35625 ou par un anticorps diminue de 60% la migration contre le MC d'adipocytes issus de souris obèses, alors que cet effet est de 30% pour le MC d'adipocytes issus de souris de poids normal (p< 0,01).

### 4) Validation de l'implication de CCR3 dans la migration en réponse aux sécrétions adipocytaires dans un modèle de lignée tumorale murine (TRAMP-C1P3)

La lignée murine syngénique TRAMP-C1P3 pour la souche de souris C57 BL/6 a été utilisée pour réaliser des modèles de greffes orthotopiques de cellules tumorales prostatiques dans des souris normopondérales ou obèses. Ce choix de lignée est justifié par le fait que la souris C57 BL/6 est un modèle sensible à l'obésité induite par le régime hyperlipidique avec une reproductibilité parfaitement caractérisée (Winzell and Ahren 2004). Les résultats (voir Fig. 4A) montrent que les cellules TRAMP-C1P3, comme les cellules tumorales prostatiques humaines, expriment le récepteur CCR3 mais pas le récepteur CCR1 (panneau de gauche). La chimiokine recombinante CCL7 est capable d'induire un chimiotactisme dose dépendant comme pour les lignées humaines à partir de 50ng/mL (panneau du milieu). Enfin, la migration induite par le MC-Ad est significativement inhibée par l'utilisation de l'UCB35625 (200nM, p<0,05) des anticorps bloquants dirigés contre CCR3 (10µg/mL, p< 0,001) ou CCL7 (10µg/mL, p< 0,01) alors que l'anticorps bloquant dirigé contre CCR1 est sans effet (panneau de droite).

Trois plasmides contenant des shRNA dirigés contre la séquence codante de CCR3 ainsi que contre une séquence non codante (shRNA contrôle) ont été construits. Ces plasmides ont été transfectés par une approche lentivirale dans la lignée TRAMP-C1P3 et 3 lignées (m4CCR3, m5CCR3, m6CCR3) invalidées pour le récepteur CCR3 ont été obtenues ainsi qu'une lignée contrôle (shCtrl). Les résultats sont représentés à la figure 4B. Comme montré dans le panneau de gauche (quadrant supérieur de la figure 4B), les 3 lignées invalidées pour CCR3 présentent une diminution significative de l'expression de ce récepteur par rapport à la lignée contrôle (immunofluorescence). Cette inhibition de l'expression de CCR3 s'accompagne d'une diminution significative de la migration contre du MC-Ad pour les trois lignées shCCR3 par rapport à la lignée contrôle (panneau de gauche, quadrant inférieur). Cette diminution de la migration est aussi retrouvée pour la lignée m6CCR3 par rapport à la lignée contrôle lorsque du milieu conditionné de TA-mu-PG ND est utilisé. Dans ces conditions, l'UCB35625 ainsi que les anticorps bloquant CCR3 ou CCL7 n'ont plus d'effet significatif sur la migration des cellules tumorales m6CCR3 contrairement à la lignée contrôle (p<0,001). Enfin, l'augmentation de la migration observée en présence de TA-mu-PG HFD n'est pas significative pour la lignée m6CCR3 contrairement à la lignée shCtrl et l'effet de l'UCB35625 ainsi que les anticorps bloquant CCR3 ou CCL7 n'est pas significatif (panneau de droite, quadrant inférieur).

### 5) Le récepteur CCR3 est impliqué dans la progression tumorale dans des modèles murins in vivo

Les résultats sont présentés à la figure 5A. L'injection de la lignée TRAMP-C1P3 dans la prostate (lobe dorsal) de souris C57BL/6 âgées de 16 semaines et nourries par un régime normolipidique (ND) (poids moyen 32 ± 3g) conduit à l'apparition de tumeurs (mesurées à 3 semaines, taille tumorale moyenne 800 ± 100 mm3, n=14). Lorsque les cellules sont injectées dans la glande prostatique de souris ayant bénéficiée d'un régime hyperlipidique (HFD) de 8 semaines et obèses (poids moyen des souris : 45 ±3 g, injection à 16 semaines comme pour les souris de poids normal), il existe une augmentation significative de la taille des tumeurs d'environ 2 fois par rapport aux souris non obèses (taille tumorale moyenne 1500 ± 80 mm3, n=14, p< 0,001). Les mêmes expériences réalisées avec la lignée m6CCR3 montre une diminution significative de la taille des tumeurs par rapport à la lignée shCtrl qu'il s'agisse des souris nourries avec un régime normolipidique (poids moyen 30 ±4 g [NS par rapport au groupe shCtrl], taille tumorale 220 ± 30 mm3, n=11, p<0,001 [par rapport au groupe shCtrl]) ou des souris obèses nourries avec un régime hyperlipidique (poids moyen 45 ± 3g) [NS par rapport au groupe shCtrl], taille tumorale 210 ± 25 mm3, n=11, p< 0,001 [par rapport au groupe shCtrl]). Pour les souris injectées avec la lignée m6CCR3, il n'existe plus de différence de taille tumorale entre les souris normonpondérées ou obèses (taille tumorale 220 ± 30 mm³ versus 200 ± 50 mm³), (panneau de gauche de la figure 5A). Une analyse anatomo-pathologique des tumeurs a été réalisée (panneau inférieur). L'analyse des coupes histologiques de ces tumeurs après coloration à l'hematoxyline-éosine (HE) confirme que les souris injectées avec la lignée contrôle (C1P3 Ctrl) présentent des tumeurs d'un volume important ayant totalement envahies le lobe dorsal de la prostate. Pour ces tumeurs on observe également une disparition du tissu adipeux périprostatique en particulier chez les souris obèses. En effet, au contact de la tumeur, les adipocytes matures se délipident et se dédifférencient ce qui conduit à leur remplacement par un tissu fibreux. En revanche, chez les souris ayant été injectées avec la lignée invalidée pour CCR3 (C1P3 m6CCR3), on observe des tumeurs d'un volume inférieur caractérisées par un envahissement du lobe dorsal de la prostate beaucoup moins important que pour la lignée C1P3 Ctrl. On retrouve en périphérie de la tumeur du tissu adipeux intègre, celui-ci présentant une hypertrophie et une hyperplasie dans les souris obèses.

### 6) L'expression du récepteur CCR3 est retrouvée dans les tumeurs prostatiques agressives et son expression est corrélée à l'extension de la tumeur en dehors de la capsule prostatique

L'expression du récepteur CCR3 dans les tumeurs humaines a été analysée dans un premier TMA (*Tumor Micro Array*) contenant 92 tumeurs prostatiques. Les résultats montrent que CCR3 est exprimé dans les tumeurs prostatiques humaines (figure représentative du résultat du marquage par immunohistochimie, panneau de gauche de la figure 6A) et que son niveau d'expression est corrélé au grade de la tumeur définie par le score de Gleason (coefficient de corrélation de 0,62, p<0,001, test Spearman) (figure 6A, panneau de droite). Dans les tumeurs de bas grade (Score de Gleason inférieur à 7), on observe une expression de CCR3 élevée (score immunohistochimique quantitatif +++) dans 10% des tumeurs contre 75% de tumeurs exprimant un CCR3 faible (score immunohistochimique quantitatif +). En revanche, dans des tumeurs plus agressives de score de Gleason égal et/ou supérieur à 7 on retrouve un marquage intense de CCR3 dans respectivement 46,8% et 76% des tumeurs contre respectivement 12,8% et 4% présentant un marquage faible. Enfin il est à noter que l'expression de CCR3 est faible voir absente dans des glandes prostatiques non tumorales.

Une deuxième série d'expériences a été réalisée sur une collection annotée de 101 patients. Les patients inclus ont tous été opérés d'un adénocarcinome prostatique dans le Département d'Urologie du CHU de Rangueil (France). Les TMA ont été réalisés en double à partir d'une zone contenant de la tumeur à des pièces de prostatectomie cryoconservées. Afin d'améliorer la quantification de CCR3, les lames des tumeurs ont été acquises numériquement et l'expression de la protéine a été quantifiée en utilisant des plugins du logiciel ImageJ pour obtenir des mesures plus précises que l'évaluation traditionnelle reposant sur la notation manuelle (Sysel *et al.*, 2013).

### 7) Immunohistochemical quantification of the vitamin B12 transport protein (TCII), cell surface receptor (TCII-R) and Ki-67 in human tumor xenografts.

Les caractéristiques cliniques de la cohorte sont présentées dans le tableau 2 ci-dessous et les caractéristiques immunohistochimiques dans le tableau 3 ci-dessous.

**Tableau 2 : Caractéristiques cliniques de la cohorte.**

| | | **Variables** | **Pourcentage parmi la cohorte** |
|---|---|---|---|
| **Taille de la cohorte (nombre de patients)** | | 101 | 100 |
| **Age à la chirurgie (années),** médiane (étendue) | | 63 (47-75) | |
| **Indice de Masse Corporelle (IMC) (Kg/m²),** médiane (étendue) | | 26.1 (19-34) | |
| **PSA post-chirurgie (ng/mL),** médiane (étendue) | | 7 (2-37) | |
| **Dernier dosage PSA (ng/mL),** médiane (étendue) | | 0 (0-4.02) | |
| **Poids de la pièce de prostatectomie (g),** médiane (étendue) | | 48 (22-93) | |
| **Durée de la surveillance (Jours),** médiane (étendue) | | 765 (62-1736) | |
| **Traitement post-chirurgie (nombre de patients)** | Surveillance seule | 71 | 70.30 |
| | Radiothérapie seule | 16 | 15.84 |
| | Hormonothérapie seule | 7 | 6.93 |
| | Radiothérapie et hormonothérapie | 7 | 6.93 |
| **Evolution post-chirurgie (nombre de patients)** | Rechute biochimique | 14 | 13.86 |
| | Echec du traitement par chirurgie^{a} | 30 | 29.70 |
| | Pas de rechute | 70 | 69.31 |
| | Décès | 1 | 0.99 |

**Tableau 3 : Caractéristiques histologiques et immunohistochimiques de la cohorte.**

| | | **Variables** | **Pourcentage parmi la cohorte** |
|---|---|---|---|
| **Score de Gleason,** nombre de patients | <7 | 18 | 17.82 |
| | =7 (3+4) | 39 | 38.62 |
| | =7 (4+3) | 36 | 35.64 |
| | >7 | 8 | 7.92 |
| **Pourcentage de contingent peu différencié (grade 4 et 5),** médiane (étendue) | | 40 (0-100) | |
| **Localisation de la tumeur,** nombre de patients | Zone de transition | 10 | 9.90 |
| | Zone périphérique | 91 | 90.10 |
| **Stade tumoral,** nombre de patients | pT2b | 6 | 5.94 |
| | pT2c | 35 | 34.65 |
| | pT3a | 44 | 42.57 |
| | pT3b | 16 | 15.84 |
| **Marges chirurgicales positives,** nombre de patients | | 22 | 21.78 |
| **Présence d'emboles lymphatiques,** nombre de patients | Non | 93 | 92.08 |
| | Oui | 8 | 7.92 |
| **Curage ganglionnaire bilatéral,** nombre de patients | | 79 | 79.21 |
| **Envahissement des ganglions lymphatiques,** nombre de patients | Nx (Pas de dissection ganglionnaire réalisée) | 22 | 21.78 |
| | N0 | 74 | 73.27 |
| | N1 | 5 | 4.95 |
| **Expression de CCR3 (unités arbitraires)**, médiane (étendue) | | 48.5 (11.4-94.7) | |

Comme dans la cohorte précédente, on retrouve une expression de CCR3 dans les tumeurs prostatiques (figure représentative du résultat du marquage par immunohistochimie, panneau de gauche de la figure 6B), et son niveau d'expression est corrélé au grade de la tumeur définie par le score de Gleason (coefficient de corrélation de 0,472, p<0,001, test Spearman) (figure 6B, panneau de droite). Une analyse extensive de la corrélation entre le niveau d'expression de CCR3 et différents paramètres cliniques et biologiques des tumeurs a été réalisée (voir résultats ci-dessous).

Il a été ainsi montré une corrélation entre le niveau d'expression de CCR3 et
- le score de Gleason (p< 0.001),
- le pourcentage de contingent tumoral histologiquement peu différencié (grade 4 et grade 5 confondus) (p < 0.01),
- le stade histologique pT équivalent au degré d'extension de la tumeur (p<0.03)
- la localisation périphérique de la tumeur (p< 0.04),
- la présence d'embols lymphatiques (p<0.02),
- la rechute biologique définie par deux dosages successifs de PSA > 0.2 mg /mL p< 0.001).

De plus, le niveau d'expression de CCR3 est augmenté chez les patients obèses (p=0.01).

L'expression du récepteur de CCR3 est un facteur de mauvais pronostic dans le cancer de la prostate. Il existe une corrélation significative entre le niveau d'expression de CCR3 et l'échec du traitement chirurgical (p <0.001). L'échec du traitement chirurgical est défini soit par une rechute biologique (deux taux de PSA successifs supérieurs à 0.2 ng/mL), soit par une récidive locorégionale ou l'apparition de métastases à distance, ou par l'utilisation d'un traitement par déprivation hormonale et/ou une radiothérapie immédiatement après la chirurgie, critère qui a été utilisé dans une étude précédente (Malavaud *et al.*, 2010).

### 8) Sphingosine kinase-1 activity and expression in human prostate cancer resection specimens.

Les valeurs de CCR3 ont été corrélées à ce critère (voir Figure 8A). Lorsque les valeurs de CCR3 ont été comparés chez les patients présentant ou non un échec du traitement chirurgical à un an de suivi dans les quatre classes de score de Gleason (*i.e*, <7, 7 [3 + 4 ou 4 + 3],> 7), elles étaient systématiquement plus élevés chez les patients présentant un échec du traitement chirurgical (Figure 8B). En outre, les différences étaient plus importantes dans le cas d'un score de Gleason faible (<7). Le taux de CCR3 pourrait donc représenter un facteur pronostique de rechute.

Dans un sous-groupe de patients, l'importance du tissu adipeux périprostatique a été mesurée. Les caractéristiques cliniques (tableau 4 ci-dessous) et histologiques et immunohistochimiques (tableau 5 ci-dessous de ce sous-groupe sont présentés.

**Tableau 4 : Caractéristiques cliniques de la cohorte pour laquelle la graisse prostatique a été mesurée.**

| | | |
|---|---|---|
| Nombre total de patient (pourcentage en colonne ; %) | | 15 (100) |
| Age (Années), Médiane (étendue) | | 62 (48-71) |
| IMC (kg/m²), Médiane (étendue) | | 28 (20-34) |
| PSA initial avant prostatectomie (ng/ml), Médiane (étendue) | | 7 (2-34) |
| Poids des pièces de prostatectomie (g), Médiane (étendue) | | 48 (32-69) |
| Traitement proposé dans les suites de la chirurgie | Surveillance seule, n (pourcentage en colonne) | 10 (66,7) |
| | Radiothérapie, n (pourcentage en colonne) | 3 (20) |
| | Hormonothérapie, n (pourcentage en colonne) | 3 (20) |
| Evolution après chirurgie | Aucune récidive biologique | 12 (80) |
| | Récidive biologique | 3 (20) |
| | Décès | 1 (10) |

**Tableau 5 : Caractéristiques histologiques et immunohistochimiques de la cohorte pour laquelle la graisse prostatique a été mesurée.**

| | | |
|---|---|---|
| Nombre total de patient (pourcentage en colone) | | 15 (100) |
| Siège de la tumeur | Transitionnel, n (pourcentage en colonne) | 1 (6,7) |
| | Périphérique, n (pourcentage en colonne) | 14 (93,3) |
| Score de Gleason sur la pièce de prostatectomie | 5, n (pourcentage en colonne) | 2 (13,3) |
| | 6, n (pourcentage en colonne) | 1 (6,7) |
| | 7, n (pourcentage en colonne) | 10 (66,7) |
| | 8, n (pourcentage en colonne) | 1 (6,7) |
| | 9, n (pourcentage en colonne) | 1 (6,7) |
| Pourcentage de contingent peu différencié (grade 4 et 5 confondus), Médiane (étendue) | | 25 (0-95) |
| Volume tumoral (pourcentage d'envahissement de la prostate), Médiane (étendue) | | 13 (6-48) |
| Présence d'embols | oui, n (pourcentage en colonne) | 2 (13,3) |
| | non, n (pourcentage en colonne) | 13 (56,7) |
| Stade pTNM | pT2a, n (pourcentage en colonne) | 1 (6,7) |
| | pT2b, n (pourcentage en colonne) | 4 (26,7) |
| | pT3a, n (pourcentage en colonne) | 7 (46,7) |
| | pT3b, n (pourcentage en colonne) | 3 (20) |
| | pT4, n (pourcentage en colonne) | 0 (0) |
| Présence de ganglions métastatiques **, n (pourcentage en colonne) | | 1 (6,7) |
| Expression de CCR3 | 0= absent, n (pourcentage en colonne) | 0 (0) |
| | 1= faible, n (pourcentage en colonne) | 2 (13,3) |
| | 2= modéré, n (pourcentage en colonne) | 10 (66,7) |
| | 3= intense, n (pourcentage en colonne) | 3 (20) |

Un TAPP abondant a été défini par une surface supérieure à 10cm2, surface pour laquelle tous les patients présentent un score de Gleason supérieur à 7 (voir tableau 6 ci-dessous).

**Tableau 6 : Corrélation entre la surface du dépôt adipeux périprostatique et différentes caractéristiques cliniques, histologiques et biochimiques de la cohorte.**

| | | Surface du tissu adipeux périprostatique | | Spearman | |
|---|---|---|---|---|---|
| | | Surface faible (<10 cm2) | Surface importante (> 10 cm²) | coefficient de corrélation | p-value |
| Age (années), Médiane (étendue) | | 61 (61-71) | 62 (48-69) | -0,017 | 0,449 |
| Score de Gleason, n (en colonne) | Gleason <7 | 3 (50) | 0 (0) | 0,5843 | 0,022 |
| | Gleason (3+4) | 3 (50) | 6 (60) | | |
| | Gleason (4+3) | 0 (0) | 2 (20) | | |
| | Gleason >7 | 0 (0) | 2 (20) | | |
| Contingent indifférencié, Médiane (étendue) | | 5 (0-25) | 25 (20-95) | 0,461 | 0,083 |
| Stade tumoral, n (en colonne) | 1= pT2b | 0 (0) | 1 (10) | 0,38 | 0,163 |
| | 2= pT2c | 2 (40) | 2 (20) | | |
| | 3= pT3a | 3 (60) | 4 (40) | | |
| | 4= pT3b | 0 (0) | 3 (30) | | |
| poids prostatique, Médiane (étendue) | | 52 (44-69) | 38 (32-68) | -0,24 | 0,39 |
| PSA (ng/mL), Médiane (étendue) | | 6.7 (7-7,5) | 7.15 (6,38-8.96) | -0,163 | 0,89 |
| Rechute biologique, n (en colonne) | réascension | 1 (20) | 2 (20) | -0,116 | 0,681 |
| IMC (kg/m2), Médiane (étendue) | | 28 (22-34) | 27 (20-31) | -0,243 | 0,3 |
| Surpoids et obésité (IMC ≥25kg/m2), n (en colonne) | | 3 (60) | 7 (70) | -0,224 | 0,38 |
| Expression de CCR3 | 1= faible, n (pourcentage en colonne) | 2 (40) | 0 (0) | 0,67 | 0,006 |
| | 2= modéré, n (pourcentage en colonne) | 3 (60) | 7 (70) | | |
| | 3= intense, n (pourcentage en colonne) | 0 (0) | 3 (30) | | |

### 9) L'expression du récepteur CCR3 joue un rôle dans la migration des cellules de cancer de la prostate induite par les adipocytes médullaires

Deux types de modèles ont été utilisés : la lignée 14F1.1 qui est une lignée de pré-adipocytes médullaires dont la différentiation (obtenue après 21 jours de confluence) *in vitro* permet l'obtention d'adipocytes contenant des lipides (Zipori, Friedman et al. 1984) (panneau de gauche de la figure 7A). Des expériences ont été aussi réalisées avec des adipocytes médullaires obtenus à partir de la différentiation *in vitro* de progéniteurs issus de la moelle de souris C57BL/6. Ces progéniteurs ont été cultivés pendant 4 semaines dans un milieu de différenciation contenant du DMEM (10% sérum) complémenté avec de la dexaméthasone à 1µM, de l'IBMX 0,45mM, de l'indométacine à 60µM et de l'insuline à 10µg/mL, permettant l'obtention d'adipocytes matures contenant des gouttelettes lipidiques (panneau du milieu de la figure 7A).

Le dosage de CCL7 par la méthode ELISA montre que les milieux conditionnés de ces cellules contiennent du CCL7 à un niveau comparable à celui des 3T3F442A différenciées *in vitro* (3 échantillons indépendants). Ces expériences ont également permis de mettre en évidence que ces adipocytes médullaires sécrètent des quantités de CCL7 supérieures à celles générées par des adipocytes viscéraux ND (p<0,001). Dans un test de migration en chambre de Boyden, les adipocytes médullaires sont capables d'induire la migration des cellules tumorales PC-3 à un niveau comparable à celui des adipocytes matures F442A (panneau du milieu). Cette migration est inhibée par l'UCB35625 (200nM) et par l'utilisation d'anticorps bloquant CCR3 et CCL7 (10µg/ml) (p<0,01). Les résultats sont présentés à la figure 7B.

La lignée RM1-BM est une lignée de cancer de la prostate capable de former des métastases osseuses dans 90% des cas chez la souris C57BL/6 après injection intracardiaque (Ohori, Egawa et al. 1994). Ces cellules comme l'ensemble des lignées tumorales prostatiques étudiées expriment le récepteur CCR3 mais non le récepteur CCR1 comme montré par cytométrie en flux (panneau de gauche de la figure 7C). La chimiokine recombinante CCL7 induit une migration significative des cellules RM1-BM à la dose de 100ng/mL par rapport au contrôle (milieu sans sérum, p< 0,01) (panneau du milieu). La migration des cellules RM1-BM contre du milieu conditionné d'adipocytes médullaires est significativement inhibé par l'UCB35625 à la dose de 200nM (p<0,05), ainsi que par les anticorps bloquant dirigés contre CCR3 et CCL7 à la dose de 10µg/ml (p<0,01) (panneau de droite). Les résultats sont présentés à la figure 7C.

### 10) Les adipocytes médullaires humains possèdent la capacité de chimioattracter les cellules tumorales prostatiques humaines, cet effet est dépendant de l'axe CCR3/CCL7 et est régulé par l'obésité

Les résultats sont présentés à la Figure 9.

Figure 9A : A partir d'échantillons de moelle jaune de patients obtenus en collaboration avec le Service de Traumatologie de l'Hôpital Pierre Paul Riquet (CHU de Toulouse), les adipocytes médullaires sont isolés et incubées 24h dans du milieu sans sérum contenant 1% de BSA. Après 24h, le milieu conditionné est prélevé. Des tests de migration ont été réalisés en utilisant des cellules tumorales PC3 comme modèle et comme milieu chimioattractant : soit du milieu de culture contenant 0% de SVF (témoin négatif), soit du milieu de culture contenant 10% de SVF (témoin positif), soit du milieu conditionné d'adipocytes médullaires humains (MC-MAd). Le taux de cellules migrantes en présence de MC-Mad est similaire à celui obtenu en présence de milieu contenant 10% de SVF, confirmant l'existence d'une migration dirigée induite par le MC-MAd et donc la présence de chimiokines dans ces sécrétions des adipocytes médullaires.

Figure 9B : Par test ELISA, il a été montré la présence de la chimiokine CCL7 dans le MC-MAd et ce en quantité quatre fois supérieure à celle présente dans le milieu conditionné de TA adipeux sous cutané humain et deux fois supérieure à celle du tissu adipeux périprostatique humain

Figures 9C et 9D : La chimiokine (CCL7) et son récepteur (CCR3) jouent un rôle dans la migration dirigée des cellules tumorales PC3 contre le MC-MAd, puisque cette migration est inhibée de près de 50% en utilisant un inhibiteur de CCR1/CCR3 (UCB35625) et de près d'un tiers en utilisant des anticorps bloquants dirigés contre CCR3 et CCL7, alors que les anticorps dirigés contre CCR3 sont sans effet.

Figures 9E et 9F : En cas d'obésité, il existe un renforcement de l'axe CCL7/CCR3. Tout d'abord, la sécrétion de CCL7 par les adipocytes médullaires, dosée par ELISA, est presque doublée chez les patients obèses (IMC> 30 mg/m²) par rapport aux patients non obèses (IMC < 25mg/m²). Le niveau de sécrétion de CCL7 dans les adipocytes médullaires est beaucoup plus important dans les adipocytes médullaires que sous-cutanés (y compris chez les sujets obèses). Par ailleurs, les sécrétions venant d'adipocytes médullaires de patients obèses sont plus chimioattractantes que celles venant des patients non-obèses et induisent à peu près 50% de migration supplémentaire. De façon intéressante, en utilisant l'inhibiteur de CCR1 et CCR3 (UCB35625), l'augmentation de migration liée à l'obésité est réversée presque totalement.

### REFERENCES

Agrawal, L., C. R. Maxwell, et al. (2009). J Gen Virol 90(Pt 3): 710-722.
Allott, E. H., E. M. Masko, et al. (2013). Eur Urol 63(5): 800-809.
Balkwill, F. (2004). Nat Rev Cancer 4(7): 540-550.
Begley, L. A., S. Kasina, et al. (2008). Cytokine 43(2): 194-199.
Cherney, R. J., R. Mo, et al. (2008). J Med Chem 51(4): 721-724.
Contreras, A. E., A. Ferrero Guadagnoli, et al. (2010). Rev Fac Cien Med Univ Nac Cordoba 67(3): 104-107.
Egawa, S., M. Ohori, et al. (1994). Nihon Hinyokika Gakkai Zasshi 85(10): 1543-1551.
Elsner, J., S. E. Escher, et al. (2004). Allergy 59(12): 1243-1258.
Elsner, J., H. Petering, et al. (1997). Eur J Immunol 27(11): 2892-2898.
Houimel, M. and L. Mazzucchelli (2013). Immunol Lett 149(1-2): 19-29.
Iacono, A. T., T. N. Masciangelo, et al. (1994). Chest 106(1): 311-313.
Inoue, K., J. W. Slaton, et al. (2000). Clin Cancer Res 6(5): 2104-2119.
Johrer, K., C. Zelle-Rieser, et al. (2005). Clin Cancer Res 11(7): 2459-2465.
Joubert, P., S. Lajoie-Kadoch, et al. (2008). J Immunol 180(2): 1268-1275.
Jung, D. W., Z. M. Che, et al. (2010). Int J Cancer 127(2): 332-344.
Kanno, A., H. Amakasu, et al. (1994). Tohoku J Exp Med 172(1): 83-90.
Kiss, D. L., J. Longden, et al. (2009). Cell Mol Biol Lett 14(4): 537-547.
Kuri-Harcuch, W. and H. Green (1978). Proc Natl Acad Sci U S A 75(12): 6107-6109.
Lee, Y. J., D. H. Kim, et al. (2010). Ann Dermatol 22(4): 412-417.
Loberg, R. D., L. L. Day, et al. (2006). Neoplasia 8(7): 578-586.
Loetscher, P., A. Pellegrino, et al. (2001). J Biol Chem 276(5): 2986-2991.
Lu, Y., Z. Cai, et al. (2007). J Cell Biochem 101(3): 676-685.
Magi-Galluzzi, C., A. J. Evans, et al. (2011). Mod Pathol 24(1): 26-38.
Malavaud, B., D. Pchejetski, et al., (2010). Eur J Cancer. 46(18):3417-24.
Menzies, N. A. and J. A. Salomon (2011). Health Econ 20(12): 1523-1531.
Ohori, M., S. Egawa, et al. (1994). Br J Urol 74(1): 72-79.
Ohori, M. and P. T. Scardino (1994). Semin Oncol 21(5): 522-526.
Ohori, M., T. M. Wheeler, et al. (1994). J Urol 152(5 Pt 2): 1714-1720.
Ohori, N. P., A. T. Iacono, et al. (1994). Am J Surg Pathol 18(12): 1192-1204.
Ouchi, N., J. L. Parker, et al. (2011). Nat Rev Immunol 11(2): 85-97.
Rosenkilde, M. M., L. O. Gerlach, et al. (2004). J Biol Chem 279(4): 3033-3041.
Sabroe, I., M. J. Peck, et al. (2000). J Biol Chem 275(34): 25985-25992.
Saitoh, Y., M. Miura, et al. (1994). "Changes of serum hepatitis C virus levels in patients with chronic hepatitis C treated with two courses of interferon administration." Tohoku J Exp Med 173(4): 361-369.
Salomon, L., D. Azria, et al. (2010). Prog Urol 20 Suppl 4: S217-251.
Santini, M. S., O. D. Salomon, et al. (2010). Rev Inst Med Trop Sao Paulo 52(4): 187-191.
Sysel, A. M, V. E. Valli, et al. (2013). Anticancer Res. 33: 4203-12.
Szymczak, W. A. and G. S. Deepe, Jr. (2009). J Immunol 183(3): 1964-1974.
Taichman, R. S., C. Cooper, et al. (2002). Cancer Res 62(6): 1832-1837.
Tourniaire, F., B. Romier-Crouzet, et al. (2013). PLoS One 8(6): e66515.
Vindrieux, D., P. Escobar, et al. (2009). Endocr Relat Cancer 16(3): 663-673.
White, J. R., J. M. Lee, et al. (1998). J Biol Chem 273(17): 10095-10098.
Winzell, M. S. and B. Ahren (2004). Diabetes 53 Suppl 3: S215-219.
Yamamura, J., G. Salomon, et al. (2011). Radiol Res Pract 2011: 616852.
Zhang, J., L. Patel, et al. (2010). Cytokine Growth Factor Rev 21(1): 41-48.
Zhu, F., P. Liu, et al. (2014). Oncol Rep 31(5): 2049-2054.
Zhu, X. H., B. Liao, et al. (2013). Zhonghua Er Bi Yan Hou Tou Jing Wai Ke Za Zhi 48(4): 316-321.
Zipori, D., A. Friedman, et al. (1984). J Cell Physiol 118(2): 143-152.

## Revendications

1. Inhibiteur de l'interaction CCL7/CCR3 pour son utilisation pour prévenir ou traiter l'extension du cancer de la prostate hors de la capsule prostatique chez un sujet ; l'inhibiteur de l'interaction CCL7/CCR3 étant choisi dans le groupe constitué par une molécule organique choisie dans le groupe constitué par les composés de formules I à DCCLXXXIV, un peptide, anticorps dirigé contre CCR3 ou CCL7, un peptidomimétique et un aptamère reconnaissant CCL7 ou CCR3.

2. Inhibiteur de l'interaction CCL7/CCR3 pour son utilisation selon la revendication 1, **caractérisé en ce qu'**il s'agit d'un antagoniste de CCR3.

3. Inhibiteur de l'interaction CCL7/CCR3 pour son utilisation selon la revendication 1 ou la revendication 2, **caractérisé en ce qu'**il s'agit du composé UCB35625 de formule DLI.

4. Inhibiteur pour son utilisation selon l'une quelconque des revendications 1 à 3, **caractérisé en ce que** le sujet est un homme obèse, ayant un indice de masse corporelle (IMC) supérieur ou égal à 30 kg/m² et/ou présentant un tissu adipeux périprostatique (TAPP) abondant, dans lequel la surface de TAPP est supérieure ou égale à 10 cm².

5. Méthode *ex vivo* et/ou *in vitro* de détermination du degré d'agressivité d'une tumeur du cancer de la prostate chez un sujet atteint d'un cancer de la prostate, ou de détermination du risque de rechute biologique d'un cancer de la prostate chez un sujet, comprenant les étapes suivantes :
a) déterminer la concentration ou le niveau d'expression du récepteur CCR3 dans un échantillon de cellules prostatiques tumorales obtenues chez ledit sujet,
b) comparer la concentration ou le niveau d'expression du récepteur CCR3 déterminée à l'étape a) avec la concentration ou le niveau d'expression de référence du récepteur CCR3 dans l'épithelium prostatique sain,
une concentration ou un niveau d'expression du récepteur CCR3, dans ledit échantillon de cellules prostatiques tumorales dudit sujet, supérieure à ladite concentration ou dudit niveau d'expression de référence étant l'indication d'une tumeur agressive du cancer de la prostate à haut potentiel de dissémination extraprostatique ou d'un risque de rechute biologique d'un cancer de la prostate.

6. Méthode selon la revendication 5, **caractérisée en ce que** la mesure de la concentration du récepteur CCR3 dans un échantillon de cellules prostatiques et/ou de cellules de l'épithélium prostatique est effectuée en mettant en oeuvre une méthode immunologique appropriée au moyen d'au moins un anticorps spécifique du récepteur CCR3.

7. Méthode selon la revendication 5 ou la revendication 6, **caractérisée en ce que** ledit sujet est un homme obèse, ayant un indice de masse corporelle (IMC) supérieur ou égal à 30 kg/m² et/ou présentant un TAPP abondant, dans lequel la surface de TAPP est supérieure ou égale à 10 cm².

8. Utilisation *ex vivo* et/ou *in vitro* d'un anticorps spécifique du récepteur CCR3 pour déterminer le degré d'agressivité d'une tumeur du cancer de la prostate chez un sujet atteint d'un cancer de la prostate.

## Patentansprüche

1. Inhibitor der CCL7/CCR3-Interaktion zu dessen Verwendung, um die Ausbreitung des Krebses der Prostata außerhalb der Prostatakapsel bei einem Patienten zu verhindern oder zu behandeln; wobei der Inhibitor der CCL7/CCR3-Interaktion ausgewählt ist aus der Gruppe bestehend aus einem organischen Molekül, ausgewählt aus der Gruppe bestehend aus den Verbindungen der Formeln I bis DCCLXXXIV, einem Peptid, gegen CCR3 oder CCL7 gerichteten Antikörpern, einem Peptidmimetikum und einem Aptamer, welches CCL7 oder CCR3 erkennt.

2. Inhibitor der CCL7/CCR3-Interaktion zu dessen Verwendung nach Anspruch 1, **dadurch gekennzeichnet, dass** es sich um einen CCR3-Antagonisten handelt.

3. Inhibitor der CCL7/CCR3-Interaktion zu dessen Verwendung nach Anspruch 1 oder Anspruch 2, **dadurch gekennzeichnet, dass** es sich um die Verbindung UCB35625 der Formel DLI handelt.

4. Inhibitor zu dessen Verwendung nach einem der Ansprüche 1 bis 3, **dadurch gekennzeichnet, dass** der Patient ein übergewichtiger Mann ist, der einen Body Mass Index (BMI) von größer als oder gleich 30 kg/m² aufweist und/oder ein übermäßiges periprostatisches Fettgewebe (ppF) aufweist, wobei die ppF-Oberfläche größer als oder gleich 10 cm² ist.

5. *Ex-vivo-* und/oder *In-vitro-*Verfahren zum Bestimmen des Aggressivitätsgrades eines Tumors des Krebses der Prostata bei einem Patienten, der an einem Krebs der Prostata erkrankt ist, oder zum Bestimmen des biologischen Rezidivrisikos eines Krebses der Prostata bei einem Patienten, das die folgenden Schritte umfasst:
a) Bestimmen der Konzentration oder des Expressionsgrades des CCR3-Rezeptors in einer Probe von Prostatatumorzellen, die vom Patienten erhalten werden,
b) Vergleichen der im Schritt a) bestimmten Konzentration oder des Expressionsgrades des CCR3-Rezeptors mit der Referenzkonzentration oder dem Referenzexpressionsgrad des CCR3-Rezeptors im gesunden Prostataepithel,
wobei eine Konzentration oder ein Expressionsgrad des CCR3-Rezeptors in der Prostatatumorzellprobe des Patienten, die/der größer ist als die Referenzkonzentration oder der Referenzexpressionsgrad, der Hinweis auf einen aggressiven Tumor des Krebses der Prostata mit hohem extraprostatischem Disseminationspotential oder auf ein biologisches Rezidivrisiko eines Krebses der Prostata ist.

6. Verfahren nach Anspruch 5, **dadurch gekennzeichnet, dass** die Messung der Konzentration des CCR3-Rezeptors in einer Probe von Prostatazellen und/oder Zellen des Prostataepithels unter Einsatz eines geeigneten immunologischen Verfahrens mittels mindestens eines Antikörpers vorgenommen wird, der für den CCR3-Rezeptor spezifisch ist.

7. Verfahren nach Anspruch 5 oder Anspruch 6, **dadurch gekennzeichnet, dass** der Patient ein übergewichtiger Mann ist, der einen Body Mass Index (BMI) von größer als oder gleich 30 kg/m² aufweist und/oder ein übermäßiges ppF aufweist, wobei die ppF-Oberfläche größer als oder gleich 10 cm² ist.

8. *Ex-vivo-* und/oder *In-vitro*-Verwendung eines für den CCR3-Rezeptor spezifischen Antikörpers, um den Aggressivitätsgrad eines Tumors des Krebses der Prostata bei einem Patienten zu bestimmen, der an einem Krebs der Prostata erkrankt ist.

## Claims

1. Inhibitor of the CCL7/CCR3 interaction for use to prevent or treat the extension of prostate cancer outside the prostatic capsule in a subject; with the inhibitor of the CCL7/CCR3 interaction being chosen from the group consisting of an organic molecule chosen from the group consisting of compounds having formulae I to DCCLXXXIV, a peptide, antibodies directed against CCR3 or CCL7, a peptidomimetic and a CCL7- or CCR3-recognising aptamer.

2. Inhibitor of the CCL7/CCR3 interaction for use according to claim 1, **characterised in that** it is a CCR3 antagonist.

3. Inhibitor of the CCL7/CCR3 interaction for use according to claim 1 or claim 2, **characterised in that** it is the compound UCB35625 having formula DLI.

4. Inhibitor for use according to any one of claims 1 to 3, **characterised in that** the subject is an obese man, having a body mass index (BMI) greater or equal to 30 kg/m² and/or having abundant periprostatic adipose tissue (PPAT), wherein the PPAT surface is greater than or equal to 10 cm².

5. *Ex vivo* and/or *in vitro* method for determining the degree of aggressiveness of a prostate cancer tumour in a subject afflicted with prostate cancer, or for determining the risk of biological relapse of prostate cancer in a subject, comprising the following steps:
a) determining the concentration or level of expression of the receptor CCR3 in a sample of prostate tumour cells obtained from said subject,
b) comparing the concentration or level of expression of the receptor CCR3 determined in step a) with the reference concentration or the level of expression of the receptor CCR3 in healthy prostatic epithelium,
a concentration or a level of expression of the receptor CCR3, in said sample of prostate tumour cells of said subject, greater than said reference concentration or said level of expression being the indication of an aggressive prostate cancer tumour with a high potential of extraprostatic dissemination or a risk of biological relapse of a prostate cancer.

6. Method according to claim 5, **characterised in that** the measurement of the concentration of the receptor CCR3 in a sample of prostatic cells and/or of prostatic epithelium cells is carried out by implementing a suitable immunological method by means of at least one antibody specific to the receptor CCR3.

7. Method according to claim 5 or claim 6, **characterised in that** said subject is an obese man, having a body mass index (BMI) greater than or equal to 30 kg/m² and/or having abundant PATT, wherein the PATT surface is greater than or equal to 10 cm².

8. *Ex vivo* and/or *in vitro* use of an antibody specific to the receptor CCR3 in order to determine the degree of aggressiveness of a prostate cancer tumour of a subject afflicted with prostate cancer.
